# EUROPEAN PATENT APPLICATION

(11) **EP 3 888 663 A1**
(43) Date of publication of application: **06.10.2021**
(21) Application number: 19890058.1
(22) Date of filing: 02.12.2019
(51) Int. Cl.: A61K 31/7105, A61K 31/713, A61K 48/00, A61P 25/28, C07H 15/04, A61K 47/54, C12N 15/113

(54) **NUCLEIC ACID COMPLEX**

(30) Priority: 30.11.2018 JP 2018225829
(71) Applicant: Kyowa Kirin Co., Ltd., Tokyo 100-0004 (JP)
(72) Inventor: IWAI, Hiroto, Tokyo 100-0004 (JP); IMAEDA, Takashi, Tokyo 100-0004 (JP); ARIYAMA, Hiroyuki, Tokyo 100-0004 (JP); MURAKAMI, Takuya, Tokyo 100-0004 (JP)
(74) Representative: D Young & Co LLP
(86) International application number: PCT/JP2019/047044
(87) International publication number: WO 2020/111280

(57) **Abstract**

The present invention provides a nucleic acid conjugate represented by the following formula 1: 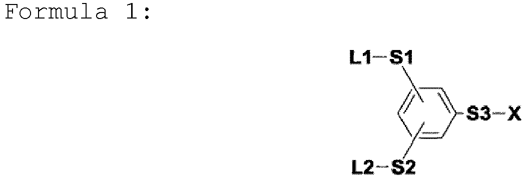 wherein X is a double-stranded nucleic acid consisting of a sense strand and an antisense strand and comprising a duplex region of at least 11 base pairs, wherein in the double-stranded nucleic acid, an oligonucleotide strand having a chain length of 17 to 30 nucleotides in the antisense strand is complementary to any of target APCS mRNA sequences described in Tables 1-1 to 1-13, and the 3' end or the 5' end of the sense strand binds to S3, L1 and L2 are each independently sugar ligand, and S1, S2 and S3 are each independently a linker.

## Description

### Technical Field

The present invention relates to a nucleic acid conjugate and a pharmaceutical composition comprising the nucleic acid conjugate, etc.

### Background Art

For example, aptamers, antisenses, decoy nucleic acids, ribozymes, siRNA, miRNA and anti-miRNA are known as nucleic acid medicines. Such nucleic acid medicines are expected to be clinically applied to various previously difficult-to-treat diseases, because of their high versatility that permits control of every gene in cells.

Also, the nucleic acid medicines are expected as next-generation medicines following antibody or low-molecular medicines, because of their high target selectivity and activity in cells.

However, a problem of the nucleic acid medicines is difficult delivery to a target tissue.

Use of a conjugate of a targeting compound and a nucleic acid (nucleic acid conjugate) has been reported as one of the methods for effectively delivering the nucleic acid medicines *in vivo.* Examples of the targeting compound include ligands capable of binding to extracellularly expressed receptors. Among others, there are a plurality of reports on a nucleic acid conjugate that utilizes N-acetyl-D-galactosamine (GalNAc) or the like as a ligand capable of binding to an asialoglycoprotein receptor (ASGPR) very highly expressed on liver cells. In recent years, nucleic acid conjugates containing such ligands bound to siRNAs have been reported to be efficiently delivered to liver cells (Non Patent Literature 1).

Patent Literatures 1 and 2 disclose, for example, the following nucleic acid conjugate as a conjugate of a targeting compound and an oligonucleotide: 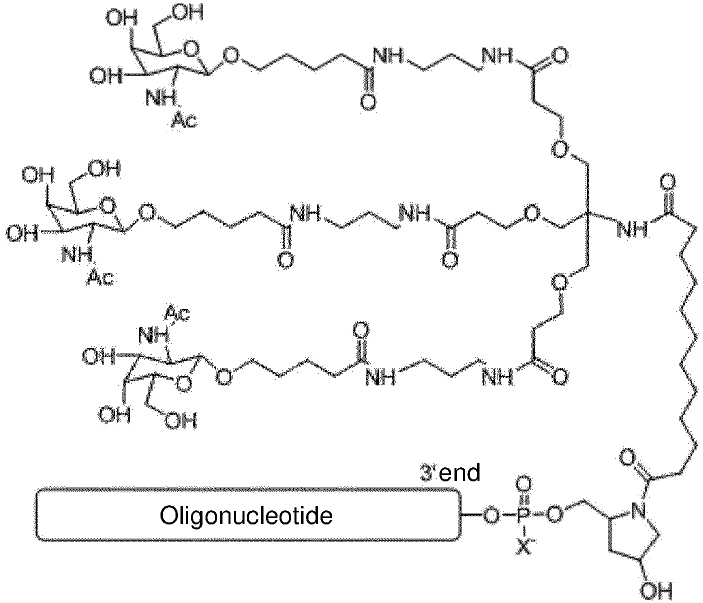 wherein Ac represents an acetyl group; hereinafter, the same holds true for the present specification.

Patent Literature 3 discloses a nucleic acid conjugate having the following structure having a sugar ligand-tether unit similar to that of the nucleic acid conjugates disclosed in Patent Literatures 1 and 2:

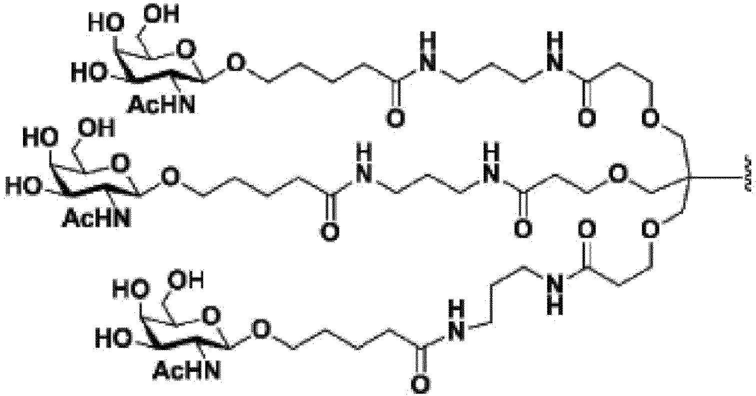

Patent Literature 4 discloses a nucleic acid conjugate having the following structure as a sugar ligand-tether unit:

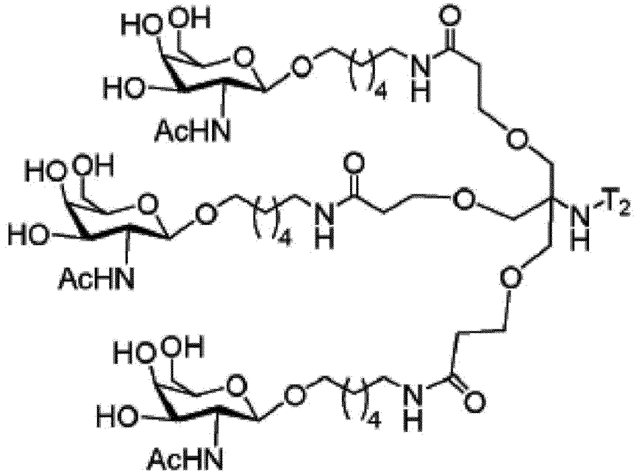

APCS (amyloid P component, serum) (also called serum amyloid P, SAP or pentraxin-2) has a pentamer structure of a glycoprotein constituted by 223 amino acids. APCS is a glycoprotein that is produced in the liver, and is present with a relatively high concentration of 30 to 50 µg/mL in blood.

APCS also has biochemical characteristics of binding to every type of amyloid fibril in a calcium-dependent manner. Patients having amyloid are known to contain APCS in an amount as large as 20,000 mg in the amyloid (Non Patent Literature 2).

APCS is present in amyloid in every patient with an amyloid-related disease and as such, is used as a diagnostic marker for amyloid-related disease patients (Non Patent Literature 3).

Amyloid-related diseases are diseases in which aberrant insoluble protein fibrils known as amyloid fibrils accumulate in tissues, causing an organ disorder.

APCS has been found to induce resistance to degradation by protease or phagocytosis by immunocytes through binding to amyloid so that the amyloid bound with APCS can be stabilized. Research using animal models and clinical research have also strongly suggested that the inhibition of the binding of APCS to amyloid can reduce amyloid accumulation in organs and tissue disorders associated therewith (Non Patent Literatures 4 and 5).

It can be expected that amyloid-related diseases can be prevented or treated by specifically inhibiting the expression of APCS. Nonetheless, any medicament specifically inhibiting the expression of APCS has not yet been reported.

### Citation List

### Patent Literature

Patent Literature 1: International Publication No. WO 2009/073809
Patent Literature 2: International Publication No. WO 2013/075035
Patent Literature 3: International Publication No. WO 2015/105083
Patent Literature 4: International Publication No. WO 2014/179620

### Non Patent Literature

Non Patent Literature 1: Journal of American Chemical Society, 2014, Vol. 136, p. 16958-16961
Non Patent Literature 2: Amyloid, 1997, Vol. 4:4, p. 274-295
Non Patent Literature 3: N. Engl. J. Med., 1990, Vol. 323, p. 508-13
Non Patent Literature 4: Nature, 2010, Vol. 468, p. 93-97
Non Patent Literature 5: N. Engl. J. Med., 2015, Vol. 373, p. 1106-1114

### Summary of Invention

### Technical Problem

An object of the present invention is to provide a nucleic acid conjugate capable of inhibiting the expression of APCS.

### Solution to Problem

The present invention relates to the following.
[1] A nucleic acid conjugate represented by the following formula 1: 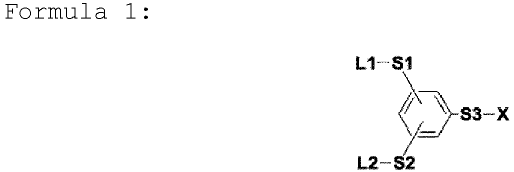 wherein
   X is a double-stranded nucleic acid consisting of a sense strand and an antisense strand and comprising a duplex region of at least 11 base pairs, wherein
   in the double-stranded nucleic acid, an oligonucleotide strand having a chain length of 17 to 30 nucleotides in the antisense strand is complementary to any of target APCS mRNA sequences described in Tables 1-1 to 1-13, and
   a 3' end or a 5' end of the sense strand binds to S3,
   L1 and L2 are each independently a sugar ligand, and
   S1, S2 and S3 are each independently a linker.
[2] The nucleic acid conjugate according to [1], wherein the nucleic acid conjugate has a structure represented by the following formula 2: 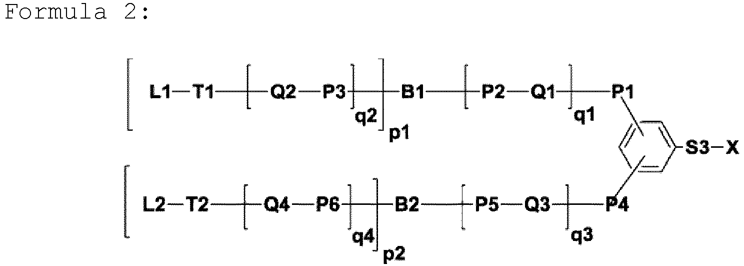 Wherein
   X, L1, L2 and S3 are each as defined above,
   P1, P2, P3, P4, P5 and P6, and T1 and T2 are each independently absent, or -CO-, -NH-, -O-, -S-, -O-CO-, - S-CO-, -NH-CO-, -CO-O-, -CO-S- or -CO-NH-,
   Q1, Q2, Q3 and Q4 are each independently absent, or substituted or unsubstituted alkylene having 1 to 12 carbon atoms or -(CH₂CH₂O)ₙ-CH₂CH₂- wherein n is an integer of 0 to 99,
   B1 and B2 are each independently a bond, or any structure represented by the following formula 2-1, wherein each of the terminal dots in each structure is a binding site to P2 or P3, or P5 or P6, and m1, m2, m3 and m4 are each independently an integer of 0 to 10: 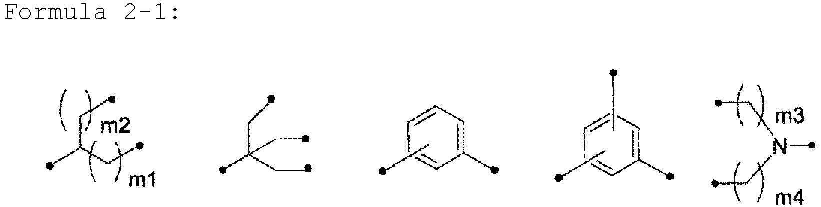
   p1 and p2 are each independently an integer of 1, 2 or 3, and
   q1, q2, q3 and q4 are each independently an integer of 0 to 10,
   provided that when each of p1 and p2 is an integer of 2 or 3, each P3 and P6, Q2 and Q4, T1 and T2 or L1 and L2 are the same or different, and when q1 to q4 are 2 to 10, combinations -[P2-Q1]-, -[Q2-P3]-, -[P5-Q3]- or -[Q4-P6]- are the same or different.
[3] The nucleic acid conjugate according to [2], wherein P1 and P4 are each independently -CO-NH-, -NH-CO- or -O-.
[4] The nucleic acid conjugate according to [2] or [3], wherein -[P2-Q1]_{q1}- and -[P5-Q3]_{q3}- are each independently absent, or any structure represented by the following formulas 3-1 to 3-3: 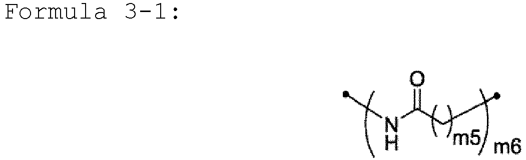 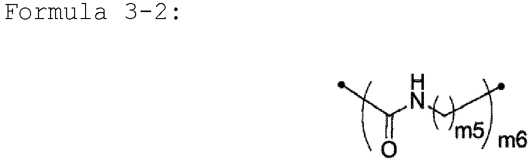 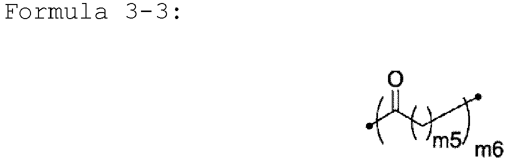 wherein
   m5 and m6 are each independently an integer of 0 to 10, and each of the terminal dots in the structures of formulas 3-1 to 3-3 is a binding site to B1 or B2, or P1 or P4.
[5] The nucleic acid conjugate according to any one of [2] to [4], wherein the nucleic acid conjugate has any structure represented by the following formulas 4-1 to 4-9: 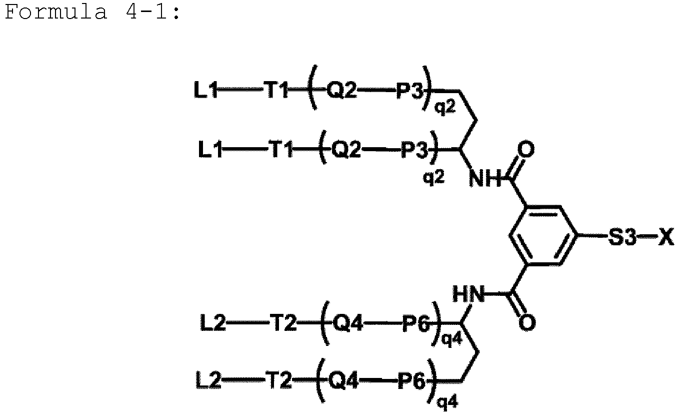 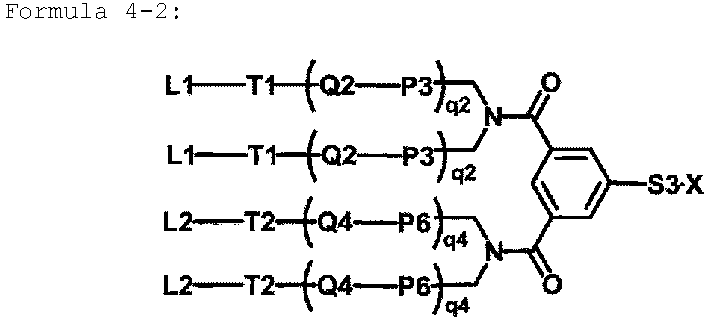 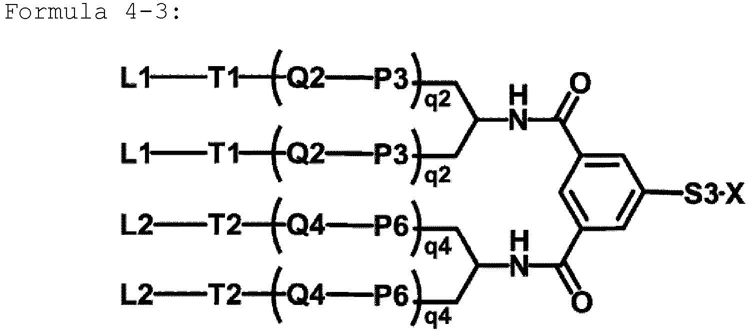 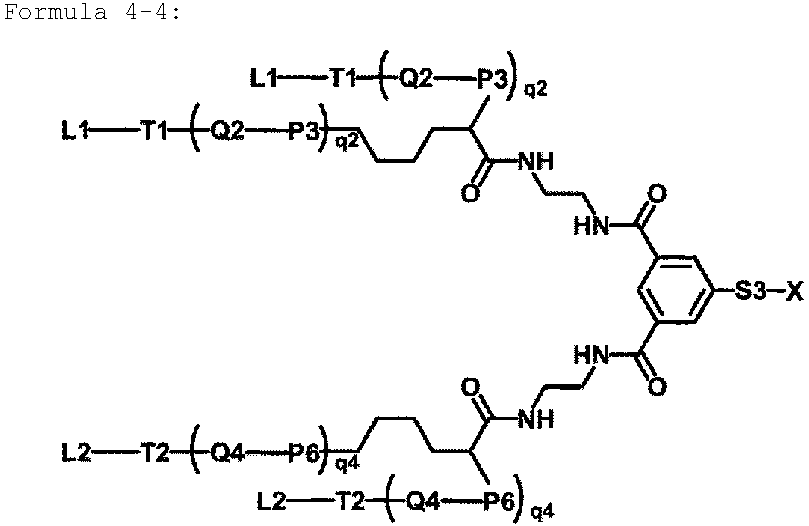 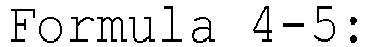 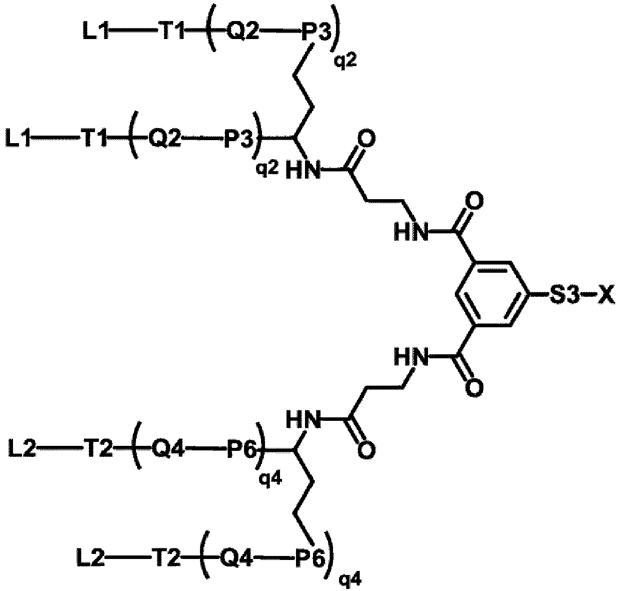 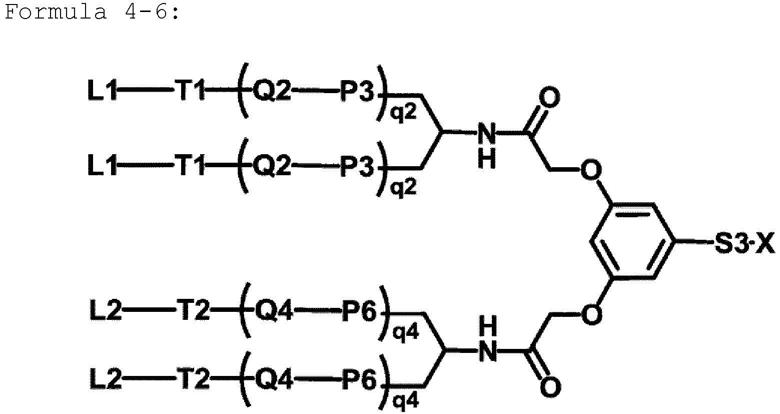 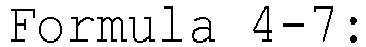 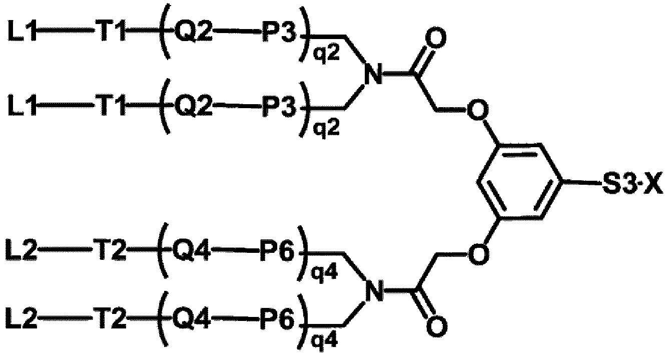 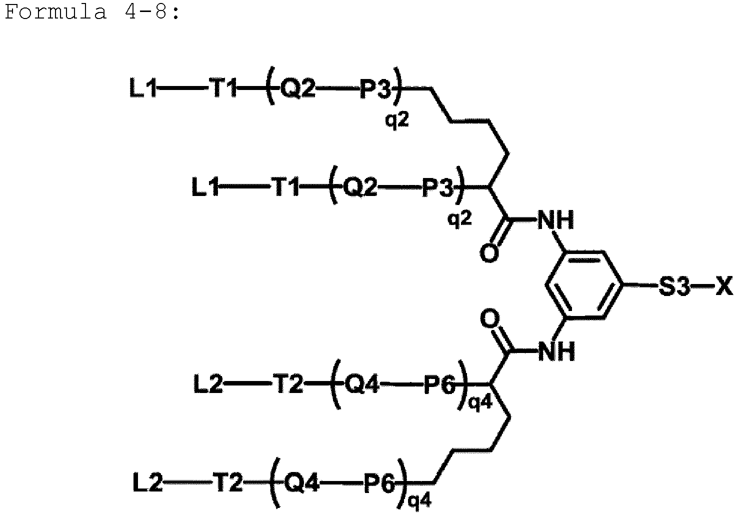 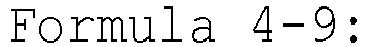 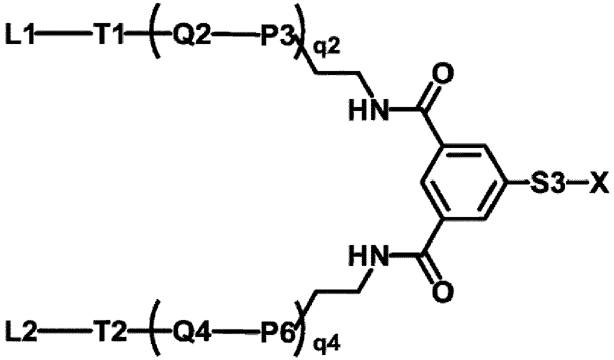 wherein
   X, L1, L2, S3, P3, P6, T1, T2, Q2, Q4, q2 and q4 are each as defined above.
[6] The nucleic acid conjugate according to [1], wherein the nucleic acid conjugate has a structure represented by the following formula 5: 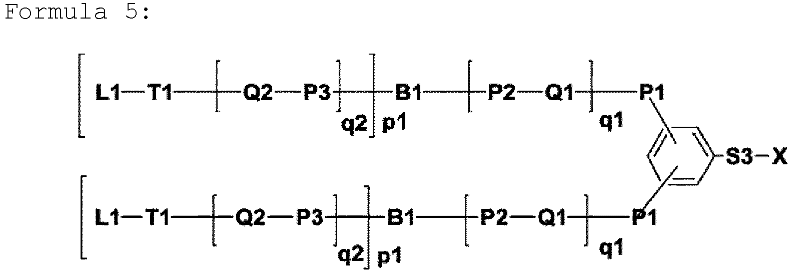 wherein
   X, S3, P1, P2, P3, Q1, Q2, B1, T1, L1, p1, q1 and q2 are each as defined above.
[7] The nucleic acid conjugate according to [6], wherein P1 is -CO-NH-, -NH-CO- or -O-.
[8] The nucleic acid conjugate according to [6] or [7], wherein the nucleic acid conjugate has any structure represented by the following formulas 6-1 to 6-9: 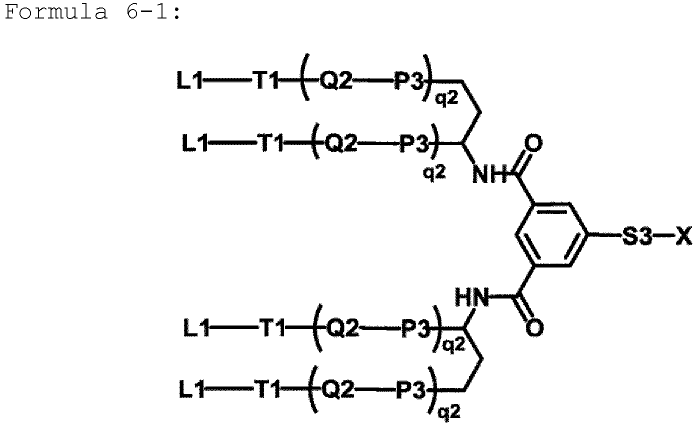 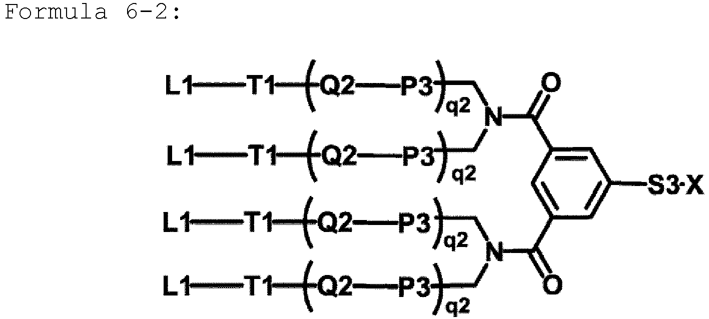 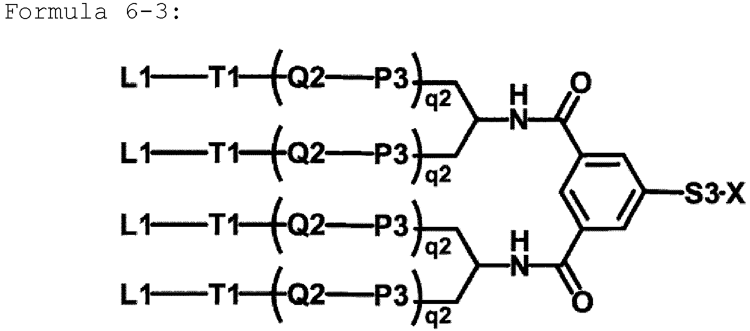 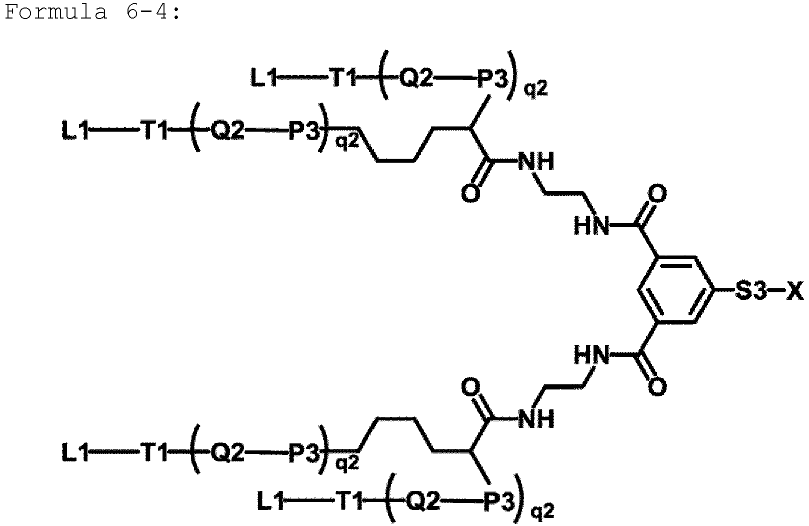 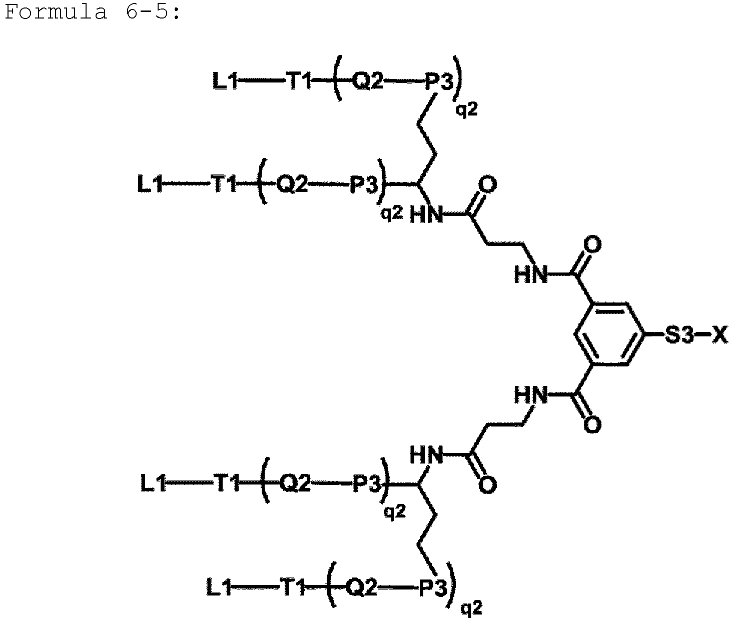 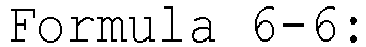 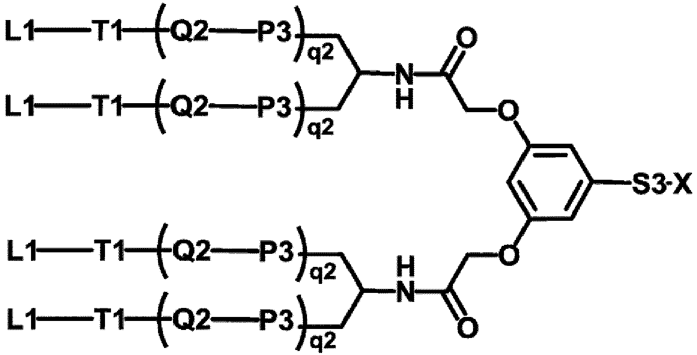 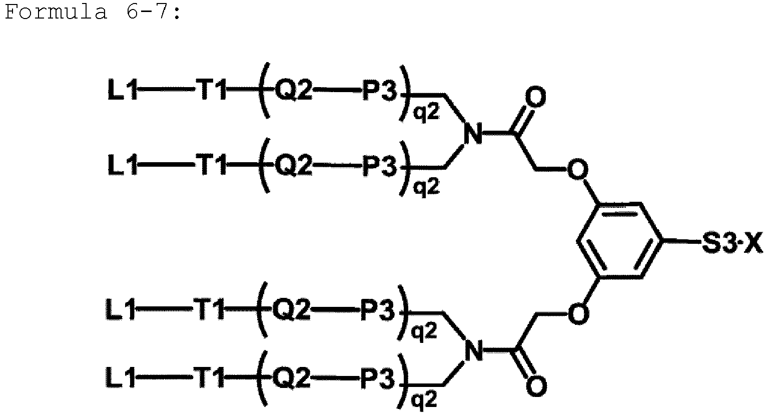 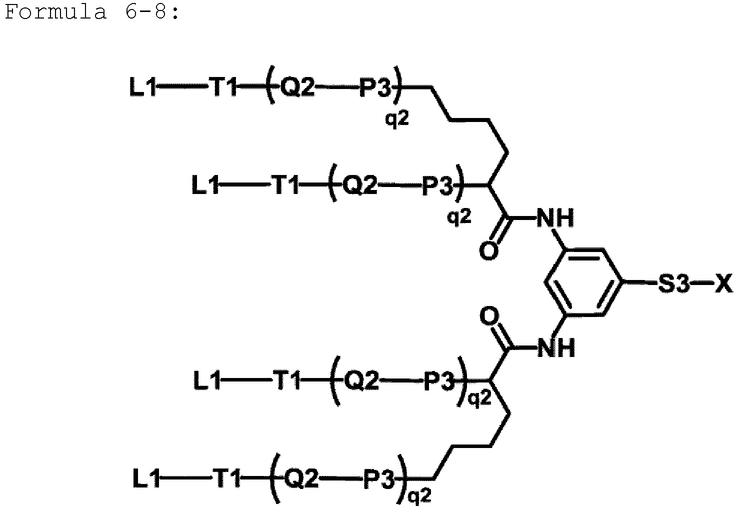 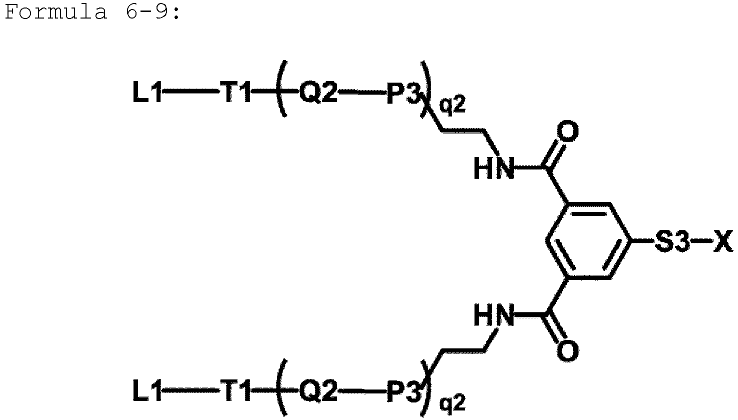 wherein
   X, S3, P3, Q2, T1, L1 and q2 are each as defined above.
[9] The nucleic acid conjugate according to any one of [2] to [5], wherein the nucleic acid conjugate has any structure represented by the following formulas 7-1 to 7-9: 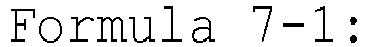 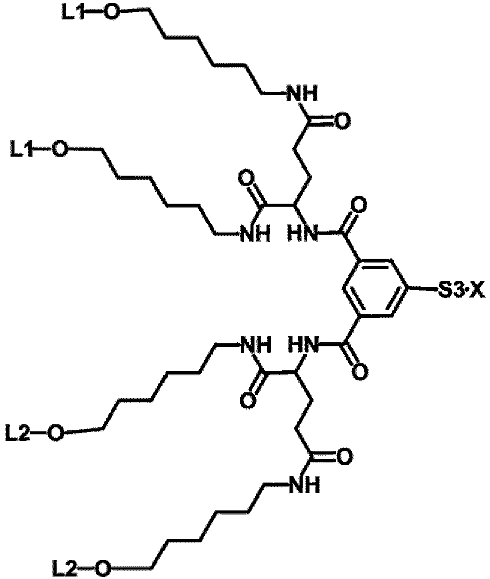 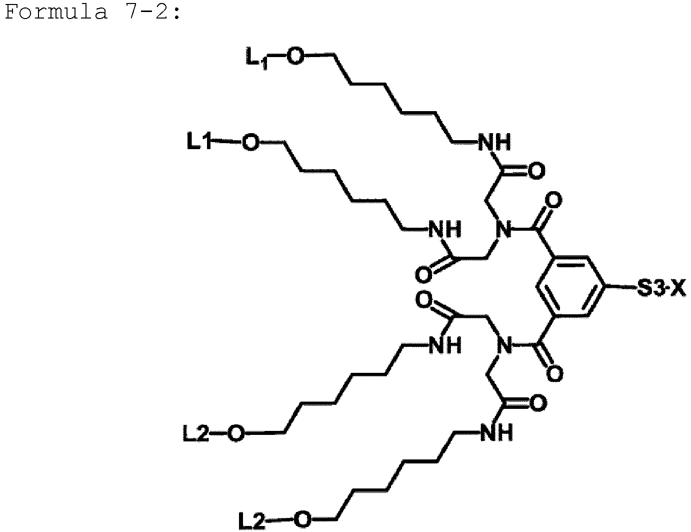 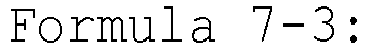 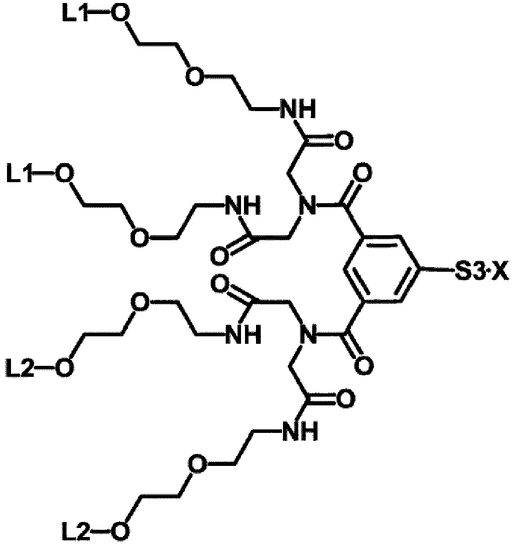 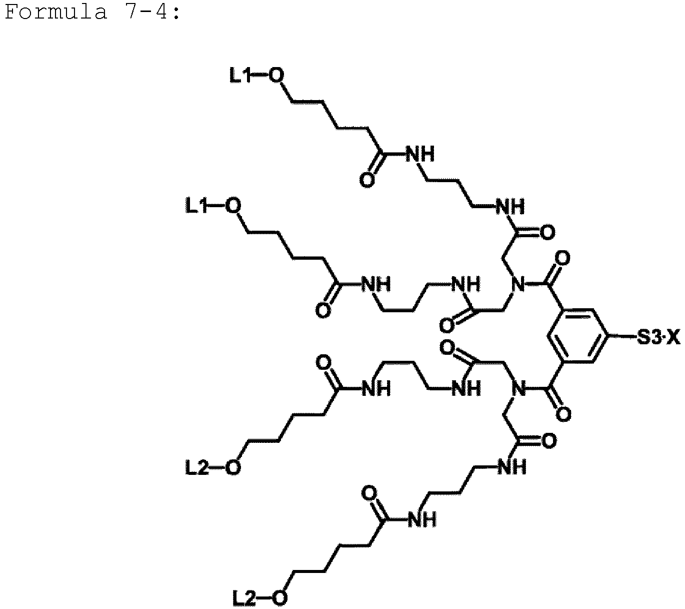 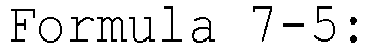 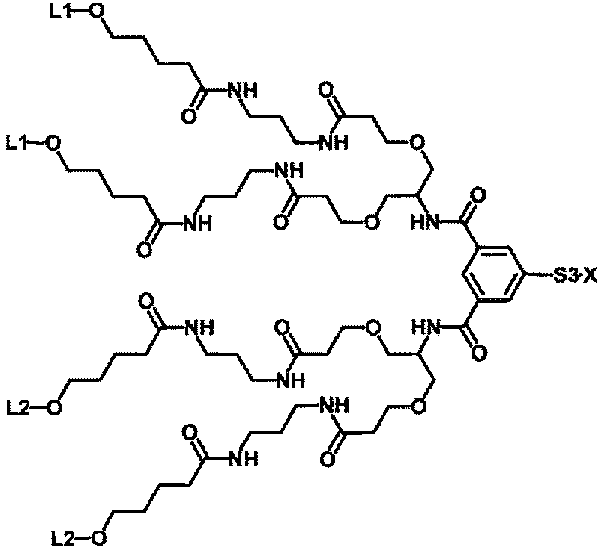 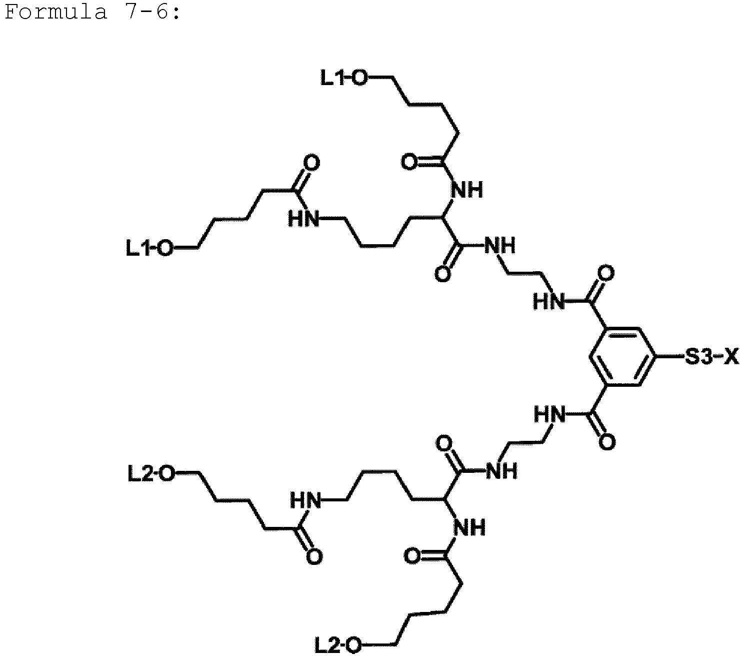 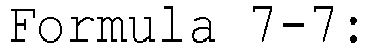 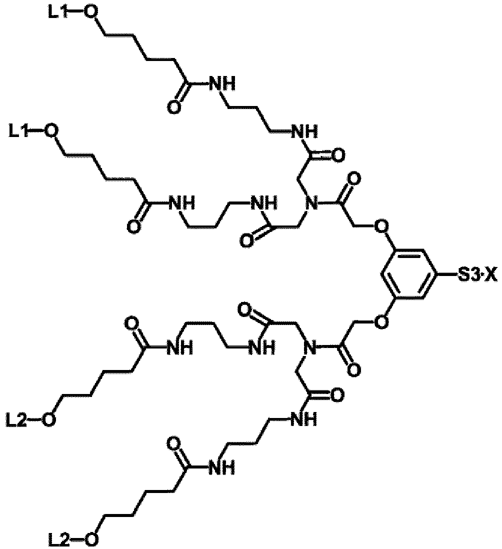 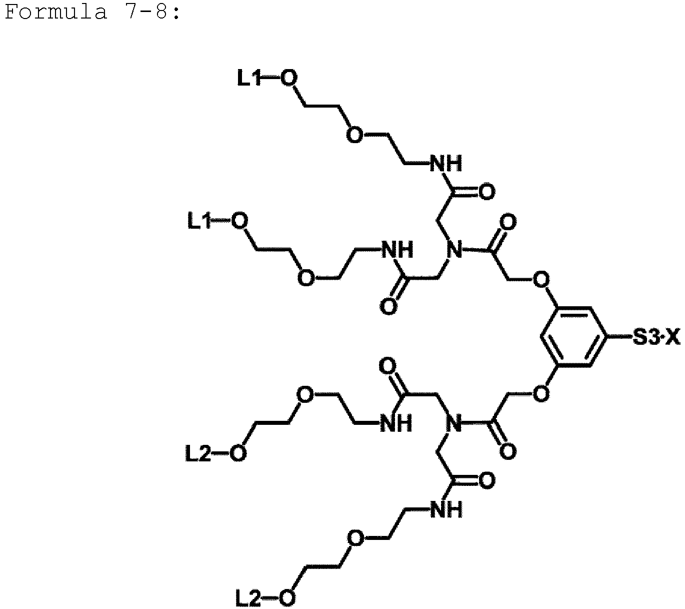 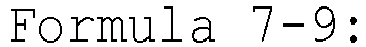 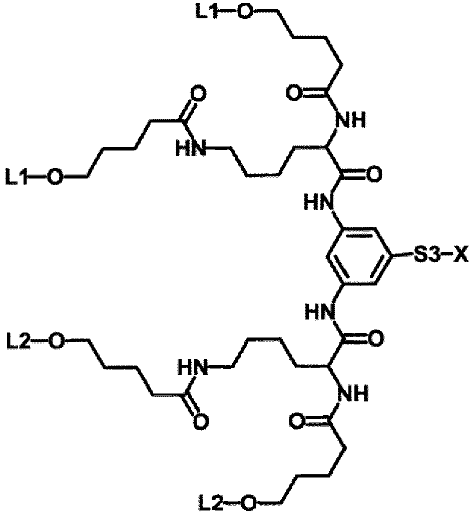 wherein
   X, S3, L1 and L2 are each as defined above.
[10] The nucleic acid conjugate according to any one of [1] to [9], wherein the sugar ligand is N-acetylgalactosamine.
[11] The nucleic acid conjugate according to any one of [1] to [10], wherein the double-stranded nucleic acid comprises a modified nucleotide.
[12] The nucleic acid conjugate according to any one of [1] to [11], wherein the 3' end of the sense strand and the 5' end of the antisense strand each form a blunt end.
[13] The nucleic acid conjugate according to [11], wherein the double-stranded nucleic acid comprises a nucleotide modified at the sugar moiety.
[14] The nucleic acid conjugate according to any one of [1] to [13], wherein the nucleic acid conjugate has a structure represented by the following formula 7-8-1: 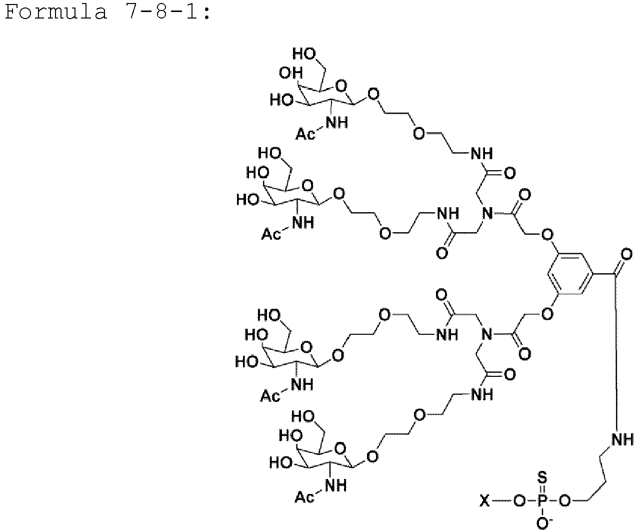 wherein X is as defined above.
[15] The nucleic acid conjugate according to any one of [1] to [14], wherein X is a pair of sense strand/antisense strand selected from the group consisting of sense strands/antisense strands described in Tables 1-1 to 1-13.
[16] The nucleic acid conjugate according to any one of [1] to [14], wherein X is a pair of sense strand/antisense strand selected from the group consisting of sense strands/antisense strands described in Tables M1-1 to M1-3, R-1 to R-2 and R-3 to R-4.
   [16-A1] The nucleic acid conjugate according to any one of [1] to [14], wherein X is a pair of sense strand/antisense strand consisting of a sense strand with its 3' end binding to S3 and an antisense strand represented by any of SEQ ID NOs: 2126, 2144 and 2146.
   [16-A2] The nucleic acid conjugate according to [16-A1], wherein the nucleic acid conjugate has any structure represented by the following formulas 6-1 to 6-9: 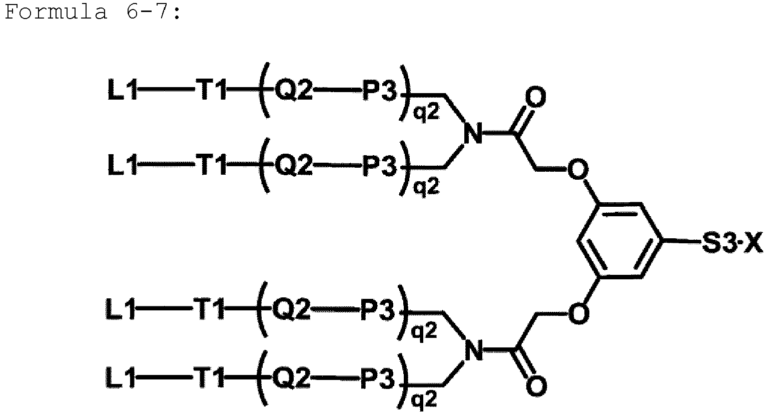 wherein
      X, S3, P3, Q2, T1, L1 and q2 are each as defined above.
   [16-A3] The nucleic acid conjugate according to [16-A1], wherein the nucleic acid conjugate has a structure represented by the following formula 7-8: 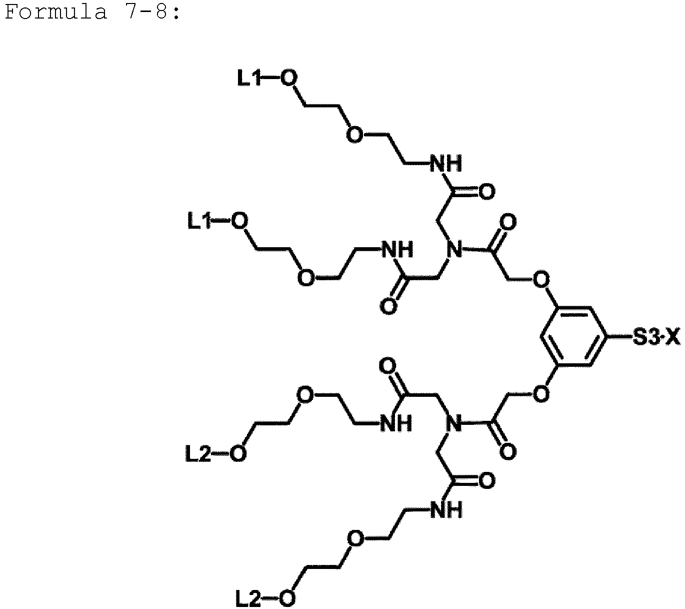 wherein
      X, S3, L1 and L2 are each as defined above.
   [16-A4] The nucleic acid conjugate according to [16-A1], wherein the nucleic acid conjugate has a structure represented by the following formula 7-8-1: 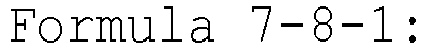 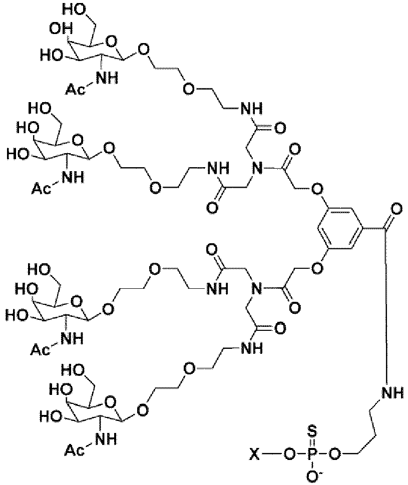 wherein X is as defined above.
   [16-B1] The nucleic acid conjugate according to any one of [1] to [14], wherein X is a pair of sense strand/antisense strand consisting of a sense strand of SEQ ID NO: 2083 and an antisense strand of SEQ ID NO: 2126, a sense strand of SEQ ID NO: 2101 and an antisense strand of SEQ ID NO: 2144, or a sense strand of SEQ ID NO: 2103 and an antisense strand of SEQ ID NO: 2146.
   [16-B2] The nucleic acid conjugate according to [16-B1], wherein the nucleic acid conjugate has any structure represented by the following formulas 6-1 to 6-9: 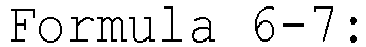 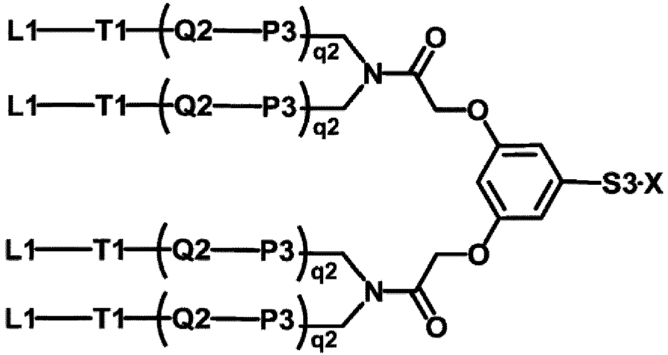 wherein
      X, S3, P3, Q2, T1, L1 and q2 are each as defined above.
   [16-B3] The nucleic acid conjugate according to [16-B1], wherein the nucleic acid conjugate has a structure represented by the following formula 7-8: 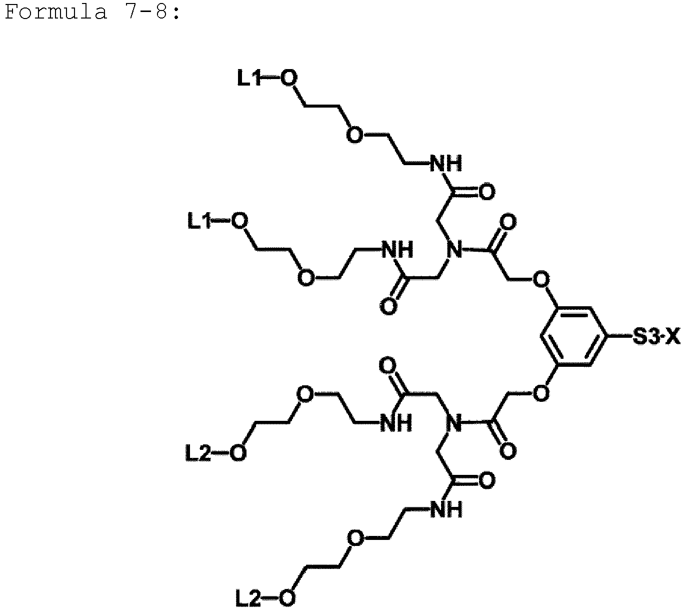 wherein
      X, S3, L1 and L2 are each as defined above.
   [16-B4] The nucleic acid conjugate according to [16-B1], wherein the nucleic acid conjugate has a structure represented by the following formula 7-8-1: 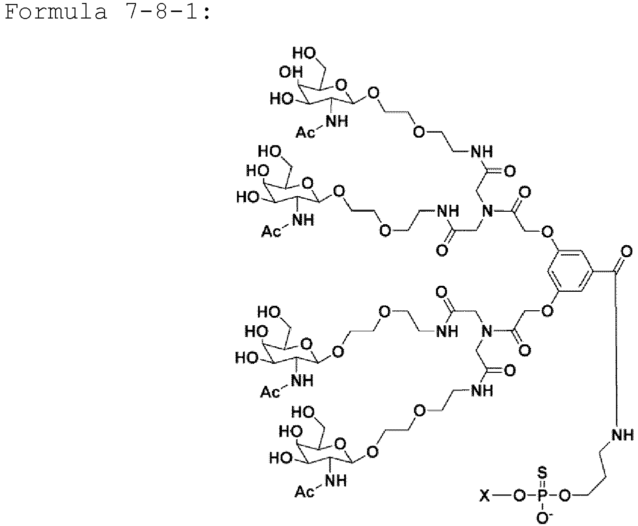 wherein X is as defined above.
[17] A pharmaceutical composition comprising a nucleic acid conjugate according to any one of [1] to [16].
[18] The pharmaceutical composition according to [17], wherein the pharmaceutical composition is for transfer into a cell.
[19] The pharmaceutical composition according to [17] or [18], wherein the pharmaceutical composition is intravenously administered or subcutaneously administered.
[20] A method for treating or preventing a disease, comprising administering a nucleic acid conjugate according to any one of [1] to [16] or a pharmaceutical composition according to any one of [17] to [19] to a patient in need thereof.
[21] A method for inhibiting the expression of APCS gene, comprising transferring a double-stranded nucleic acid into a cell using a nucleic acid conjugate according to any one of [1] to [16] or a pharmaceutical composition according to any one of [17] to [19].
[22] A method for treating an amyloid-related disease, comprising administering a nucleic acid conjugate according to any one of [1] to [16] or a pharmaceutical composition according to any one of [17] to [19] to a mammal.
[23] A medicament for use in the treatment of an amyloid-related disease, comprising a nucleic acid conjugate according to any one of [1] to [16] or a pharmaceutical composition according to any one of [17] to [19].
[24] A therapeutic agent for an amyloid-related disease, comprising a nucleic acid conjugate according to any one of [1] to [16] or a pharmaceutical composition according to any one of [17] to [19].
[25] The treatment method according to [22], wherein the amyloid-related disease is a disease caused by a disorder mediated by amyloid fibrils containing APCS.
[26] The medicament according to [23], wherein the amyloid-related disease is a disease caused by a disorder mediated by amyloid fibrils containing APCS.
[27] The therapeutic agent according to [24], wherein the amyloid-related disease is a disease caused by a disorder mediated by amyloid fibrils containing APCS.

### Advantageous Effects of Invention

For example, a pharmaceutical composition comprising the nucleic acid conjugate of the present invention can be administered to mammals to treat various related diseases *in vivo.*

### Brief Description of Drawing

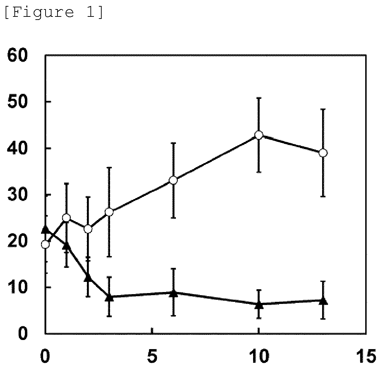

[Figure 1] Figure 1 is a diagram showing changes in human APCS concentration in blood of each mouse group in Test Example 2. In the diagram, the APCS concentration of Day 0 refers to a value obtained 6 days before administration, and the date of initial administration is defined as Day 0. The abscissa depicts the number of lapsed days with the date of initial administration defined as Day 0, and the ordinate depicts the human APCS concentration (µg/mL) in blood. The open circles depict a control group, and the filled triangles depict a 10 mg/kg administration group of compound 5-2. The error bars in the diagram depict standard deviation (SD).

### Description of Embodiments

The nucleic acid conjugate of the present invention is a nucleic acid conjugate represented by the following formula 1:

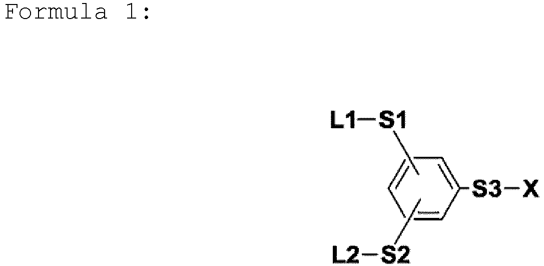

In formula 1,
X is a double-stranded nucleic acid consisting of a sense strand and an antisense strand and comprising a duplex region of at least 11 base pairs, wherein
in the double-stranded nucleic acid, an oligonucleotide strand having a chain length of 17 to 30 nucleotides in the antisense strand is complementary to any of target APCS mRNA sequences described in Tables 1-1 to 1-13, and
the 3' end or the 5' end of the sense strand binds to S3,
L1 and L2 are each independently a sugar ligand, and
S1, S2 and S3 are each independently a linker.

In the present invention, S1 and S2 can each be bonded to the benzene ring at an ortho-, meta- or para-position with respect to the substitution position of S3 on the benzene ring. A nucleic acid conjugate represented by formula 1-1 given below is preferred. The bonds of S1 and S2 to the benzene ring in formula 1 mean that the bonds can be at arbitrary positions other than the substitution position of S3 on the benzene ring.

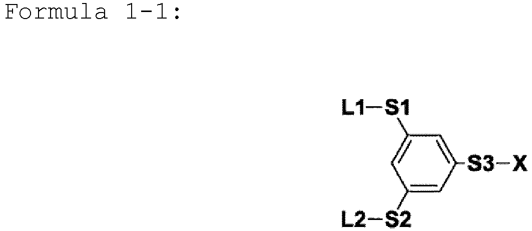

In formula 1-1,
X, L1, L2, S1, S2 and S3 are each as defined above.

In the present specification, the phrase "as defined above" means that, when formula 1-1 is taken as an example, each of X, L1, L2, S1 and S2 in formula 1-1 can be the same group as in the definition about each of X, L1, L2, S1 and S2 described above in formula 1.

In the present invention, X is a double-stranded nucleic acid consisting of a sense strand and an antisense strand and comprising a duplex region of at least 11 base pairs.

In the double-stranded nucleic acid, an oligonucleotide strand having a chain length of 17 to 30 nucleotides in the antisense strand is complementary to any of target APCS mRNA sequences described in Tables 1-1 to 1-13 mentioned later.

The 3' end or the 5' end of the sense strand binds to S3.

L1 and L2 are each independently a sugar ligand.

In the present invention, the sugar ligand means a group derived from a saccharide (monosaccharide, disaccharide, trisaccharide and polysaccharide, etc.) capable of binding to a receptor expressed on a target cell. In the present invention, when the sugar ligand is bonded to linker S1 or S2 through an O- bond, the sugar ligand means a group derived from a saccharide as a moiety, except for a hydroxy group, involved in the binding of the saccharide constituting the sugar ligand.

In the present invention, the sugar ligand targeted by the oligonucleotide can be selected.

Examples of the monosaccharide include allose, aldose, arabinose, cladinose, erythrose, erythrulose, fructose, D-fucitol, L-fucitol, fucosamine, fucose, fuculose, galactosamine, D-galactosaminitol, N-acetylgalactosamine, galactose, glucosamine, N-acetylglucosamine, glucosaminitol, glucose, glucose-6-phosphate, gulose, glyceraldehyde, L-glycero-D-manno-heptose, glycerol, glycerone, gulose, idose, lyxose, mannosamine, mannose, mannose-6-phosphate, psicose, quinovose, quinovosamine, rhamnitol, rhamnosamine, rhamnose, ribose, ribulose, sedoheptulose, sorbose, tagatose, talose, tartaric acid, threose, xylose, and xylulose.

Examples of the disaccharide, the trisaccharide, and the polysaccharide include abequose, acarbose, amicetose, amylopectin, amylose, apiose, arcanose, ascarylose, ascorbic acid, boivinose, cellobiose, cellotriose, cellulose, chacotriose, chalcose, chitin, colitose, cyclodextrin, cymarose, dextrin, 2-deoxyribose, 2-deoxyglucose, diginose, digitalose, digitoxose, evalose, evemitrose, fructo-oligosaccharide, galto-oligosaccharide, gentianose, gentiobiose, glucan, glycogen, hamamelose, heparin, inulin, isolevoglucosenone, isomaltose, isomaltotriose, isopanose, kojibiose, lactose, lactosamine, lactosediamine, laminarabiose, levoglucosan, levoglucosenone, β-maltose, maltriose, mannanoligosaccharide, manninotriose, melicitose, melibiose, muramic acid, mycarose, mycinose, neuraminic acid, sialic acid-containing sugar chains, nigerose, nojirimycin, nobiose, oleandrose, panose, paratose, planteose, primeverose, raffinose, rhodinose, rutinose, sarmentose, sedoheptulose, sedoheptulosan, solatriose, sophorose, stachyose, streptose, sucrose, α,α-trehalose, trehalosamine, turanose, tyvelose, xylobiose, and umbelliferose.

Each monosaccharide as the saccharide may be in a D form or a L form and may be a mixture of D and L forms at an arbitrary ratio.

The saccharide may contain deoxysugar (derived by the replacement of an alcoholic hydroxy group with a hydrogen atom), aminosugar (derived by the replacement of an alcoholic hydroxy group with an amino group), thiosugar (derived by the replacement of an alcoholic hydroxy group with thiol, the replacement of C=O with C=S, or the replacement of ring oxygen with sulfur), selenosugar, tellurosugar, azasugar (derived by the replacement of ring carbon with nitrogen), iminosugar (derived by the replacement of ring oxygen with nitrogen), phosphano-sugar (derived by the replacement of ring oxygen with phosphorus), phospha-sugar (derived by the replacement of ring carbon with phosphorus), C-substituted monosaccharide (derived by the replacement of a hydrogen atom on a nonterminal carbon atom with a carbon atom), unsaturated monosaccharide, alditol (derived by the replacement of a carbonyl group with a CHOH group), aldonic acid (derived by the replacement of an aldehyde group with a carboxy group), ketoaldonic acid, uronic acid, aldaric acid, or the like.

Examples of the aminosugar which is a monosaccharide include amino monosaccharides as the saccharide, such as galactosamine, glucosamine, mannosamine, fucosamine, quinovosamine, neuraminic acid, muramic acid, lactosediamine, acosamine, bacillosamine, daunosamine, desosamine, forosamine, gallosamine, kanosamine, kansosamine, mycaminose, mycosamine, perosamine, pneumosamine, purpurosamine, and rhodosamine. The amino group of the aminosugar may be substituted with an acetyl group or the like.

Examples of the sialic acid-containing sugar chains include sugar chains containing NeuAc at their nonreducing ends and specifically include sugar chains containing NeuAc-Gal-GlcNAc, and sugar chains containing Neu5Acα(2-6)Galβ(1-3)GlcNAc.

Each monosaccharide as the saccharide may be substituted with a substituent as long as the monosaccharide is capable of binding to a receptor expressed on a target cell. For example, the monosaccharide may be substituted with a hydroxy group, or one or more hydrogen atoms in each monosaccharide may be replaced with azide and/or an optionally substituted aryl group.

The sugar ligand is preferably selected as a sugar ligand binding to a receptor expressed on the surface of a target cell according to each targeted organ. When the target cell is, for example, a liver cell, the sugar ligand is preferably a sugar ligand against a receptor expressed on the surface of the liver cell, more preferably a sugar ligand against an asialoglycoprotein receptor (ASGPR).

The sugar ligand against ASGPR is preferably mannose or N-acetylgalactosamine, more preferably N-acetylgalactosamine.

For example, sugar derivatives described in Bioorganic Medicinal Chemistry, 17, 7254 (2009), and Journal of American Chemical Society, 134, 1978 (2012) are known as sugar ligands having higher affinity for ASGPR, and these sugar derivatives may be used.

In the present invention, each of S1, S2 and S3 is a linker.

S1 and S2 are not particularly limited as long as their structures link sugar ligands L1 and L2 to the benzene ring. A structure known in the art for use in nucleic acid conjugates may be adopted. S1 and S2 may be the same or may be different.

Sugar ligands L1 and L2 are preferably linked to S1 and S2 through glycoside bonds. S1 and S2 may each be linked to the benzene ring, for example, through a -CO-, -NH-, -O-, -S-, -O-CO-, -S-CO-, -NH-CO-, -CO-O-, -CO-S- or -CO-NH- bond.

S3 is not particularly limited as long as its structure links double-stranded nucleic acid X to the benzene ring. A structure known in the art for use in nucleic acid conjugates may be adopted. Oligonucleotide X is preferably linked to S3 through a phosphodiester bond. S3 may be linked to the benzene ring, for example, through a -CO-, -NH-, -O-, -S-, -O-CO-, -S-CO-, -NH-CO-, -CO-O-, -CO-S- or -CO-NH- bond.

For example, structures disclosed in International Publication Nos. WO 2009/073809, WO 2013/075035, WO 2015/105083, WO 2014/179620, and WO 2015/006740 may be adopted as linkers S1, S2 and S3.

In the present invention, the nucleic acid conjugate is preferably a nucleic acid conjugate having a structure represented by the following formula 2:

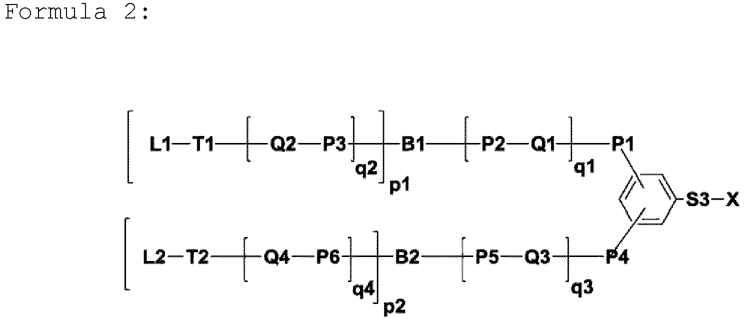

In formula 2,
X, L1, L2 and S3 are each as defined above,
P1, P2, P3, P4, P5 and P6, and T1 and T2 are each independently absent, or -CO-, -NH-, -O-, -S-, -O-CO-, - S-CO-, -NH-CO-, -CO-O-, -CO-S- or -CO-NH-, and
Q1, Q2, Q3 and Q4 are each independently absent, or substituted or unsubstituted alkylene having 1 to 12 carbon atoms or -(CH₂CH₂O)ₙ-CH₂CH₂- wherein n is an integer of 0 to 99.

P1 and P4 are each independently absent, or -CO-, - NH-, -O-, -S-, -O-CO-, -S-CO-, -NH-CO-, -CO-O-, -CO-S- or -CO-NH- and are each preferably -O-, -O-CO-, -NH-CO- or - CO-NH-, more preferably -O-, -NH-CO- or -CO-NH-, further preferably -NH-CO-.

When P1 or P4 is, for example, -NH-CO-, a substructure -NH-CO-benzene ring is present.

Q1, Q2, Q3 and Q4 are each independently absent, or substituted or unsubstituted alkylene having 1 to 12 carbon atoms or -(CH₂CH₂O)ₙ-CH₂CH₂- wherein n is an integer of 0 to 99, and are each preferably substituted or unsubstituted alkylene having 1 to 12 carbon atoms, more preferably unsubstituted alkylene having 1 to 12 carbon atoms, further preferably unsubstituted alkylene having 1 to 6 carbon atoms, still further preferably unsubstituted alkylene having 1 to 4 carbon atoms.

P2 and P5 are each independently absent, or -CO-, - NH-, -O-, -S-, -O-CO-, -S-CO-, -NH-CO-, -CO-O-, -CO-S- or -CO-NH- and are each preferably absent, -CO-O- or -CO-NH-, more preferably absent or -CO-NH-. When each of P2 and P5 is, for example, -CO-NH-, substructures B1-CO-NH-Q1 and B2-CO-NH-Q3 are present.

-[P2-Q1]_{q1}- and -[P5-Q3]_{q3}- are each independently preferably absent, or any structure represented by the following formulas 3-1 to 3-3:

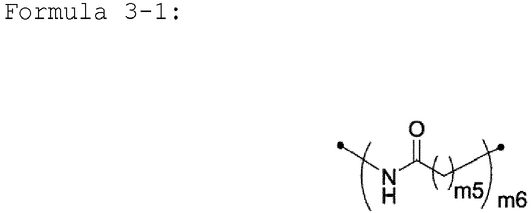

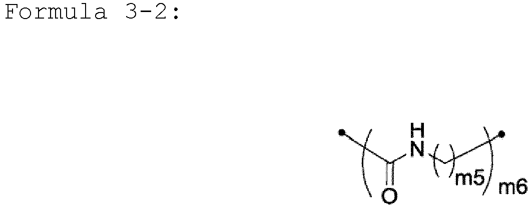

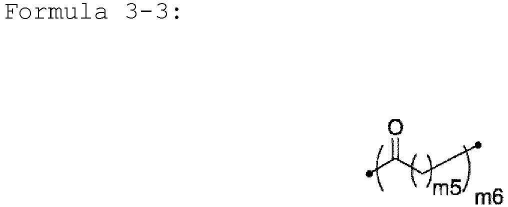

In formulas 3-1 to 3-3,
m5 and m6 are each independently an integer of 0 to 10, and each of the terminal dots in the structures of formulas 3-1 to 3-3 is a binding site to B1 or B2, or P1 or P4.

B1 and B2 are each independently a bond, or any structure represented by the following formulas, wherein each of the terminal dots in each structure is a binding site to P2 or P3, or P5 or P6, and m1, m2, m3 and m4 are each independently an integer of 0 to 10:

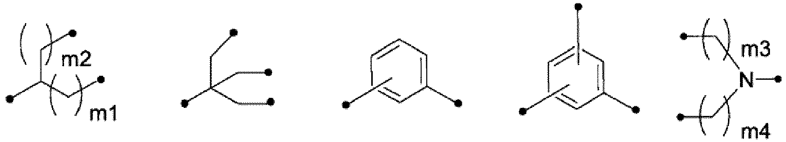

Each of B1 and B2 is preferably a group derived from an amino acid such as glutamic acid, aspartic acid, lysine, including non-natural amino acids such as iminodiacetic acid, or an amino alcohol such as 2-amino-1,3-propanediol. When B1 and B2 are groups derived from glutamic acid or aspartic acid, it is preferred that the amino group of each glutamic acid or aspartic acid should be bonded while P2 and P5 should be -NH-CO- bonds. When B1 and B2 are groups derived from lysine, it is preferred that the carboxyl group of each lysine should be bonded while P2 and P5 should be -CO-NH- bonds. When B1 and B2 are groups derived from iminodiacetic acid, it is preferred that the amino group of each iminodiacetic acid should be bonded while P2 and P5 should be -CO- bonds. Specifically, each of B1 and B2 preferably has any of the following structures:

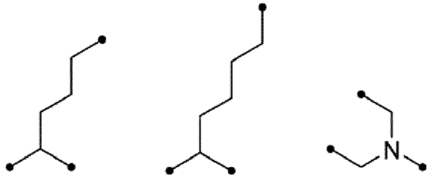

When each of p1 and p2 is an integer of 2 or 3, each P3 and P6, Q2 and Q4, T1 and T2 or L1 and L2 are the same or different.

When q1 to q4 are 2 to 10, combinations -[P2-Q1]-, - [Q2-P3]-, -[P5-Q3]- or -[Q4-P6]- are the same or different. The same or different combinations -[P2-Q1]-, -[Q2-P3]-, -[P5-Q3]- or -[Q4-P6]- mean that 2 to 10 units each of -[P2-Q1]-,-[Q2-P3]-,-[P5-Q3]-, and -[Q4-P6]- may be the same or may be different.

In the present invention, the nucleic acid conjugate is preferably a nucleic acid conjugate having any structure represented by the following formulas 4-1 to 4-9:

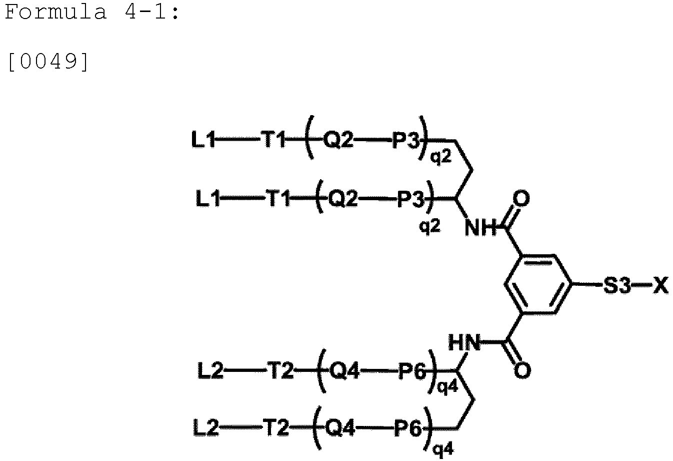

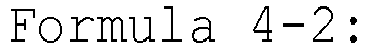

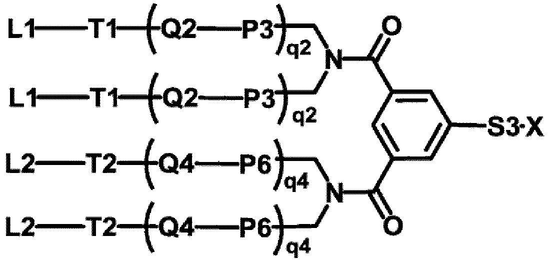

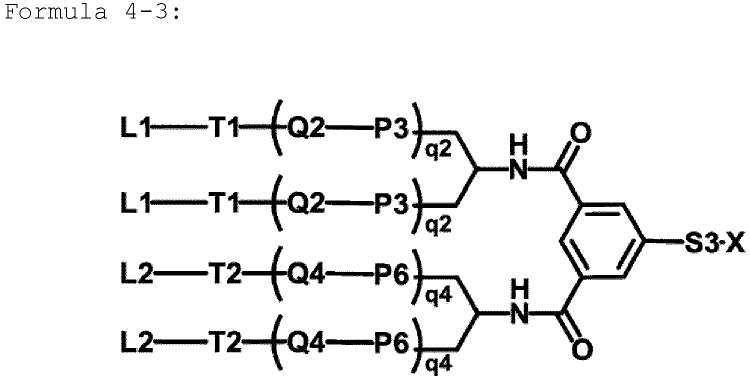

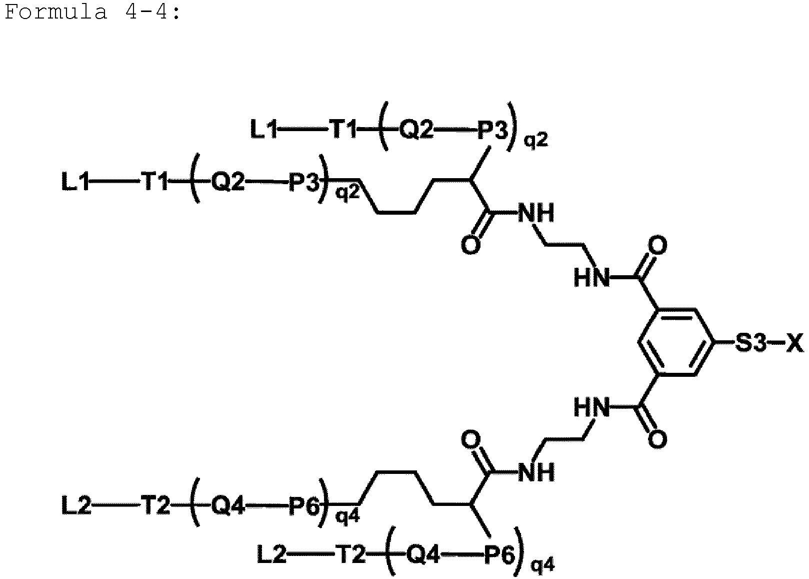

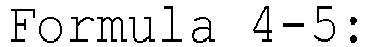

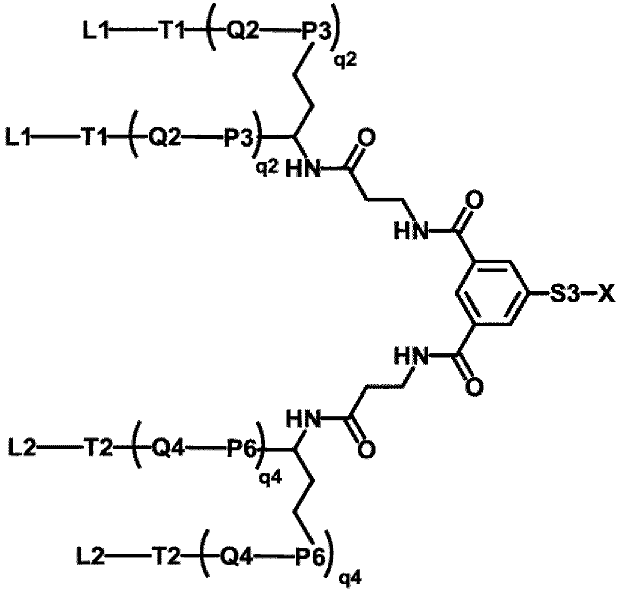

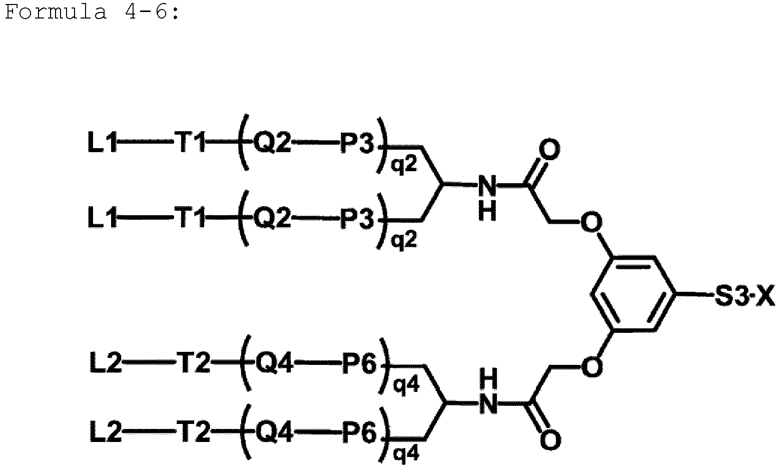

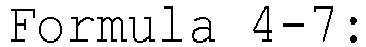

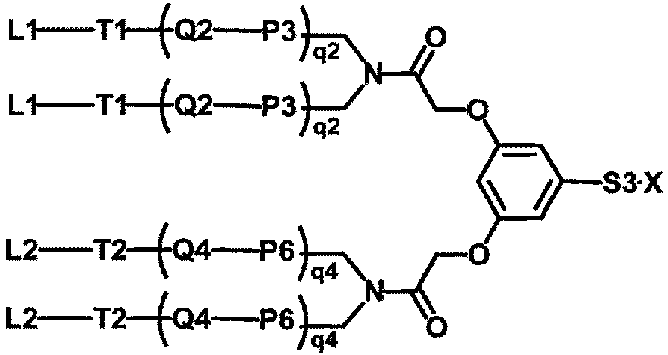

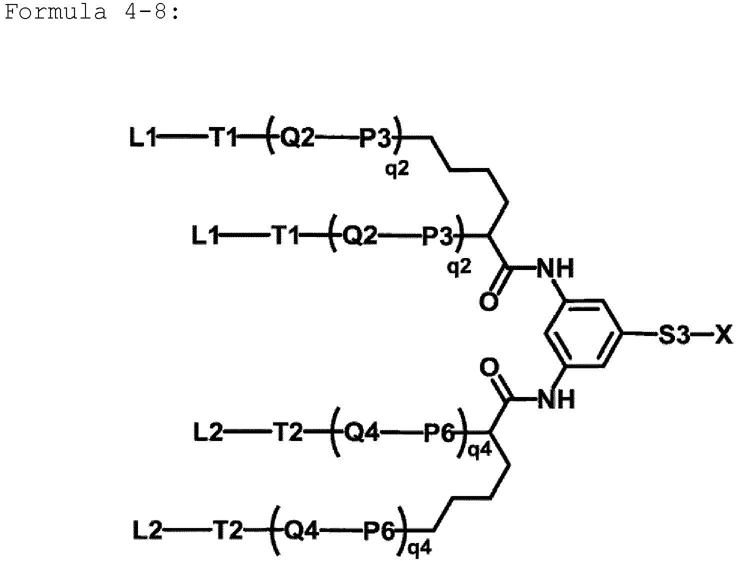

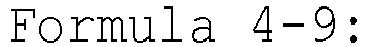

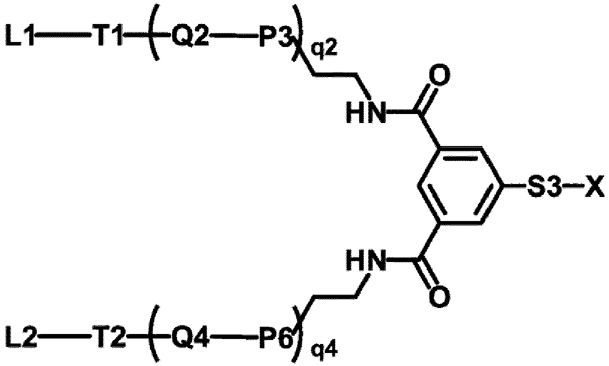

In formulas 4-1 to 4-9,
X, L1, L2, S3, P3, P6, T1, T2, Q2, Q4, q2 and q4 are each as defined above.

P3 and P6 are each independently absent, or -CO-, - NH-, -O-, -S-, -O-CO-, -S-CO-, -NH-CO-, -CO-O-, -CO-S- or -CO-NH- and are each preferably -O-CO- or -NH-CO-, more preferably -NH-CO-. When each of P3 and P6 is, for example, -NH-CO-, substructures B1-NH-CO-Q2 and B2-NH-CO-Q4 are present.

T1 and T2 are each independently absent, or -CO-, - NH-, -O-, -S-, -O-CO-, -S-CO-, -NH-CO-, -CO-O-, -CO-S- or -CO-NH- and are each preferably -O- or -S-, more preferably -O-.

In the present invention, the nucleic acid conjugate is preferably a nucleic acid conjugate having a structure represented by formula 5 given below.

In formula 5, P1 and P4 in formula 2 are the same; P2 and P5 in formula 2 are the same; P3 and P6 in formula 2 are the same; Q1 and Q3 in formula 2 are the same; Q2 and Q4 in formula 2 are the same; B1 and B2 in formula 2 are the same; T1 and T2 in formula 2 are the same; L1 and L2 in formula 2 are the same; p1 and p2 in formula 2 are the same; q1 and q3 in formula 2 are the same; and q2 and q4 in formula 2 are the same.

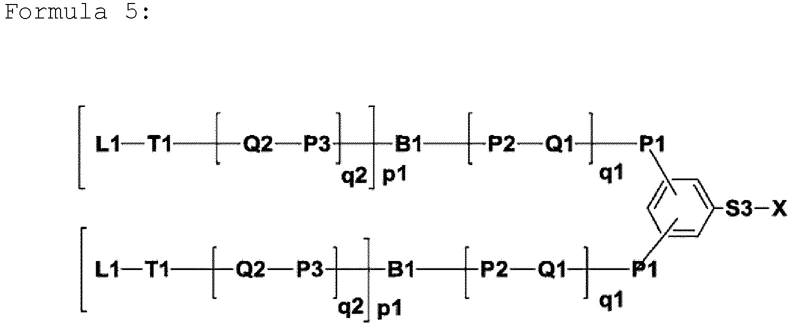

In formula 5,
X, S3, P1, P2, P3, Q1, Q2, B1, T1, L1, p1, q1 and q2 are each as defined above.
X, S3, P1, P2, P3, Q1, Q2, B1, T1, L1, p1, q1 and q2 in formula 5 can each be any of the preferred groups mentioned above. P1 is preferably -CO-NH-, -NH-CO- or - O-.
-(P2-Q1)_{q1}- in formula 5 is preferably absent, or any structure represented by formulas 3-1 to 3-3 described above.

In the present invention, the nucleic acid conjugate is preferably a nucleic acid conjugate having any structure represented by the following formulas 6-1 to 6-9:

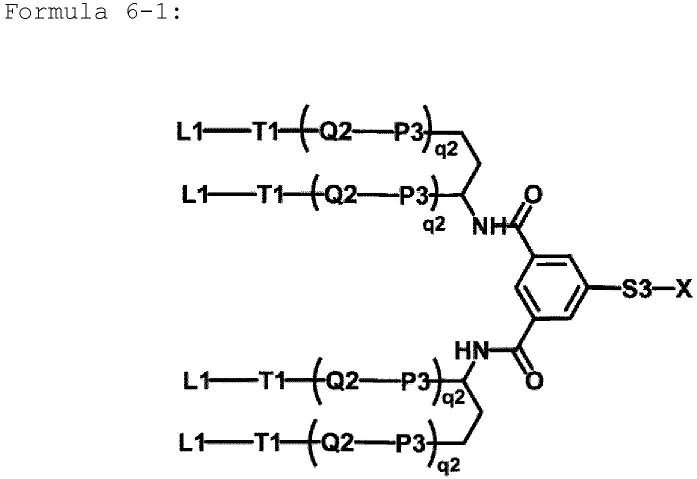

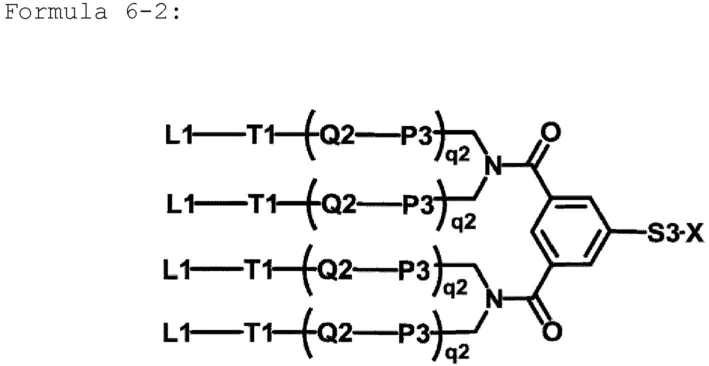

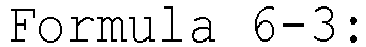

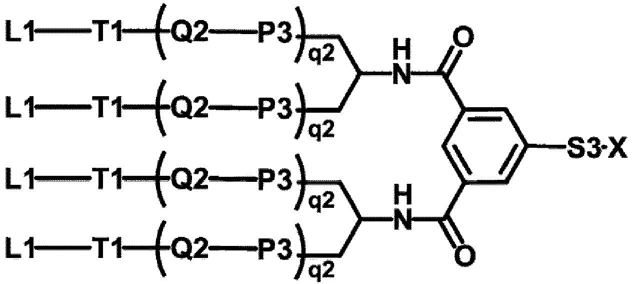

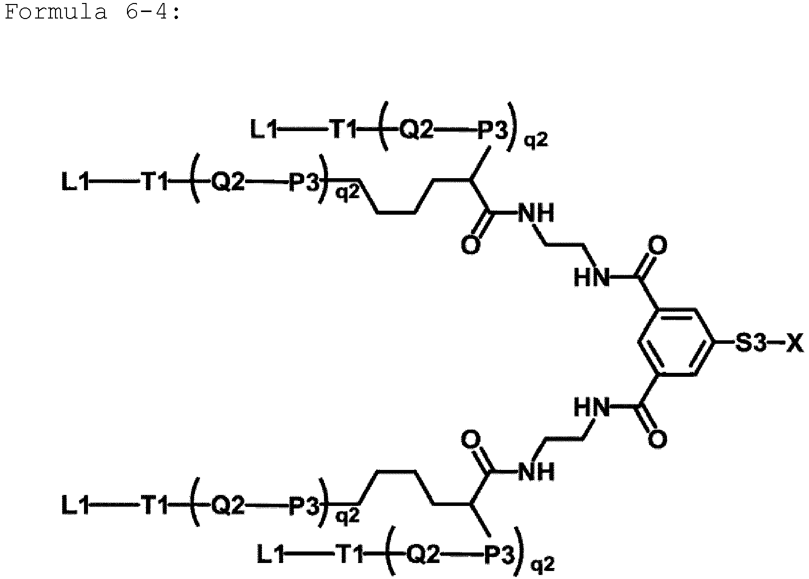

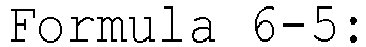

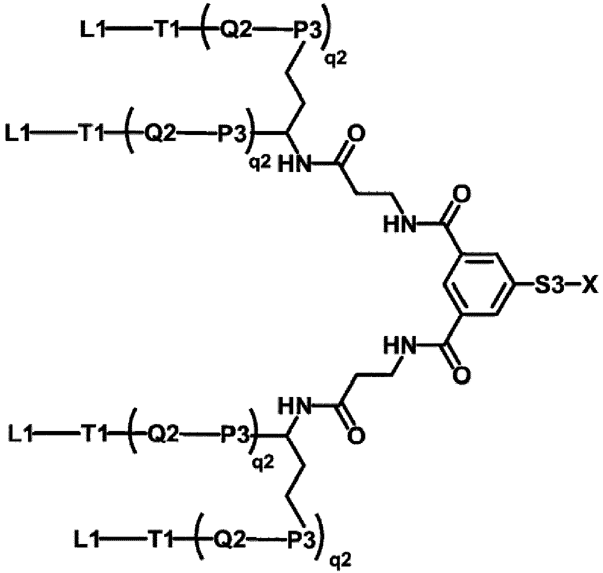

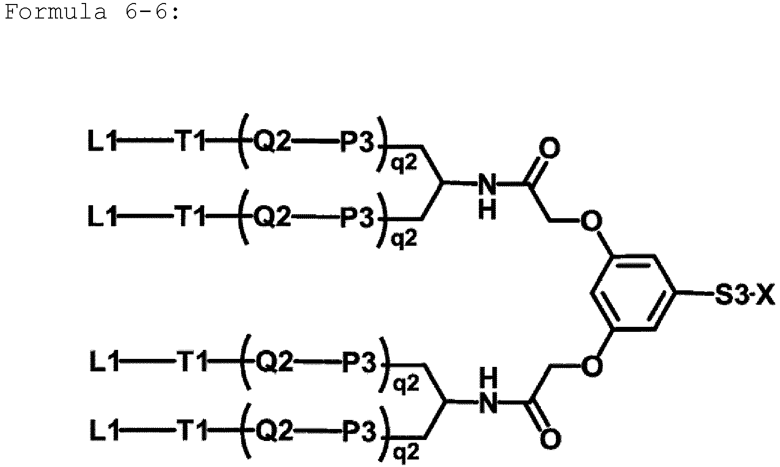

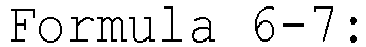

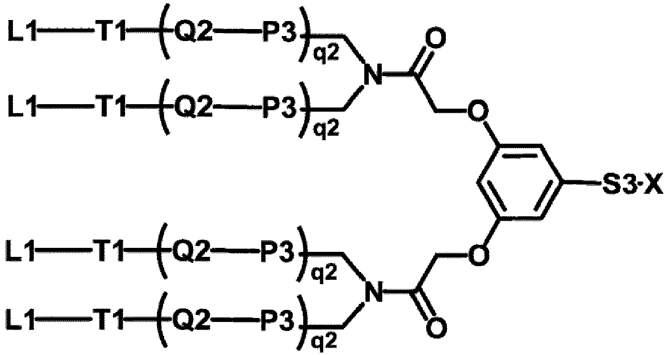

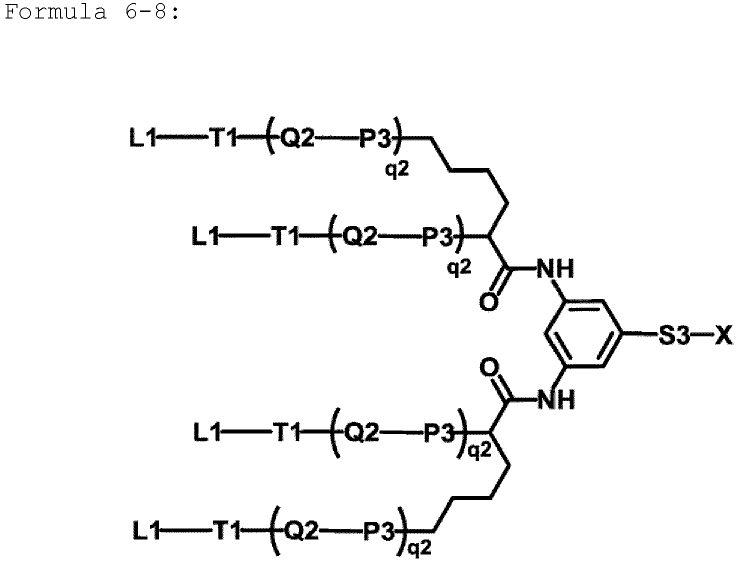

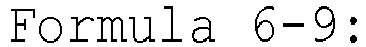

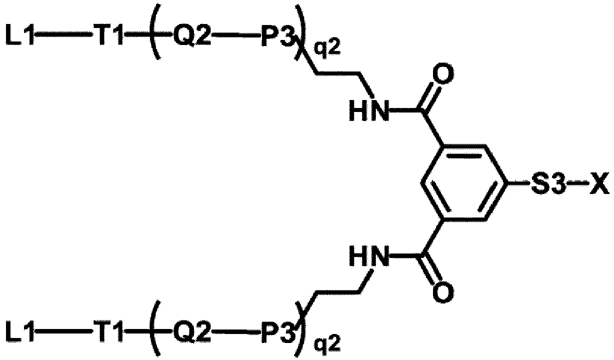

In formulas 6-1 to 6-9,
X, S3, P3, Q2, T1, L1 and q2 are each as defined above.

In the present invention, the nucleic acid conjugate is preferably a nucleic acid conjugate having any structure represented by the following formulas 7-1 to 7-9:

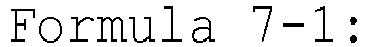

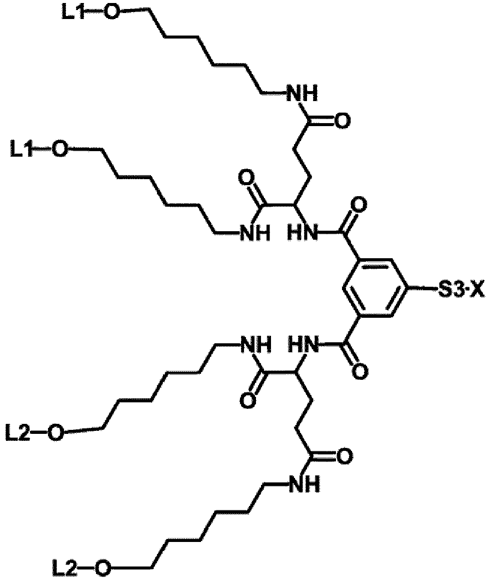

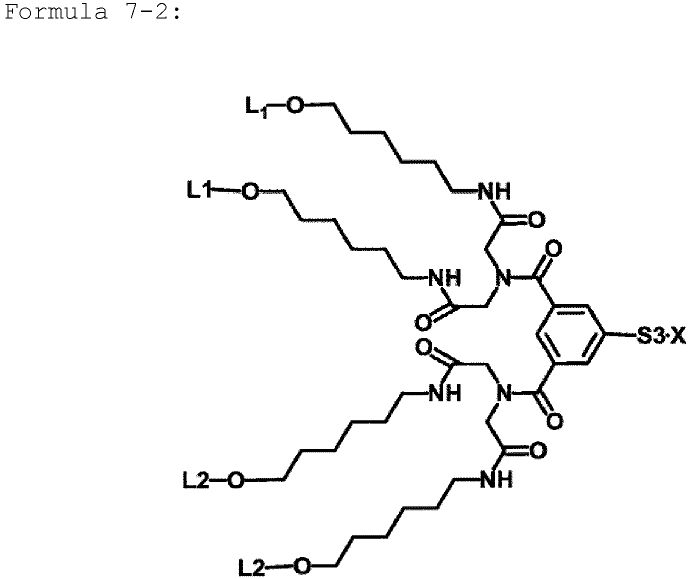

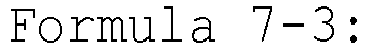

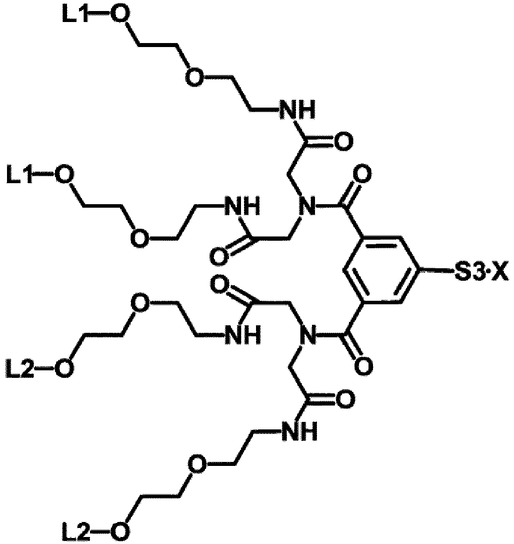

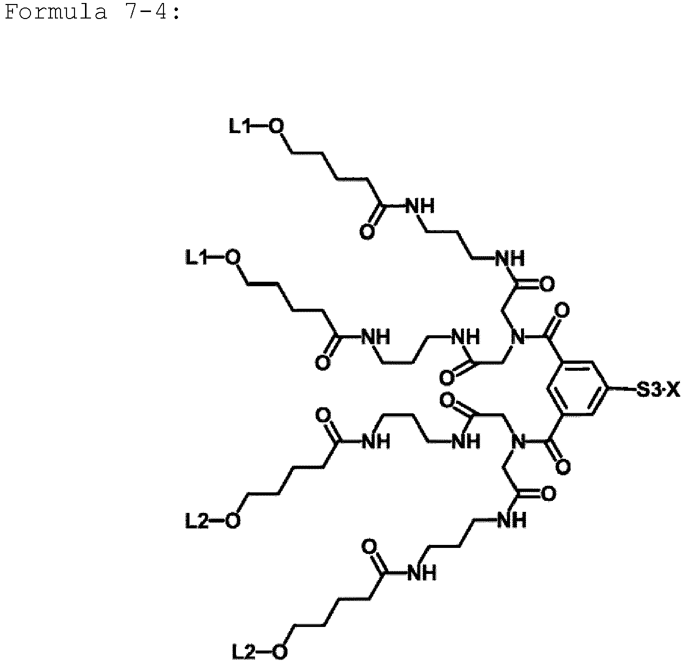

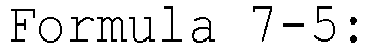

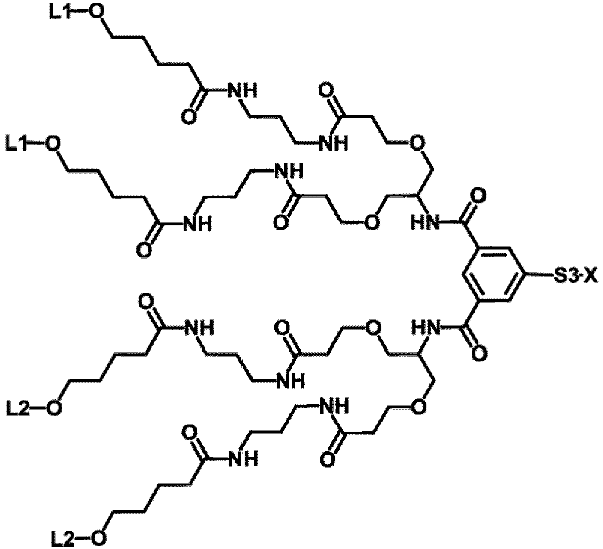

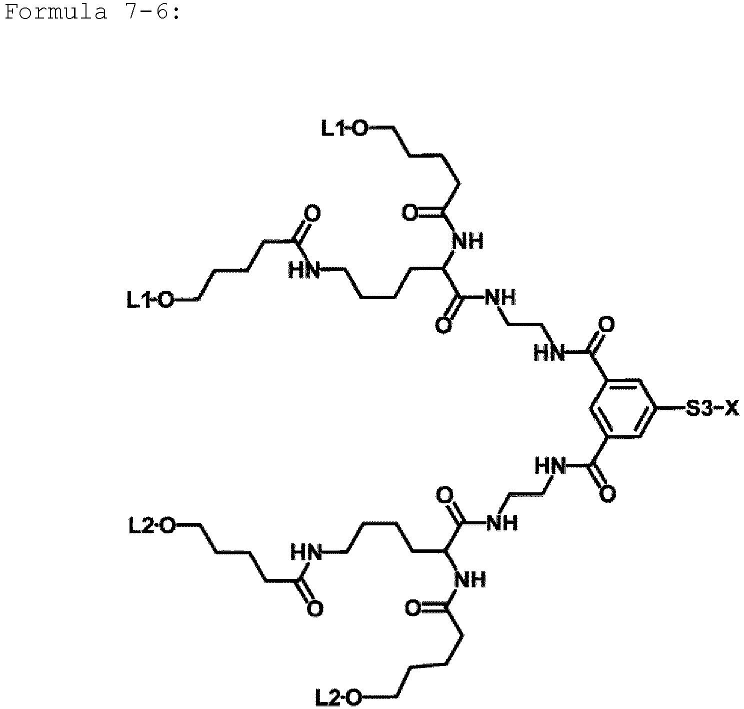

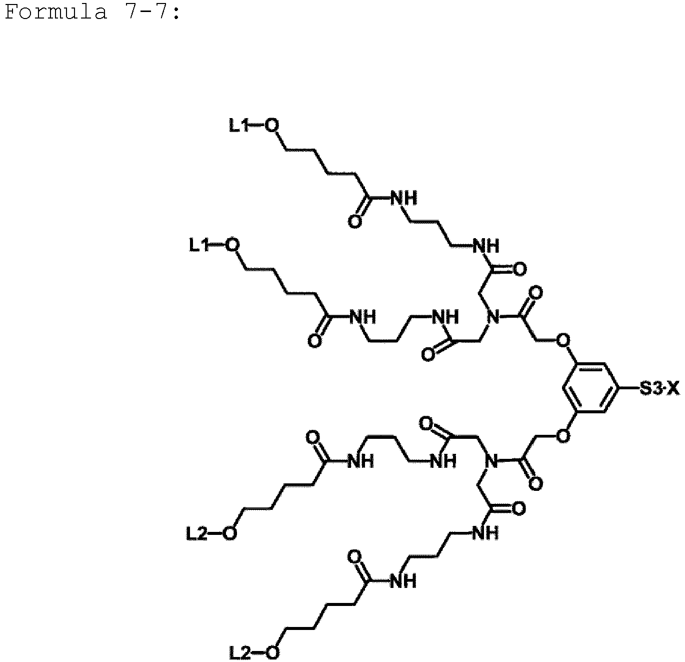

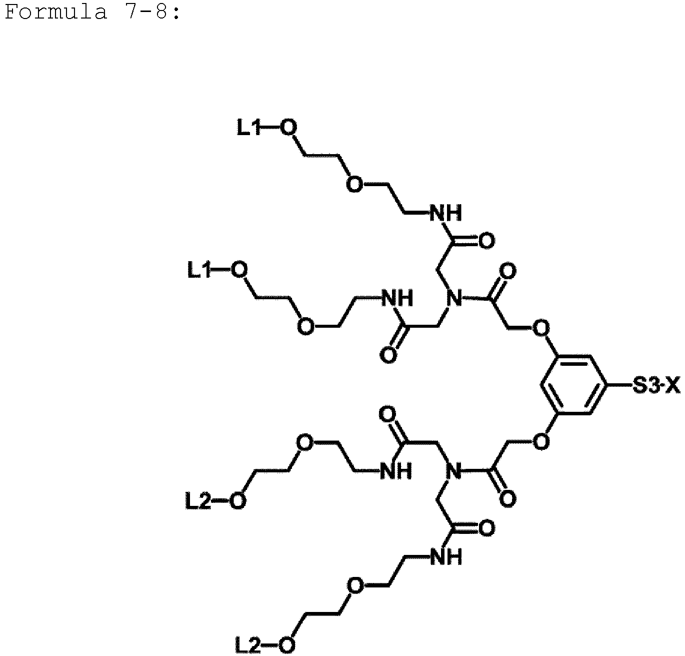

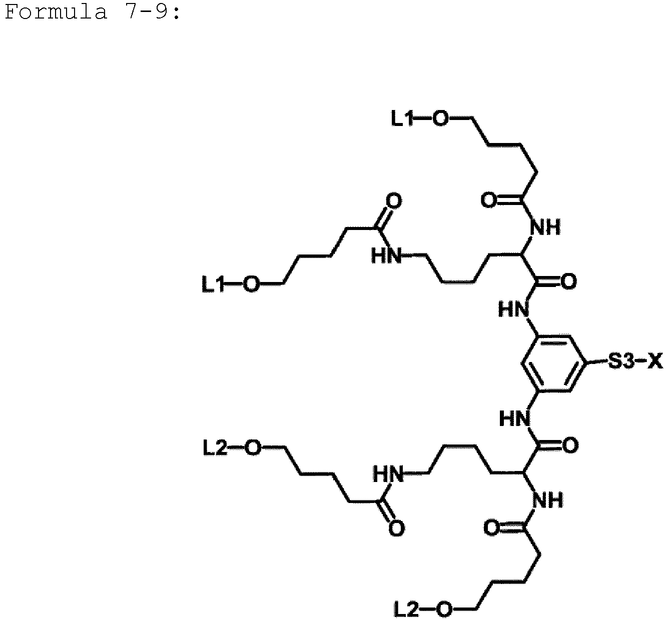

In formulas 7-1 to 7-9,
X, L1, L2 and S3 are each as defined above. L1 and L2 may be the same or may be different and is preferably the same.

A nucleic acid derivative other than the nucleic acid conjugate having any structure represented by formulas 7-1 to 7-9 can also be produced by introducing alkylene chains differing in chain length as each alkylene group moiety in formulas 7-1 to 7-9, or by replacing an amide bond or the like with another bond.

In the present invention, the nucleic acid conjugate is preferably a nucleic acid conjugate having a structure represented by the following formula 11:

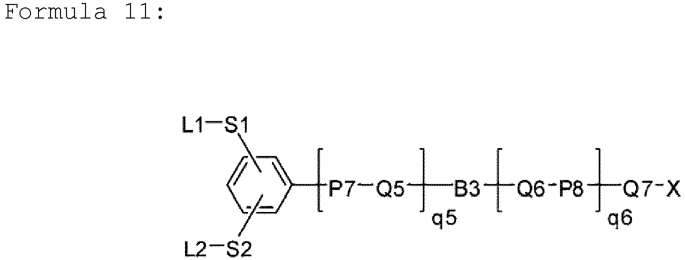

In formula 11,
L1, L2, S1S2 and X are each as defined above,
P7 and P8 are each independently absent, or -CO-, - NH-, -O-, -S-, -O-CO-, -S-CO-, -NH-CO-, -CO-O-, -CO-S- or -CO-NH-,
Q5, Q6 and Q7 are each independently absent, or substituted or unsubstituted alkylene having 1 to 12 carbon atoms or -(CH₂CH₂O)ₙ₈-CH₂CH₂- wherein n8 is an integer of 0 to 99,
B3, which is referred to as a brancher unit in the present specification, is any structure represented by the following formula 11-1, wherein the broken lines respectively mean bonds to Q5 and Q6.

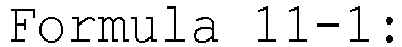

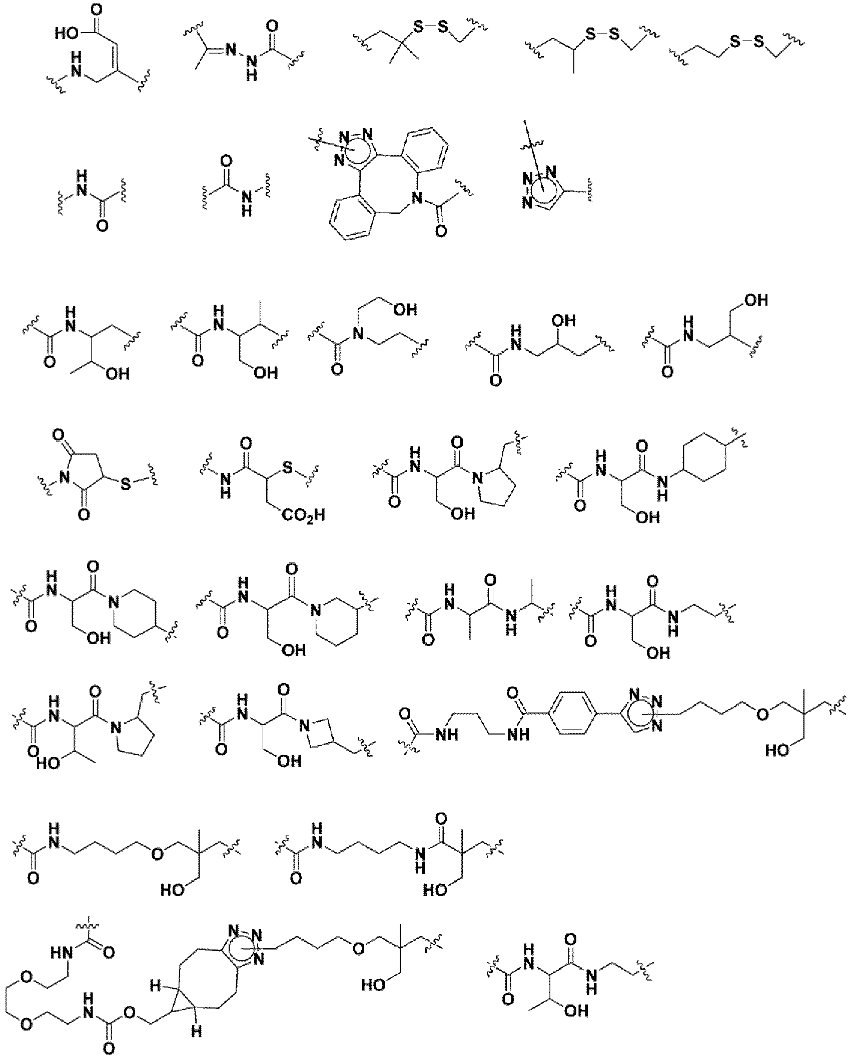

In formula 11-1, substitution in a group having a triazole ring occurs at any of nitrogen atoms at positions 1 and 3 of the triazole ring. q5 and q6 are each independently an integer of 0 to 10.

P7 is absent, or -CO-, -NH-, -O-, -S-, -O-CO-, -S-CO-, -NH-CO-, -CO-O-, -CO-S- or -CO-NH- and is preferably -O-, -NH-CO- or -CO-NH-, more preferably -O- or -NH-CO-. When P7 is, for example, -O-, a substructure benzene ring-O- is present.

P8 is absent, or -CO-, -NH-, -O-, -S-, -O-CO-, -S-CO-, -NH-CO-, -CO-O-, -CO-S- or -CO-NH-. When P8 is present, P8 is preferably -CO-O- or -CO-NH-, more preferably -CO-NH-. When P8 is, for example, -CO-NH-, a substructure Q6-CO-NH- is present.

Q5, Q6 and Q7 are each independently absent, or substituted or unsubstituted alkylene having 1 to 12 carbon atoms or -(CH₂CH₂O)ₙ₈-CH₂CH₂- wherein n8 is an integer of 0 to 99, and are each preferably substituted or unsubstituted alkylene having 1 to 12 carbon atoms, more preferably unsubstituted alkylene having 1 to 12 carbon atoms, further preferably unsubstituted alkylene having 1 to 6 carbon atoms, still further preferably unsubstituted alkylene having 1 to 4 carbon atoms.

Preferably, -(P7-Q5)_{q5}- is -O-(CH₂)ₘ₁₅-NH- or -NH-CO-(CH₂)ₘ₁₆-NH-, and m15 and m16 are each independently an integer of 1 to 10.

In the present invention, the nucleic acid conjugate is preferably a nucleic acid conjugate having any structure represented by the following formulas 12-1 to 12-12:

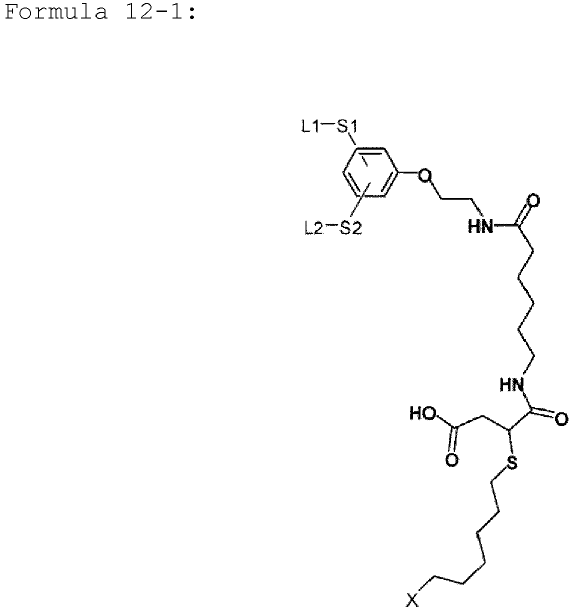

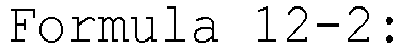

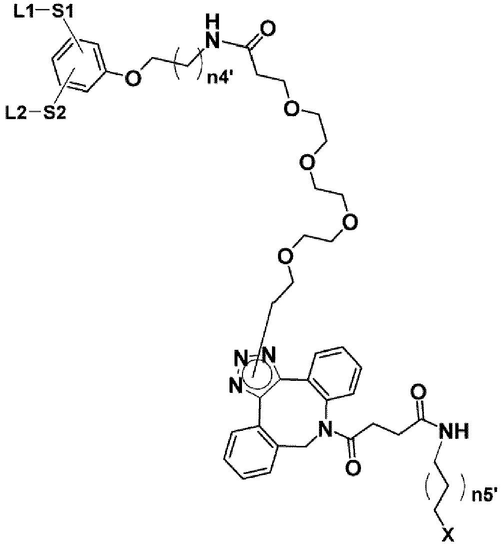

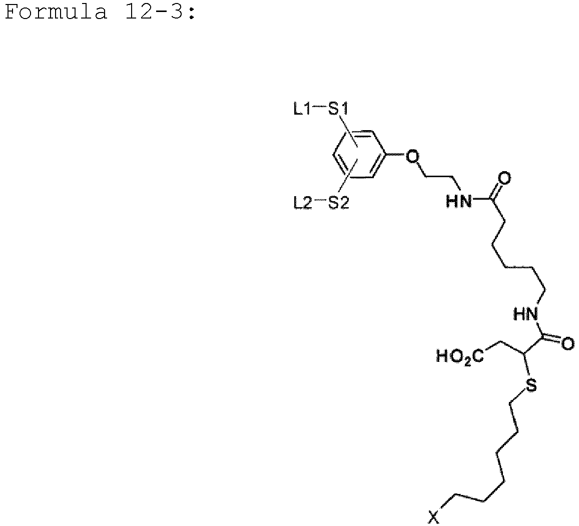

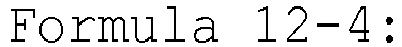

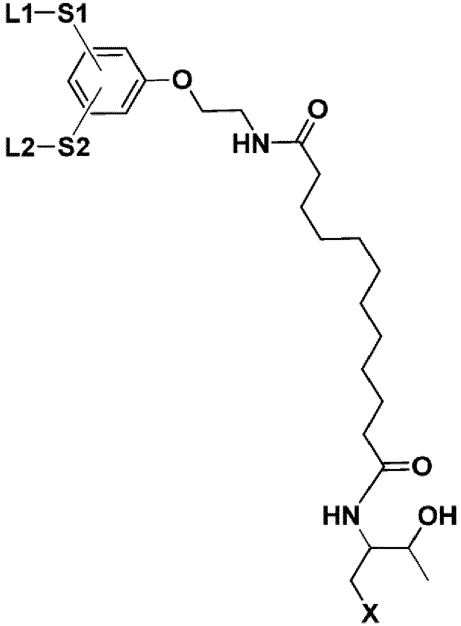

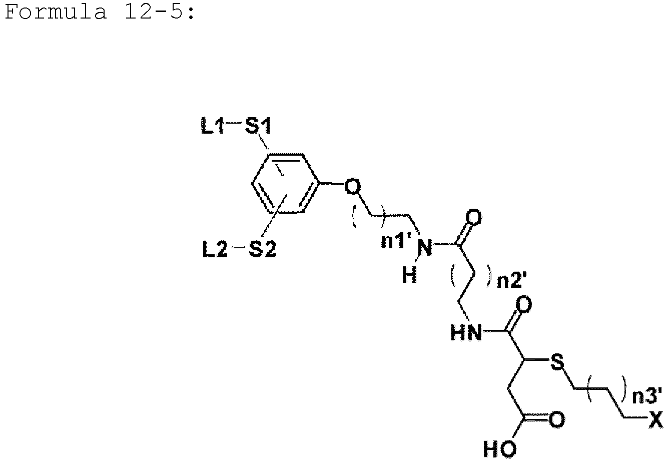

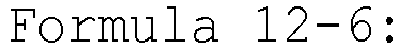

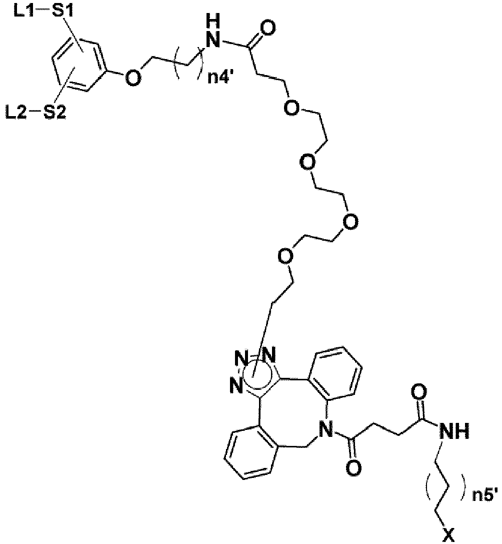

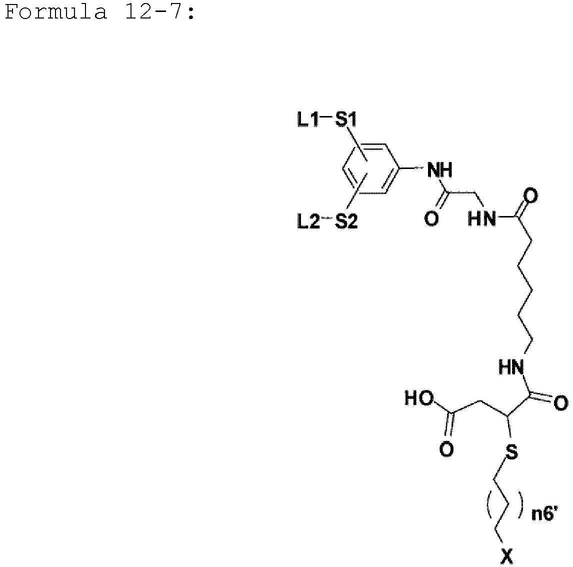

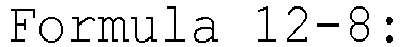

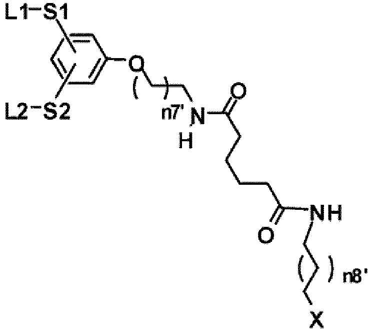

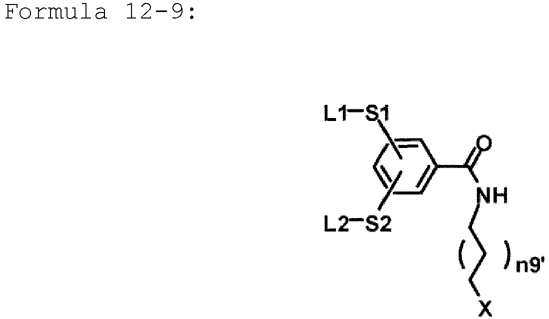

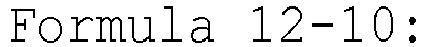

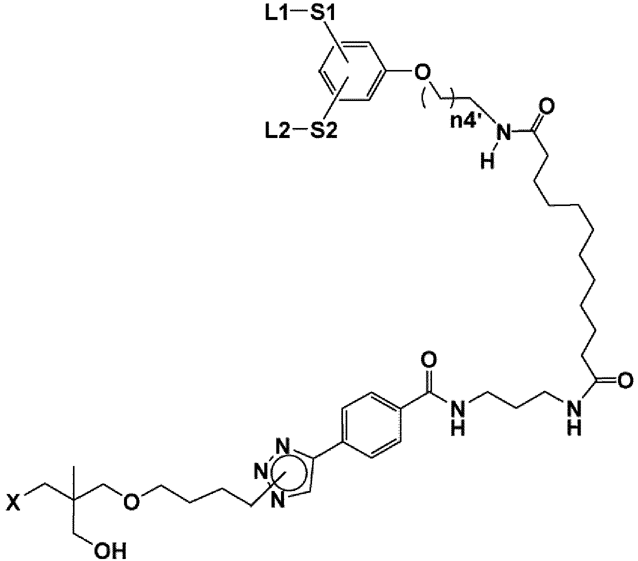

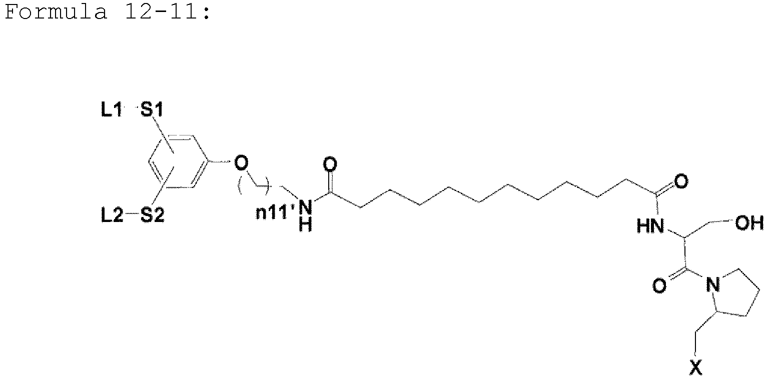

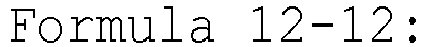

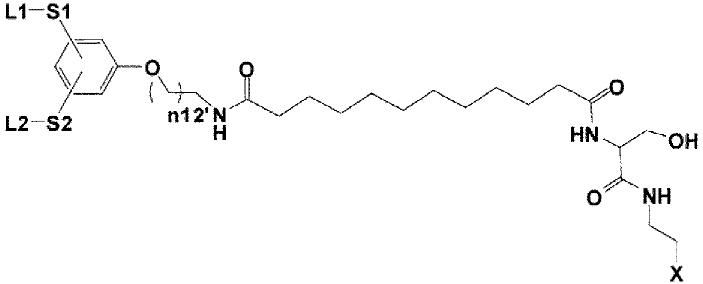

In formulas 12-1 to 12-12,
X, L1, L2, S1 and S2 are each as defined above, and n1' to n12' are each independently an integer of 1 to 10.

The nucleic acid conjugate of the present invention is preferably a nucleic acid conjugate having a structure represented by formula 2 corresponding to S1 and S2 and a structure represented by formula 11 corresponding to S3 in combination in the nucleic acid conjugate represented by formula 1. Formula 2 may be any of formula 4-1 to formula 4-9, may be any of formula 6-1 to formula 6-9, or may be any of formula 7-1 to formula 7-9. When formula 2 is any of formula 4-1 to formula 4-9, formula 6-1 to formula 6-9, or formula 7-1 to formula 7-9, formula 11 may be any of formula 12-1 to formula 12-12. The nucleic acid conjugate of the present invention is more preferably a nucleic acid conjugate having any one structure represented by formula 4-1 to formula 4-9 corresponding to S1 and S2, and any one structure represented by formula 12-1 to formula 12-12 corresponding to S3 in combination, a nucleic acid conjugate having any one structure represented by formula 6-1 to formula 6-9 corresponding to S1 and S2, and any one structure represented by formula 12-1 to formula 12-12 corresponding to S3 in combination, a nucleic acid conjugate having any one structure represented by formula 7-1 to formula 7-9 corresponding to S1 and S2, and any one structure represented by formula 12-1 to formula 12-12 corresponding to S3 in combination in the nucleic acid conjugate represented by formula 1.

The nucleic acid conjugate of the present invention is preferably represented by the following formula 7-8-1: 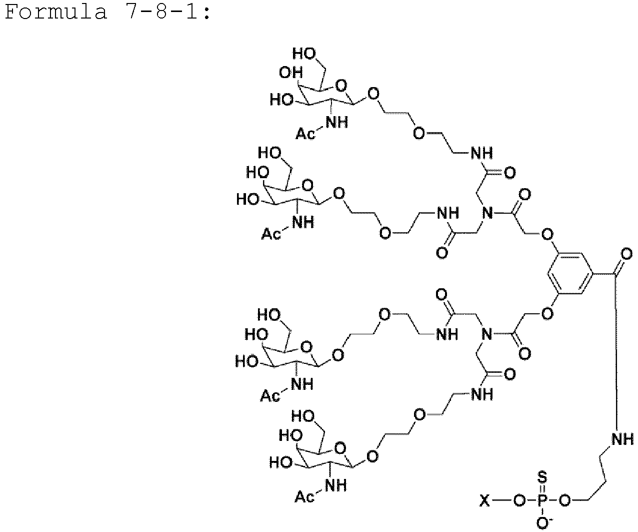 wherein X is as defined above.

X in formula 1 is a double-stranded nucleic acid consisting of a sense strand and an antisense strand and comprising a duplex region of at least 11 base pairs. In the double-stranded nucleic acid, an oligonucleotide strand having a chain length of 17 to 30 nucleotides in the antisense strand is complementary to any of target APCS mRNA sequences described in Tables 1-1 to 1-13. The 3' end or the 5' end of the sense strand binds to S3. Therefore, in formula 1, X binding to S3 is the sense strand constituting the double-stranded nucleic acid and is a sense strand represented by any of sense strand sequences in Tables 1-1 to 1-13, M1-1 to M1-3 and R-1 to R-2 mentioned later.

In the present invention, a nucleic acid comprising a nucleotide sequence complementary to APCS mRNA is also referred to as an antisense strand nucleic acid, and a nucleic acid comprising a nucleotide sequence complementary to the nucleotide sequence of the antisense strand nucleic acid is also referred to as a sense strand nucleic acid.

The double-stranded nucleic acid constituting the nucleic acid conjugate of the present invention is a double-stranded nucleic acid having the ability to decrease or arrest the expression of APCS gene when transferred to mammalian cells, and is a double-stranded nucleic acid having a sense strand and an antisense strand. The sense strand and the antisense strand have at least 11 base pairs. An oligonucleotide strand having a chain length of at least 17 nucleotides and at most 30 nucleotides, i.e., 17 to 30 nucleotides, in the antisense strand is complementary to a target APCS mRNA sequence selected from a group described in Tables 1-1 to 1-13.

The double-stranded nucleic acid constituting the nucleic acid conjugate of the present invention can be any double-stranded nucleic acid which is a polymer of nucleotides or molecules functionally equivalent to nucleotides. Examples of such a polymer include RNA which is a polymer of ribonucleotides, DNA which is a polymer of deoxyribonucleotides, a chimeric nucleic acid consisting of RNA and DNA, and a nucleotide polymer derived from any of these nucleic acids by the replacement of at least one nucleotide with a molecule functionally equivalent to the nucleotide. A derivative of any of these nucleic acids containing at least one molecule functionally equivalent to the nucleotide is also included in the double-stranded nucleic acid as a drug used in the present invention. Uracil (U) and thymine (T) in DNA can be used interchangeably with each other.

Examples of the molecules functionally equivalent to nucleotides include nucleotide derivatives. The nucleotide derivative may be any molecule as long as the molecule is prepared by modifying a nucleotide. For example, a modified ribonucleotide or deoxyribonucleotide molecule is suitably used for improving or stabilizing nuclease resistance, for enhancing affinity for a complementary strand nucleic acid, for enhancing cell permeability, or for visualizing the molecule, as compared with RNA or DNA.

Examples of the molecule prepared by modifying a nucleotide include nucleotides modified at the sugar moiety, nucleotides modified at the phosphodiester bond, nucleotides modified at the base, and nucleotides modified at at least one of a sugar moiety, a phosphodiester bond and a base.

The nucleotide modified at the sugar moiety can be any nucleotide in which a portion or the whole of the chemical structure of its sugar is modified or substituted with an arbitrary substituent or substituted with an arbitrary atom. A 2'-modified nucleotide is preferably used.

The 2'-modified nucleotide is a nucleotide in which the 2'-OH group of ribose is substituted with a substituent selected from the group consisting of H, OR, R, R'OR, SH, SR, NH₂, NHR, NR₂, N₃, CN, F, Cl, Br and I (R is alkyl or aryl, preferably alkyl having 1 to 6 carbon atoms, and R' is alkylene, preferably alkylene having 1 to 6 carbon atoms), more preferably a nucleotide in which the 2'-OH group is substituted with H, F or a methoxy group, further preferably a nucleotide in which the 2'-OH group is substituted with F or a methoxy group. Further examples thereof include nucleotides in which the 2'-OH group is substituted with a substituent selected from the group consisting of a 2-(methoxy)ethoxy group, a 3-aminopropoxy group, a 2-[(N,N-dimethylamino)oxy]ethoxy group, a 3-(N,N-dimethylamino)propoxy group, a 2-[2-(N,N-dimethylamino)ethoxy]ethoxy group, a 2-(methylamino)-2-oxoethoxy group, a 2-(N-methylcarbamoyl)ethoxy group and a 2-cyanoethoxy group.

The double-stranded nucleic acid preferably contains 50 to 100%, more preferably 70 to 100%, further preferably 90 to 100%, of the 2'-modified nucleotide with respect to the nucleotides within the double-stranded nucleic acid region. The sense strand preferably contains 20 to 100%, more preferably 40 to 100%, further preferably 60 to 100%, of the 2'-modified nucleotide with respect to the nucleotides of the sense strand. The antisense strand preferably contains 20 to 100%, more preferably 40 to 100%, further preferably 60 to 100%, of the 2'-modified nucleotide with respect to the nucleotides of the antisense strand.

The nucleotide modified at the phosphodiester bond can be any nucleotide in which a portion or the whole of the chemical structure of its phosphodiester bond is modified or substituted with an arbitrary substituent or substituted with an arbitrary atom. Examples thereof include a nucleotide resulting from the substitution of the phosphodiester bond with a phosphorothioate bond, a nucleotide resulting from the substitution of the phosphodiester bond with a phosphorodithioate bond, a nucleotide resulting from the substitution of the phosphodiester bond with an alkyl phosphonate bond, and a nucleotide resulting from the substitution of the phosphodiester bond with a phosphoramidate bond.

The nucleotide modified at the base can be any nucleotide in which a portion or the whole of the chemical structure of its base is modified or substituted with an arbitrary substituent or substituted with an arbitrary atom. Examples thereof include a nucleotide resulting from the substitution of an oxygen atom in the base with a sulfur atom, a nucleotide resulting from the substitution of a hydrogen atom with an alkyl group having 1 to 6 carbon atoms, halogen, or the like, a nucleotide resulting from the substitution of a methyl group with hydrogen, hydroxymethyl, an alkyl group having 2 to 6 carbon atoms, or the like, and a nucleotide resulting from the substitution of an amino group with an alkyl group having 1 to 6 carbon atoms, an alkanoyl group having 1 to 6 carbon atoms, an oxo group, a hydroxy group, or the like.

Examples of the nucleotide derivatives also include nucleotides or nucleotides modified at at least one of a sugar moiety, a phosphodiester bond and a base which contain an additional chemical substance, such as peptide, protein, sugar, lipid, phospholipid, phenazine, folate, phenanthridine, anthraquinone, acridine, fluorescein, rhodamine, coumarin, or a dye, added thereto directly or via a linker, and specifically include 5'-polyamine-added nucleotide derivatives, cholesterol-added nucleotide derivatives, steroid-added nucleotide derivatives, bile acid-added nucleotide derivatives, vitamin-added nucleotide derivatives, Cy5-added nucleotide derivatives, Cy3-added nucleotide derivatives, 6-FAM-added nucleotide derivatives, and biotin-added nucleotide derivatives.

The nucleotide derivative may form a bridged structure, such as an alkylene structure, a peptide structure, a nucleotide structure, an ether structure, an ester structure, and a structure combined with at least one of these structures, with another nucleotide or nucleotide derivative within the nucleic acid.

In the present specification, the term "complementation" means a relationship capable of forming a base pair between two bases and refers to the formation of a double helix structure as the whole duplex region via a mild hydrogen bond, for example, the relationship between adenine and thymine or uracil, and the relationship between guanine and cytosine.

In the present specification, the term "complementary" not only means the case where two nucleotide sequences are completely complementary to each other, but means that 0 to 30%, 0 to 20% or 0 to 10% of a mismatch base can be present between the nucleotide sequences, and, for example, an antisense strand complementary to APCS mRNA may contain the substitution of one or more bases in its nucleotide sequence completely complementary to a partial nucleotide sequence of the mRNA. Specifically, the antisense strand may have 1 to 8, preferably 1 to 6, 1 to 4 or 1 to 3, particularly, 2 or 1 mismatch bases for a target sequence of a target gene. For example, when the antisense strand is 21 bases long, the antisense strand may have 6, 5, 4, 3, 2 or 1 mismatch bases for a target sequence of a target gene. The position of the mismatch may be the 5' end or the 3' end of each sequence.

Also, the term "complementary" is meant to encompass the case where two nucleotide sequences, one of which is completely complementary to the other nucleotide sequence, have the addition and/or deletion of one or more bases. For example, APCS mRNA and the antisense strand nucleic acid of the present invention may have 1 or 2 bulge bases in the antisense strand and/or target APCS mRNA region by the addition and/or deletion of base(s) in the antisense strand.

The double-stranded nucleic acid as a drug used in the present invention may be constituted by any nucleotide or derivative thereof as long as the nucleotide or the derivative is a nucleic acid comprising a nucleotide sequence complementary to a partial nucleotide sequence of APCS mRNA and/or a nucleic acid comprising a nucleotide sequence complementary to the nucleotide sequence of the nucleic acid. The double-stranded nucleic acid of the present invention can have any length as long as the nucleic acid comprising a nucleotide sequence complementary to the target APCS mRNA sequence and the nucleic acid comprising a nucleotide sequence complementary to the nucleotide sequence of the nucleic acid can form a duplex of at least 11 base pairs. The sequence length that allows formation of the duplex is usually 11 to 27 bases, preferably 15 to 25 bases, more preferably 17 to 23 bases, further preferably 19 to 23 bases.

A nucleic acid comprising a nucleotide sequence complementary to the target APCS mRNA sequence is used as the antisense strand of the nucleic acid conjugate of the present invention. A nucleic acid derived from the nucleic acid by the deletion, substitution or addition of 1 to 3 bases, preferably 1 to 2 bases, more preferably 1 base, may be used.

A single-stranded nucleic acid that consists of a nucleic acid comprising a nucleotide sequence complementary to the target APCS mRNA sequence and inhibits the expression of APCS, or a double-stranded nucleic acid that consists of a nucleic acid comprising a nucleotide sequence complementary to the target APCS mRNA sequence and a nucleic acid comprising a nucleotide sequence complementary to the nucleotide sequence of the nucleic acid and inhibits the expression of APCS is suitably used as a nucleic acid inhibiting the expression of APCS.

The antisense strand nucleic acid and the sense strand nucleic acid constituting the double-stranded nucleic acid are the same or different and each usually consist of 11 to 30 bases, and are the same or different and each preferably consist of 17 to 27 bases, more preferably 17 to 25 bases, further preferably 19 to 25 bases, still further preferably 21 or 23 bases.

The double-stranded nucleic acid used as a drug in the present invention may have a non-duplex-forming additional nucleotide or nucleotide derivative on the 3' or 5' side subsequent to the duplex region. This non-duplex-forming moiety is referred to as an overhang. When the double-stranded nucleic acid has an overhang, the nucleotide constituting the overhang may be a ribonucleotide, a deoxyribonucleotide or a derivative thereof.

A double-stranded nucleic acid having an overhang consisting of 1 to 6 bases, usually 1 to 3 bases, at the 3' or 5' end of at least one of the strands is used as the double-stranded nucleic acid having the overhang. A double-stranded nucleic acid having an overhang consisting of 2 bases is preferably used. Examples thereof include double-stranded nucleic acids having an overhang consisting of dTdT (dT represents deoxythymidine) or UU (U represents uridine). The overhang can be present in only the antisense strand, only the sense strand, and both the antisense strand and the sense strand. In the present invention, a double-stranded nucleic acid having an overhang in the antisense strand is preferably used. In the antisense strand, an oligonucleotide strand consisting of 17 to 30 nucleotides comprising the duplex region and the overhang subsequent thereto is sufficiently complementary to a target APCS mRNA sequence selected from a group described in Tables 1-1 to 1-13. Alternatively, for example, a nucleic acid molecule that forms a double-stranded nucleic acid by the action of ribonuclease such as Dicer (WO2005/089287), a double-stranded nucleic acid having blunt ends formed without having 3' terminal and 5' terminal overhangs, or a double-stranded nucleic acid having a protruding sense strand (US2012/0040459) may be used as the double-stranded nucleic acid of the present invention.

A nucleic acid consisting of a sequence identical to the nucleotide sequence of a target gene or the nucleotide sequence of its complementary strand may be used in the double-stranded nucleic acid constituting the nucleic acid conjugate of the present invention. A double-stranded nucleic acid consisting of a nucleic acid derived from the nucleic acid by the truncation of 1 to 4 bases from the 5' or 3' end of at least one strand, and a nucleic acid comprising a nucleotide sequence complementary to the nucleotide sequence of the nucleic acid may be used.

The double-stranded nucleic acid constituting the nucleic acid conjugate of the present invention may be double-stranded RNA (dsRNA) comprising a RNA duplex, double-stranded DNA (dsDNA) comprising a DNA duplex, or a hybrid nucleic acid comprising a RNA-DNA duplex. Alternatively, the double-stranded nucleic acid may be a chimeric nucleic acid having two strands, one or both of which consists of DNA and RNA. Double-stranded RNA (dsRNA) is preferred.

Preferably, the 2nd nucleotide counted from the 5' end of the antisense strand of the nucleic acid conjugate of the present invention is complementary to the 2nd deoxyribonucleotide counted from the 3' end of the target APCS mRNA sequence. More preferably, the 2nd to 7th nucleotides counted from the 5' end of the antisense strand are completely complementary to the 2nd to 7th deoxyribonucleotides counted from the 3' end of the target APCS mRNA sequence. Further preferably, the 2nd to 11th nucleotides counted from the 5' end of the antisense strand are completely complementary to the 2nd to 11th deoxyribonucleotides counted from the 3' end of the target APCS mRNA sequence. Also preferably, the 11th nucleotide counted from the 5' end of the antisense strand in the nucleic acid of the present invention is complementary to the 11th deoxyribonucleotide counted from the 3' end of the target APCS mRNA sequence. More preferably, the 9th to 13th nucleotides counted from the 5' end of the antisense strand are completely complementary to the 9th to 13th deoxyribonucleotides counted from the 3' end of the target APCS mRNA sequence. Further preferably, the 7th to 15th nucleotides counted from the 5' end of the antisense strand are completely complementary to the 7th to 15th deoxyribonucleotides counted from the 3' end of the target APCS mRNA sequence.

The antisense strand and the sense strand of the nucleic acid conjugate of the present invention can be designed on the basis of, for example, the nucleotide sequence (SEQ ID NO: 1) of cDNA (sense strand) of full-length mRNA of human APCS registered under GenBank Accession No. NM_001639.3.

The double-stranded nucleic acid can be designed so as to interact with a target sequence within the APCS gene sequence.

The sequence of one strand of the double-stranded nucleic acid is complementary to the sequence of the target site described above. The double-stranded nucleic acid can be chemically synthesized by use of a method described in the present specification.

RNA may be produced enzymatically or by partial or total organic synthesis. A modified ribonucleotide can be introduced enzymatically or by organic synthesis *in vitro.* In one aspect, each strand is chemically prepared. A method for chemically synthesizing a RNA molecule is known in the art [see Nucleic Acids Research, 1998, Vol. 32, p. 936-948]. In general, the double-stranded nucleic acid can be synthesized by use of a solid-phase oligonucleotide synthesis method (see, for example, Usman et al., U.S. Patent No. 5,804,683; U.S. Patent No. 5,831,071; U.S. Patent No. 5,998,203; U.S. Patent No. 6,117,657; U.S. Patent No. 6,353,098; U.S. Patent No. 6,362,323; U.S. Patent No. 6,437,117; and U.S. Patent No. 6,469,158; Scaringe et al., U.S. Patent No. 6,111,086; U.S. Patent No. 6,008,400; and U.S. Patent No. 6,111,086).

The single-stranded nucleic acid is synthesized by use of a solid-phase phosphoramidite method [see Nucleic Acids Research, 1993, Vol. 30, p. 2435-2443], deprotected, and desalted on NAP-5 column (Amersham Pharmacia Biotech Ltd., Piscataway, NJ). The oligomer is purified by ionexchange high-performance liquid chromatography (IE-HPLC) on Amersham Source 15Q column (1.0 cm, height: 25 cm; Amersham Pharmacia Biotech Ltd., Piscataway, NJ) using a linear gradient in a 15-minute step. The gradient shifts from buffer solution A:B of 90:10 to buffer solution A:B of 52:48. The buffer solution A is 100 mmol/L Tris, pH 8.5, and the buffer solution B is 100 mmol/L Tris, pH 8.5 (1 mol/L NaCl). A sample is monitored at 260 nm, and a peak corresponding to full-length oligonucleotide species is collected, pooled, desalted on NAP-5 column, and freeze-dried.

The purity of each single-stranded nucleic acid is determined by capillary electrophoresis (CE) using Beckman PACE 5000 (Beckman Coulter, Inc., Fullerton, Calif). The CE capillary has an inside diameter of 100 µm and contains ssDNA 100R Gel (Beckman-Coulter, Inc.). Typically, approximately 0.6 nmole of the oligonucleotide is injected to the capillary, and CE is carried out in an electric field of 444 V/cm, followed by the detection of UV absorbance at 260 nm. An electrophoresis buffer solution containing modified Tris-borate and 7 mol/L urea is purchased from Beckman Coulter, Inc. A single-stranded nucleic acid having at least 90% purity evaluated by CE is obtained for use in an experiment mentioned below. Compound identity is verified by matrix-assisted laser desorption/ionization time of flight (MALDI-TOF) mass spectrometry using Voyager DE(TM) Biospectometry workstation (Applied Biosystems, Inc., Foster City, Calif.) according to manufacturer's recommended protocol. The relative molecular mass of the single-stranded nucleic acid can be obtained within 0.2% of a predicted molecular mass.

The single-stranded nucleic acid is resuspended at a concentration of 100 (µmol/L in a buffer solution consisting of 100 mmol/L potassium acetate and 30 mmol/L HEPES, pH 7.5. The complementary sense strand and the antisense strand are mixed in equimolar amounts to obtain a final solution of 50 (µmol/L double-stranded nucleic acid. The sample is heated to 95°C for 5 minutes and cooled to room temperature before use. The double-stranded nucleic acid is preserved at -20°C. The single-stranded nucleic acid is freeze-dried or stored at -80°C in nuclease-free water.

A double-stranded nucleic acid comprising a sequence selected from the group consisting of antisense strands described in Tables M1-1 to M1-3, R-3, R-4 and 1-1 to 1-13, a double-stranded nucleic acid comprising a sequence selected from the group consisting of sense strands described in Tables M1-1 to M1-3, R-1, R-2 and 1-1 to 1-13 mentioned later, or double-stranded nucleic acid comprising the sequences of a pair of sense strand/antisense strand selected from the group consisting of sense strands/antisense strands described in Tables M1-1 to M1-3, R-1 to R-4 and 1-1 to 1-13 can be used as the double-stranded nucleic acid according to the present invention consisting of a sense strand and an antisense strand and comprising a duplex region of at least 11 base pairs, wherein an oligonucleotide strand having a chain length of 11 to 30 nucleotides in the antisense strand is complementary to a target APCS mRNA sequence selected from a group described in Tables 1-1 to 1-13 mentioned later.

A specific example of the double-stranded nucleic acid constituting the nucleic acid conjugate used in the present invention is a double-stranded nucleic acid consisting of any sense strand and antisense strand in Tables M1-1 to M1-3, R-1 to R-4 and 1-1 to 1-13. In Tables M1-1 to M1-3 and R-1 to R-4, N(M) represents 2'-O-methyl-modified RNA; N(F) represents 2'-fluorine-modified RNA; and ^ represents phosphorothioate. The 5' terminal nucleotides of antisense strand sequences described in Tables 1-1 to 1-13, M1-1 to M1-3, R-3 and R-4 may or may not be phosphorylated and are preferably phosphorylated.

The double-stranded nucleic acid comprising the sequences of any sense strand/antisense strand described in Tables 1-1 to 1-13 attains a relative APCS expression level of preferably 0.50 or less, more preferably 0.30 or less, further preferably 0.20 or less, most preferably 0.10 or less, as compared with conditions that are not supplemented with the double-stranded nucleic acid in the measurement of knockdown activity when added at 1 nM.

The oligonucleotide according to the present invention can be any double-stranded nucleic acid comprising a 2'-modified nucleotide and is preferably any of those described in Tables M1 to M3. The double-stranded nucleic acid attains a relative APCS expression level of more preferably 0.10 or less as compared with conditions that are not supplemented with the double-stranded nucleic acid in the measurement of knockdown activity when added at 1 nM.

A nucleic acid conjugate of any of the preferred double-stranded nucleic acids combined with a ligand linker known in the art is also included in the present invention. Examples of the ligand linker known in the art include those disclosed in International Publication Nos. WO 2009/073809 and WO 2013/075035.

The nucleic acid conjugate of the present invention is preferably any nucleic acid conjugate described in Table S1 (compounds 1-2 to 43-2) and is more preferably a nucleic acid conjugate that attains a relative APCS expression level of more preferably 0.50 or less as compared with a negative control that is not supplemented with the nucleic acid conjugate in the measurement of knockdown activity when added at a final concentration of 3 nM. Specific examples of the nucleic acid conjugate include compounds 1-2, 5-2, 9-2, 10-2, 15-2, 20-2, 33-2 and 35-2.

A method for producing the nucleic acid conjugate of the present invention will be described. In the production methods given below, if defined groups react under conditions of the production methods or are unsuitable for carrying out the production methods, the compounds of interest can be produced by use of methods for introducing and removing protective groups commonly used in organic synthetic chemistry [e.g., methods described in Protective Groups in Organic Synthesis, third edition, T.W. Greene, John Wiley & Sons Inc. (1999)] or the like. If necessary, the order of reaction steps including substituent introduction and the like may be changed.

The nucleic acid conjugate represented by formula 1 can also be synthesized by solid-phase synthesis.

The nucleic acid conjugate represented by formula 1 can be synthesized with reference to a method for synthesizing a linker structure known in the art for nucleic acid conjugates.

The synthesis of a Ll-benzene ring unit having linker S1 or a L2-benzene ring unit having linker S2 in the nucleic acid conjugate represented by formula 1 will be described by taking the nucleic acid conjugate represented by formula 2 as an example.

The Ll-benzene ring unit and the L2-benzene ring unit in the nucleic acid conjugate represented by formula 2 has linkages by P1, P2, P3, P4, P5, and P6, and T1 and T2.

The -CO-, -NH-, -O-, -S-, -O-CO-, -S-CO-, -NH-CO-, - CO-O-, -CO-S- or -CO-NH- bond represented by P1, P2, P3, P4, P5, and P6, and T1 and T2 can be appropriately synthesized by selecting a starting material suitable for forming the structure represented by formula 2 with reference to methods for binding reaction described in, for example, The Fourth Series of Experimental Chemistry 19, "Synthesis of Organic Compound I", Maruzen Co., Ltd. (1992) and The Fourth Series of Experimental Chemistry 20, "Synthesis of Organic Compound II", Maruzen Co., Ltd. (1992).

A substructure of the Ll-benzene ring unit can be produced by sequentially bonding a compound having Q1 as a substructure and a compound having B1 as a substructure to the benzene ring.

The Ll-benzene ring unit structure can be produced by separately synthesizing a compound having L1 and Q2 as a substructure, and bonding the compound having L1 and Q2 as a substructure to a compound having a substructure of a Ll-benzene ring unit having the benzene ring, Q1 and B1 as a substructure.

Likewise, a substructure of the L2-benzene ring unit can be produced by sequentially bonding a compound having Q3 as a substructure and a compound having B2 as a substructure to the benzene ring.

The L2-benzene ring unit structure can be produced by separately synthesizing a compound having L2 and Q4 as a substructure, and bonding the compound having L2 and Q4 as a substructure to a compound having a substructure of a L2-benzene ring unit having the benzene ring, Q3 and B2 as a substructure.

Examples of the compound having Q1 as a substructure and the compound having Q3 as a substructure include compounds having a hydroxy group, a carboxyl group, an amino group, and/or a thiol group at both ends of alkylene having 1 to 10 carbon atoms or -(CH₂CH₂O)ₙ-CH₂CH₂-.

Examples of the compound having B1 as a substructure and the compound having B2 as a substructure include compounds having any structure represented by the following formula 2-1 and having a hydroxy group, a carboxyl group, an amino group, or a thiol group at each of the terminal dots in each structure:

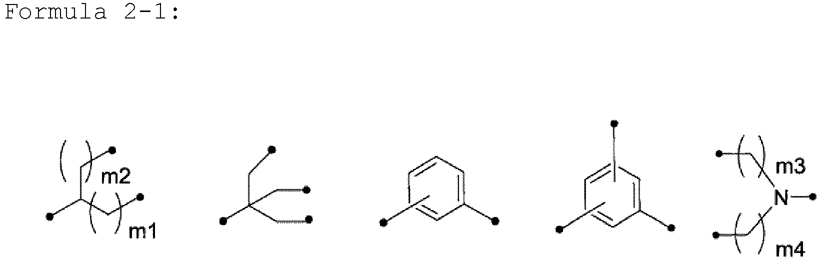

Specific examples of the compound having B1 as a substructure and the compound having B2 as a substructure include glycol, glutamic acid, aspartic acid, lysine, Tris, iminodiacetic acid, and 2-amino-1,3-propanediol. Glutamic acid, aspartic acid, lysine, or iminodiacetic acid are preferred. Specifically, each of B1 and B2 is preferably any of the following structures:

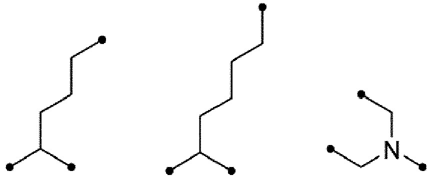

The Ll-benzene ring unit structure may be produced by synthesizing a compound having L1, Q2 and B1 as a substructure and then bonding this compound to a compound having Q1 and the benzene ring.

The L2-benzene ring unit structure may be produced by synthesizing a compound having L2, Q4 and B2 as a substructure and then bonding this compound to a compound having Q3 and the benzene ring.

In the present invention, the substructure [L1-T1-(Q2-P3)_{q2}-]ₚ₁-B1-(P2-Q1)_{q1}-P1- and the substructure [L2-T2-(Q3-P6)_{q4}-]ₚ₂-B2-(P5-Q3)_{q3}-P2- may be the same or different and are preferably the same.

Examples of the unit corresponding to L1-T1-Q2 in the sugar ligand include L3-T1-Q2-COOH and L3-T1-(Q2-P3)_{q2-1}-Q2-NH₂. Specific examples thereof include L3-O-alkylene having 1 to 12 carbon atoms-COOH and L3-alkylene having 1 to 12 carbon atoms-CO-NH-alkylene having 2 to 12 carbon atoms-NH₂.

L3 is not particularly limited as long as L3 is a sugar ligand derivative that is converted to L1 by deprotection. The substituent on the sugar ligand is not particularly limited as long as the substituent is routinely used in the field of carbohydrate chemistry. An Ac group is preferred.

Specifically, the Ll-benzene ring unit having linker S1 or the L2-benzene ring unit having linker S2 can be synthesized by appropriately increasing or decreasing the number of carbon atoms of an alkylene chain, and using a compound with a terminal amino group or a terminal carboxyl group converted to a group capable of forming a -CO-, -NH-, -O-, -S-, -O-CO-, -S-CO-, -NH-CO-, -CO-O-, - CO-S- or -CO-NH- bond, with reference to a method described in Examples. Mannose or N-acetylgalactosamine is taken as an example of sugar ligand L1 in Examples. However, sugar ligand L1 may be changed to other sugar ligands for the practice.

The synthesis of an X-benzene ring unit having linker S3 in the nucleic acid conjugate represented by formula 1 will be described by taking the nucleic acid conjugate represented by formula 12 as an example.

The X-benzene ring unit in the nucleic acid conjugate represented by formula 12 has bonds represented by P7 and P8 in addition to the bond of the oligonucleotide.

The -CO-, -NH-, -O-, -S-, -O-CO-, -S-CO-, -NH-CO-, - CO-O-, -CO-S- or -CO-NH- bond represented by P7 and P8 can be appropriately synthesized by selecting a starting material suitable for forming the structure represented by formula 12 with reference to methods for binding reaction described in, for example, The Fourth Series of Experimental Chemistry 19, "Synthesis of Organic Compound I", Maruzen Co., Ltd. (1992) and The Fourth Series of Experimental Chemistry 20, "Synthesis of Organic Compound II", Maruzen Co., Ltd. (1992).

A substructure of the X-benzene ring unit can be produced by sequentially bonding a compound having Q5 as a substructure and a compound having B3 as a substructure to the benzene ring.

The X-benzene ring unit structure can be produced by separately synthesizing a compound having X and Q7 as a substructure or a compound having X and Q6 as a substructure, and bonding the compound having X and Q7 as a substructure or the compound having X and Q6 as a substructure to a compound having a substructure of a X-benzene ring unit having the benzene ring and Q5 as a substructure to construct the B3 moiety.

Specifically, the case of having an azide group at the end of the compound having a substructure of an X-benzene ring unit having the benzene ring and Q5 as a substructure will be taken as an example. The X-benzene ring unit structure can be produced by reacting an oligonucleotide allowed to have a terminal binding functional group as disclosed in Examples so that a triazole ring is formed by cycloaddition to construct the B3 moiety.

Examples of the compound having Q5 as a substructure, the compound having Q6 as a substructure, and the compound having Q7 as a substructure include compounds having a hydroxy group, a carboxyl group, an amino group, and/or a thiol group at both ends of alkylene having 1 to 10 carbon atoms or - (CH₂CH₂O)ₙ₈-CH₂CH₂-.

The Ll-benzene ring unit structure, the L2-benzene ring unit structure, and the X-benzene ring unit structure can be sequentially produced. It is preferred to synthesize the Ll-benzene ring unit structure and the L2-benzene ring unit structure and then bond the X-benzene ring unit structure thereto. Particularly, it is preferred to introduce X having the oligonucleotide moiety into the compound near the final step of sugar ligand conjugate synthesis.

In the present invention, a compound represented by the following formulas 8 to 10 is obtained as an intermediate of synthesis: 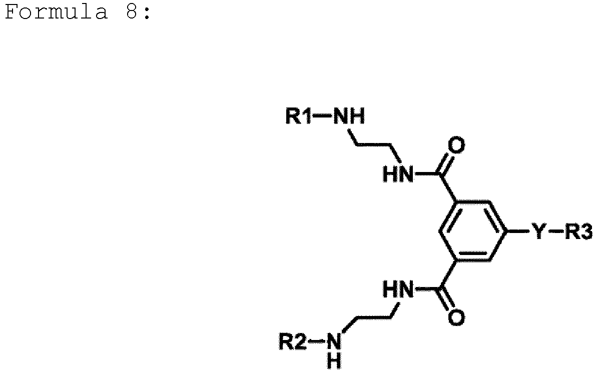 wherein
R1 and R2 are each independently a hydrogen atom, a t-butoxycarbonyl group (Boc group), a benzyloxycarbonyl group (Z group), a 9-fluorenylmethyloxycarbonyl group (Fmoc group), -CO-R4, or -CO-B4-[(P9-Q8)_{q7}-T3-L3]ₚ₃,
P9 and T3 are each independently absent, or -CO-, - NH-, -O-, -S-, -O-CO-, -S-CO-, -NH-CO-, -CO-O-, -CO-S- or -CO-NH-,
Q8 is absent, or substituted or unsubstituted alkylene having 1 to 12 carbon atoms or - (CH₂CH₂O)ₙ₁-CH₂CH₂- wherein n1 is an integer of 0 to 99,
B4 is a bond, or any structure represented by the following formula 8-1, wherein each of the terminal dots in each structure is a binding site to a carbonyl group or P9, and m7, m8, m9 and m10 are each independently an integer of 0 to 10:

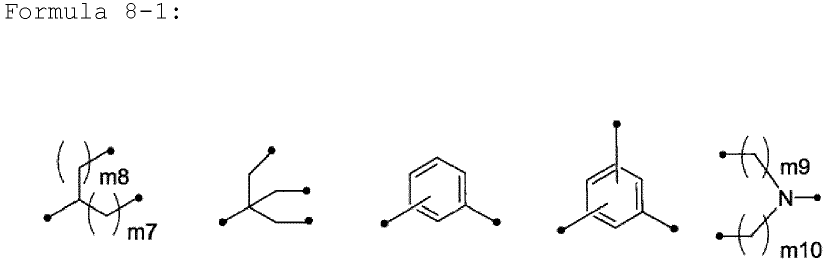

p3 is an integer of 1, 2 or 3,
q7 is an integer of 0 to 10,
L3 is a sugar ligand,
Y is -O-(CH₂)ₘ₁₁-NH-or -NH-CO-(CH₂)ₘ₁₂-NH- wherein m11 and m12 are each independently an integer of 1 to 10,
R3 is a hydrogen atom, a t-butoxycarbonyl group, a benzyloxycarbonyl group, a 9-fluorenylmethyloxycarbonyl group, -CO-R4, -CO- (CH₂CH₂O)ₙ₂-CH₂CH₂-N₃, or -CO-Q9-B5-(Q10-P10)_{q8}-X1 wherein n2 is an integer of 0 to 99,
P10 is absent, or -CO-, -NH-, -O-, -S-, -O-CO-, -S-CO-, -NH-CO-, -CO-O-, -CO-S- or -CO-NH-,
Q9 and Q10 are each independently absent, or substituted or unsubstituted alkylene having 1 to 12 carbon atoms or - (CH₂CH₂O)ₙ₃-CH₂CH₂- wherein n3 is an integer of 0 to 99,
B5 is any structure represented by the following formula 8-2, wherein the broken lines respectively mean bonds to Q9 and Q10: 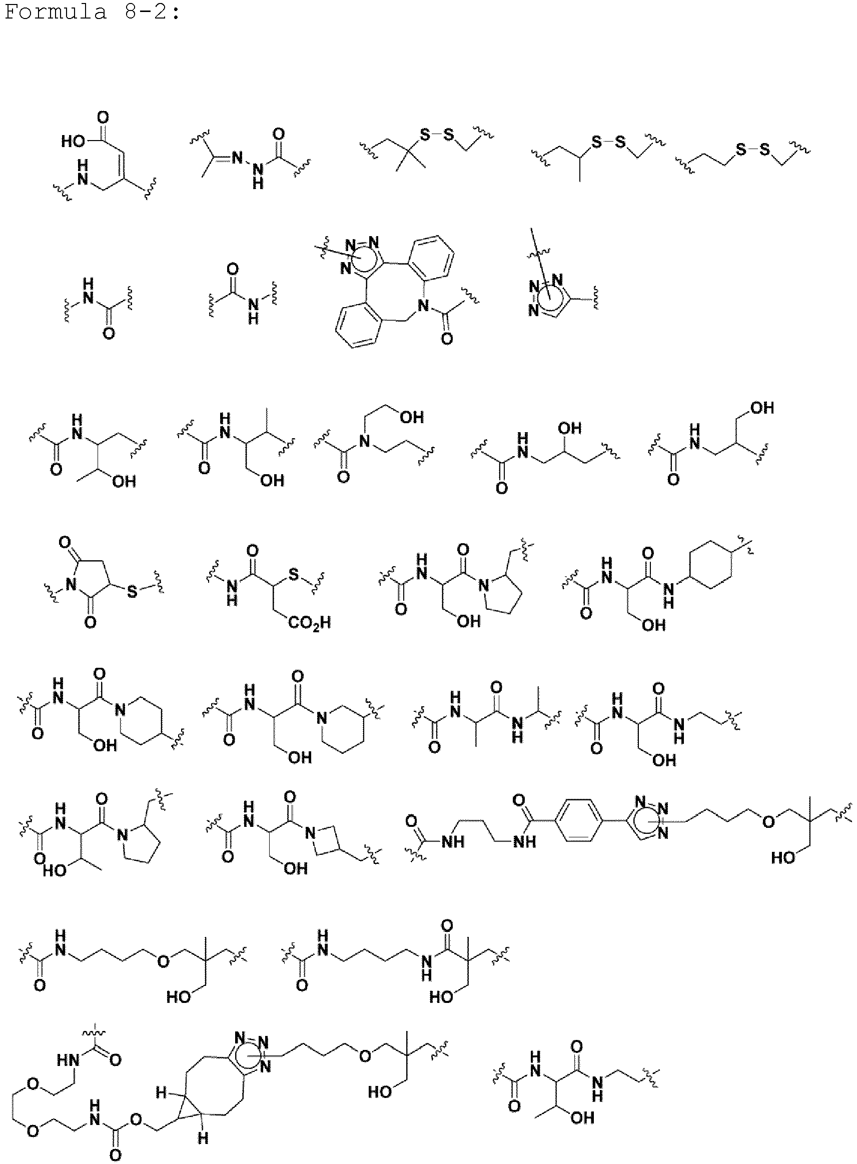 wherein substitution in a group having a triazole ring occurs at any of nitrogen atoms at positions 1 and 3 of the triazole ring,
   q8 is an integer of 0 to 10,
   X1 is a hydrogen atom or a solid-phase support, and
   R4 is an alkyl group having 2 to 10 carbon atoms substituted with 1 or 2 substituents selected from the group consisting of an amino group unsubstituted or substituted with a t-butoxycarbonyl group, a benzyloxycarbonyl group or a 9-fluorenylmethyloxycarbonyl group, a carboxy group, a maleimide group, and an aralkyloxycarbonyl group. 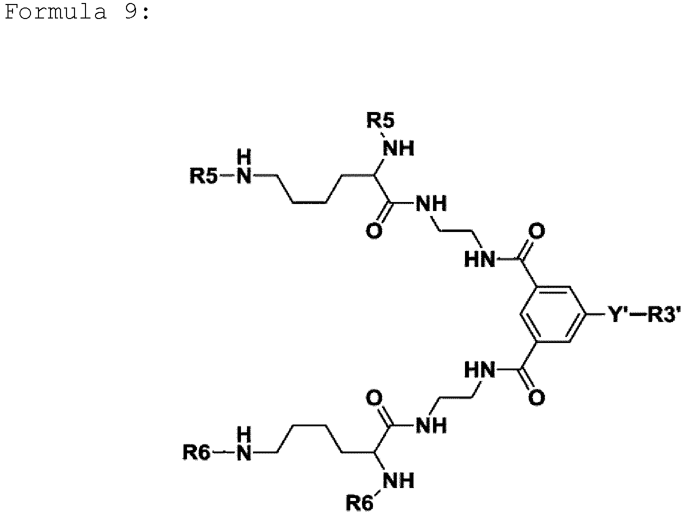
wherein
R5 and R6 are each independently a hydrogen atom, a t-butoxycarbonyl group, a benzyloxycarbonyl group, a 9-fluorenylmethyloxycarbonyl group, -CO-R4', or -CO-Q11-(P11-Q11')_{q9}-T4-L4,
P11 and T4 are each independently absent, or -CO-, - NH-, -O-, -S-, -O-CO-, -S-CO-, -NH-CO-, -CO-O-, -CO-S- or -CO-NH-,
each of Q11 and Q11' is absent, or substituted or unsubstituted alkylene having 1 to 12 carbon atoms or - (CH₂CH₂O)ₙ₄-CH₂CH₂- wherein n4 is an integer of 0 to 99,
q9 is an integer of 0 to 10,
L4 is a sugar ligand,
Y' is -O-(CH₂)_{m11'}-NH- or -NH-CO-(CH₂)_{m12'}-NH- wherein m11' and m12' are each independently an integer of 1 to 10,
R3' is a hydrogen atom, a t-butoxycarbonyl group, a benzyloxycarbonyl group, a 9-fluorenylmethyloxycarbonyl group, -CO-R4', -CO- (CH₂CH₂O)ₙ₂-CH₂CH₂-N₃, or -CO-Q9'-B5'-(Q10'-P10')_{q8'}-X1' wherein n2' is an integer of 0 to 99,
P10' is absent, or -CO-, -NH-, -O-, -S-, -O-CO-, -S-CO-, -NH-CO-, -CO-O-, -CO-S- or -CO-NH-,
Q9' and Q10' are each independently absent, or substituted or unsubstituted alkylene having 1 to 12 carbon atoms or - (CH₂CH₂O)_{n3'}-CH₂CH₂- wherein n3' is an integer of 0 to 99,
B5' is any structure represented by the following formula 9-1, wherein the broken lines respectively mean bonds to Q9' and Q10': 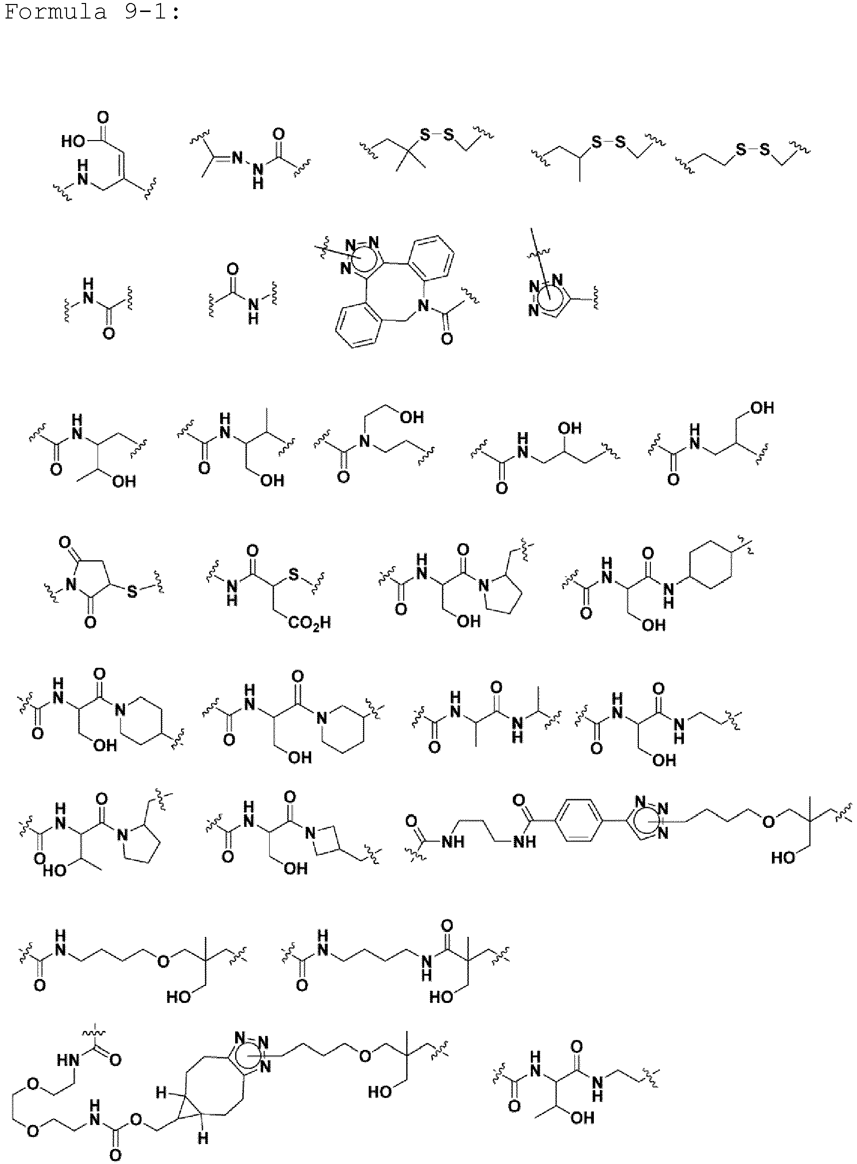 wherein substitution in a group having a triazole ring occurs at any of nitrogen atoms at positions 1 and 3 of the triazole ring,
   q8' is an integer of 0 to 10,
   X1' is a hydrogen atom or a solid-phase support, and
   R4' is an alkyl group having 2 to 10 carbon atoms substituted with 1 or 2 substituents selected from the group consisting of an amino group unsubstituted or substituted with a t-butoxycarbonyl group, a benzyloxycarbonyl group or a 9-fluorenylmethyloxycarbonyl group, a carboxy group, a maleimide group, and an aralkyloxycarbonyl group. 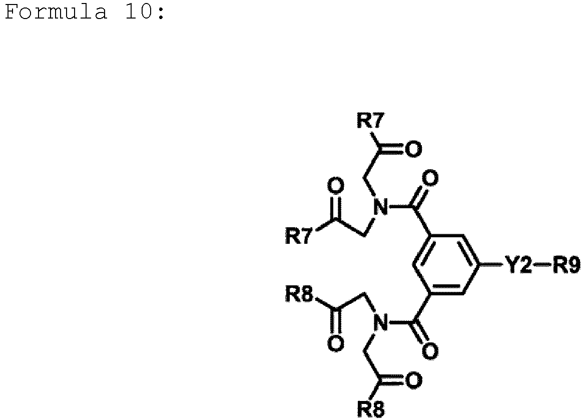
wherein
R7 and R8 are each independently a hydroxy group, a t-butoxy group, a benzyloxy group, -NH-R10, or -NH-Q12-(P12-Q12')_{q10}-T4-L4,
P12 and T4 are each independently absent, or -CO-, - NH-, -O-, -S-, -O-CO-, -S-CO-, -NH-CO-, -CO-O-, -CO-S- or -CO-NH-,
each of Q12 and Q12' is absent, or substituted or unsubstituted alkylene having 1 to 12 carbon atoms or - (CH₂CH₂O)n2-CH₂CH₂- wherein n2 is an integer of 0 to 99,
L4 is a sugar ligand,
Y2 is -O-(CH₂)ₘ₉-NH- or -NH-CO-(CH₂)ₘ₁₀-NH- wherein m9 and m10 are each independently an integer of 1 to 10,
q10 is an integer of 0 to 10,
R9 is a hydrogen atom, a t-butoxycarbonyl group, a benzyloxycarbonyl group, a 9-fluorenylmethyloxycarbonyl group, -CO-R10, -CO- (CH₂CH₂O) ₙ₆-CH₂CH₂-N₃, or -CO-Q13-B6-(Q14-P13)_{q11}-X2 wherein n6 is an integer of 0 to 99,
P13 is absent, or -CO-, -NH-, -O-, -S-, -O-CO-, -S-CO-, -NH-CO-, -CO-O-, -CO-S- or -CO-NH-,
Q13 and Q14 are each independently absent, or substituted or unsubstituted alkylene having 1 to 12 carbon atoms or - (CH₂CH₂O) ₙ₇-CH₂CH₂- wherein n7 is an integer of 0 to 99,
B6 is any structure represented by the following formula 10-1, wherein the broken lines respectively mean bonds to Q13 and Q14: 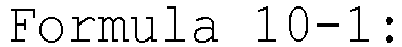 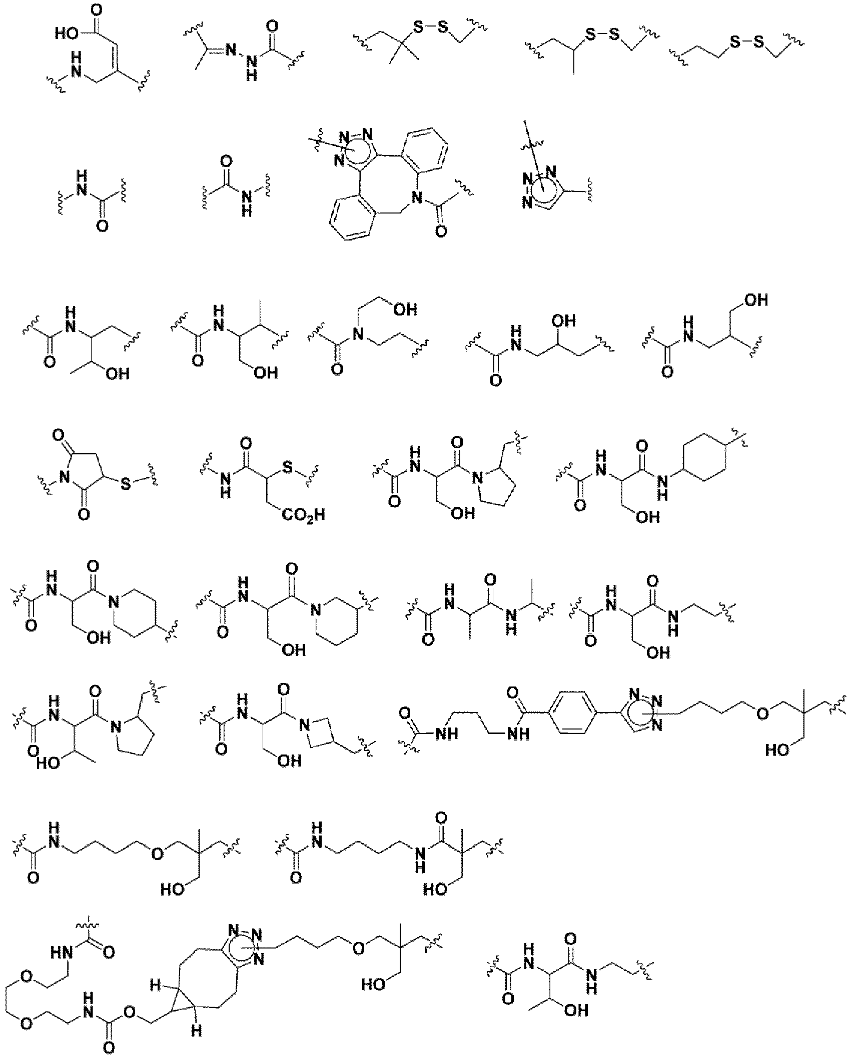
wherein substitution in a group having a triazole ring occurs at any of nitrogen atoms at positions 1 and 3 of the triazole ring,
q11 is an integer of 0 to 10,
X2 is a hydrogen atom or a solid-phase support, and
R10 is an alkyl group having 2 to 10 carbon atoms substituted with 1 or 2 substituents selected from the group consisting of an amino group unsubstituted or substituted with a t-butoxycarbonyl group, a benzyloxycarbonyl group or a 9-fluorenylmethyloxycarbonyl group, a carboxy group, a maleimide group, and an aralkyloxycarbonyl group.

Hereinafter, exemplary production methods will be given in relation to the present invention. In the description about production methods 1 to 17 given below, the same symbols as those representing groups in the compounds represented by formulas 1 to 12 in the nucleic acid derivative, etc. of the present invention may be used. These symbols in production methods 1 to 17 should be understood separately from those in the compounds represented by formulas 1 to 12. The present invention should not be restrictively interpreted by the description of the groups about production methods 1 to 12. For the nucleic acid derivative according to the present invention, X representing the oligonucleotide is described as -O-X in production methods 1 to 17.

### Production method 1

For the nucleic acid derivative according to the present invention, the production method can be taken as an example of a method for producing a compound having a substructure represented by formula (I'): 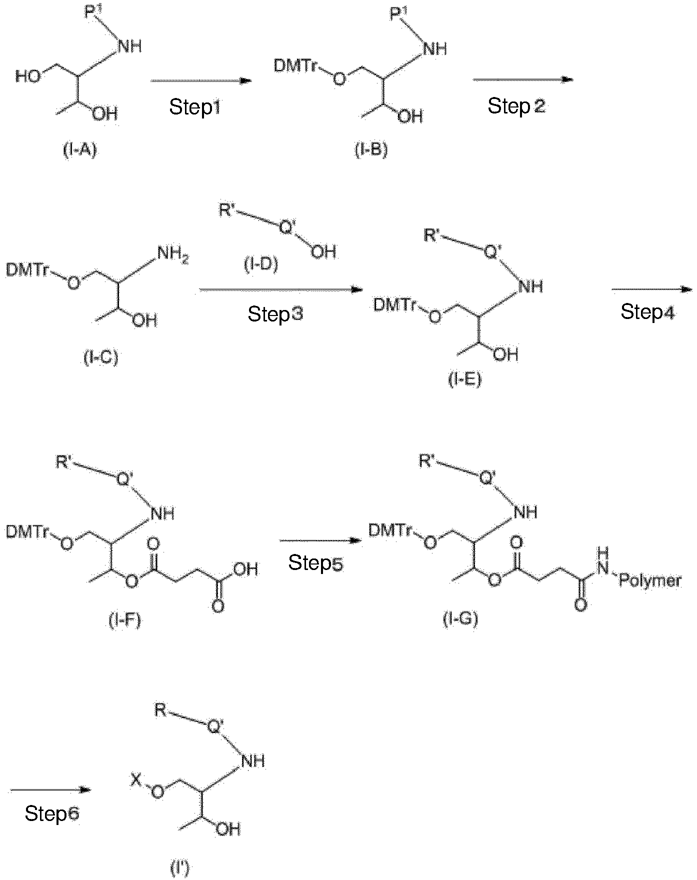 wherein P1 is a base-deprotectable protective group such as Fmoc, DMTr represents a p,p'-dimethoxytrityl group, R represents a sugar ligand-tether unit, R' represents a group in which each hydroxy group of the sugar ligand in R is protected with a base-deprotectable protective group such as an acetyl group, Polymer represents a solid-phase support, and Q' is -CO-.

### Step 1

Compound (I-B) can be produced by reacting compound (I-A) with p,p'-dimethoxytrityl chloride at a temperature between 0°C and 100°C for 5 minutes to 100 hours in a solvent such as pyridine, if necessary, in the presence of a cosolvent.

Examples of the cosolvent include methanol, ethanol, dichloromethane, chloroform, 1,2-dichloroethane, toluene, ethyl acetate, acetonitrile, diethyl ether, tetrahydrofuran, 1,2-dimethoxyethane, dioxane, N,N-dimethylformamide (DMF), N,N-dimethylacetamide, N-methylpyrrolidone, pyridine, and water. These cosolvents may be used alone or as a mixture.

### Step 2

Compound (I-C) can be produced by reacting compound (I-B) at a temperature between room temperature and 200°C for 5 minutes to 100 hours in the presence of 1 to 1000 equivalents of a secondary amine without a solvent or in a solvent.

Examples of the solvent include methanol, ethanol, dichloromethane, chloroform, 1,2-dichloroethane, toluene, ethyl acetate, acetonitrile, diethyl ether, tetrahydrofuran, 1,2-dimethoxyethane, dioxane, N,N-dimethylformamide (DMF), N,N-dimethylacetamide, N-methylpyrrolidone, pyridine, and water. These solvents may be used alone or as a mixture.

Examples of the secondary amine include diethylamine and piperidine.

### Step 3

Compound (1-E) can be produced by reacting compound (I-C) with compound (I-D) at a temperature between room temperature and 200°C for 5 minutes to 100 hours in the presence of 1 to 30 equivalents of a base, a condensing agent and, if necessary, 0.01 to 30 equivalents of an additive without a solvent or in a solvent.

Examples of the solvent include those listed in step 2.

Examples of the base include cesium carbonate, potassium carbonate, potassium hydroxide, sodium hydroxide, sodium methoxide, potassium tert-butoxide, triethylamine, diisopropylethylamine, N-methylmorpholine, pyridine, 1,8-diazabicyclo[5.4.0]-7-undecene (DBU), and N,N-dimethyl-4-aminopyridine (DMAP).

Examples of the condensing agent include 1,3-dicyclohexanecarbodiimide (DCC), 1-ethyl-3-(3-dimethylaminopropyl)carbodiimide hydrochloride (EDC), carbonyldiimidazole, benzotriazol-1-yloxytris(dimethylamino)phosphonium hexafluorophosphate, (benzotriazol-1-yloxy)tripyrrolidinophosphonium hexafluorophosphate, O-(7-azabenzotriazol-1-yl)-N,N,N',N'-tetramethyluronium hexafluorophosphate (HATU), O-(benzotriazol-1-yl)-N,N,N',N'-tetramethyluronium hexafluorophosphate (HBTU), and 2-chloro-1-methylpyridinium iodide.

Examples of the additive include 1-hydroxybenzotriazole (HOBt) and 4-dimethylaminopyridine (DMAP).

Compound (I-D) can be obtained by a method known in the art (see, for example, Journal of American Chemical Society, 136, 16958, (2014) or a method equivalent thereto.

### Step 4

Compound (I-F) can be produced by reacting compound (I-E) with succinic anhydride at a temperature between room temperature and 200°C for 5 minutes to 100 hours in the presence of 1 to 30 equivalents of a base in a solvent.

Examples of the solvent include those listed in step 2.

Examples of the base include those listed in step 3.

### Step 5

Compound (I-G) can be produced by reacting compound (I-F) with a terminally aminated solid-phase support at a temperature between room temperature and 200°C for 5 minutes to 100 hours in the presence of 1 to 30 equivalents of a base, a condensing agent and, if necessary, 0.01 to 30 equivalents of an additive without a solvent or in a solvent, and then reacting the resultant with a solution of acetic anhydride in pyridine at a temperature between room temperature and 200°C for 5 minutes to 100 hours.

Examples of the solvent include those listed in step 2.

Examples of the base, the condensing agent and the additive include those respectively listed in step 3.

Examples of the aminated solid-phase support include long-chain alkylamine controlled pore glass (LCAA-CPG). Such an aminated solid-phase support can be obtained as a commercially available product.

### Step 6

The nucleic acid conjugate having the sugar ligand-tether-brancher unit represented by formula (I') can be produced by elongating a corresponding nucleotide strand by a chemical oligonucleotide synthesis method known in the art using compound (I-G), followed by dissociation from the solid phase, deprotection of the protective group and purification.

Examples of the chemical oligonucleotide synthesis method known in the art can include a phosphoramidite method, a phosphorothioate method, a phosphotriester method, and a CEM method (see Nucleic Acids Research, 35, 3287 (2007)). The nucleotide strand can be synthesized using, for example, ABI3900 high-throughput nucleic acid synthesizer (manufactured by Applied Biosystems, Inc.).

The dissociation from the solid phase and the deprotection can be performed by treatment with a base at a temperature between -80°C and 200°C for 10 seconds to 72 hours in a solvent or without a solvent after the chemical oligonucleotide synthesis.

Examples of the base include ammonia, methylamine, dimethylamine, ethylamine, diethylamine, isopropylamine, diisopropylamine, piperidine, triethylamine, ethylenediamine, 1,8-diazabicyclo[5.4.0]-7-undecene (DBU), and potassium carbonate.

Examples of the solvent include water, methanol, ethanol, and THF.

The oligonucleotide can be purified using a C18 reverse-phase column or an anion-exchange column, preferably these two approaches in combination. The purity of the nucleic acid conjugate thus purified is desirably 90% or higher, preferably 95% or higher.

In step 3 described above, if necessary, compound (I-D) may be divided into two units and condensed with compound (I-C) at two separate stages. Specifically, when R-Q' is, for example, R-NH-CO-Q4'-CO- (Q4' is substituted or unsubstituted alkylene having 1 to 12 carbon atoms), in step 3, compound (I-C) and CH₃CH₂-O-CO-Q4'-CO-OH (Q4' is as defined above) are condensed in the same way as in step 3, and ethyl ester of the obtained compound can be hydrolyzed with a base such as lithium hydroxide in a solvent such as ethanol or water, followed by further condensation with R'-NH₂ (R' is as defined above) to obtain the compound of interest. CH₃CH₂-O-CO-Q4'-CO-OH (Q4' is as defined above) and R'-NH₂ (R' is as defined above) can be obtained by a method known in the art (see, for example, Journal of American Chemical Society, 136, 16958 (2014)) or a method equivalent thereto. In this context, the substituent and the alkylene moiety in the substituted or unsubstituted alkylene having 1 to 12 carbon atoms, represented by Q4' are as defined above.

Although the case where Q is -CO- is taken as an example above, a compound in which Q is not -CO- can also be prepared according to the same method as above or a method known in the art, or a combination thereof by appropriately changing the structure of Q and appropriately changing the reaction conditions.

### Production method 2

For the nucleic acid derivative according to the present invention, the production method can be taken as an example of a method for producing a compound having a substructure represented by formula (II'): 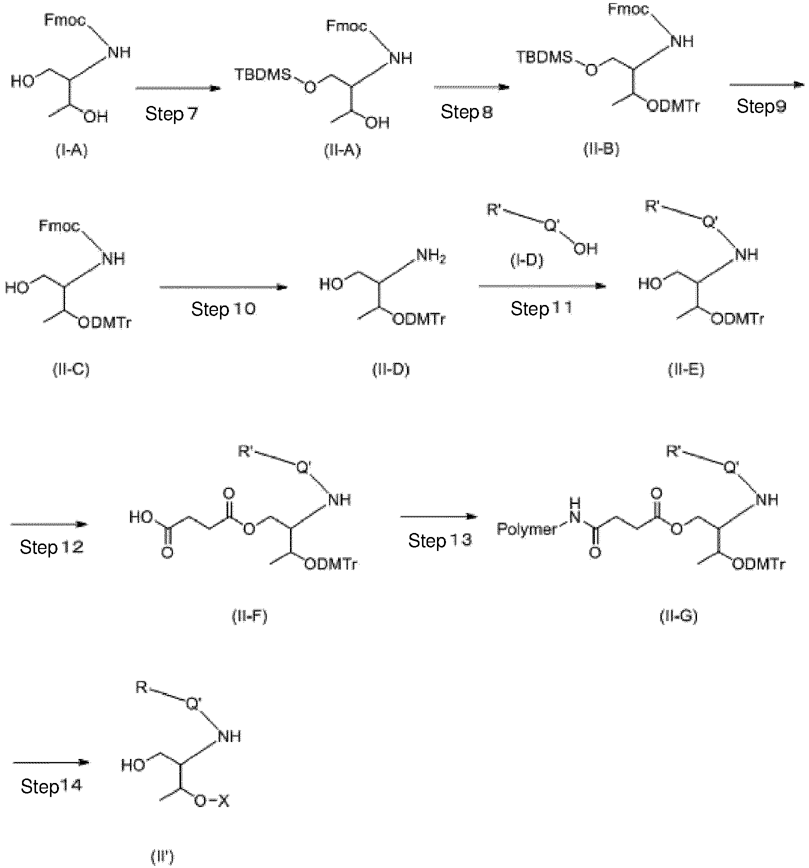 wherein DMTr, R, R', X, Q', and Polymer are as defined above, TBDMS represents a t-butyldimethylsilyl group, and Fmoc represents a 9-fluorenylmethyloxycarbonyl group.

### Step 7

Compound (II-A) can be produced by reacting compound (I-A) with t-butyldimethylsilyl chloride and dimethylaminopyridine at a temperature between 0°C and 100°C for 5 minutes to 100 hours in a solvent such as N,N-dimethylformamide (DMF), preferably in the presence of 2 equivalents of a base.

Examples of the base include those listed in step 3 of production method 1.

### Step 8

Compound (II-B) can be produced under the same conditions as in step 1 of production method 1 using compound (II-A).

### Step 9

Compound (II-C) can be produced by reacting compound (II-B) with n-tetrabutylammonium fluoride (TBAF) at a temperature between room temperature and 200°C for 5 minutes to 100 hours in a solvent.

Examples of the solvent include those listed in step 2.

### Step 10

Compound (II-D) can be produced under the same conditions as in step 2 of production method 1 using compound (II-C).

### Step 11

Compound (II-E) can be produced under the same conditions as in step 3 of production method 1 using compound (II-D) and compound (I-D).

### Steps 12 to 14

Compound (II') can be produced under the same conditions as in steps 4 to 6 of production method 1 using compound (II-E).

In step 11 described above, if necessary, compound (I-D) may be divided into two units and condensed with compound (II-C) at two separate stages. Specifically, when R-Q' is, for example, R-NH-CO-Q4'-CO- (Q4' is substituted or unsubstituted alkylene having 1 to 12 carbon atoms), in step 11, compound (II-C) and CH₃CH₂-O-CO-Q4'-CO-OH (Q4' is as defined above) are condensed in the same way as in step 11, and ethyl ester of the obtained compound can be hydrolyzed with a base such as lithium hydroxide in a solvent such as ethanol or water, followed by further condensation with R'-NH₂ (R' is as defined above) to obtain the compound of interest. CH₃CH₂-O-CO-Q4'-CO-OH (Q4' is as defined above) and R'-NH₂ (R' is as defined above) can be obtained by a method known in the art (see, for example, Journal of American Chemical Society, 136, 16958 (2014)) or a method equivalent thereto.

Although the case where Q is -CO- is taken as an example above, a compound in which Q is not -CO- can also be prepared according to the same method as above or a method known in the art, or a combination thereof by appropriately changing the structure of Q and appropriately changing the reaction conditions.

### Production method 3

For the nucleic acid derivative according to the present invention, the production method can be taken as an example of a method for producing a compound having a substructure represented by formula (III'): 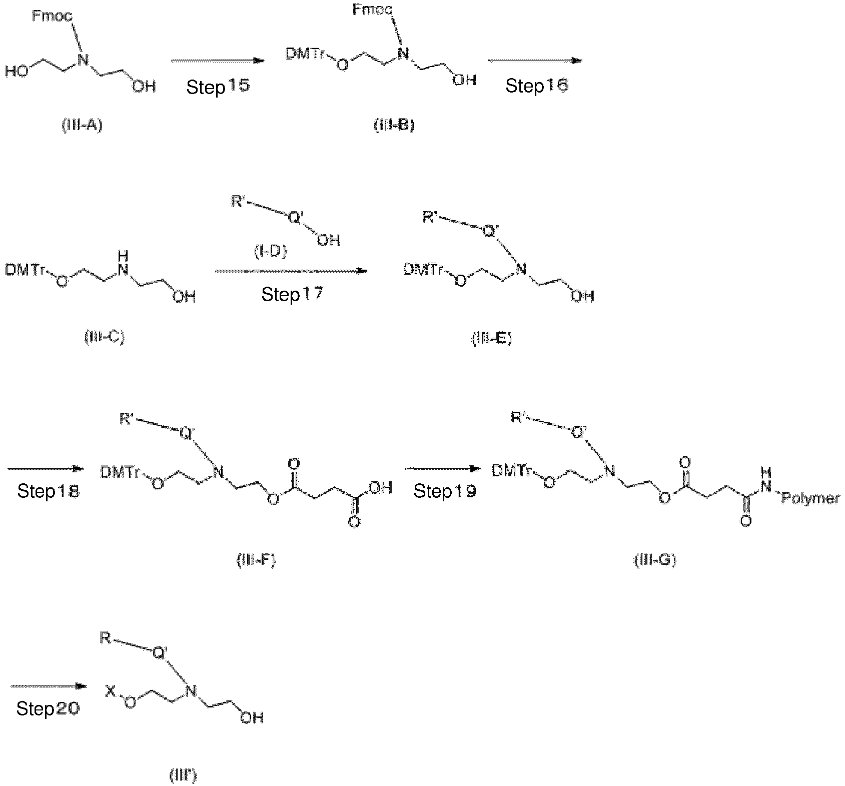 wherein DMTr, Fmoc, R, R', Q', X and Polymer are as defined above.

Compound (III') can be produced under the same conditions as in steps 1 to 6 of production method 1 using compound (III-A). Compound (III-A) can be obtained as a commercially available product.

### Step 15

Compound (III-B) can be produced under the same conditions as in step 1 of production method 1 using compound (III-A).

Compound (III-A) can be purchased as a commercially available product.

### Step 16

Compound (III-C) can be produced under the same conditions as in step 2 of production method 1 using compound (III-B).

### Step 17

Compound (III-E) can be produced under the same conditions as in step 3 of production method 1 using compound (III-C).

### Steps 18 to 20

Compound (III') can be produced under the same conditions as in steps 4 to 6 of production method 1 using compound (III-E).

In step 17 described above, if necessary, compound (I-D) may be divided into two units and condensed with compound (III-C) at two separate stages. Specifically, when R-Q' is, for example, -NH-CO-Q4'-CO- (Q4' is substituted or unsubstituted alkylene having 1 to 12 carbon atoms), in step 17, compound (III-C) and CH₃CH₂-O-CO-Q4'-CO-OH (Q4' is as defined above) are condensed in the same way as in step 17, and ethyl ester of the obtained compound can be hydrolyzed with a base such as lithium hydroxide in a solvent such as ethanol or water, followed by further condensation with R'-NH₂ (R' is as defined above) to obtain the compound of interest. CH₃CH₂-O-CO-Q4'-CO-OH (Q4' is as defined above) and R'-NH₂ (R' is as defined above) can be obtained by a method known in the art (see, for example, Journal of American Chemical Society, 136, 16958 (2014)) or a method equivalent thereto.

Although the case where Q is -CO- is taken as an example above, a compound in which Q is not -CO- can also be prepared according to the same method as above or a method known in the art, or a combination thereof by appropriately changing the structure of Q and appropriately changing the reaction conditions.

### Production method 4

For the nucleic acid derivative according to the present invention, the production method can be taken as an example of a method for producing a compound having a substructure represented by formula (IV'): 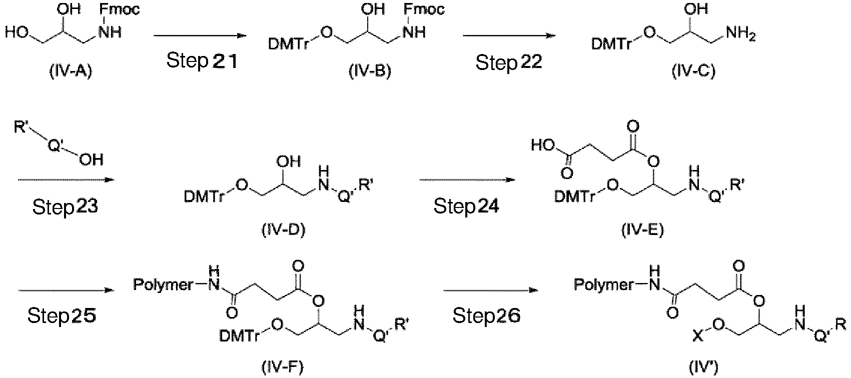 wherein DMTr, Fmoc, R, R', Q', X and Polymer are as defined above.

Compound (IV') can be produced under the same conditions as in steps 1 to 6 of production method 1 using compound (IV-A). Compound (IV-A) can be obtained as a commercially available product.

In step 23 described above, if necessary, compound (I-D) may be divided into two units and condensed with compound (IV-C) at two separate stages. Specifically, when R'-Q' is, for example, -NH-CO-Q4'-CO- (Q4' is substituted or unsubstituted alkylene having 1 to 12 carbon atoms), in step 23, compound (IV-C) and CH₃CH₂-O-CO-Q4'-CO-OH (Q4' is as defined above) are condensed in the same way as in step 23, and ethyl ester of the obtained compound can be hydrolyzed with a base such as lithium hydroxide in a solvent such as ethanol or water, followed by further condensation with R'-NH₂ (R' is as defined above) to obtain the compound of interest. CH₃CH₂-O-CO-Q4'-CO-OH (Q4' is as defined above) and R'-NH₂ (R' is as defined above) can be obtained by a method known in the art (see, for example, Journal of American Chemical Society, 136, 16958 (2014)) or a method equivalent thereto.

Although the case where Q is -CO- is taken as an example above, a compound in which Q is not -CO- can also be prepared according to the same method as above or a method known in the art, or a combination thereof by appropriately changing the structure of Q and appropriately changing the reaction conditions.

### Production method 5

For the nucleic acid derivative according to the present invention, the production method can be taken as an example of a method for producing a compound having a substructure represented by formula (V'): 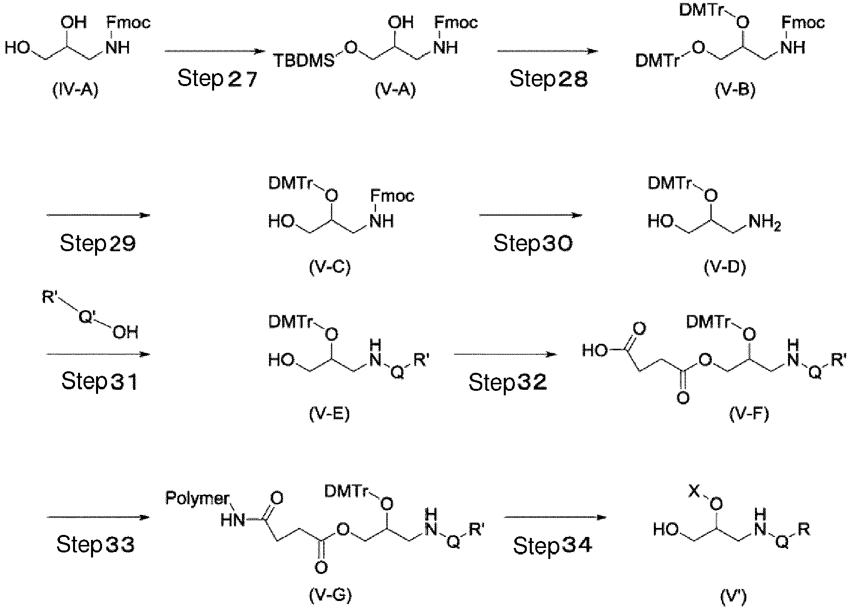 wherein DMTr, R, R', X, Q', TBDMS, Fmoc and Polymer are as defined above.

Compound (V') can be produced under the same conditions as in steps 1 to 7 of production method 2 using compound (IV-A). Compound (IV-A) can be obtained as a commercially available product.

In step 31 described above, if necessary, compound (I-D) may be divided into two units and condensed with compound (V-D) at two separate stages. Specifically, when R'-Q' is, for example, -NH-CO-Q4'-CO- (Q4' is substituted or unsubstituted alkylene having 1 to 12 carbon atoms), in step 31, compound (V-D) and CH₃CH₂-O-CO-Q4'-CO-OH (Q4' is as defined above) are condensed in the same way as in step 31, and ethyl ester of the obtained compound can be hydrolyzed with a base such as lithium hydroxide in a solvent such as ethanol or water, followed by further condensation with R'-NH₂ (R' is as defined above) to obtain the compound of interest. CH₃CH₂-O-CO-Q4'-CO-OH (Q4' is as defined above) and R'-NH₂ (R' is as defined above) can be obtained by a method known in the art (see, for example, Journal of American Chemical Society, 136, 16958 (2014)) or a method equivalent thereto.

Although the case where Q is -CO- is taken as an example above, a compound in which Q is not -CO- can also be prepared according to the same method as above or a method known in the art, or a combination thereof by appropriately changing the structure of Q-OH and appropriately changing the reaction conditions.

### Production method 6

An exemplary method for producing the nucleic acid conjugate of the present invention in which a sugar ligand-tether-brancher unit is bonded to the 5' end of the oligonucleotide will be given below. 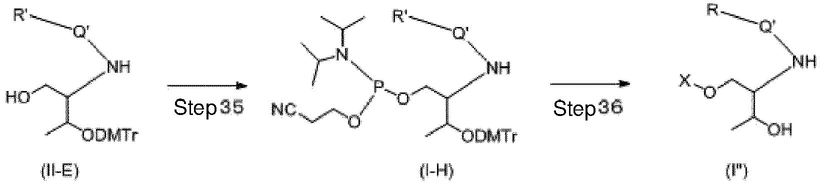 wherein R, R', Q', DMTr and X are as defined above.

### Step 35

Compound (I-H) can be produced by reacting compound (II-E) with 2-cyanoethyl-N,N,N',N'-tetraisopropylphosphodiamidite at a temperature between room temperature and 200°C for 5 minutes to 100 hours in the presence of a base and a reaction accelerator without a solvent or in a solvent.

Examples of the solvent include those listed in step 2 of production method 1.

Examples of the base include those listed in step 3 of production method 1.

Examples of the reaction accelerator include 1H-tetrazole, 4,5-dicyanoimidazole, 5-ethylthiotetrazole, and 5-benzylthiotetrazole. These reaction accelerators can be purchased as commercially available products.

### Step 36

Compound (I'') can be produced by elongating an oligonucleotide strand and finally modifying the 5' end of the oligonucleotide with a sugar ligand-tether-brancher unit using compound (I-H), followed by dissociation from the solid phase, deprotection of the protective group and purification. In this context, the dissociation from the solid phase, the deprotection of the protective group and the purification can each be performed in the same way as in step 7 of production method 1.

### Production method 7

An exemplary method for producing the nucleic acid conjugate of the present invention in which a sugar ligand-tether-brancher unit is bonded to the 5' end of the oligonucleotide will be given below.

The nucleic acid conjugate can be produced under the same conditions as in steps 35 and 36 of production method 6. 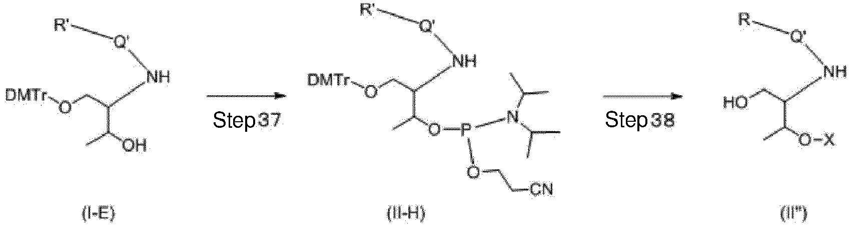 wherein R, R', Q', DMTr and X are as defined above.

### Production method 8

An exemplary method for producing the nucleic acid conjugate of the present invention in which a sugar ligand-tether-brancher unit is bonded to the 5' end of the oligonucleotide will be given below.

The nucleic acid conjugate can be produced under the same conditions as in steps 35 and 36 of production method 6. 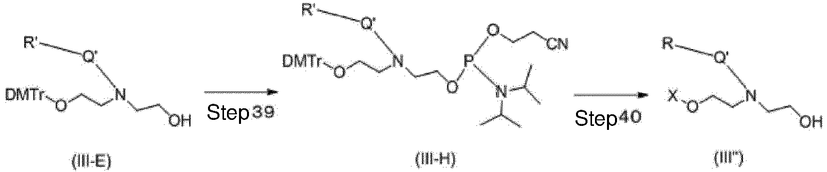 wherein R, R', Q', DMTr and X are as defined above.

### Production method 9

An exemplary method for producing the nucleic acid conjugate of the present invention in which a sugar ligand-tether-brancher unit is bonded to the 5' end of the oligonucleotide will be given below.

The nucleic acid conjugate can be produced under the same conditions as in steps 35 and 36 of production method 6. 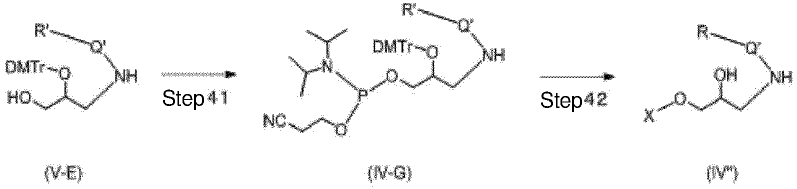 wherein R, R', Q', DMTr and X are as defined above.

### Production method 10

An exemplary method for producing the nucleic acid conjugate of the present invention in which a sugar ligand-tether-brancher unit is bonded to the 5' end of the oligonucleotide will be given below.

The nucleic acid conjugate can be produced under the same conditions as in steps 35 and 36 of production method 6. 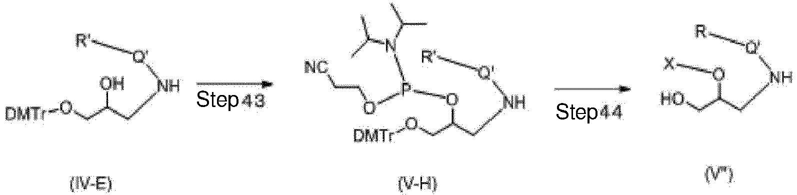 wherein R, R', Q', DMTr and X are as defined above.

### Production method 11

A nucleic acid conjugate having a double-stranded nucleic acid can be obtained by dissolving each of a sense strand having a sugar ligand-tether-brancher unit at the 3' or 5' end of a sense strand constituting the double-stranded nucleic acid, and an antisense strand constituting the double-stranded nucleic acid, in water or an appropriate buffer solution, and mixing the solutions.

Examples of the buffer solution include acetate buffer solutions, Tris buffer solutions, citrate buffer solutions, phosphate buffer solutions, and water. These buffer solutions are used alone or as a mixture.

The mixing ratio between the sense strand and the antisense strand is preferably 0.5 to 2 equivalents, more preferably 0.9 to 1.1 equivalents, further preferably 0.95 equivalents to 1.05 equivalents, of the antisense strand with respect to 1 equivalent of the sense strand.

The sense strand and the antisense strand thus mixed may be appropriately subjected to annealing treatment. The annealing treatment can be performed by heating the mixture of the sense strand and the antisense strand to preferably 50 to 100°C, more preferably 60 to 100°C, further preferably 80 to 100°C, followed by slow cooling to room temperature.

The antisense strand can be obtained in conformity to the aforementioned oligonucleotide synthesis method known in the art.

### Production method 12

For the nucleic acid derivative according to the present invention, the production method can be taken as an example of a method for producing a compound having a substructure represented by formula (VI'): 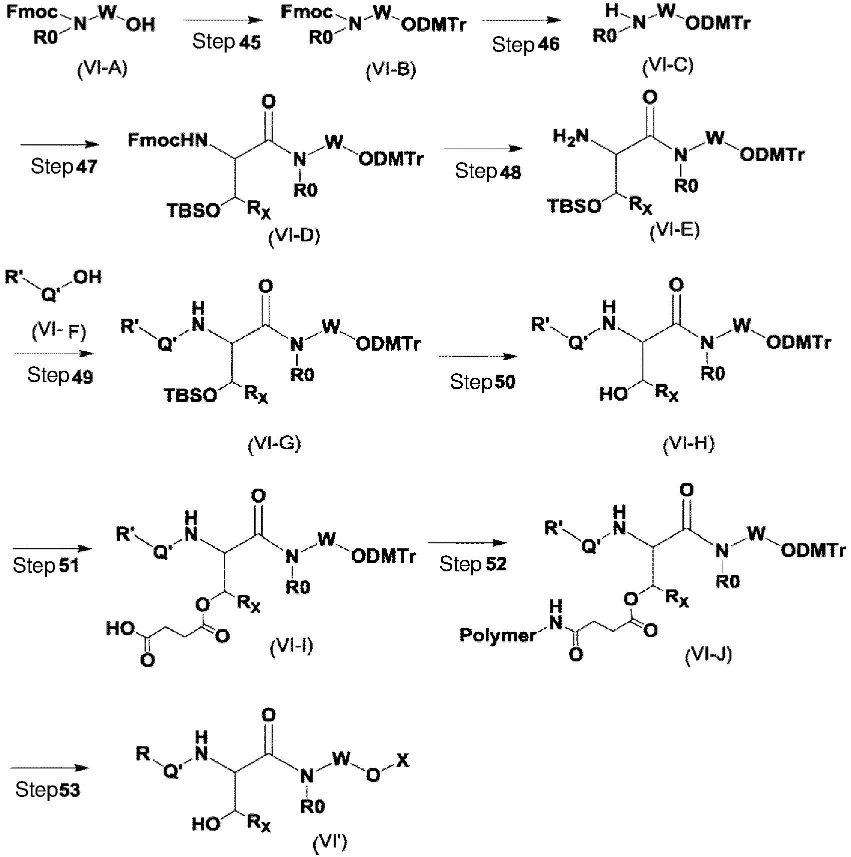 wherein DMTr, R, R', X, Q', Polymer, and Fmoc are as defined above, TBS represents a t-butyldimethylsilyl group, R0 and Rx are the same or different and each represent a hydrogen atom, C1-C10 alkylene or C3-C8 cycloalkylene, and W is C1-C10 alkylene or C3-C8 cycloalkylene or may form a C4-C8 nitrogen-containing heterocyclic ring together with R0.

### Step 45

Compound (VI-B) can be produced under the same conditions as in step 1 of production method 1 using compound (VI-A).

Compound (VI-A) can be obtained as a commercially available product, or by a method known in the art (e.g., Bioorganic & Medicinal Chemistry Letters, Vol. 11, p. 383-386) or a method equivalent thereto.

### Step 46

Compound (VI-C) can be produced under the same conditions as in step 2 of production method 1 using compound (VI-B).

### Step 47

Compound (VI-D) can be produced under the same conditions as in step 3 of production method 1 using compound (VI-C).

### Step 48

Compound (VI-E) can be produced under the same conditions as in step 2 of production method 1 using compound (VI-D).

### Step 49

Compound (VI-G) can be produced under the same conditions as in step 3 of production method 1 using compound (VI-E) and compound (VI-F).

### Step 50

Compound (VI-H) can be produced under the same conditions as in step 9 of production method 2 using compound (VI-G).

### Steps 51 to 53

Compound (VI') can be produced under the same conditions as in steps 4 to 6 of production method 1 using compound (VI-H), compound (VI-I) and compound (VI-J) .

Steps 45 to 53 can also be carried out by a method known in the art (e.g., a method described in International Publication No. WO 2015/105083) or a method equivalent thereto.

Compound (VI-F) can be obtained by a method known in the art (e.g., a method described in Journal of American Chemical Society, Vol. 136, p. 16958, 2014) or a method equivalent thereto.

### Production method 13

A sugar ligand-tether unit in which each of P1 and P4 in formula 2 is -NH-CO-, -O-CO- or -S-CO- can be produced by the following method. 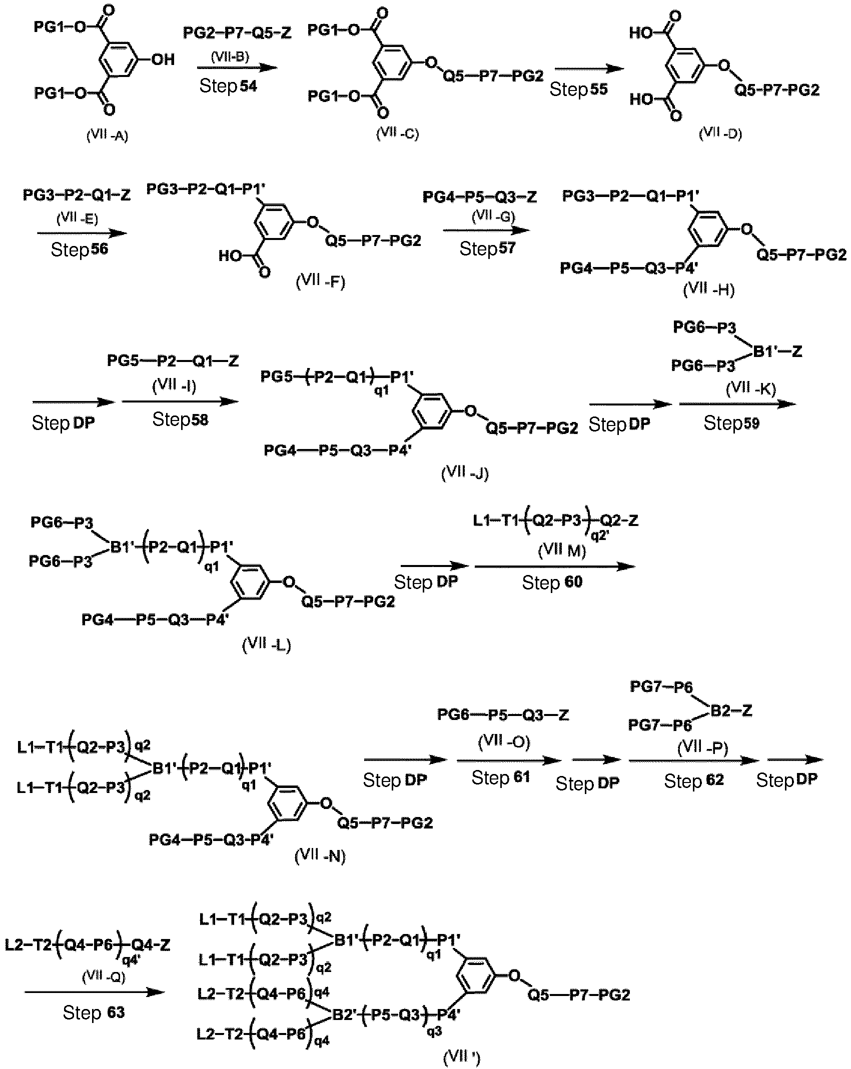 wherein Q1, Q2, Q3, Q4, Q5, P2, P3, P5, P6, P7, T1, T2, L1, L2, q1, q2, q3 and q4 are each as defined above, q2' represents an integer smaller by 1 than q2, q4' represents an integer smaller by 1 than q4, P1' and P4' each independently represent -NH-CO-, -O-CO- or -S-CO-, Z represents H, OH, NH₂, SH, a chlorine atom, a bromine atom, an iodine atom, methanesulfonyloxy, p-toluenesulfonyloxy or carboxylic acid, B1' and B2' each represent any one structure of the following formulas, and PG1, PG2, PG3, PG4, PG5, PG6 and PG7 each represent an appropriate protective group.
Formulas: 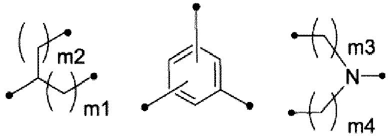 m1, m2, m3 and m4 each independently represent an integer of 0 to 10.

### Step 54

Compound (VII-C) can be produced by adding polymer-supported triphenylphosphine to compound (VII-A) with compound (VII-B) in a solvent such as tetrahydrofuran, and reacting the mixture with a solution of diisopropyl azodicarboxylate in toluene under ice cooling.

Examples of the solvent include those listed in step 2 of production step 1.

Compound (VII-A) can be obtained as a commercially available product.

### Step 55

Compound (VII-D) can be produced by reacting compound (VII-C) under ice cooling in the presence of a base in a solvent such as methanol.

Examples of the solvent include those listed in step 2 of production step 1.

Examples of the base include those listed in step 3 of production step 1.

### Step 56

Compound (VII-F) can be produced under the same conditions as in step 3 of production step 1 using compound (VII-D) and compound (VII-E).

### Step 57

Compound (VII-H) can be produced under the same conditions as in step 3 of production step 1 using compound (VII-F) and compound (VII-G).

### Step 58

Compound (VII-J) can be produced under the same conditions as in step 3 of production step 1 using compound (VII-H) and compound (VII-I).

Also, compound (VII-J) having a desired value of q1 can be produced by repetitively performing step DP1 described below and step 58.

### Step 59

Compound (VII-L) can be produced under the same conditions as in step 3 of production step 1 using compound (VII-J) and compound (VII-K).

### Step 60

Compound (VII-N) can be produced under the same conditions as in step 3 of production step 1 using compound (VII-L) and compound (VII-M).

### Steps 61 to 63

Compound (VII') can be produced under the same conditions as in step 3 of production step 1 using compound (VII-O), compound (VII-P) and compound (VII-Q).

Also, compound (VII') having a desired value of q3 can be produced by repetitively performing step DP1 described below and step 61.

### Step DP1

A desired compound can be produced by appropriately using a method commonly used in organic synthetic chemistry [e.g., a method described in Protective Groups in Organic Synthesis, third edition, T.W. Greene, John Wiley & Sons Inc. (1999)].

Compound (VII-B), compound (VII-E), compound (VII-G), compound (VII-I), compound (VII-K), compound (VII-M), compound (VII-O), compound (VII-P) and compound (VII-Q) can be obtained as commercially available products, or by methods described in "The Fourth Series of Experimental Chemistry, Organic Synthesis, p. 258, Maruzen Co., Ltd. (1992)" and "March's Advanced Organic Chemistry: Reactions, Mechanisms, and Structure, 7th Edition" in combination or methods equivalent thereto.

### Production method 14

A unit in which P7 in formula 4 is -O- can be produced by the following method. 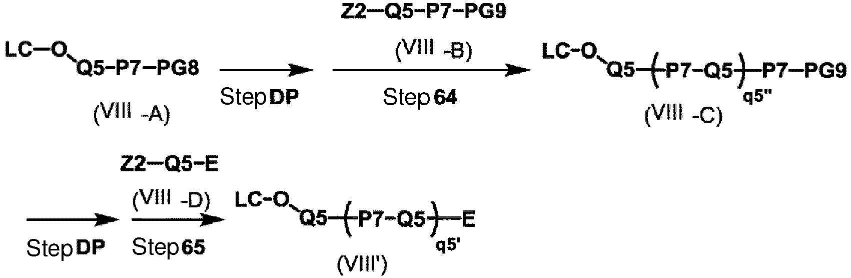 wherein Q5, P7 and q5 are each as defined above, q5'' represents an integer smaller by 2 than q5, q5' represents an integer smaller by 1 than q5, Z2 represents H, OH, NH₂ or SH, PG8 and PG9 each represent an appropriate protective group, LC represents a sugar ligand-tether unit, and E represents carboxylic acid or maleimide.

### Step 64

Compound (VIII-C) can be produced under the same conditions as in step 3 of production step 1 using compound (VIII-A) and compound (VIII-B).

Also, compound (VIII-C) having a desired value of q5'' can be produced by repetitively performing step DP2 described below and step 64. Compound (VIII-B) can be obtained as a commercially available product, or by methods described in "The Fourth Series of Experimental Chemistry, Organic Synthesis, p. 258, Maruzen Co., Ltd. (1992)" and "March's Advanced Organic Chemistry: Reactions, Mechanisms, and Structure, 7th Edition" in combination or methods equivalent thereto.

### Step 65

Compound (VIII') can be produced under the same conditions as in step 3 of production step 1 using compound (VIII-C) and compound (VIII-D).

Compound (VIII-D) can be obtained as a commercially available product, or by methods described in "The Fourth Series of Experimental Chemistry, Organic Synthesis, p. 258, Maruzen Co., Ltd. (1992)" and "March's Advanced Organic Chemistry: Reactions, Mechanisms, and Structure, 7th Edition" in combination or methods equivalent thereto.

### Step DP2

A desired compound can be produced by appropriately using a method commonly used in organic synthetic chemistry [e.g., a method described in Protective Groups in Organic Synthesis, third edition, T.W. Greene, John Wiley & Sons Inc. (1999)].

### Production method 15

A sugar ligand-tether unit in which each of P1 and P4 in formula 2 is -O- can be produced by the following method. 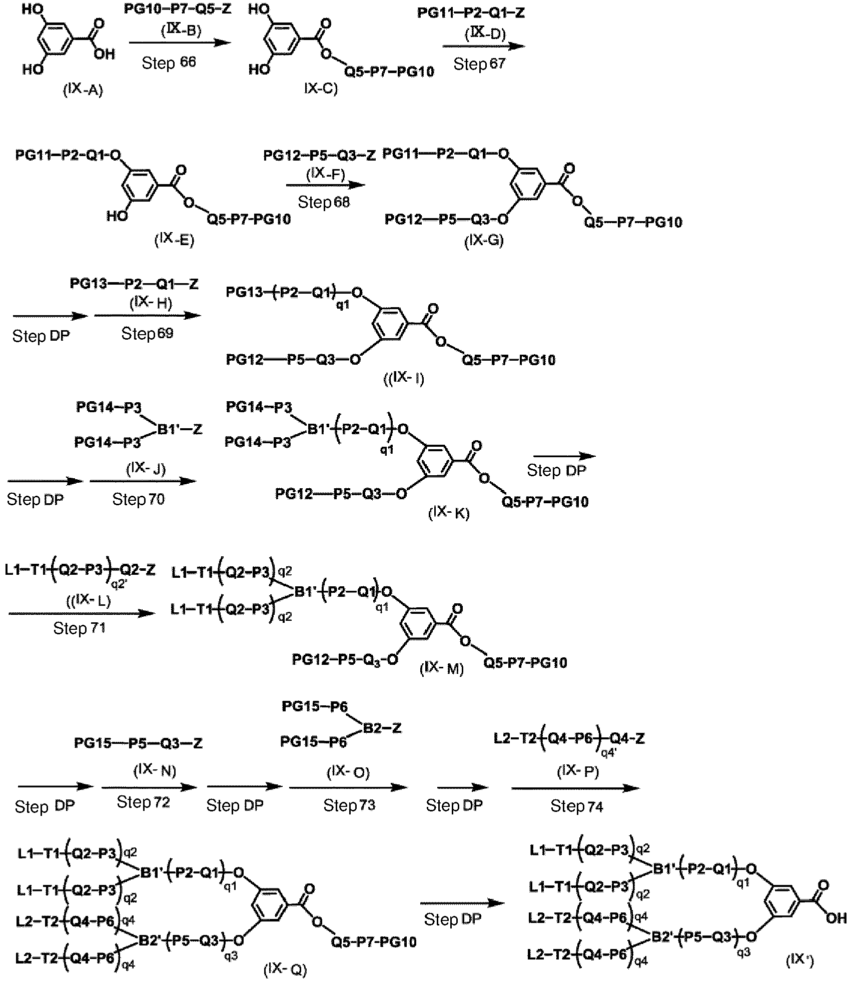 wherein Q1, Q2, Q3, Q4, P2, P3, P5, P6, T1, T2, L1, L2, q1, q2, q3, q4, q2', q4', Z, B1' and B2' are each as defined above, and PG10, PG11, PG12, PG13, PG14 and PG15 each represent an appropriate protective group. Formulas: 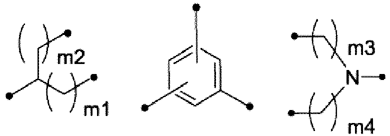 m1, m2, m3 and m4 are as defined above.

### Step 66

Compound (IX-C) can be produced by dissolving compound (IX-A) and compound (IX-B) in a solvent such as N,N'-dimethylformamide, adding a base such as potassium bicarbonate to the solution, and reacting the mixture at room temperature to 200°C for 5 minutes to 100 hours.

Examples of the solvent include those listed in step 2 of production step 1.

Examples of the base include those listed in step 3 of production step 1.

### Step 67

Compound (IX-E) can be produced by dissolving compound (IX-C) and compound (IX-D) in a solvent such as N,N'-dimethylformamide, adding a base such as potassium bicarbonate to the solution, and reacting the mixture at room temperature to 200°C for 5 minutes to 100 hours.

Examples of the solvent include those listed in step 2 of production step 1.

Examples of the base include those listed in step 3 of production step 1.

Compound (IX-A) can be obtained as a commercially available product.

### Step 68

Compound (IX-G) can be produced under the same conditions as in step 3 of production step 1 using compound (IX-E) and compound (IX-F).

### Step 69

Compound (IX-I) can be produced under the same conditions as in step 3 of production step 1 using compound (IX-G) and compound (IX-H).

Also, compound (VII-J) having a desired value of q1 can be produced by repetitively performing step DP and step 69.

### Step 70

Compound (IX-K) can be produced under the same conditions as in step 3 of production step 1 using compound (IX-I) and compound (IX-J).

### Step 71

Compound (IX-M) can be produced under the same conditions as in step 3 of production step 1 using compound (IX-K) and compound (IX-L).

### Steps 72 to 74

Compound (IX') can be produced under the same conditions as in step 3 of production step 1 using compound (IX-M), compound (IX-N), compound (IX-O) and compound (IX-P).

Also, compound (IX') having a desired value of q3 can be produced by repetitively performing step DP3 described below and step 72.

### Step DP3

A desired compound can be produced by appropriately using a method commonly used in organic synthetic chemistry [e.g., a method described in Protective Groups in Organic Synthesis, third edition, T.W. Greene, John Wiley & Sons Inc. (1999)].

Compound (IX'-B), compound (IX'-D), compound (IX'-F), compound (IX'-H), compound (IX'-J), compound (IX'-L), compound (IX'-N), compound (IX'-O) and compound (IX'-P) can be obtained as commercially available products, or by methods described in "The Fourth Series of Experimental Chemistry, Organic Synthesis, p. 258, Maruzen Co., Ltd. (1992)" and "March's Advanced Organic Chemistry: Reactions, Mechanisms, and Structure, 7th Edition" in combination or methods equivalent thereto.

### Production method 16

The following method can also be used as a method for producing the nucleic acid conjugates of formulas 1 to 8. 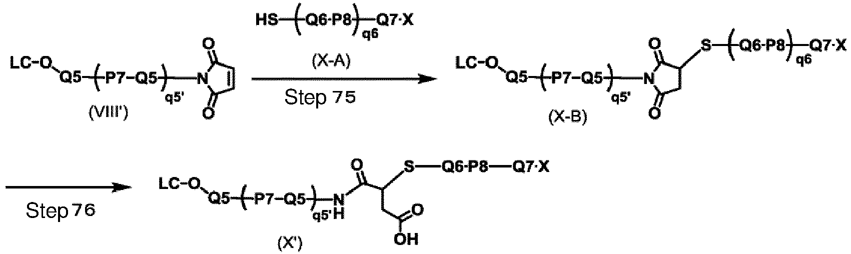 wherein LC, Q5, Q6, Q7, P7, P8, q5', q6 and X are as defined above.

### Step 75

Compound (X-B) can be produced by reacting compound (XIII'') with compound (X-A) at 0°C to 100°C for 10 seconds to 100 hours in a solvent.

Examples of the solvent include water, phosphate buffer solutions, sodium acetate buffer solutions, and dimethyl sulfoxide. These solvents may be used alone or as a mixture.

Compound (VIII') can be obtained by use of production method 14.

Compound (X-A) can also be obtained by a method known in the art (e.g., Bioconjugate Chemistry, Vol. 21, p. 187-202, 2010; and Current Protocols in Nucleic Acid Chemistry, Sep. 2010; CHAPTER: Unit 4.41) or a method equivalent thereto.

### Step 76

Compound (X') can be produced by reacting compound (X-B) at a temperature between room temperature and 200°C for 5 minutes to 100 hours under conditions of pH 8 or higher such as in an aqueous sodium carbonate solution or ammonia water.

### Production method 17

The following method can also be used as a method for producing the nucleic acid conjugates of formulas 1 to 8. 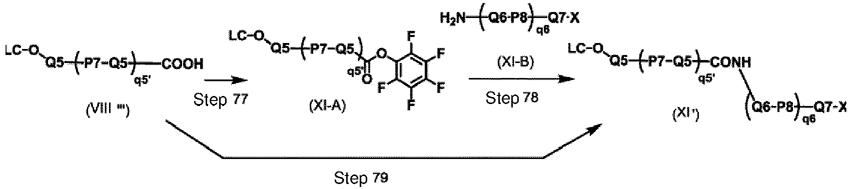 wherein LC, Q5, Q6, Q7, P7, P8, q5', q6 and X are as defined above.

### Step 77

Compound (XI-A) can be obtained by using compound (VIII''') and a method known in the art (e.g., a method described in Bioconjugate Chemistry, Vol. 26, p. 1451-1455, 2015) or a method equivalent thereto.

Compound (VIII '' ') can be obtained by use of production method 14.

### Step 78

Compound (XI') can be obtained by using compound (XI-A) and compound (XI-B) and a method known in the art (e.g., a method described in Bioconjugate Chemistry, Vol. 26, p. 1451-1455, 2015) or a method equivalent thereto.

Compound (XI-B) can be obtained by a method described in Bioconjugate Chemistry, Vol. 26, p. 1451-1455, 2015) or a method equivalent thereto.

### Step 79

In another method, compound (XI') can be obtained directly from compound (XI-A) by a method known in the art (see, for example, Bioconjugate Chemistry, Vol. 22, p. 1723-1728, 2011) or a method equivalent thereto.

The nucleic acid conjugate described in the present specification may be obtained as a salt, for example, an acid-addition salt, a metal salt, an ammonium salt, an organic amine-addition salt, or an amino acid-addition salt.

Examples of the acid-addition salt include: inorganic acid salts such as hydrochloride, sulfate, and phosphate; and organic acid salts such as acetate, maleate, fumarate, citrate, and methanesulfonate. Examples of the metal salt include: alkali metal salts such as sodium salt and potassium salt; alkaline earth metal salts such as magnesium salt and calcium salt; and aluminum salt and zinc salt. Examples of the ammonium salt include salts of ammonium, tetramethylammonium, and the like. Examples of the organic amine-addition salt include addition salts of morpholine, piperidine, and the like. Examples of the amino acid-addition salt include addition salts of lysine, glycine, phenylalanine, and the like.

In the case of preparing the salt of the nucleic acid conjugate described in the present specification, the conjugate obtained in the form of the desired salt can be purified directly, or the conjugate obtained in a free form can be dissolved or suspended in an appropriate solvent, and a corresponding acid or base is added to the solution or the suspension, followed by isolation or purification. In order to convert counter ions forming the conjugate salt to different counter ions, the conjugate salt can be dissolved or suspended in an appropriate solvent, and then, several equivalents to a large excess of an acid, a base and/or a salt (e.g., an inorganic salt such as sodium chloride or ammonium chloride) is added to the solution or the suspension, followed by isolation or purification.

Some nucleic acid conjugates described in the present specification may have stereoisomers such as geometric isomers and optical isomers, tautomers, or the like. All possible isomers and mixtures thereof are also encompassed in the present invention.

The nucleic acid conjugate described in the present specification may be present in the form of an adduct with water or various solvents. These adducts are also encompassed the nucleic acid conjugate of the present invention.

The nucleic acid conjugate of the present invention further encompasses molecules in which a portion or the whole of the atoms is substituted with an atom having an atomic mass number different therefrom (isotope) (e.g., a deuterium atom).

The pharmaceutical composition of the present invention comprises the nucleic acid conjugate represented by formula 1. The nucleic acid conjugate of the present invention, owing to having sugar ligands L1 and L2, is recognized by a target cell and transferred into the cell.

The nucleic acid conjugate of the present invention can be used in the treatment of diseases related to a target gene by inhibiting (reducing or silencing) the expression of the target gene *in vivo* when administered to a mammal.

In the case of using the nucleic acid conjugate of the present invention as a therapeutic agent or a prophylactic agent, the administration route is not particularly limited, and an administration route most effective for treatment is desirably used. Examples thereof include intravenous administration, subcutaneous administration, intramuscular administration. Subcutaneous administration is preferred.

The dose differs depending on the pathological condition or age of the recipient, the administration route, etc. The dose can be, for example, a daily dose of 0.1 µg to 1000 mg, more preferably 1 to 100 mg, in terms of the amount of the double-stranded oligonucleotide.

Examples of the preparation appropriate for intravenous administration or intramuscular administration include injections. A prepared liquid formulation may be used directly in the form of, for example, an injection. Alternatively, the liquid formulation may be used after removal of the solvent by, for example, filtration or centrifugation, or the liquid formulation may be used after being freeze-dried and/or may be used after being supplemented with, for example, an excipient such as mannitol, lactose, trehalose, maltose, or glycine and then freeze-dried.

In the case of an injection, the liquid formulation or the solvent-free or freeze-dried composition is preferably mixed with, for example, water, an acid, an alkali, various buffer solutions, physiological saline, or an amino acid transfusion, to prepare the injection. Alternatively, the injection may be prepared by the addition of, for example, an antioxidant such as citric acid, ascorbic acid, cysteine, or EDTA or a tonicity agent such as glycerin, glucose or sodium chloride. Also, the injection can also be cryopreserved by the addition of a cryopreserving agent such as glycerin.

The composition of the present invention can be administered to a mammalian cell so that the double-stranded nucleic acid in the composition of the present invention can be transferred into the cell.

The *in vivo* method for transferring the nucleic acid conjugate of the present invention to a mammalian cell can be performed according to transfection procedures known in the art that can be performed *in vivo.* The composition of the present invention can be intravenously administered to a mammal including a human and thereby delivered to the liver so that the double-stranded nucleic acid in the composition of the present invention can be transferred into the liver or a liver cell.

The double-stranded nucleic acid in the composition of the present invention thus transferred into the liver or a liver cell decreases the expression of APCS gene in the liver cell and can treat or prevent an amyloid-related disease. Examples of the amyloid-related disease include diseases caused by a disorder mediated by amyloid fibrils containing APCS. Specifically, the amyloid-related disease is a disease in which aberrant insoluble protein fibrils known as amyloid fibrils accumulate in tissues, causing an organ disorder. More specifically, examples of the amyloid-related disease include AL amyloidosis, AA amyloidosis, ATTR amyloidosis, dialysis-related amyloidosis, cardiac failure involving amyloid accumulation, nephropathy involving amyloid accumulation, senile amyloidosis and carpal-tunnel syndrome which are systemic amyloidosis, and Alzheimer's dementia, brain amyloid angiopathy and type II diabetes mellitus which are localized amyloidosis. The recipient of the nucleic acid conjugate of the present invention is a mammal, preferably a human.

In the case of using the nucleic acid conjugate of the present invention as a therapeutic agent or a prophylactic agent for an amyloid-related disease, the administration route used is desirably an administration route most effective for treatment. Examples thereof preferably include intravenous administration, subcutaneous administration and intramuscular administration. Subcutaneous administration is more preferred.

The dose differs depending on the pathological condition or age of the recipient, the administration route, etc. The dose can be, for example, a daily dose of 0.1 µg to 1000 mg, preferably 1 to 100 mg, in terms of the amount of the double-stranded nucleic acid.

The present invention also provides a nucleic acid conjugate for use in the treatment of a disease; a pharmaceutical composition for use in the treatment of a disease; use of a nucleic acid conjugate for treating a disease; use of a nucleic acid conjugate in the production of a medicament for treatment of a disease; a nucleic acid conjugate for use in the production of a medicament for treatment of a disease; and a method for treating or preventing a disease, comprising administering an effective amount of a nucleic acid conjugate to a subject in need thereof.

### Examples

Next, the present invention will be specifically described with reference to Reference Examples, Examples and Test Examples. However, the present invention is not limited by these Examples and Test Examples. Proton nuclear magnetic resonance spectra (¹H NMR) shown in Examples and Reference Examples were measured at 270 MHz, 300 MHz or 400 MHz, and no exchangeable proton may be clearly observed depending on compounds and measurement conditions. Signal multiplicity is indicated as usually used, and br means broad and represents an apparently broad signal.

UPLC analysis employed the following conditions:
Mobile phase A: aqueous solution containing 0.1% formic
acid, B: acetonitrile solution
Gradient: linear gradient from 10% to 90% of mobile phase B (3 min)
Column: ACQUITY UPLC BEH C18 manufactured by Waters Corp.
(1.7 µm, inside diameter: 2.1 x 50 mm)
Flow rate: 0.8 mL/min
PDA detection wavelength: 254 nm (detection range: 190 to 800 nm)

Reference Example compounds are shown in Tables X-1 to X-4.

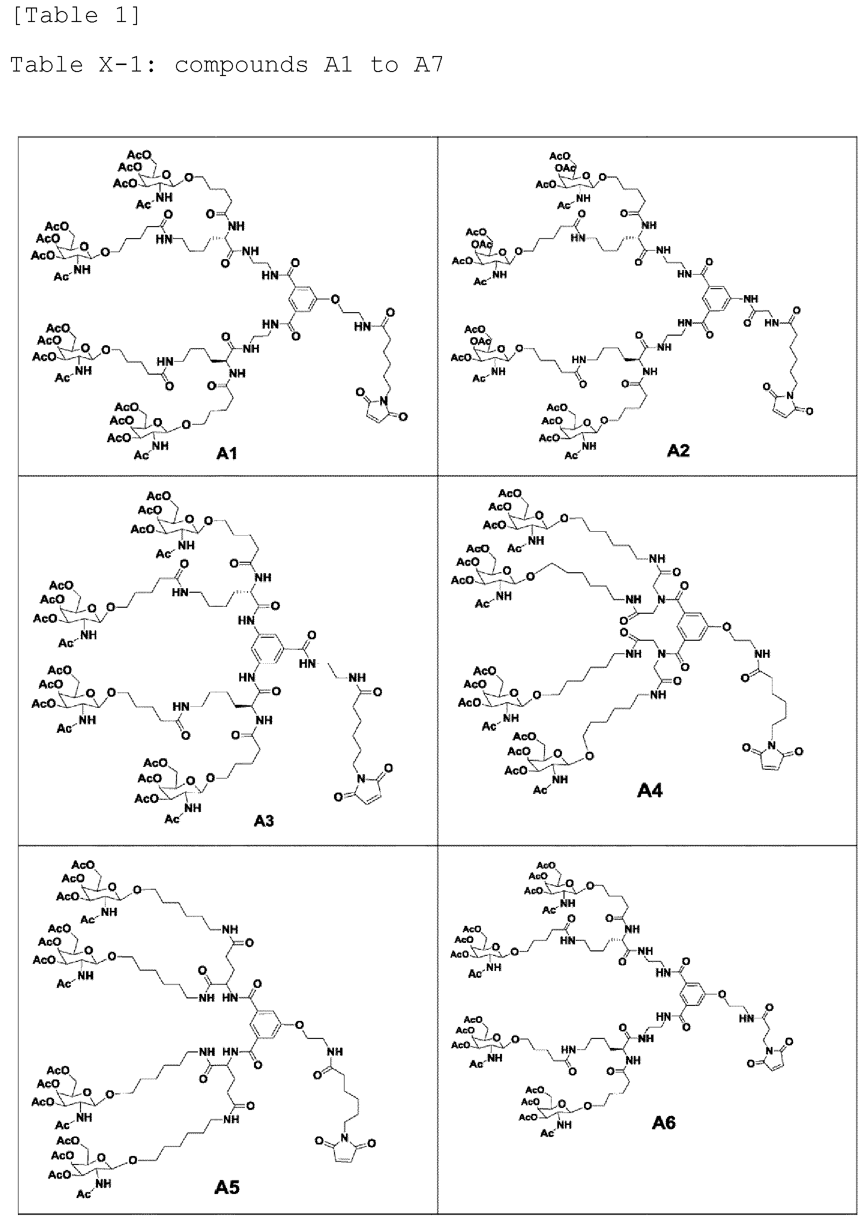

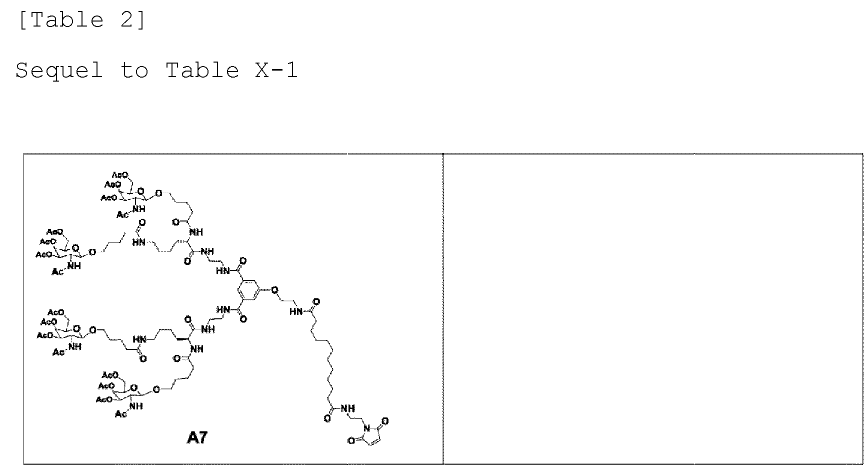

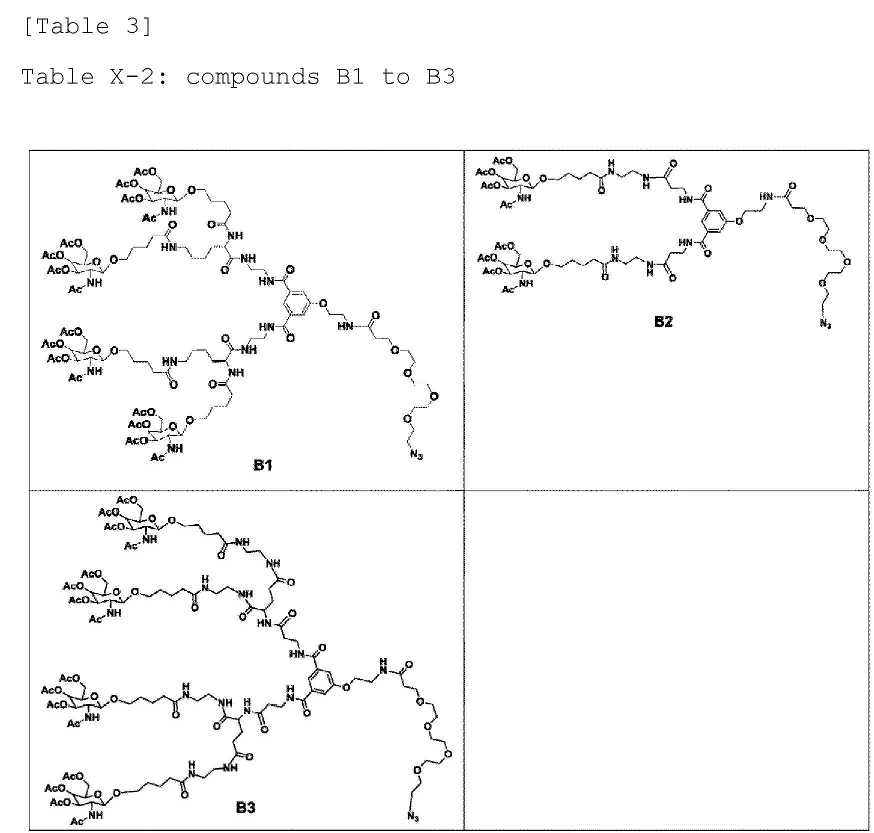

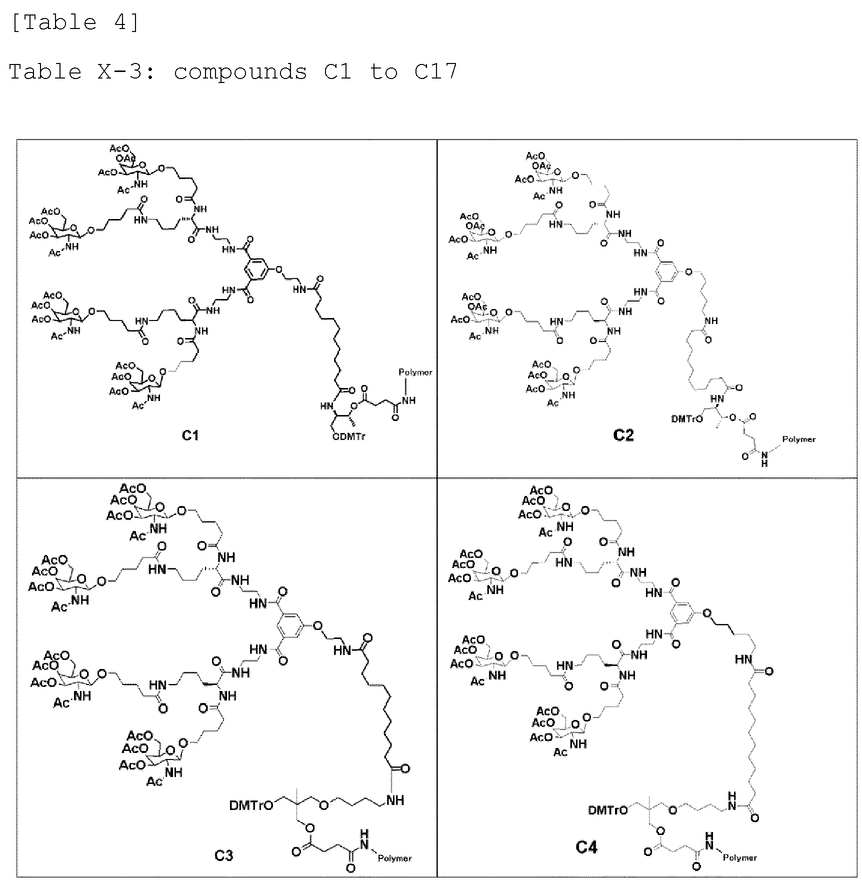

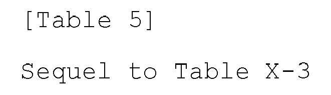

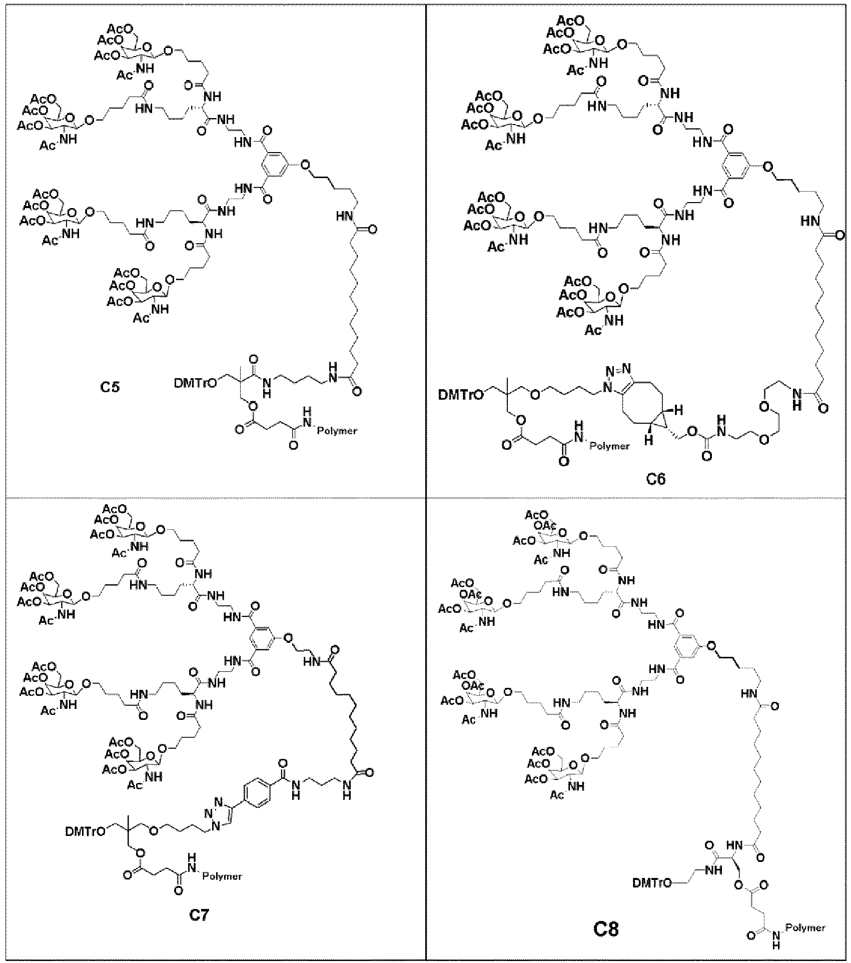

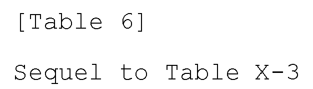

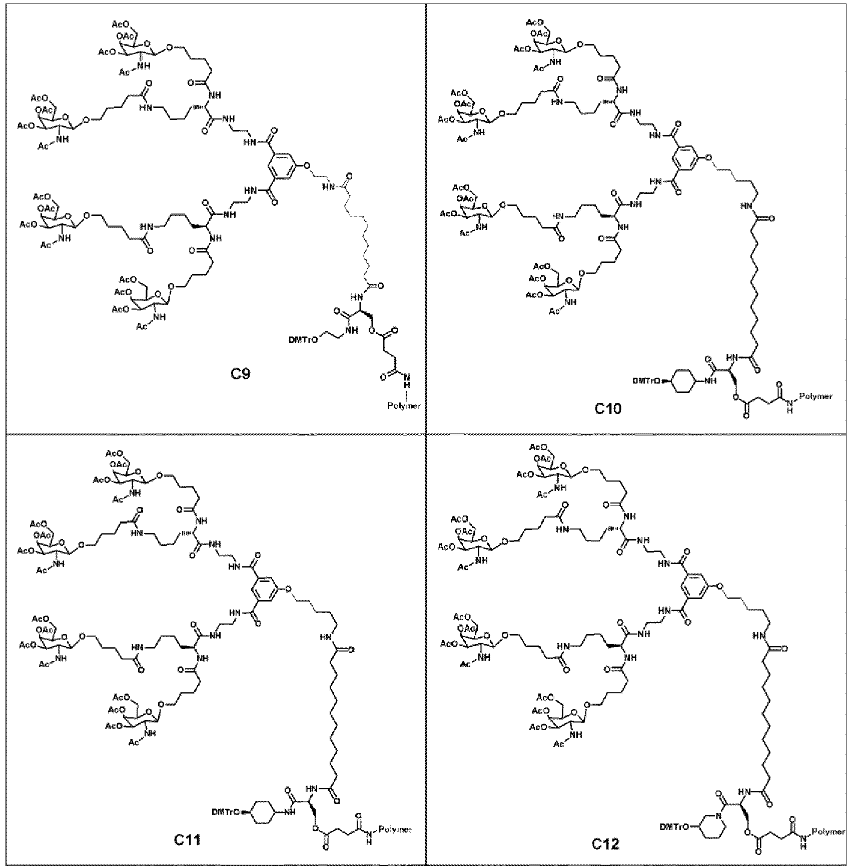

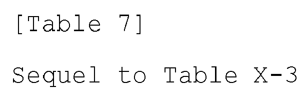

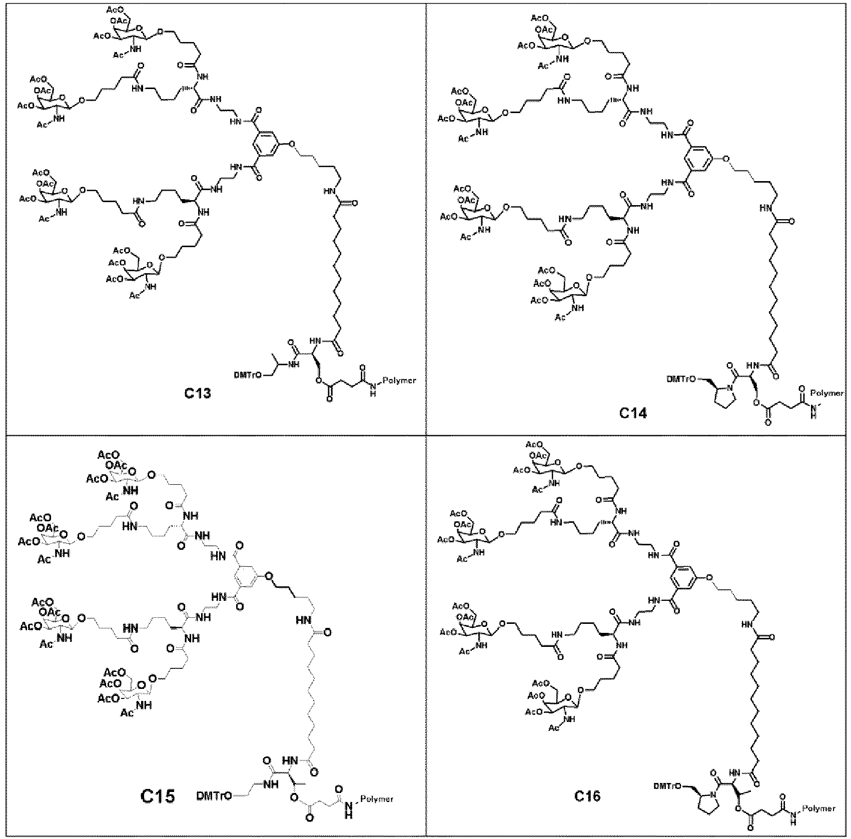

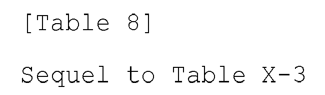

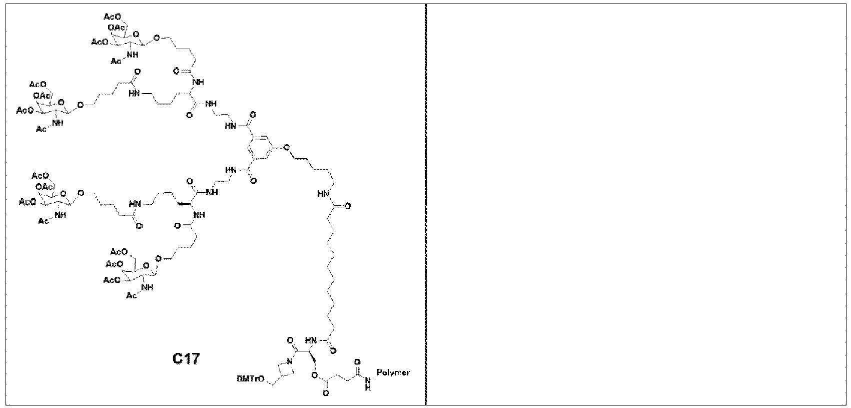

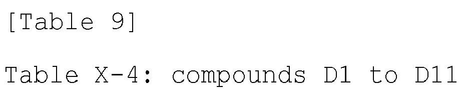

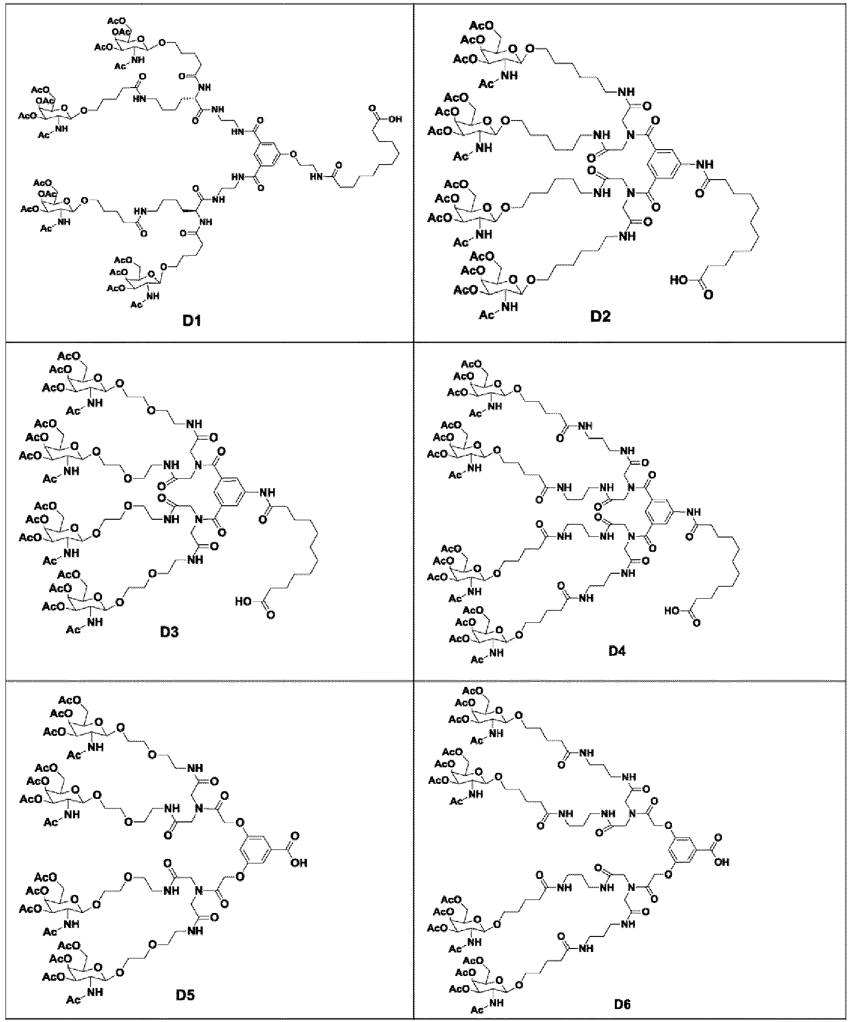

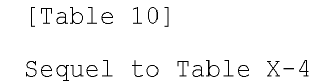

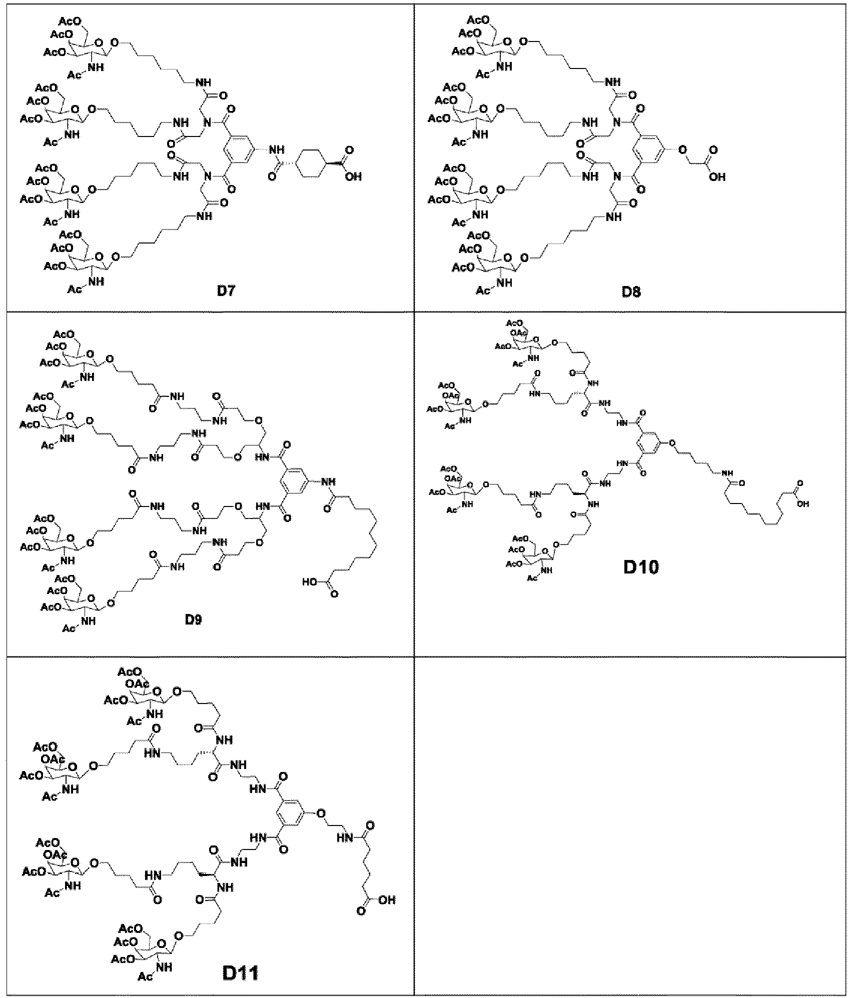

### Reference Example 1

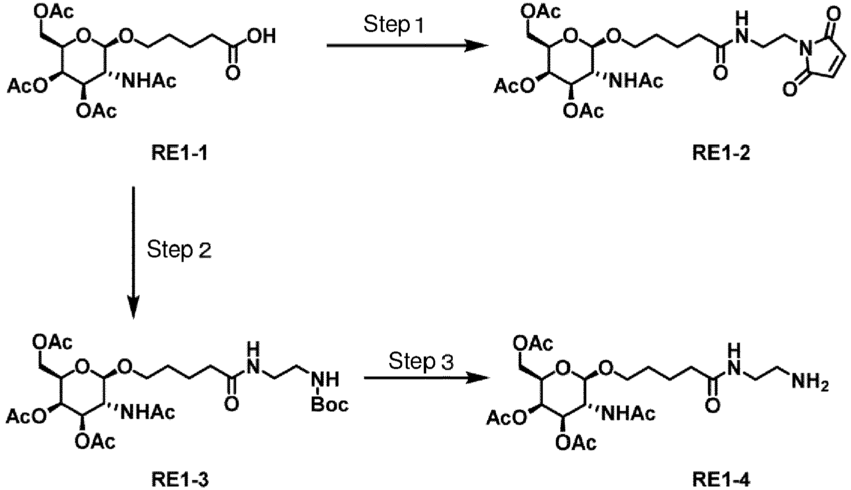

### Synthesis of compound RE1-2

### Step 1 of Reference Example 1

Compound RE1-1 (0.8755 g, 1.9567 mmol) synthesized by the method described in Journal of American Chemical Society, Vol. 136, p. 16958-16961, 2014 was dissolved in tetrahydrofuran (10 mL). To the solution, 1,3-dicyclohexanecarbodiimide (DCC, 0.4247 g, 2.0584 mmol) and N-hydroxysuccinimide (0.2412 g, 2.0958 mmol) were added, and the mixture was stirred overnight at room temperature. A precipitated solid was removed from the reaction mixture, and the solvent was distilled off under reduced pressure. The obtained mixture was dissolved in N,N'-dimethylformamide (DMF). To the solution, 2-aminoethyl maleimide bromate (0.6479 g, 2.5491 mmol) and diisopropylethylamine (1.7 mL, 9.7835 mmol) were added, and then, the mixture was stirred overnight at room temperature. The solvent in the reaction solution was distilled off under reduced pressure, followed by elution by reverse-phase column chromatography (water/methanol = 80/20) to obtain compound RE1-2 (0.8502 g, yield: 76%). ESI-MS m/z: 570 (M + H)⁺;
¹H-NMR (DMSO-D₆) δ: 1.45-1.56 (4H, m), 1.78 (3H, s), 1.90 (3H, s), 1.97 (2H, t, J = 7.0 Hz), 2.00 (3H, s), 2.11 (3H, s), 3.18-3.19 (2H, m), 3.38-3.45 (3H, m), 3.64-3.71 (1H, m), 3.85-3.89 (1H, m), 4.01-4.04 (3H, m), 4.48 (1H, d, J = 8.6 Hz), 4.95-4.98 (1H, m), 5.21 (1H, d, J = 3.5 Hz), 6.99 (2H, s), 7.81-7.87 (2H, m).

### Synthesis of compound RE1-4

### Step 2 of Reference Example 1

Compound RE1-1 (0.9602 g, 2.1460 mmol) was dissolved in N,N'-dimethylformamide (10 mL). To the solution, N-Boc-ethylenediamine (manufactured by Sigma-Aldrich Co. LLC, 0.6877 g, 4.292 mmol), diisopropylethylamine (1.90 mL, 10.87 mmol), and 2-(1H-benzotriazol-1-yl)-1,1,3,3-tetramethyluronium hexafluorophosphate (manufactured by Wako Pure Chemical Industries, Ltd., 1.6437 g, 4.3229 mmol) were added, and the mixture was stirred overnight at room temperature. Water was added to the reaction solution, followed by extraction with chloroform twice. Then, the organic layer was washed with saturated saline and dried over anhydrous magnesium sulfate. The solvent was distilled off under reduced pressure to obtain a crude product of compound RE1-3.
ESI-MS m/z: 590 (M + H)⁺

### Step 3 of Reference Example 1

Compound RE1-3 (1.2654 g, 2.1460 mmol) synthesized in step 1 in the synthesis of compound RE1-4 was dissolved in dichloromethane (15 mL). To the solution, trifluoroacetic acid (4 mL) was added, and the mixture was stirred overnight at room temperature. Water was added to the reaction solution, followed by extraction with ethyl acetate. Then, the organic layer was washed with saturated saline and dried over anhydrous magnesium sulfate. The solvent was distilled off under reduced pressure, followed by elution by reverse-phase column chromatography (water/methanol = 80/20) to obtain compound RE1-4 (0.3879 g, yield: 37%).
ESI-MS m/z: 490 (M + H)⁺;
¹H-NMR (DMSO-D₆) δ: 1.46-1.52 (4H, m), 1.78 (3H, s), 1.90 (3H, s), 2.00 (3H, s), 2.08 (2H, t, J = 7.4 Hz), 2.11 (3H, s), 2.85 (2H, t, J = 6.3 Hz), 3.27 (2H, dd, J = 12.3, 6.2 Hz), 3.67-3.69 (1H, m), 3.68-3.73 (1H, m), 3.86-3.90 (1H, m), 4.01-4.04 (3H, m), 4.49 (1H, d, J = 8.4 Hz), 4.97 (1H, dd, J = 11.3, 3.4 Hz), 5.22 (1H, d, J = 3.5 Hz), 7.86 (1H, d, J = 9.1 Hz), 7.95-8.02 (1H, m).

### Reference Example 2

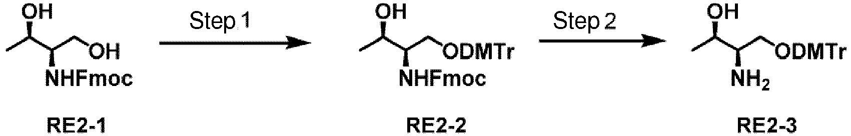

### Step 1 of Reference Example 2

(9H-Fluoren-9-yl)methyl ((2R,3R)-1,3-dihydroxybutan-2-yl)carbamate (compound RE2-1, manufactured by Chem-Impex International, Inc., 1.50 g, 4.58 mmol) was dissolved in pyridine (20 mL). To the solution, 4,4'-dimethoxytrityl chloride (manufactured by Tokyo Chemical Industry Co., Ltd., 1.71 g, 5.04 mmol) was added under ice cooling, and then, the mixture was stirred at room temperature for 2 hours. The reaction solution was ice-cooled, and a 10% aqueous citric acid solution was added thereto, followed by extraction with ethyl acetate. Then, the organic layer was washed with saturated saline and dried over anhydrous magnesium sulfate. The solvent was distilled off under reduced pressure, and the residue was purified by silica gel column chromatography (hexane/ethyl acetate = 90/10) to obtain compound RE2-2 (1.07 g, yield: 37%).
ESI-MS m/z: 630 (M + H)⁺

### Step 2 of Reference Example 2

Compound RE2-2 (1.07 g, 1.699 mmol) synthesized in step 1 of Reference Example 2 was dissolved in N,N-dimethylformamide (10 mL). To the solution, piperidine (0.336 mL, 3.40 mmol) was added at room temperature, and the mixture was stirred for 3 hours. Water was added to the reaction solution, followed by extraction with ethyl acetate. Then, the organic layer was washed with saturated saline and dried over anhydrous magnesium sulfate. The solvent was distilled off under reduced pressure, and the residue was purified by amino silica gel column chromatography (chloroform/methanol = 90/10) to obtain compound RE2-3 (0.59 g, yield: 85%).
ESI-MS m/z: 408 (M + H)⁺

### Reference Example 3

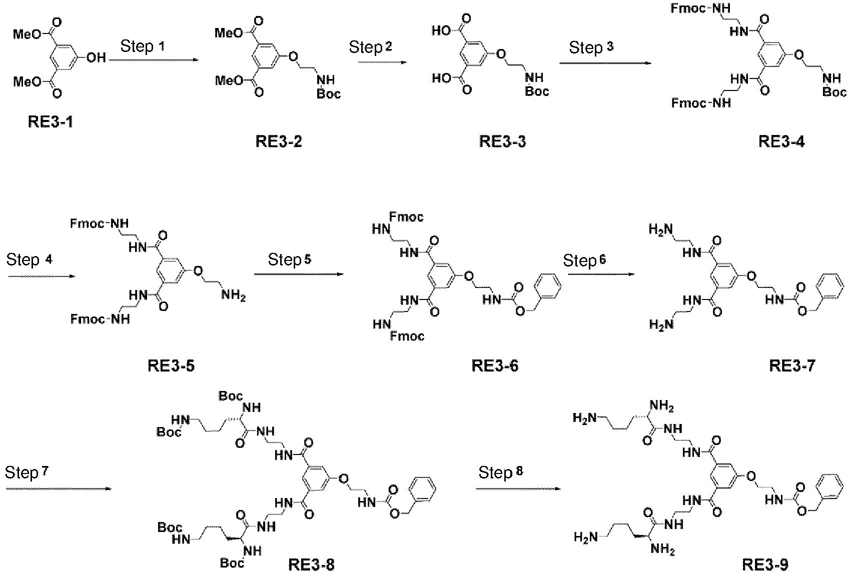

### Step 1 of Reference Example 3

Dimethyl 5-hydroxyisophthalate (compound RE3-1, manufactured by Wako Pure Chemical Industries, Ltd., 5.0443 g, 24 mmol) was dissolved in tetrahydrofuran (manufactured by Wako Pure Chemical Industries, Ltd., 25 mL). To the solution, 2-(tert-butoxycarbonylamino)-1-ethanol (manufactured by Tokyo Chemical Industry Co., Ltd., 4.0343 g, 25.03 mmol), and polymer-supported triphenylphosphine (manufactured by Sigma-Aldrich Co. LLC, 6.61 g, 25.2 mmol) were added, then a 40% solution of diisopropyl azodicarboxylate (DIAD) in toluene (manufactured by Tokyo Chemical Industry Co., Ltd., 13.26 mL, 25.2 mmol) was added under ice cooling, and the mixture was stirred overnight at room temperature. The reaction solution was filtered, and the solvent in the filtrate was distilled off under reduced pressure. Then, the residue was purified by amino silica gel column chromatography (hexane/ethyl acetate = 95/5 to 80/20) to obtain compound RE3-2 (5.3071 g, yield: 63%).
ESI-MS m/z: 254 (M + H)⁺, detected as a Boc-deprotected form

### Step 2 of Reference Example 3

Compound RE3-2 (5.3071 g, 15.02 mmol) synthesized in step 1 of Reference Example 3 was dissolved in methanol (25 mL). To the solution, a 2 mol/L aqueous sodium hydroxide solution (manufactured by Wako Pure Chemical Industries, Ltd., 13 mL) was added under ice cooling, and the mixture was stirred at room temperature for 4 hours. The reaction solution was ice-cooled, and a 10% aqueous citric acid solution was added thereto, followed by extraction with ethyl acetate. Then, the organic layer was washed with saturated saline and dried over anhydrous magnesium sulfate. The solvent was distilled off under reduced pressure to quantitatively obtain compound RE3-3. ESI-MS m/z: 324 (M - H)⁻

### Step 3 of Reference Example 3

Compound RE3-3 (1.9296 g, 5.93 mmol) synthesized in step 2 of Reference Example 3 was dissolved in N,N'-dimethylformamide (70 mL). To the solution, N-1-(9H-fluoren-9-ylmethoxycarbonyl)-ethylenediamine hydrochloride (3.3493 g, 11.86 mmol), diisopropylethylamine (5.18 mL, 29.7 mmol), and 2-(1H-benzotriazol-1-yl)-1,1,3,3-tetramethyluronium hexafluorophosphate (4.5168 g, 11.88 mmol) were added, and the mixture was stirred at room temperature for 4 hours. The reaction solution was ice-cooled, and a 10% aqueous citric acid solution was added thereto, followed by extraction with chloroform. Then, the organic layer was washed with a saturated aqueous solution of sodium bicarbonate and saturated saline and dried over anhydrous magnesium sulfate. The solvent was distilled off under reduced pressure, and the residue was purified by silica gel column chromatography (chloroform/methanol) to obtain compound RE3-4 (3.4407 g, yield: 68%).
ESI-MS m/z: 898 (M + HCOO)⁻

### Step 4 of Reference Example 3

Compound RE3-4 (1.6087 g, 1.884 mmol) synthesized in step 3 of Reference Example 3 was dissolved in dichloromethane (20 mL). To the solution, trifluoroacetic acid (5 mL, 64.9 mmol) was added under ice cooling, and the mixture was stirred at room temperature for 4 hours. The solvent in the reaction solution was distilled off under reduced pressure to quantitatively obtain compound RE3-5 (2.4079 g).
ESI-MS m/z: 798 (M + HCOO)⁻

### Step 5 of Reference Example 3

Compound RE3-5 (386 mg, 0.512 mmol) synthesized in step 4 of Reference Example 3 was dissolved in tetrahydrofuran (10 mL). To the solution, benzoyl chloride (175 mg, 1.024 mmol) was added under ice cooling, and the mixture was stirred at room temperature for 1 hour. The solvent in the reaction solution was distilled off under reduced pressure, and the residue was purified by silica gel column chromatography(chloroform/methanol) to obtain compound RE3-6 (373 mg, yield: 82%).
ESI-MS m/z: 888 (M + H)⁺

### Step 6 of Reference Example 3

Compound RE3-6 (108 mg, 0.122 mmol) synthesized in step 5 of Reference Example 3 was dissolved in dichloromethane (5 mL). To the solution, diethylamine (0.5 mL, 4.8 mmol) was added at room temperature, and the mixture was stirred for 1 hour. The solvent was distilled off under reduced pressure to quantitatively obtain compound RE3-7 (54.9 mg).
ESI-MS m/z: 444 (M + H)⁺

### Step 7 of Reference Example 3

Compound RE3-7 (180 mg, 0.406 mmol) synthesized in step 6 of Reference Example 3, Nα,Nε-bis(tert-butoxycarbonyl)-L-lysine (manufactured by Novabiochem/Merck Millipore, 295 mg, 0.852 mmol), diisopropylethylamine (0.354 mL, 2.029 mmol), and 2-(1H-benzotriazol-1-yl)-1,1,3,3-tetramethyluronium hexafluorophosphate (324 mg, 0.852 mmol) were added, and the mixture was stirred overnight at room temperature. The reaction solution was ice-cooled, and a 10% aqueous citric acid solution was added thereto, followed by extraction with chloroform. Then, the organic layer was washed with saturated saline and dried over anhydrous magnesium sulfate. The solvent was distilled off under reduced pressure, and the residue was purified by amino silica gel column chromatography (chloroform/methanol) to quantitatively obtain compound RE3-8 (450 mg).
ESI-MS m/z: 1101 (M + H)⁺

### Step 8 of Reference Example 3

Compound RE3-8 (2.1558 g, 1.9593 mmol) synthesized in step 7 of Reference Example 3 was dissolved in dichloromethane (20 mL). To the solution, trifluoroacetic acid (5 mL) was added under ice cooling, and the mixture was stirred at room temperature for 4 hours. The solvent in the reaction solution was distilled off under reduced pressure to quantitatively obtain compound RE3-9.
ESI-MS m/z: 700 (M + H)⁺

### Reference Example 4

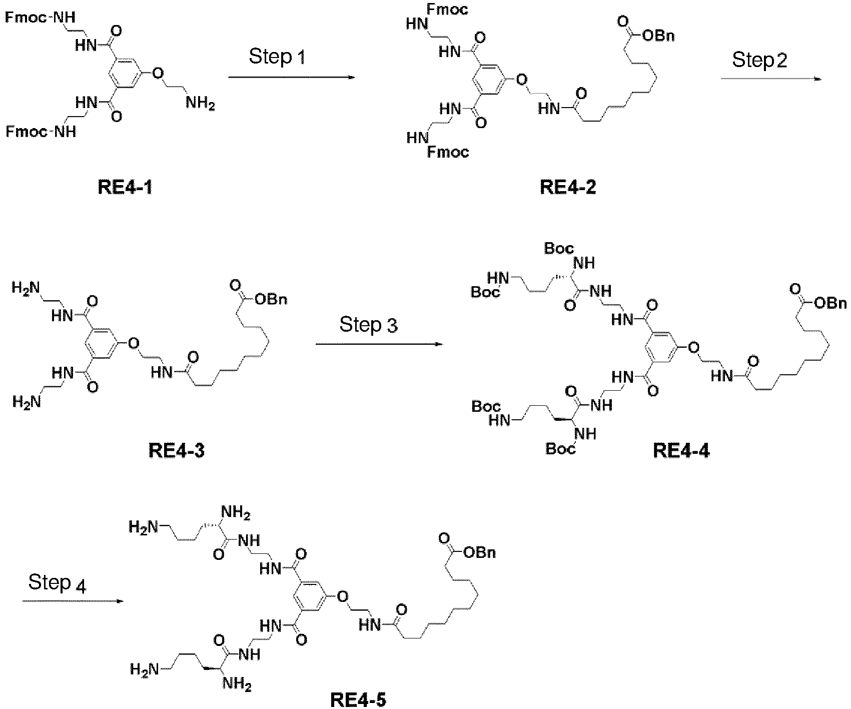

wherein Bn represents a benzyl group.

### Step 1 of Reference Example 4

Compound RE4-1 (compound RE3-5 in Reference Example 3, 0.5716 g, 0.7582 mmol) synthesized by the method described in Reference Example 3, dodecanoic acid monobenzyl ester (0.4859 g, 1.5164 mmol) synthesized by the method described in Bioconjugate Chemistry, Vol. 22, p. 690-699, 2011, diisopropylethylamine (0.662 mL, 3.79 mmol), and 2-(1H-benzotriazol-1-yl)-1,1,3,3-tetramethyluronium hexafluorophosphate (0.5766 g, 1.516 mmol) were dissolved in N,N-dimethylformamide (12 mL). The solution was stirred at room temperature for 1 hour. The reaction solution was ice-cooled, and a saturated aqueous solution of citric acid was added thereto, followed by extraction with chloroform. Then, the organic layer was washed with saturated saline and dried over anhydrous magnesium sulfate. The solvent was distilled off under reduced pressure, and the residue was purified by silica gel column chromatography (chloroform/methanol) to obtain compound RE4-2 (0.88 g, yield: 84%)
ESI-MS m/z: 1057(M + H)⁺

### Step 2 of Reference Example 4

Compound RE4-2 (0.7545 g, 0.714 mmol) synthesized in step 1 of Reference Example 4 was dissolved in a mixed solvent of dichloromethane and tetrahydrofuran (20 mL). To the solution, diethylamine (5 mL, 47.9 mmol) was added at room temperature, and the mixture was stirred overnight. The solvent was distilled off under reduced pressure to quantitatively obtain compound RE4-3.
ESI-MS m/z: 612 (M + H)⁺

### Step 3 of Reference Example 4

Compound RE4-3 (0.437 g, 0.7143 mmol) synthesized in step 2 of Reference Example 4, Nα,Nε-bis(tert-butoxycarbonyl)-L-lysine (manufactured by Novabiochem/Merck Millipore, 0.5483 g, 1.583 mmol), diisopropylethylamine (0.624 mL, 3.57 mmol), and 2-(1H-benzotriazol-1-yl)-1,1,3,3-tetramethyluronium hexafluorophosphate (0.5703 g, 1.5 mmol) were added, and the mixture was stirred room at temperature for 2 hours. A 10% aqueous citric acid solution was added to the reaction solution, followed by extraction with ethyl acetate. Then, the organic layer was washed with saturated saline and dried over anhydrous magnesium sulfate. The solvent was distilled off under reduced pressure to obtain a crude product of compound RE4-4.
ESI-MS m/z: 1269 (M + H)⁺

### Step 4 of Reference Example 4

Compound RE4-4 (0.906 g, 0.7143 mmol) synthesized in step 3 of Reference Example 4 was dissolved in dichloromethane (12 mL). To the solution, trifluoroacetic acid (3 mL, 38.9 mmol) was added under ice cooling, and the mixture was stirred at room temperature for 4 hours. The solvent in the reaction solution was distilled off under reduced pressure to obtain a crude product of compound RE4-5.
ESI-MS m/z: 869 (M + H)⁺

### Reference Example 5

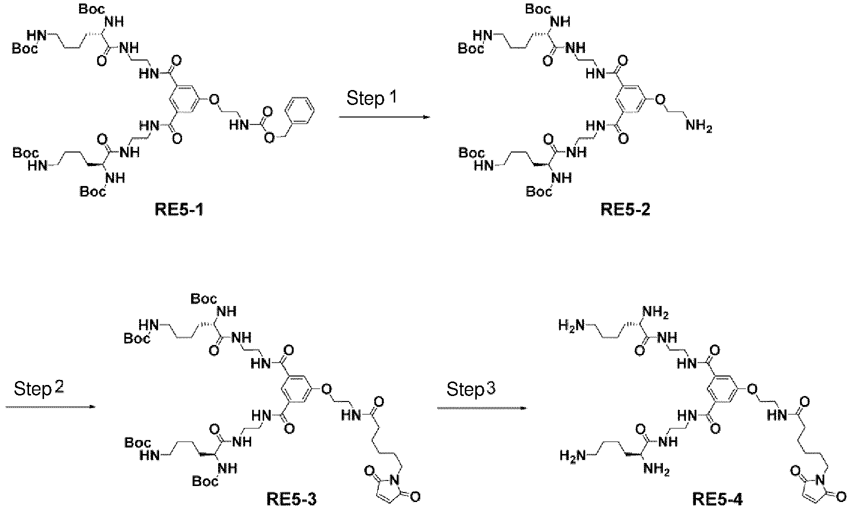

### Step 1 of Reference Example 5

Compound RE5-1 (RE3-8 in Reference Example 3, 100 mg, 0.091 mmol) synthesized by the method described in Reference Example 3 was dissolved in methanol (3 mL). To the solution, acetic acid (2 µL) was added, followed by catalytic hydrogen reduction using palladium/carbon. The solvent in the obtained solution fraction was distilled off under reduced pressure to quantitatively obtain compound RE5-2.
ESI-MS m/z: 967 (M + H)⁺

### Step 2 of Reference Example 5

Compound RE5-2 (50 mg, 0.052 mmol) and N-(6-maleimidocaproyloxy)succinimide (48 mg, 0.155 mmol) were dissolved in tetrahydrofuran (2 mL). To the solution, diisopropylethylamine (0.045 mL, 0.259 mmol) was added, and the mixture was stirred overnight at room temperature. The solvent was distilled off under reduced pressure, and the residue was purified by silica gel column chromatography (chloroform/methanol) to obtain compound RE5-3 (18 mg, yield: 30%).
ESI-MS m/z: 1160 (M + H)⁺

### Step 3 of Reference Example 5

Compound RE5-3 (18 mg, 0.016 mmol) synthesized in step 2 of Reference Example 5 was dissolved in dichloromethane (2 mL). To the solution, trifluoroacetic acid (0.2 mL, 2.6 mmol) was added under ice cooling, and the mixture was stirred overnight at room temperature. The solvent in the reaction solution was distilled off under reduced pressure, and the residue was crystallized from diethyl ether to obtain compound RE5-4 (7.5 mg, yield: 64%) as a white solid.
ESI-MS m/z: 759 (M + H)⁺

### Reference Example 6

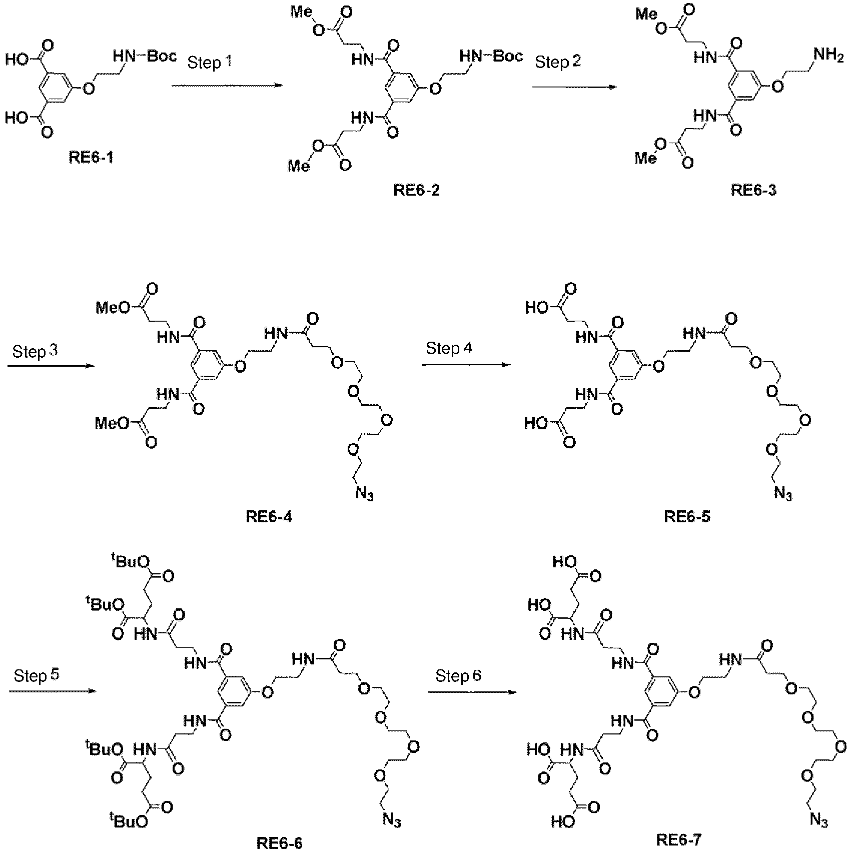

### Step 1 of Reference Example 6

Compound RE6-2 was quantitatively obtained in the same way as in step 3 of Reference Example 3 using compound RE6-1 (compound RE3-2 in Reference Example 3, 0.9372 g, 2.8809 mmol) synthesized by the method described in Reference Example 3 and β-alanine methyl ester hydrochloride (manufactured by Tokyo Chemical Industry Co., Ltd., 0.8082 g, 5.7902 mmol).
ESI-MS m/z: 495 (M + H)⁺

### Step 2 of Reference Example 6

Compound RE6-3 was quantitatively obtained in the same way as in step 4 of Reference Example 3 using compound RE6-2 (0.9622 g, 1.952 mmol) synthesized in step 1 of Reference Example 6.
ESI-MS m/z: 396 (M + H)⁺

### Step 3 of Reference Example 6

Compound RE6-4 was quantitatively obtained in the same way as in step 2 of Reference Example 5 using compound RE6-3 (0.1146 g, 0.290 mmol) synthesized in step 2 of Reference Example 6 and N-succinimidyl 15-azido-4,7,10,13-tetraoxapentadecanoic acid (N3-PEG4-NHS, manufactured by Tokyo Chemical Industry Co., Ltd., 0.0750 g, 0.1931 mmol).
ESI-MS m/z: 669 (M + H)⁺

### Step 4 of Reference Example 6

Compound RE6-5 was quantitatively obtained in the same way as in step 2 of Reference Example 3 using compound RE6-4 (0.1291 g, 0.193 mmol) synthesized in step 3 of Reference Example 6.
ESI-MS m/z: 641 (M + H)⁺

### Step 5 of Reference Example 6

Compound RE6-6 (0.0521 g, yield: 24%) was obtained in the same way as in step 3 of Reference Example 3 using compound RE6-5 (0.1252 g, 0.193 mmol) synthesized in step 4 of Reference Example 6 and L-glutamic acid di-tert-butyl ester (manufactured by Watanabe Chemical Industries, Ltd., 0.1180 g, 0.399 mmol).
ESI-MS m/z: 1124 (M + H)⁺

### Step 6 of Reference Example 6

Compound RE6-7 (36 mg, yield: 86%) was obtained in the same way as in step 4 of Reference Example 3 using compound RE6-6 (0.0521 g, 0.0464 mmol) synthesized in step 5 of Reference Example 6.
ESI-MS m/z: 899 (M + H)⁺

### Reference Example 7

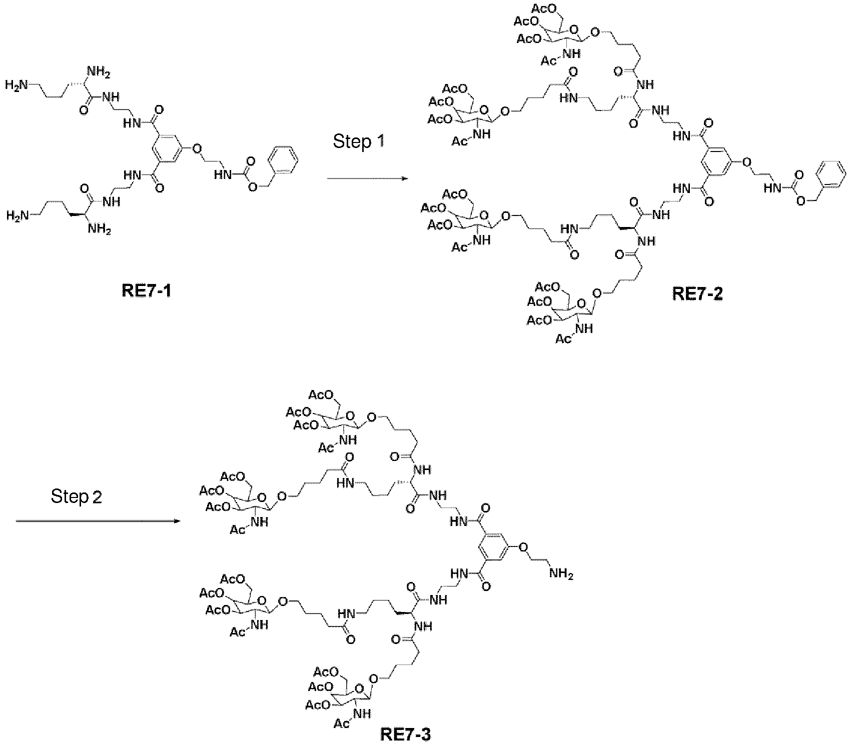

### Step 1 of Reference Example 7

Compound RE7-2 (0.5272 g, yield: 61%) was obtained in the same way as in step 3 of Reference Example 3 using compound RE7-1 (RE3-9 in Reference Example 3, 0.2586 g, 0.3695 mmol) synthesized by the method described in Reference Example 3 and compound RE-1 (0.8559 g, 1.7927 mmol) of Reference Example 1 synthesized by the method described in Journal of American Chemical Society, Vol. 136, p. 16958-16961, 2014.
ESI-MS m/z: 2418 (M + H)⁺

### Step 2 of Reference Example 7

Compound RE7-3 (0.1524 g, yield: 61%) was obtained in the same way as in step 1 of Reference Example 5 using compound RE7-2 (0.2653 g, 0.1097 mmol) synthesized in step 1 of Reference Example 7.
ESI-MS m/z: 2284 (M + H)⁺

### Reference Example 8: Synthesis of compounds A1 and B1

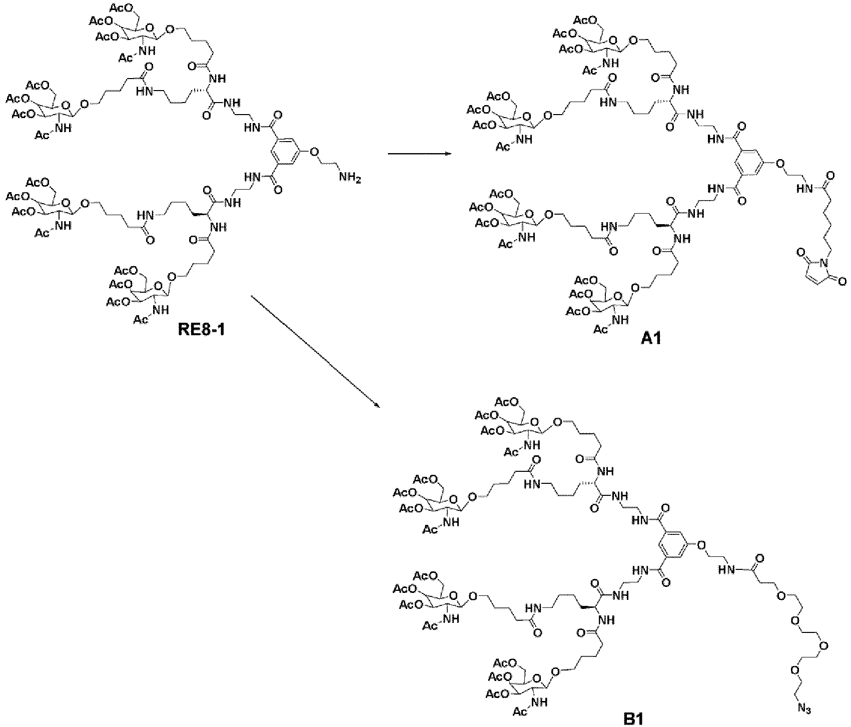

### Synthesis of compound A1

Compound A1 (0.0077 g, yield: 47%) was obtained in the same way as in step 2 of Reference Example 5 using compound RE8-1 (compound RE7-3 in Reference Example 7, 0.0152 g, 0.006657 mmol) synthesized by the method described in Reference Example 7.
ESI-MS m/z: 1239(M + 2H)²⁺
¹H-NMR (DMSO-D₆) δ: 1.11-1.66 (34H, m), 1.77 (12H, d, J = 1.5 Hz), 1.89 (12H, s), 2.01-2.14 (10H, m), 2.01 (12H, s), 2.10 (12H, s), 2.92-2.99 (4H, m), 3.16-3.54 (14H, m), 3.65-3.74 (4H, m), 3.81-3.91 (4H, m), 3.98-4.08 (14H, m), 4.11-4.24 (4H, m), 4.48 (4H, dd, J = 8.4, 1.8 Hz), 4.93-5.00 (4H, m), 5.21 (4H, d, J = 3.5 Hz), 6.99 (2H, s), 7.52 (2H, s), 7.66-7.75 (2H, m), 7.78-7.87 (6H, m), 7.91 (1H, br s), 8.01-8.08 (3H, br m), 8.54-8.60 (2H, br m).

### Synthesis of compound B1

Compound B1 (0.0062 g, yield: 37%) was obtained in the same way as in step 3 of Reference Example 3 using compound RE8-1 (compound RE7-3 in Reference Example 7, 0.0150 g, 0.00657 mmol).
ESI-MS m/z: 1279(M + 2H)²⁺
¹H-NMR (DMSO-D₆) δ: 1.11-1.66 (30H, m), 1.77 (12H, s), 1.89 (12H, s), 2.01-2.14 (8H, m), 2.01 (12H, s), 2.10 (12H, s), 2.33-2.38 (2H, m), 2.92-2.99 (4H, m), 3.16-3.54 (14H, m), 3.58-3.63 (16H, m), 3.65-3.74 (4H, m), 3.81-3.91 (4H, m), 3.98-4.08 (12H, m), 4.11-4.24 (4H, m), 4.48 (4H, dd, J = 8.4, 1.8 Hz), 4.93-5.00 (4H, m), 5.21 (4H, d, J = 3.5 Hz), 7.52 (2H, s), 7.66-7.75 (2H, m), 7.78-7.87 (6H, m), 7.91 (1H, br s), 8.01-8.08 (3H, br m), 8.54-8.60 (2H, br m) .

### Reference Example 9: Synthesis of compounds B2 and B3

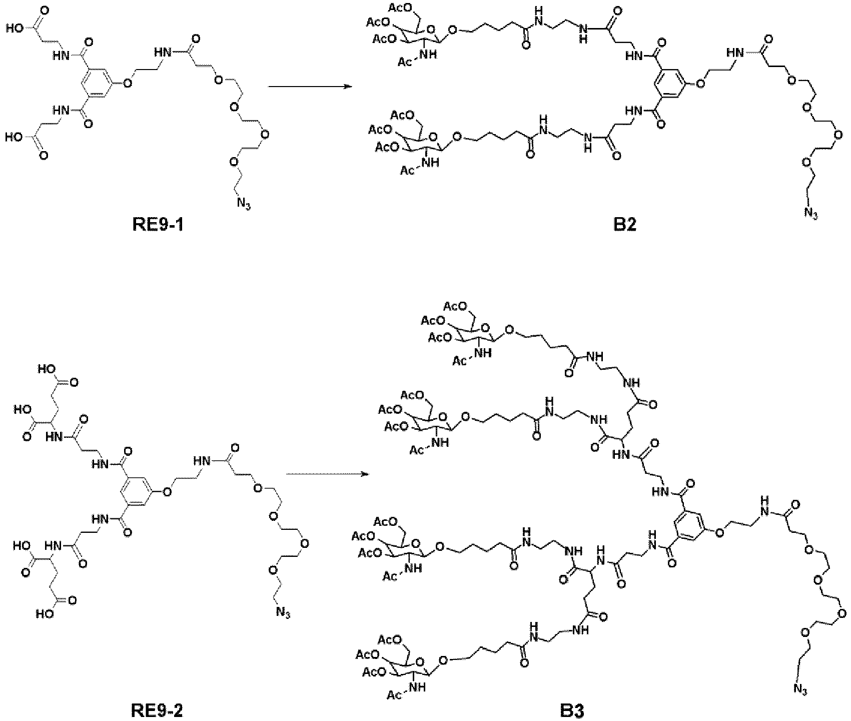

### Synthesis of compound B2

A crude product of compound B2 was obtained in the same way as in step 3 of Reference Example 3 using compound RE9-1 (compound RE6-5 in Reference Example 6, 0.00436 g, 0.00681 mmol) synthesized by the method described in Reference Example 6 and compound RE1-4 (0.010 g, 0.020 mmol) of Reference Example 1.
ESI-MS m/z: 1584(M + H)⁺

### Synthesis of compound B3

Compound B3 (0.0223 g, yield: 72%) was obtained in the same way as in step 3 of Reference Example 3 using compound RE9-2 (compound RE6-7 in Reference Example 6, 0.0100 g, 0.01112 mmol) synthesized by the method described in Reference Example 6.
ESI-MS m/z: 1393(M + 2H)²⁺

### Reference Example 10: Synthesis of compounds C1 and D1

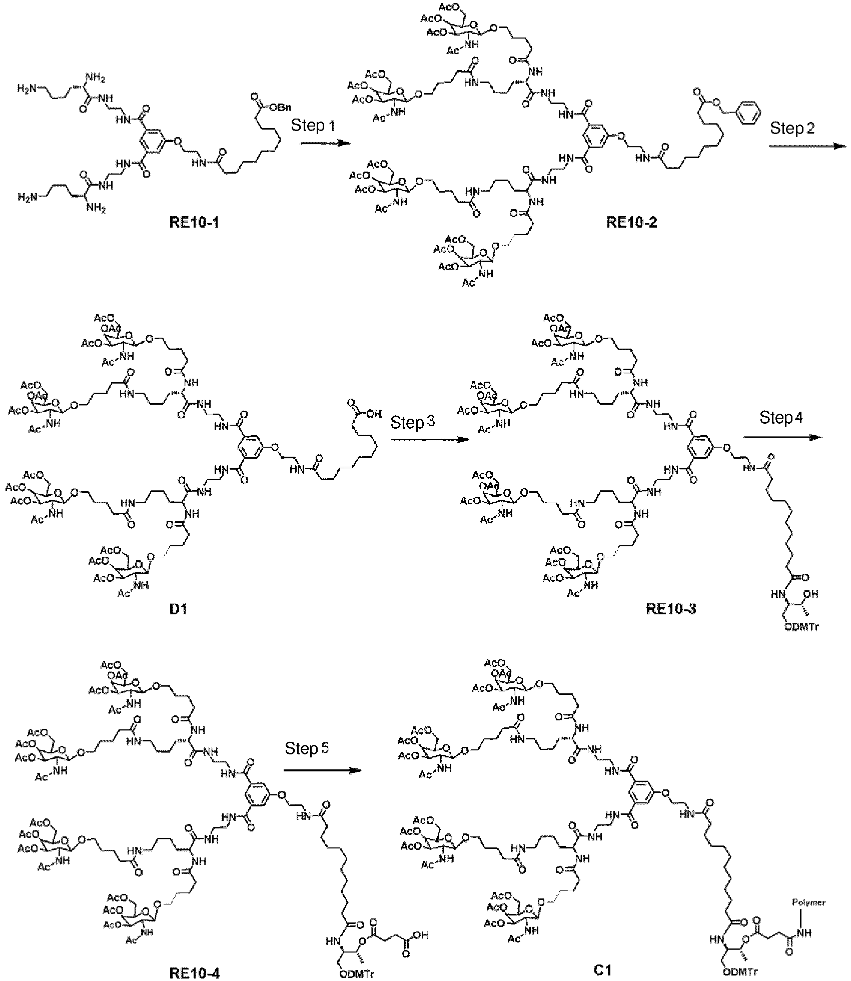

### Step 1 of Reference Example 10

Compound RE10-2 (334.8 mg, yield: 58%) was obtained in the same way as in step 3 of Reference Example 3 using compound RE10-1 (compound RE4-5 in Reference Example 4, 0.1952 g, 0.225 mmol) synthesized by the method described in Reference Example 4 and compound RE1-1 (0.4162 g, 0.93 mmol) of Reference Example 1 synthesized by the method described in Journal of American Chemical Society, Vol. 136, p. 16958-16961, 2014.
ESI-MS m/z: 1294 (M + 2H)²⁺

### Step 2 of Reference Example 10

Compound D1 (112 mg, yield: 80%) was obtained in the same way as in step 1 of Reference Example 5 using compound RE10-2 (0.1459 g, 0.056 mmol) synthesized in step 1 of Reference Example 10.
ESI-MS m/z: 1249 (M + 2H)²⁺
¹H-NMR (DMSO-D₆) δ: 1.11-1.66 (44H, m), 1.77 (12H, d, J = 1.5 Hz), 1.89 (12H, s), 2.01-2.20 (12H, m), 2.01 (12H, s), 2.10 (12H, s), 2.92-2.99 (4H, m), 3.16-3.54 (10H, m), 3.58-3.64 (4H, m), 3.65-3.74 (4H, m), 3.81-3.91 (4H, m), 3.98-4.08 (12H, m), 4.11-4.24 (4H, m), 4.48 (4H, dd, J = 8.4, 1.8 Hz), 4.93-5.00 (4H, m), 5.21 (4H, d, J = 3.5 Hz), 6.99 (2H, s), 7.52 (2H, s), 7.66-7.75 (2H, m), 7.78-7.87 (6H, m), 7.91 (1H, br s), 8.01-8.08 (3H, br m), 8.54-8.60 (2H, br m) .

### Step 3 of Reference Example 10

Compound RE10-3 was obtained as a crude product in the same way as in step 3 of Reference Example 3 using compound D1 (0.1091 g, 0.044 mmol) synthesized in step 2 of Reference Example 10 and compound RE2-3 (0.0748 g, 0.184 mmol) of Reference Example 2.
ESI-MS m/z: 1292 (M + 2H)²⁺, detected as a DMTr-deprotected form

### Step 4 of Reference Example 10

Compound RE10-3 (0.161 g, 0.05586 mmol) synthesized in step 3 of Reference Example 10 was dissolved in dichloromethane (5 mL). To the solution, succinic anhydride (manufactured by Tokyo Chemical Industry Co., Ltd., 0.1182 g, 1.181 mmol), N,N-dimethylaminopyridine (0.0224 g, 0.183 mmol), and triethylamine (0.55 mL, 3.95 mmol) were added, and the mixture was stirred overnight at room temperature. The reaction solution was ice-cooled, and water was added thereto, followed by extraction with ethyl acetate twice. Then, the organic layer was washed with saturated saline and dried over anhydrous magnesium sulfate. The solvent was distilled off under reduced pressure to obtain a crude product of compound RE10-4.
ESI-MS m/z: 1342 (M + 2H)²⁺, detected as a DMTr-deprotected form

### Step 5 of Reference Example 10

Compound RE10-4 (0.0816 g, 0.02734 mmol) synthesized in step 4 of Reference Example 10, O-(benzotriazol-1-yl)-N,N,N',N'-tetramethyluronium hexafluorophosphate (0.0221 g, 0.05827 mmol), and diisopropylethylamine (0.02 mL, 0.1094 mmol) were dissolved in N,N-dimethylformamide (4 mL). To the solution, LCAA-CPG (manufactured by ChemGenes Corp., 0.4882 g) was added, and the mixture was stirred overnight at room temperature. The mixture was collected by filtration, washed with dichloromethane, a 10% solution of methanol in dichloromethane, and diethyl ether in this order and then allowed to act on a solution of acetic anhydride in pyridine to obtain compound C1 (49.5 (µmol/g, yield: 89%). The yield was calculated from the rate of introduction to a solid-phase support which can be calculated from absorption derived from a DMTr group by adding a 1% solution of trifluoroacetic acid in dichloromethane to the form supported by the solid phase.

### Reference Example 11

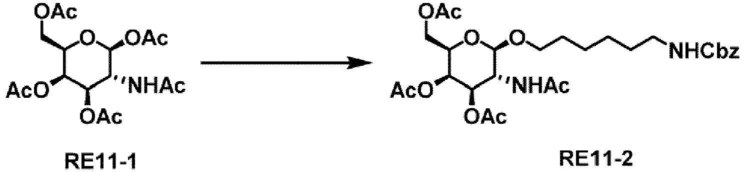

Compound RE11-2 (1.050 g, yield: 50%) was synthesized from compound RE11-1 (1.200 g, 3.640 mmol) by the method described in Journal of Medicinal Chemistry, Vol. 59, p. 2718-2733, 2016.
ESI-MS m/z: 582 (M + H)⁺

### Reference Example 12

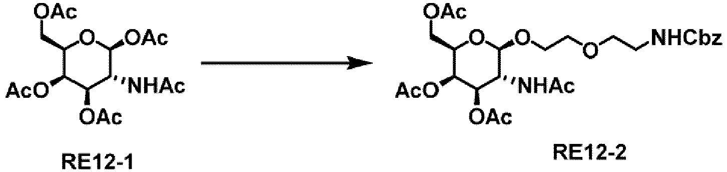

Compound RE12-1 (4.000 g, 10.27 mmol) was dissolved in dichloromethane (60 mL). To the solution, benzyl-2-(2-hydroxyethoxy)ethyl carbamate (2.700 g, 11.30 mmol) and trifluoromethanesulfonic acid (0.1360 mL, 1.541 mmol) were added, and the mixture was stirred overnight under reflux conditions. A 10 wt% aqueous potassium carbonate solution was added to the reaction solution, and the mixture was separated into aqueous and organic layers with dichloromethane. Then, the organic layer was washed with water and dried over anhydrous sodium sulfate. The solvent was distilled off under reduced pressure, and the residue was solvent-replaced with 2-methyltetrahydrofuran and concentrated. The residue was added dropwise to heptane, and the obtained crystals were filtered to obtain compound RE12-2 (5.130 g, yield: 88%).
ESI-MS m/z: 570 (M + H)⁺

### Reference Example 13

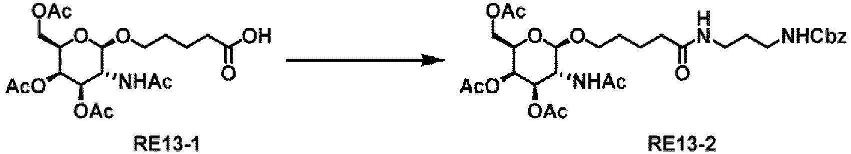

Compound RE13-1 (compound RE1-1 in Reference Example 1, 898.0 mg, 2.007 mmol) was dissolved in dichloromethane (15 mL). To the solution, 1-hydroxybenzotriazole monohydrate (338.0 mg, 2.208 mmol), 1-(3-dimethylaminopropyl)-3-ethylcarbodiimide hydrochloride (343 mg, 2.208 mmol), and N-1-Z-1,3-diaminopropane hydrochloride (0.4910 mL, 2.208 mmol) were added, and the mixture was stirred at room temperature for 3 hours. Water was added to the reaction solution, and the mixture was separated into aqueous and organic layers with dichloromethane. Then, the organic layer was washed with a saturated aqueous solution of sodium bicarbonate and dried over anhydrous sodium sulfate. The solvent was distilled off under reduced pressure, and the residue was purified by silica gel column chromatography (chloroform/methanol = 90/10) to obtain compound RE13-2 (873.0 mg, yield: 68%).
ESI-MS m/z: 639 (M + H)⁺

### Reference Example 14

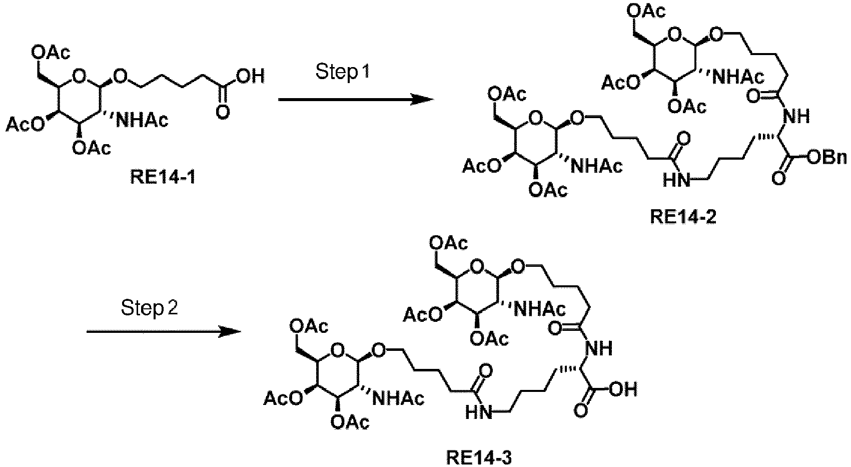

### Step 1 of Reference Example 14

Compound RE14-1 (compound RE1-1 in Reference Example 1, 3.00 g, 6.70 mmol) was dissolved in dichloromethane (60 mL). To the solution, L-lysine benzyl ester di-p-toluenesulfonate (1.75 g, 3.02 mmol), triethylamine (0.935 mL, 6.70 mmol), 1-(3-dimethylaminopropyl)-3-ethylcarbodiimide hydrochloride (1.29 g, 6.70 mmol), and 1-hydroxybenzotriazole monohydrate (103 mg, 0.670 mmol) were added at room temperature, and the mixture was stirred for 2.5 hours. The reaction solution was washed with water and a saturated aqueous solution of sodium bicarbonate and dried over anhydrous sodium sulfate. The solvent was distilled off under reduced pressure, and the residue was purified by silica gel column chromatography (chloroform/methanol = 90/10) to quantitatively obtain compound RE14-2.
ESI-MS m/z: 1096 (M + H)⁺

### Step 2 of Reference Example 14

Compound RE14-2 (2.30 g, 2.10 mmol) was dissolved in tetrahydrofuran (46 mL). To the solution, a 10% palladium-carbon powder (water-containing product, 54.29%; 424 mg) was added at room temperature, and the mixture was stirred overnight in a hydrogen atmosphere. The reaction solution was filtered, and the solvent was distilled off under reduced pressure to quantitatively obtain compound RE14-3.
ESI-MS m/z: 1006 (M + H)⁺

### Reference Example 15

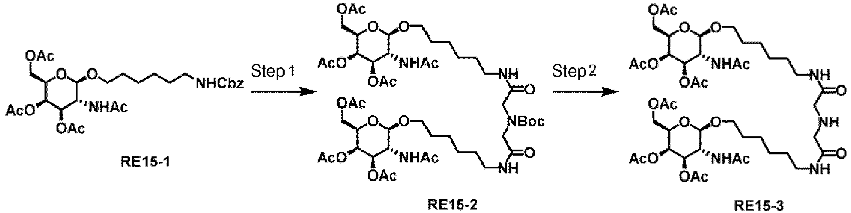

### Step 1 of Reference Example 15

Iminodiacetic acid (manufactured by Tokyo Chemical Industry Co., Ltd., 1.5 g, 6.43 mmol,) was dissolved in methylene chloride (30 mL). To the solution, pentafluorotrifluoroacetic acid (manufactured by Tokyo Chemical Industry Co., Ltd., 2.75 mL, 16.08 mmol) and triethylamine (4.48 mL, 32.2 mmol) were added, and the mixture was stirred for 4 hours. A 10% aqueous citric acid solution was added to the reaction solution, followed by extraction with chloroform. Then, the organic layer was washed with saturated saline and dried over anhydrous magnesium sulfate. A solution of compound RE15-1 (compound RE11-2 in Reference Example 11, 2 g, 3.45 mmol) synthesized by the method described in Reference Example 11, dissolved in a mixed solution of ethyl acetate (10 mL) and acetonitrile (10 mL) was added to the residue, followed by catalytic hydrogen reduction using palladium/carbon. The solvent in the obtained solution portion was distilled off under reduced pressure to obtain a crude product of compound RE15-2.
ESI-MS m/z: 1091 (M + H)⁺

### Step 2 of Reference Example 15

Compound RE15-3 was quantitatively obtained in the same way as in step 3 of Reference Example 1 using compound RE15-2 (1.5 g, 1.37 mmol).
ESI-MS m/z: 990 (M + H)⁺

### Reference Example 16

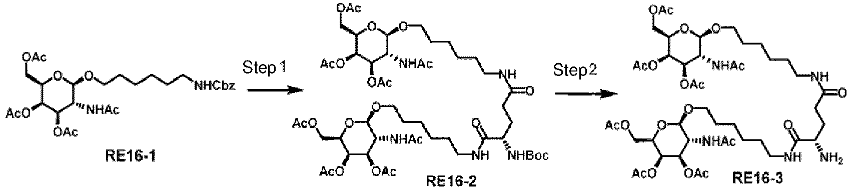

### Step 1 of Reference Example 16

A crude product of compound RE16-2 was obtained in the same way as in step 1 of Reference Example 15 using N-(t-butoxycarbonyl)-L-glutamic acid (manufactured by Tokyo Chemical Industry Co., Ltd.) and compound RE16-1 (1.855 g, 3.19 mmol) synthesized by the method described in Reference Example 11.
ESI-MS m/z: 1105 (M + H)⁺

### Step 2 of Reference Example 16

Compound RE16-3 was quantitatively obtained in the same way as in step 3 of Reference Example 1 using compound RE16-2 (1.421 g, 1.2866 mmol).
ESI-MS m/z: 1004 (M + H)⁺

### Reference Example 17

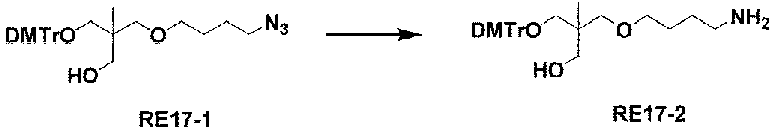

Compound RE17-1 (90 mg, 0.173 mmol) synthesized by the method described in Journal of Organic Chemistry, Vol. 74, p. 6837-6842, 2009 was dissolved in tetrahydrofuran (1 mL). To the solution, polymer-supported triphenylphosphine (manufactured by Sigma-Aldrich Co. LLC, 63 mg, 0.189 mmol) was added, and the mixture was stirred for 3 hours under heating to reflux. The reaction solution was filtered, and the solvent was distilled off under reduced pressure to obtain compound RE17-2 (70 mg, yield: 82%).
ESI-MS m/z: 516 (M+Na)⁺
¹H-NMR (400 MHz, CDCl₃): δ 0.89 (3H, s), 1.42-1.48 (2H, m), 1.52-1.61 (2H, m), 1.85 (1H, br s), 2.68 (2H, t, J = 7.2 Hz), 3.06-3.07 (2H, m), 3.39-3.44 (3H, m), 3.51-3.55 (3H, m), 3.78 (6H, s), 6.80-6.85 (4H, m), 7.17-7.23 (1H, m), 7.27-7.33 (6H, m), 7.41-7.43 (2H, m).

### Reference Example 18

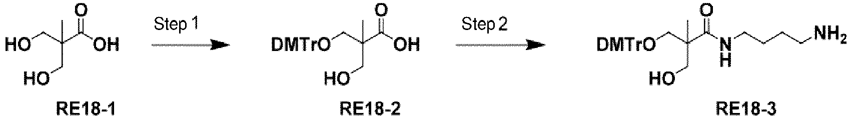

### Step 1 of Reference Example 18

A crude product of compound RE18-2 (1.5 g) was obtained in the same way as in step 1 of Reference Example 2 using compound RE18-1 (manufactured by Tokyo Chemical Industry Co., Ltd., 0.500 g, 3.73 mmoL).
ESI-MS m/z: 435 (M - H)-

### Step 2 of Reference Example 18

Compound RE18-3 (0.18 g, 2-step yield: 10%) was obtained in the same way as in step 3 of Reference Example 3 using a crude product of compound RE18-2 (1.5 g) and 1,4-diaminobutane (manufactured by Tokyo Chemical Industry Co., Ltd., 3.29 g, 37.3 mmol).
ESI-MS m/z: 551 (M+HCOO)⁻
¹H-NMR (400 MHz, MeOD) : δ 1.09 (3H, s), 1.45-1.52 (4H, m), 2.80 (2H, t, J = 7.2 Hz), 2.91 (2H, s), 3.05 (1H, d, J = 8.8 Hz), 3.12-3.16 (4H, m), 3.24 (1H, s), 3.43 (1H, d, J = 10.8 Hz), 3.62-3.66 (7H, m), 6.71-6.76 (4H, m), 7.05-7.11 (1H, m), 7.12-7.20 (6H, m), 7.28-7.32 (2H, m).

### Reference Example 19

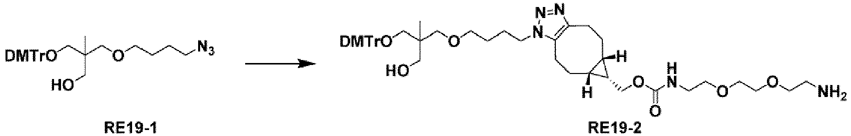

Compound RE19-1 (110 mg, 0.212 mmol) synthesized by the method described in Journal of Organic Chemistry, Vol. 74, p. 6837-6842, 2009 was dissolved in tetrahydrofuran (2 mL). To the solution, N-(1R,8S,9s)-bicyclo[6.1.0]non-4-yn-9-ylmethyloxycarbonyl-1,8-diamino-3,6-dioxaoctane (manufactured by Tokyo Chemical Industry Co., Ltd., 72 mg, 0.222 mmol) was added, and the mixture was stirred at room temperature for 1 hour. Water was added to the reaction solution, followed by extraction with chloroform. Then, the organic layer was washed with saturated saline and dried over anhydrous magnesium sulfate. The solvent was distilled off under reduced pressure, and the residue was purified by amino silica gel column chromatography (chloroform/methanol = 90/10) to obtain compound RE19-2 (160 mg, yield: 90%). ESI-MS m/z: 845 (M+H)⁺
¹H-NMR (400 MHz, CDCl₃): δ 0.88 (3H, s), 0.91-1.09 (3H, m), 1.20-1.25 (1H, m), 1.52-1.59 (4H, m), 1.80-1.85 (2H, m), 2.19-2.25 (4H, m), 2.59-2.68 (1H, m), 2.84-2.90 (4H, m), 3.02-3.11 (3H, m), 3.35-3.44 (5H, m), 3.49-3.53 (5H, m), 3.54-3.58 (2H, m), 3.62 (5H, s), 3.78 (6H, s), 4.13 (2H, d, J = 6.4 Hz), 4.21 (2H, t, J = 7.2 Hz), 6.79-6.84 (4H, m), 7.18-7.21 (1H, m), 7.24-7.27 (2H, m), 7.28-7.32 (4H, m), 7.39-7.44 (2H, m).

### Reference Example 20

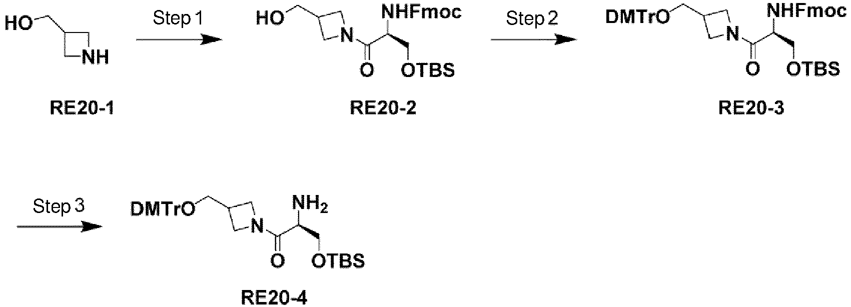

### Step 1 of Reference Example 20

Compound RE20-2 (410 mg, yield: 70%) was obtained in the same way as in step 3 of Reference Example 3 using compound RE20-1 (manufactured by AstaTech Inc., 100 mg, 1.148 mmol) and Fmoc-Ser(tBuMe2Si)-OH (manufactured by Watanabe Chemical Industries, Ltd., 532 mg, 1.205 mmol). ESI-MS m/z: 511 (M+H)⁺
¹H-NMR (400 MHz, CDCl₃): δ 0.06 (6H, s), 0.90 (9H, s), 2.76-2.85 (1H, m), 3.65-3.86 (5H, m), 4.02-4.23 (3H, m), 4.32-4.40 (4H, m), 5.55 (1H, d, J = 8.0 Hz), 7.31 (2H, t, J = 7.6 Hz), 7.40 (2H, t, J = 7.6 Hz), 7.59 (2H, d, J = 7.6 Hz), 7.76 (2H, d, J = 7.6 Hz).

### Step 2 of Reference Example 20

A crude product of compound RE20-3 (680 mg) was obtained in the same way as in step 1 of Reference Example 2 using compound RE20-2 (410 mg, 0.803 mmol). ESI-MS m/z: 814 (M + H)⁺

### Step 3 of Reference Example 20

Compound RE20-4 (330 mg, 2-step yield: 70%) was obtained in the same way as in step 5 of Reference Example 2 using a crude product of compound RE20-3 (680 mg).
ESI-MS m/z: 519 (M+H)⁺
¹H-NMR (400 MHz, CDCl₃) : δ 0.02-0.09 (6H, m), 0.89 (9H, d, J = 28.8 Hz), 2.84-2.94 (1H, m), 3.24-3.30 (2H, m), 3.46 (1H, t, J = 7.2 Hz), 3.52-3.68 (2H, m), 3.75-3.80 (1H, m), 3.82 (6H, d, J = 2.4 Hz), 3.89-3.96 (1H, m), 4.05-4.17 (1H, m), 4.27-4.37 (1H, m), 6.82-6.89 (4H, m), 7.22-7.27 (1H, m), 7.29-7.34 (6H, m), 7.41-7.45 (2H, m)

### Reference Example 21

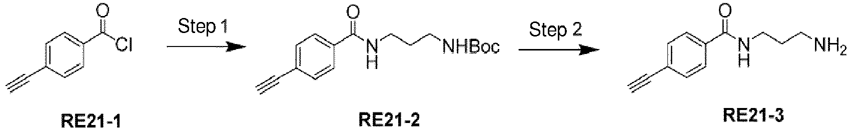

### Step 1 of Reference Example 21

N-(tert-Butoxycarbonyl)-1,3-diaminopropane (manufactured by Tokyo Chemical Industry Co., Ltd., 1.788 g, 10.26 mmol) was dissolved in dichloromethane (22.8 mL). To the solution, triethylamine (1.907 mL, 13.68 mmol) was added, and the mixture was stirred at room temperature for 15 minutes. A solution of compound RE21-1 (1.126 g, 6.84 mmol) synthesized by the method described in Organic Letter, Vol. 16, p. 6318-6321, 2014 in dichloromethane (5 mL) was added dropwise to the reaction solution, and the mixture was stirred at room temperature for 2 hours. Water was added to the reaction solution, followed by extraction with chloroform. Then, the organic layer was washed with saturated saline and dried over anhydrous magnesium sulfate. The solvent was distilled off under reduced pressure, and the residue was purified by silica gel column chromatography (heptane/ethyl acetate = 35/65) to obtain compound RE21-2 (1.65 g, yield: 80%).
ESI-MS m/z: 303 (M + H)⁺

### Step 2 of Reference Example 21

Compound RE21-3 (1.10 g, yield: 100%) was obtained in the same way as in step 2 of Reference Example 1 using compound RE21-2 (1.65 g, 5.46 mmol).
ESI-MS m/z: 203 (M+H)⁺
¹H-NMR (400 MHz, CDCl₃) : δ 1.74 (2H, dt, J = 12.0, 6.0 Hz), 2.95 (2H, t, J = 6.0 Hz), 3.18 (1H, s), 3.60 (2H, td, J = 6.0, 5.2 Hz), 7.54 (2H, dt, J = 8.4, 1.8 Hz), 7.76 (2H, dt, J = 8.4, 1.8 Hz), 7.97 (1H, br s).

### Reference Example 22

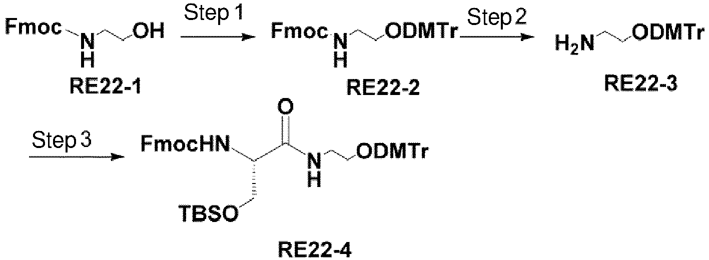

### Step 1 of Reference Example 22

A crude product of compound RE22-2 was obtained in the same way as in step 1 of Reference Example 2 using compound RE22-1 (manufactured by Tokyo Chemical Industry Co., Ltd., 1.2 g, 4.24 mmol).
ESI-MS m/z: 608 (M + Na)⁺

### Step 2 of Reference Example 22

Compound R22-3 (1.34 g, 2-step yield: 52%) was obtained in the same way as in step 5 of Reference Example 2 using a crude product of compound RE22-2. ESI-MS m/z: 386 (M+Na)⁺
¹H-NMR (400 MHz, CDCl₃): δ 3.34 (2H, t, J = 6.4 Hz), 3.47 (2H, t, J = 6.4 Hz), 3.79 (6H, s), 6.78-6.84 (4H, m), 7.17-7.21 (1H, m), 7.27-7.35 (6H, m), 7.42-7.46 (2H, m).

### Step 3 of Reference Example 22

Compound RE22-4 (560 mg, yield: 31%) was obtained in the same way as in step 3 of Reference Example 3 using compound RE22-3 (1.15 g, 3.16 mmol) and Fmoc-Ser(tBuMe2Si)-OH (manufactured by Watanabe Chemical Industries, Ltd., 1.677 g, 3.8 mmol).
1H-NMR (400 MHz, CDCl₃) δ: 0.00-0.07 (6H, m), 0.83-0.89 (9H, m), 3.18-3.26 (2H, m), 3.39-3.46 (2H, m), 3.61-3.68 (1H, m), 3.76 (6H, s), 3.89 (1H, dd, J = 10.0, 4.0 Hz), 4.03 (1H, dd, J = 10.0, 4.0 Hz), 4.15-4.20 (1H, m), 4.22-4.28 (1H, m), 4.32-4.40 (2H, m), 5.65-5.88 (1H, m), 6.76-6.85 (4H, m), 7.16-7.23 (1H, m), 7.25-7.34 (8H, m), 7.36-7.44 (4H, m), 7.50-7.64 (2H, m), 7.72-7.79 (2H, m)·

### Reference Example 23

Compounds RE23-6 to RE23-10 described in Table Y-2 were obtained in the same way as in Reference Example 22 using compounds RE23-1 to RE23-5 described in Table Y-1 and Fmoc-Ser(tBuMe₂Si)-OH.

Compound RE23-11 described in Table Y-2 was obtained in the same way as in Reference Example 22 using compound RE22-3 of Reference Example 22 and Fmoc-Thr(tBuMe₂Si)-OH.

Compound RE23-12 described in Table Y-2 was obtained in the same way as in Reference Example 22 using compound RE23-1 described in Table Y-1 and Fmoc-Thr(tBuMe₂Si)-OH.

NMR analysis data on the compounds synthesized in this Example are shown in Table Y-3.

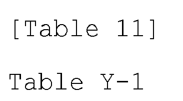

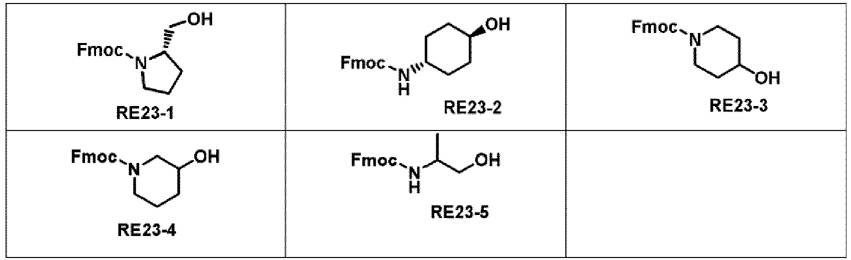

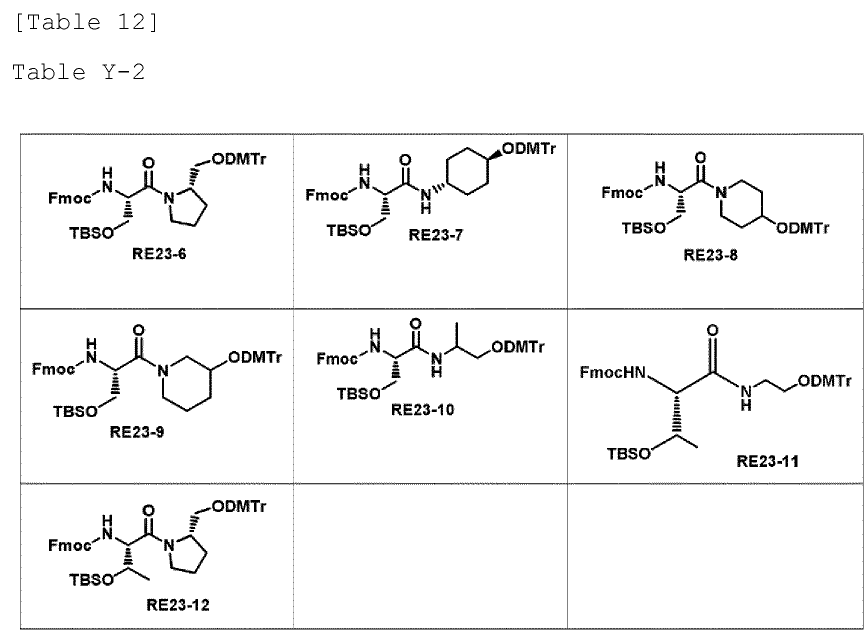

### [Table 13]

**Table Y-3**

| Compounds of Reference Example 23 | NMR spectrum |
|---|---|
| RE23-6 | ¹H-NMR (400 MHz, CDCl₃): δ 0.07-0.03 (6H, m), 0. 89-0.87 (9H, m H), 2.0 4-1.65 (4H, m), 3.86-3.58 (11H, m), 4.37-4. 18 (4H, m), 4.69-4.68 (1 H, m), 5.67-5.65 (1H, m), 6.84-6.80 (4H, m),7.18-7.16 (3H, m), 7.3 3-7.25 (7H, m), 7.42-7.38 (3H, m), 7.60-7.58 (2H, m), 7.78-7.75 (2H, m). |
| RE23-7 | ¹H NMR (400 MHz, CDCl₃) : δ 0.11-0.05 (6H, m), 0.91-0.87 (9H, m), 1.43-1.19 (4H, m), 2.00-1.80 (311, m), 3.74-3.36 (211, m), 3.81-3.75 (611, m), 4.41-3.96 (5H, m), 5.71-5.64 (111, m), 6.37-6.36 (111, m), 6.84-6.80 (4H, m), 7.20-7.16 (2H, m), 7.42-7.25 (10H, m), 7.59-7.48 (3H, m), 7.78-7.75 (2H, m). |
| RE23-8 | ¹H-NMR (400 MHz, CDCl₃): δ 0.08-0.03 (6H, m), 0.98-0.81 (9H, m), 1.20-1.07 (211, m), 1.39-1.30 (2H, m), 1.42-1.40 (111, m), 1.71-1.50 (2H, m), 3.36-3.14 (1H, m), 3.73-3.60 (3H, m), 3.80-3.77 (6H, m), 4.33-4.18 (1H, m), 4.35-4.34 (2H, m), 4.78-4.77 (1H, m), 5.75-5.74 (1H, m), 6.83-6.81 (4H, m), 7.35-7.26 (5H, m), 7.39-7.37 (611, m), 7.50-7.48 (211, m), 7.61-7.57 (2H, m), 7.77-7.74 (2H, m) |
| RE23-9 | ¹H-NMR (400 MHz, CDCl₃) : δ 0.04-0.00 (611, m), 0.87-0.80 (9H, m), 1.47-1.11 (3H, m), 1.92-1.61 (1H, m), 4.83-2.99 (16H, m), 5.88-5.72 (1H, m), 6.89-6.82 (4H, m), 7.21-7.14 (1H, m),7.49-7.28 (12H, m), 7.62-7.59 (2H, m), 7.77-7.75 (2H, m) |
| RE23-10 | ¹H-NMR (400 MHz, CDCl₃): δ 0.01-0.05 (6H, m), 0.66-0.92 (911, m), 1.11-1.12 (3H, m), 3.02-3.06 (1H, m), 3.55-3.62 (1H, m), 3. 69-3.75 (6H, m), 3.91-4.35 (6H, m), 5.62 (1H, s), 6.74-6.78 (4H, m), 7.10-7.12 (2H, m), 7.14-7.27 (7H, m), 7.30-7.36 (4H, m), 7.43-7.53 (2H, m), 7. 68-7.71 (2H, m). |
| RE23-11 | ¹H-NMR (400 MHz, CDCl₃): δ 0.14-0.08 (611, m), 1.10-0.87 (9H, m), 1.56-1.55 (3H, m), 3.48-3.41 (2H, m), 3.81-3.73 (7H, m), 4.25-4.14 (2H, m), 4.45-4.39 (3H, m), 5.77-5.76 (1H, m), 6.84-6.79 (4H, m), 7.18-7.16 (3H, m), 7.42-7.26 (10H, m), 7.61-7.56 (211, m), 7.78-7.74 (211, m). |
| RE23-12 | ¹H-NMR (400 MHz, CDCl₃): δ-0.10-0.00 (6H, m), 0.81-0.72 (9H, m), 1.12 -1.11 (3H, m), 1.47-1. 54 (1H, m), 1. 83-1.77 (3H, m), 3.71-3.47 (9H, m), 4.39-4.00 (6H, m) , 5.55-5. 53 (1H, m), 6. 74-6.71 (4H, m), 7.08-7.06 (3H, m), 7.32-7.16 (10H, m), 7.52-7.48 (2H, m), 7.67-7.65 (2H, m). |

### Reference Example 24

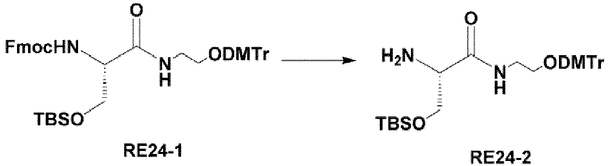

Compound RE24-2 (1.2 g, yield: 67%) was obtained in the same way as in step 2 of Reference Example 2 using compound RE24-1 (compound RE22-4 in Reference Example 22, 2.487 g, 3.16 mmol) synthesized by the method described in Reference Example 22.
ESI-MS m/z: 587(M+Na)⁺
¹H-NMR (400 MHz, CDCl₃):δ -0.01-0.07 (6H, m), 0.86-0.90 (9H, m), 3.15-3.21 (2H, m), 3.41-3.48 (3H, m), 3.72 (1H, dd, J = 10.0, 6.4 Hz), 3.79 (6H, s), 3.84 (1H, dd, J = 10.0, 4.8 Hz), 6.79-6.84 (4H, m), 7.18-7.23 (1H, m), 7.27-7.33 (6H, m), 7.40-7.44 (2H, m), 7.72-7.75 (1H, br m) .

### Reference Example 25

Compounds RE25-1 to RE25-7 described in Table Z-1 were obtained in the same way as in Reference Example 24 using compounds RE23-6 to RE23-12 described in Table Y-2.

The mass spectrometry results of the compounds synthesized in this Example are shown in Table Z-2.

### [Table 14]

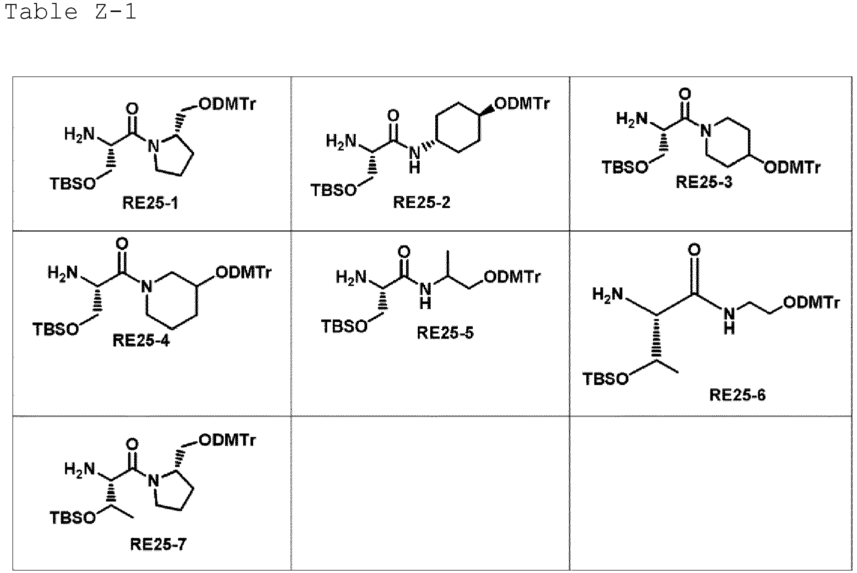

### [Table 15]

**Table Z-2**

| Compounds of Reference Example 25 | ESI-MS m/z |
|---|---|
| RE25-1 | 605 (M+H)+ |
| RE25-2 | 619 (M+H)+ |
| RE25-3 | 303 (M+H)+, DMTr-deprotected product detected |
| RE25-4 | 649 (M+HCOO)- |
| RE25 5 | 577 (M-H) |
| RE25-6 | 623 (M+HCOO)- |
| RE25-7 | 317 (M+H)+, DMTr-deprotected product detected |

### Reference Example 26

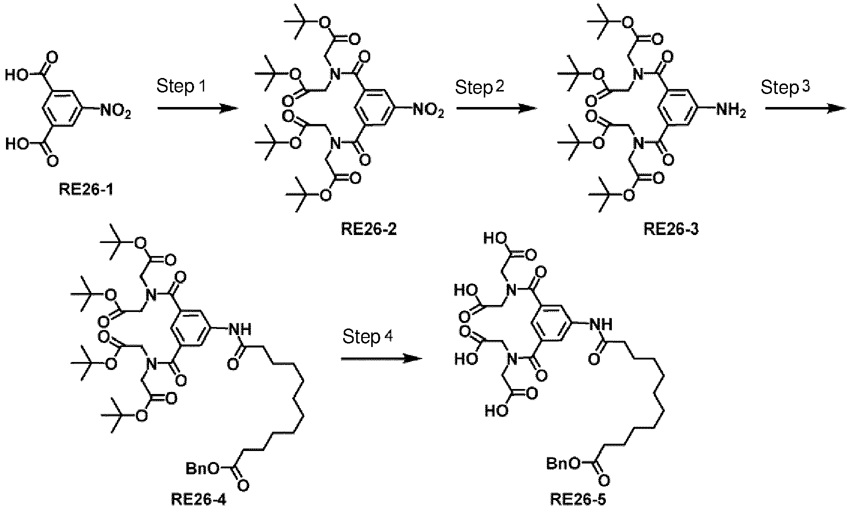

### Step 1 of Reference Example 26

Compound RE26-1 (2.00 g, 9.47 mmol) was dissolved in N,N'-dimethylformamide (40 mL). To the solution, iminodiacetic acid di-tert-butyl ester (5.11 g, 20.84 mmol), 1-(3-dimethylaminopropyl)-3-ethylcarbodiimide hydrochloride (4.00 g, 20.84 mmol), and 1-hydroxybenzotriazole monohydrate (145 mg, 0.947 mmol) were added at room temperature, and the mixture was stirred for 2 hours. Water was added to the reaction solution, followed by extraction with ethyl acetate. Then, the organic layer was washed with a saturated aqueous solution of sodium bicarbonate and saturated saline and dried over anhydrous sodium sulfate. The solvent was distilled off under reduced pressure, and the residue was purified by silica gel column chromatography (heptane/ethyl acetate = 50/50). The purified product was further slurry-purified with methanol to obtain compound RE26-2 (4.07 g, yield: 65%).
ESI-MS m/z: 664 (M - H)⁻

### Step 2 of Reference Example 26

Compound RE26-2 (2.66 g, 4.00 mmol) was dissolved in tetrahydrofuran (53 mL). To the solution, a 10% palladium-carbon powder (water-containing product, 54.29%; 490 mg) was added at room temperature, and the mixture was stirred for 3 hours in a hydrogen atmosphere. The reaction solution was filtered, and the solvent was distilled off under reduced pressure to obtain compound RE26-3 (2.86 g, yield: 113%).
ESI-MS m/z: 634 (M - H)⁻

### Step 3 of Reference Example 26

Compound RE26-3 (871.0 mg, 1.370 mmol) was dissolved in N,N'-dimethylformamide (17 mL). To the solution, O-(7-azabenzotriazol-1-yl)-N,N,N',N'-tetramethyluronium hexafluorophosphate (625.0 mg, 1.644 mmol), diisopropylethylamine (0.5730 mL, 3.290 mmol), and dodecanedioic acid monobenzyl ester (527.0 mg, 1.644 mmol) were added, and the mixture was stirred overnight at room temperature. Water was added to the reaction solution, followed by extraction with ethyl acetate. Then, the organic layer was washed with a saturated aqueous solution of sodium bicarbonate and saturated saline and dried over anhydrous sodium sulfate. The solvent was distilled off under reduced pressure, and the residue was purified by silica gel column chromatography (heptane/ethyl acetate = 60/40) to obtain compound RE26-4 (1.030 g, yield: 80%).
ESI-MS m/z: 939 (M + H)⁺

### Step 4 of Reference Example 26

Compound RE26-4 (1.030 g, 1.098 mmol) was dissolved in dichloromethane (10 mL). To the solution, trifluoroacetic acid (10.00 mL, 130.0 mmol) was added, and the mixture was stirred at room temperature for 1 hour. The solvent was distilled off under reduced pressure to obtain a crude product of compound RE26-5. ESI-MS m/z: 713 (M - H)⁻

### Reference Example 27

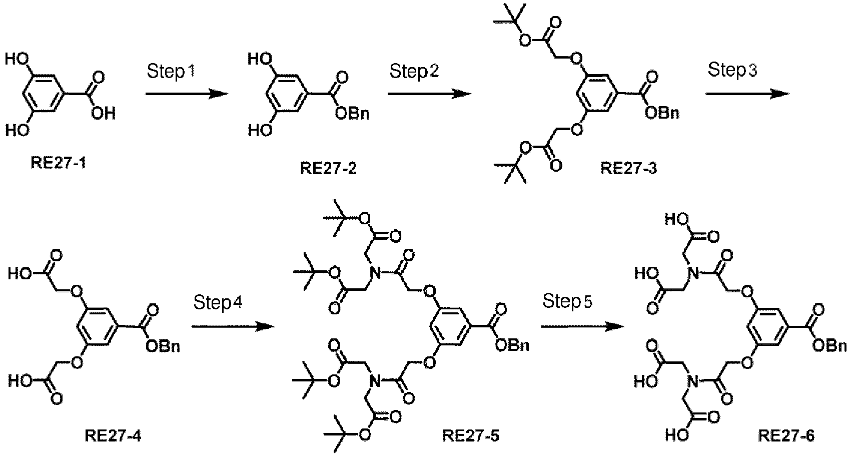

### Step 1 of Reference Example 27

Compound RE27-1 (2.000 g, 12.98 mmol) was dissolved in N,N'-dimethylformamide (30 mL). To the solution, potassium bicarbonate (1.559 g, 15.57 mmol) and benzyl chloride (2.328 mL, 19.47 mmol) were added, and the mixture was stirred at room temperature for 4 hours. Saturated ammonium chloride aqueous solution was added to the reaction solution, followed by extraction with dichloromethane. Then, the organic layer was washed with water and dried over anhydrous sodium sulfate. The solvent was distilled off under reduced pressure, and the residue was purified by silica gel column chromatography (heptane/ethyl acetate = 50/50) to obtain compound RE27-2 (2.850 g, yield: 90%).
ESI-MS m/z: 243 (M - H)⁻

### Step 2 of Reference Example 27

Compound RE27-2 (2.500 g, 10.24 mmol) was dissolved in N,N'-dimethylformamide (30 mL). To the solution, potassium carbonate (5.660 g, 40.90 mmol) and tert-butyl bromoacetic acid (3.300 mL, 22.52 mmol) were added, and the mixture was stirred at 90°C for 4 hours. Saturated ammonium chloride aqueous solution was added to the reaction solution, followed by extraction with dichloromethane. Then, the organic layer was washed with saturated saline and dried over anhydrous sodium sulfate. The solvent was distilled off under reduced pressure, and the residue was purified by silica gel column chromatography (heptane/ethyl acetate = 75/25) to obtain compound RE27-3 (4.300 g, yield: 89%).
ESI-MS m/z: 472 (M - H)⁻

### Step 3 of Reference Example 27

Compound RE27-3 (1.000 g, 2.116 mmol) was dissolved in dichloromethane (10 mL). To the solution, trifluoroacetic acid (10.00 mL, 130.0 mmol) was added, and the mixture was stirred at room temperature for 6 hours. The solvent was distilled off under reduced pressure to obtain a crude product of compound RE27-4. ESI-MS m/z: 359 (M - H)⁻

### Step 4 of Reference Example 27

Compound RE27-4 (350.0 mg, 0.9710 mmol) was dissolved in N,N'-dimethylformamide (7 mL). To the solution, 1-hydroxybenzotriazole monohydrate (327.0 mg, 2.137 mmol), 1-(3-dimethylaminopropyl)-3-ethylcarbodiimide hydrochloride (410.0 mg, 2.137 mmol), and iminodiacetic acid di-tert-butyl ester (524.0 mg, 2.137 mmol) were added, and the mixture was stirred at room temperature for 5 hours. Water was added to the reaction solution, followed by extraction with ethyl acetate. Then, the organic layer was washed with a saturated aqueous solution of sodium bicarbonate and saturated saline and dried over anhydrous sodium sulfate. The solvent was distilled off under reduced pressure, and the residue was purified by silica gel column chromatography (heptane/ethyl acetate = 60/40) to obtain compound RE27-5 (617.0 mg, yield: 78%).
ESI-MS m/z: 814 (M - H)⁻

### Step 5 of Reference Example 27

Compound RE27-5 (610.0 mg, 0.7490 mmol) was dissolved in dichloromethane (6 mL). To the solution, trifluoroacetic acid (6 mL, 78.00 mmol) was added, and the mixture was stirred at room temperature for 1 hour. The solvent was distilled off under reduced pressure to obtain a crude product of compound RE27-6.
ESI-MS m/z: 590 (M + H)⁺

### Reference Example 28

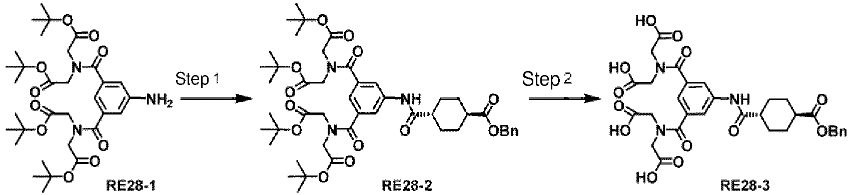

### Step 1 of Reference Example 28

Compound RE28-1 (compound RE26-3 in Reference Example 26, 474 mg, 0.744 mmol) synthesized by the method described in Reference Example 26 was dissolved in N,N'-dimethylformamide (10 mL). To the solution, trans-cyclohexane-1,4-dicarboxylic acid monobenzyl ester (0.234 mg, 0.893 mmol) synthesized by the method described in Journal of Medicinal Chemistry, Vol. 54, p. 2433-2446, 2011, diisopropylethylamine (0.312 mL, 1.79 mmol), and O-(7-azabenzotriazol-1-yl)-N,N,N',N'-tetramethyluronium hexafluorophosphate (339 mg, 0.893 mmol) were added at room temperature, and the mixture was stirred for 6 hours. Water was added to the reaction solution, followed by extraction with ethyl acetate. Then, the organic layer was washed with a saturated aqueous solution of sodium bicarbonate and saturated saline and dried over anhydrous sodium sulfate. The solvent was distilled off under reduced pressure, and the residue was purified by silica gel column chromatography (heptane/ethyl acetate = 50/50) to obtain compound RE28-2 (448 mg, yield: 68%).
ESI-MS m/z: 879 (M - H)⁻

### Step 2 of Reference Example 28

Compound RE28-2 (341 mg, 0.387 mmol) was dissolved in dichloromethane (3.4 mL). To the solution, trifluoroacetic acid (3.4 mL) was added at room temperature, and the mixture was stirred overnight. The reaction solution was concentrated under reduced pressure and subjected to azeotropy with ethyl acetate, and the residue was slurry-purified with heptane to obtain compound RE28-3 (254 mg, yield: 100%).
ESI-MS m/z: 656 (M + H)⁺

### Reference Example 29

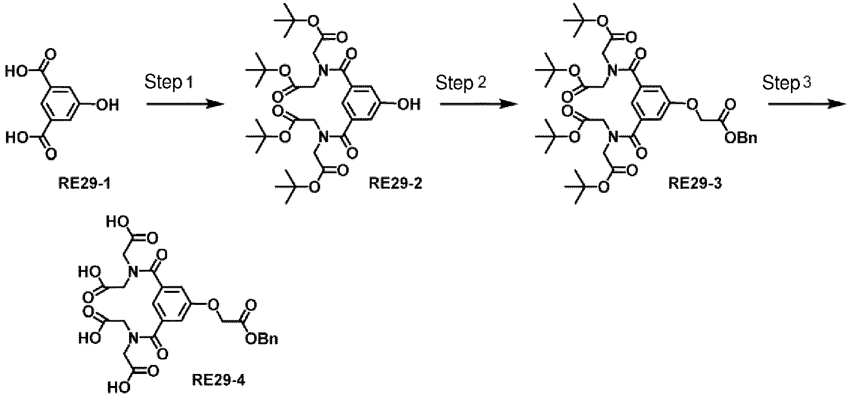

### Step 1 of Reference Example 29

Compound RE29-1 (500 mg, 2.75 mmol) was dissolved in N,N'-dimethylformamide (10 mL). To the solution, iminodiacetic acid di-tert-butyl ester (1.48 g, 6.04 mmol), 1-(3-dimethylaminopropyl)-3-ethylcarbodiimide hydrochloride (1.16 g, 6.04 mmol), and 1-hydroxybenzotriazole monohydrate (42.0 mg, 0.275 mmol) were added at room temperature, and the mixture was stirred for 4 hours. Water was added to the reaction solution, followed by extraction with ethyl acetate. Then, the organic layer was washed with saturated saline and dried over anhydrous sodium sulfate. The solvent was distilled off under reduced pressure, and the residue was purified by silica gel column chromatography (heptane/ethyl acetate = 50/50) to obtain compound RE29-2 (329 mg, yield: 19%).
ESI-MS m/z: 635 (M - H)⁻

### Step 2 of Reference Example 29

Compound RE29-2 (323 mg, 0.507 mmol) was dissolved in N,N'-dimethylformamide (6.5 mL). To the solution, potassium carbonate (84.0 mg, 0.609 mmol) and benzyl bromoacetate (139 mg, 0.609 mmol) were added at room temperature, and the mixture was stirred for 3 hours. Water was added to the reaction solution, followed by extraction with ethyl acetate. Then, the organic layer was washed with saturated saline and dried over anhydrous sodium sulfate. The solvent was distilled off under reduced pressure, and the residue was purified by silica gel column chromatography (heptane/ethyl acetate = 50/50) to obtain compound RE29-3 (313 mg, yield: 79%).
ESI-MS m/z: 783 (M - H)-

### Step 3 of Reference Example 29

Compound RE29-3 (312 mg, 0.398 mmol) was dissolved in dichloromethane (3.1 mL). To the solution, trifluoroacetic acid (3.1 mL) was added at room temperature, and the mixture was stirred overnight. The reaction solution was concentrated under reduced pressure and subjected to azeotropy with ethyl acetate to obtain compound RE29-4 (252 mg, quantitative).
ESI-MS m/z: 561 (M + H)⁺

### Reference Example 30

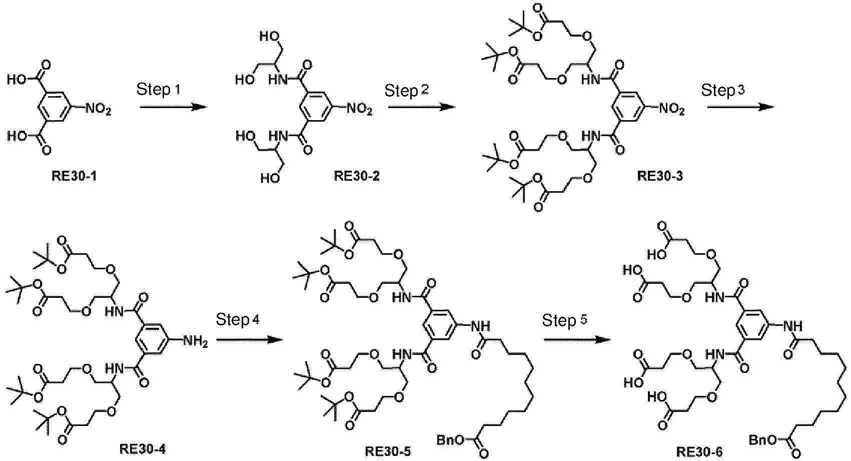

### Step 1 of Reference Example 30

Compound RE30-1 (2.00 g, 9.47 mmol) was dissolved in N,N'-dimethylformamide (40 mL). To the solution, 2-amino-1,3-propanediol (1.90 g, 20.84 mmol), 1-(3-dimethylaminopropyl)-3-ethylcarbodiimide hydrochloride (4.00 g, 20.84 mmol), and 1-hydroxybenzotriazole monohydrate (145 mg, 0.947 mmol) were added at room temperature, and the mixture was stirred for 2 hours.
The solvent was distilled off under reduced pressure, and the residue was purified by silica gel column chromatography (ethyl acetate/methanol = 70/30). The purified product was further slurry-purified with ethyl acetate to obtain compound RE30-2 (2.68 g, yield: 79%). ESI-MS m/z: 356 (M - H)⁻

### Step 2 of Reference Example 30

Compound RE30-2 (500 mg, 1.40 mmol) was suspended in acetonitrile (10 mL). To the suspension, acrylic acid tert-butyl ester (3.59 g, 28.0 mmol) and benzyltrimethylammonium hydroxide (40% aqueous solution; 1.76 mL, 702 mmol) were added at room temperature, and the mixture was stirred overnight. The solvent was distilled off under reduced pressure, and water was added to the residue, followed by extraction with ethyl acetate. Then, the organic layer was washed with saturated saline and dried over anhydrous sodium sulfate. The solvent was distilled off under reduced pressure, and the residue was purified by silica gel column chromatography (heptane/ethyl acetate = 50/50) to obtain compound RE30-3 (300 mg, yield: 24%).
ESI-MS m/z: 871 (M + H)⁺

### Step 3 of Reference Example 30

Compound RE30-3 (340 mg, 0391 mmol) was dissolved in tetrahydrofuran (6.8 mL). To the solution, a 10% palladium-carbon powder (water-containing product, 54.29%; 31.3 mg) was added at room temperature, and the mixture was stirred for 6 hours in a hydrogen atmosphere. The reaction solution was filtered, and the solvent was distilled off under reduced pressure. The residue was purified by silica gel column chromatography (heptane/ethyl acetate = 30/70) to obtain compound RE30-4 (235 mg, yield: 72%).
ESI-MS m/z: 841 (M + H)⁺

### Step 4 of Reference Example 30

Compound RE30-4 (232 mg, 0.276 mmol) was dissolved in N,N'-dimethylformamide (4.6 mL). To the solution, dodecanoic acid monobenzyl ester (0.133 mg, 0.414 mmol), diisopropylethylamine (0.145 mL, 0.829 mmol), and O-(7-azabenzotriazol-1-yl)-N,N,N',N'-tetramethyluronium hexafluorophosphate (158 mg, 0.414 mmol) were added at room temperature, and the mixture was stirred overnight. Water was added to the reaction solution, followed by extraction with ethyl acetate. Then, the organic layer was washed with a saturated aqueous solution of sodium bicarbonate and saturated saline and dried over anhydrous sodium sulfate. The solvent was distilled off under reduced pressure, and the residue was purified by silica gel column chromatography (heptane/ethyl acetate = 30/70) to obtain compound RE30-5 (274 mg, yield: 87%).
ESI-MS m/z: 1141 (M - H)⁻

### Step 5 of Reference Example 30

Compound RE30-5 (273 mg, 0.239 mmol) was dissolved in dichloromethane (2.7 mL). To the solution, trifluoroacetic acid (2.7 mL) was added at room temperature, and the mixture was stirred overnight. The reaction solution was concentrated under reduced pressure and subjected to azeotropy with ethyl acetate to obtain compound RE30-6 (231 mg, quantitative).
ESI-MS m/z: 919 (M + H)⁺

### Reference Example 31

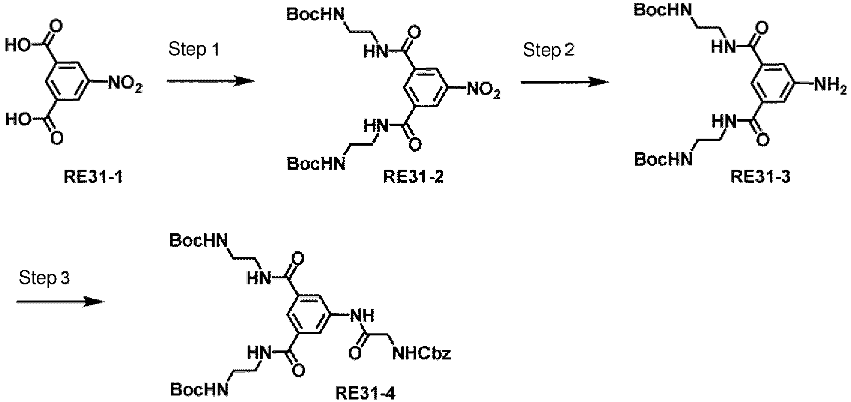

### Step 1 of Reference Example 31

4-Nitroisophthalic acid RE31-1 (500 mg, 2.37 mmol) and N-Boc-ethylenediamine (808 mg, 5.21 mmol) were dissolved in N,N'-dimethylformamide (10 mL). To the solution, triethylamine (0.90 mL, 7.11 mmol), 1-hydroxybenzotriazole monohydrate (703 mg, 5.21 mmol) and 1-(3-dimethylaminopropyl)-3-ethylcarbodiimide hydrochloride (1.36 g, 7.11 mmol) were added at room temperature, and the mixture was stirred for 16 hours. The reaction solution was aftertreated, and the crude product was purified by silica gel column chromatography to obtain compound RE31-2 (650 mg, yield: 55%).

### Step 2 of Reference Example 31

Compound RE31-2 (500 mg, 1.01 mmol) and a zinc powder (330 mg, 5.05 mmol) were suspended in methanol (3.5 mL) and tetrahydrofuran (3.5 mL). To the suspension, an aqueous solution of ammonium chloride (378 mg, 7.07 mmol) was added dropwise at 0°C, and the mixture was stirred at room temperature for 24 hours. The reaction solution was aftertreated, and the crude product was purified by silica gel column chromatography to obtain compound RE31-3 (160 mg, yield: 34%).

### Step 3 of Reference Example 31

Compound RE31-3 (200 mg, 0.430 mmol) and N-benzyloxycarbonyl-glycine (benzyloxycarbonyl is also referred to as Cbz) (90.0 mg, 0.430 mmol) were dissolved in N,N'-dimethylformamide (2.0 mL). To the solution, diisopropylethylamine (0.220 mL, 1.29 mmol) and O-(7-azabenzotriazol-1-yl)-N,N,N',N'-tetramethyluronium hexafluorophosphate (245 mg, 0.645 mmol) were added at room temperature, and the mixture was stirred for 16 hours. The reaction solution was aftertreated, and the crude product was purified by silica gel column chromatography to obtain compound RE31-4 (180 mg, yield: 64%) .
ESI-MS m/z: 657 (M + H)⁺

### Reference Example 32

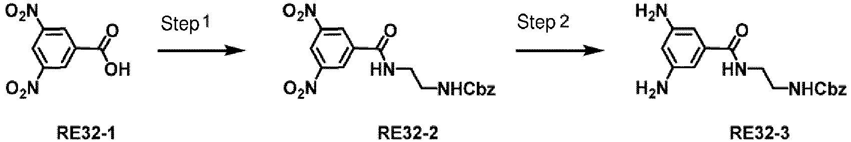

### Step 1 of Reference Example 32

3,5-Dinitrobenzoic acid RE32-1 (500 mg, 2.36 mmol) and N-Cbz-ethylenediamine (588 mg, 2.83 mmol) were dissolved in N,N'-dimethylformamide (5.0 mL). To the solution, triethylamine (0.65 mL, 4.72 mmol), 1-hydroxybenzotriazole monohydrate (380 mg, 2.83 mmol) and 1-(3-dimethylaminopropyl)-3-ethylcarbodiimide hydrochloride (675 mg, 3.54 mmol) were added at room temperature, and the mixture was stirred for 16 hours. The reaction solution was aftertreated, and the crude product was purified by silica gel column chromatography to obtain compound RE32-2 (445 mg, yield: 48%).

### Step 2 of Reference Example 32

Compound RE32-2 (200 mg, 0.515 mmol) was dissolved in ethanol (5.0 mL). To the solution, tin(II) chloride (584 mg, 3.09 mmol) and concentrated hydrochloric acid (0.2 mL) were added at room temperature, and the mixture was stirred at 80°C for 16 hours. The reaction solution was aftertreated to obtain compound RE32-3 (180 mg, quantitative).
ESI-MS m/z: 329 (M + H)⁺

### Reference Example 33

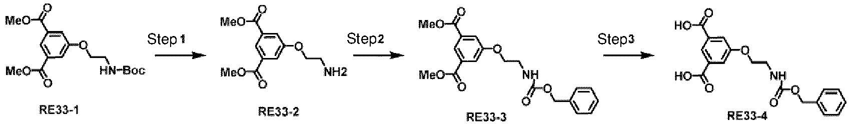

### Step 1 of Reference Example 33

Compound RE33-2 (3.7 g, yield: 63%) was obtained in the same way as in step 4 of Reference Example 3 using compound RE33-1 (compound RE3-2 in Reference Example 3, 8.17 g, 23.12 mmol) synthesized by the method described in Reference Example 3.
ESI-MS m/z: 254(M+H)⁺

### Step 2 of Reference Example 33

Compound RE33-3 (3.82 g, yield: 67%) was obtained in the same way as in step 5 of Reference Example 3 using compound RE33-2 (3.7 g, 14.63 mmol).
ESI-MS m/z: 432(M+HCOO)⁻

### Step 3 of Reference Example 33

Compound RE33-4 (3.08 g, yield: 87%) was obtained in the same way as in step 2 of Reference Example 1 using compound RE33-3 (3.82 g, 9.86 mmol).
ESI-MS m/z: 360(M+H)⁺

### Reference Example 34

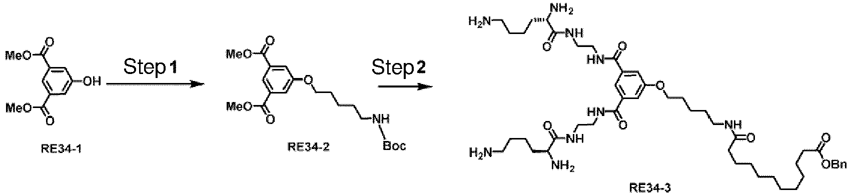

### Step 1 of Reference Example 34

Compound RE34-2 (2.40 g, yield: 63%) was obtained in the same way as in step 1 of Reference Example 3 using compound RE34-1 (2 g, 9.53 mmol) and tert-butoxycarbonylamino)-1-pentanol (manufactured by Tokyo Chemical Industry Co., Ltd., 2 g, 10 mmol).
ESI-MS m/z: 296(M+H)⁺, detected as a Boc-deprotected form

### Step 2 of Reference Example 34

Compound RE34-3 (1.579 g, yield: 21%) was obtained in the same way as in steps 2 to 4 of Reference Example 3 and steps 1 to 4 of Reference Example 4 using compound RE34-2.
ESI-MS m/z: 910 (M+H)⁺

### Reference Example 35: Synthesis of compound D2

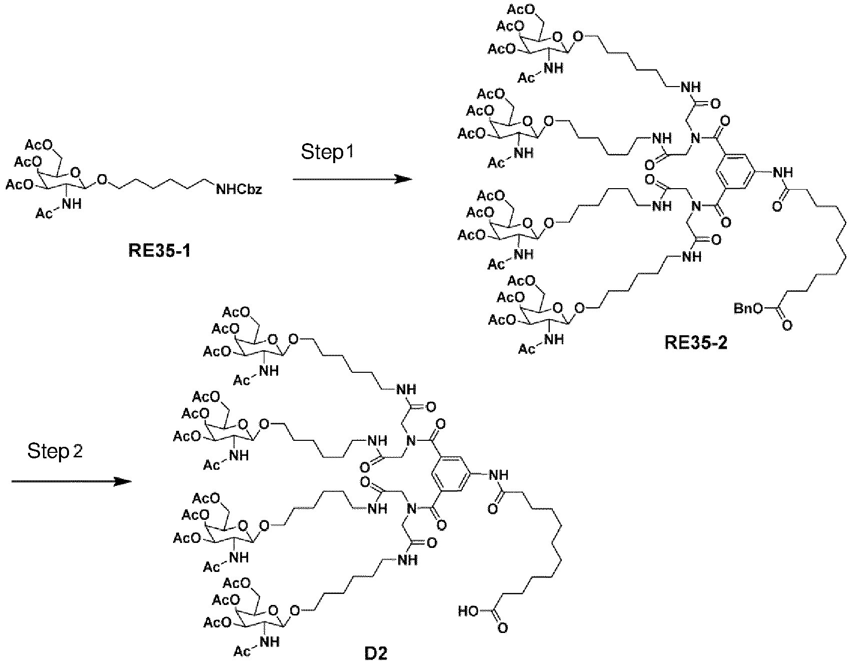

### Step 1 of Reference Example 35

Compound RE35-1 (compound RE11-2 in Reference Example 11, 1.015 g, 1.748 mmol) synthesized by the method described in Reference Example 11 was dissolved in N,N'-dimethylformamide (12 mL). To the solution, a 10% palladium-carbon powder (water-containing product, 54.29%; 187 mg) was added at room temperature, and the mixture was stirred for 6 hours in a hydrogen atmosphere. The reaction solution was filtered. Compound RE26-5 (250.0 mg, 0.350 mmol) synthesized in Reference Example 26, 1-hydroxybenzotriazole monohydrate (26.80 mg, 0.1750 mmol), and 1-(3-dimethylaminopropyl)-3-ethylcarbodiimide hydrochloride (402.0 mg, 2.099 mmol) were added to the filtrate, and the mixture was stirred overnight at room temperature. Water was added to the reaction solution, followed by extraction with ethyl acetate. Then, the organic layer was washed with a saturated aqueous solution of sodium bicarbonate and saturated saline and dried over anhydrous sodium sulfate. The solvent was distilled off under reduced pressure, and the residue was purified by silica gel column chromatography (chloroform/methanol = 87/13) to obtain compound RE35-2 (617.0 mg, yield: 88%).
ESI-MS m/z: 1215 (M + 2H)²⁺

### Step 2 of Reference Example 35

Compound RE35-2 (0.7380 g, 0.3040 mmol) was dissolved in tetrahydrofuran (7 mL). To the solution, a 10% palladium-carbon powder (water-containing product, 54.29%; 135.90 mg) was added at room temperature, and the mixture was stirred overnight in a hydrogen atmosphere. The reaction solution was filtered, and the solvent was distilled off under reduced pressure. The residue was purified by silica gel column chromatography (chloroform/methanol = 87/13) to obtain compound D2 (581 mg, yield: 82%).
ESI-MS m/z: 1170 (M + 2H) ²⁺
¹H-NMR (400 MHz, DMSO-d6, δ): 1.12-2.36 (106H, m), 2.91-3.19 (8H, m), 3.23-3.55 (14H, m), 3.60-3.76 (4H, m), 3.78-3.94 (8H, m), 3.95-4.10 (16H, m), 4.47 (4H, d, J = 8.8 Hz), 4.92-5.01 (4H, m), 5.17-5.24 (4H, m), 6.98 (1H, s), 7.64 (2H, s), 7.81-7.95 (4H, m), 8.28-8.38 (2H, m), 8.44-8.56 (2H, m), 10.13 (1H, s)

### Reference Example 36: Synthesis of compound D3

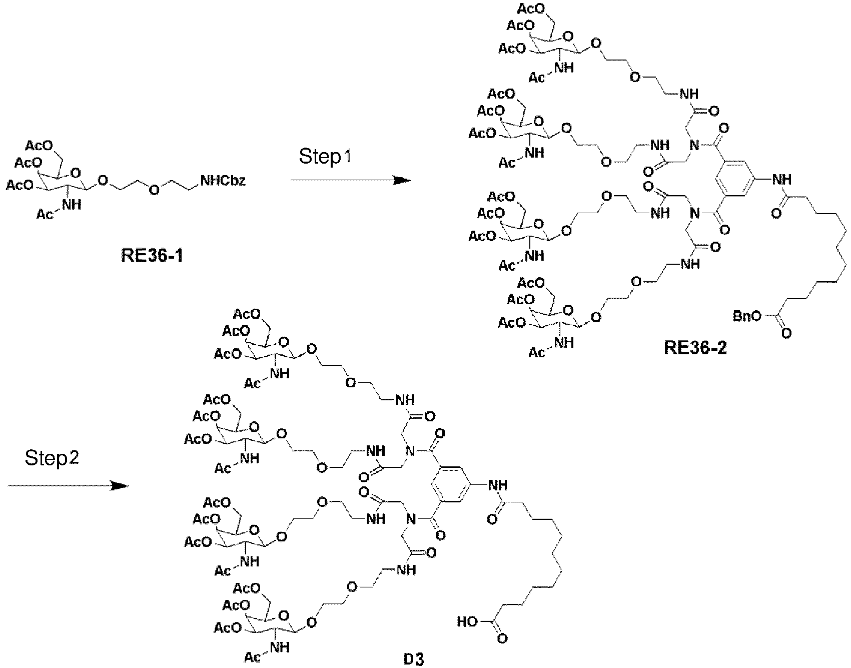

### Step 1 of Reference Example 36

Compound RE36-1 (compound RE12-2 in Reference Example 12, 500 mg, 0.879 mmol) synthesized by the method described in Reference Example 12 was dissolved in N,N'-dimethylformamide (6.5 mL). To the solution, a 10% palladium-carbon powder (water-containing product, 54.29%; 94 mg) was added at room temperature, and the mixture was stirred for 4 hours in a hydrogen atmosphere. The reaction solution was filtered. Compound RE26-5 (126.0 mg, 0.176 mmol) of Reference Example 26, 1-hydroxybenzotriazole monohydrate (13.47 mg, 0.088 mmol), and 1-(3-dimethylaminopropyl)-3-ethylcarbodiimide hydrochloride (202.0 mg, 1.055 mmol) were added to the filtrate, and the mixture was stirred overnight at room temperature. The solvent in the reaction solution was distilled off under reduced pressure, and the residue was purified by reverse-phase column chromatography (water/acetonitrile) to obtain compound RE36-2 (249.7 mg, yield: 60%).
ESI-MS m/z: 1191 (M + 2H)²⁺

### Step 2 of Reference Example 36

Compound RE36-2 (0.242 g, 0.102 mmol) was dissolved in tetrahydrofuran (3.6 mL) and water (1.2 mL). To the solution, a 10% palladium-carbon powder (water-containing product, 54.29%; 45 mg) was added at room temperature, and the mixture was stirred for 4 hours in a hydrogen atmosphere. The reaction solution was filtered, and the solvent was distilled off under reduced pressure to obtain compound D3 (216 mg, yield: 93%).
ESI-MS m/z: 1146 (M + 2H) 2+
1H-NMR (400 MHz, DMSO-d6, δ): 1.15-1.65 (20H, m), 1.68-2.15 (52H, m), 3.13-3.29 (6H, m), 3.40-3.67 (16H, m), 3.71-3.96 (11H, m), 3.98-4.14 (16H, m), 4.55 (4H, t, J = 8.8 Hz), 4.93-5.06 (4H, m), 5.12-5.28 (4H, m), 6.56 (1H, s), 6.98 (1H. s), 7.64 (2H, s), 7.77-7.93 (4H, m), 8.26-8.49 (3H, m), 10.10 (1H, s)

### Reference Example 37: Synthesis of compound D4

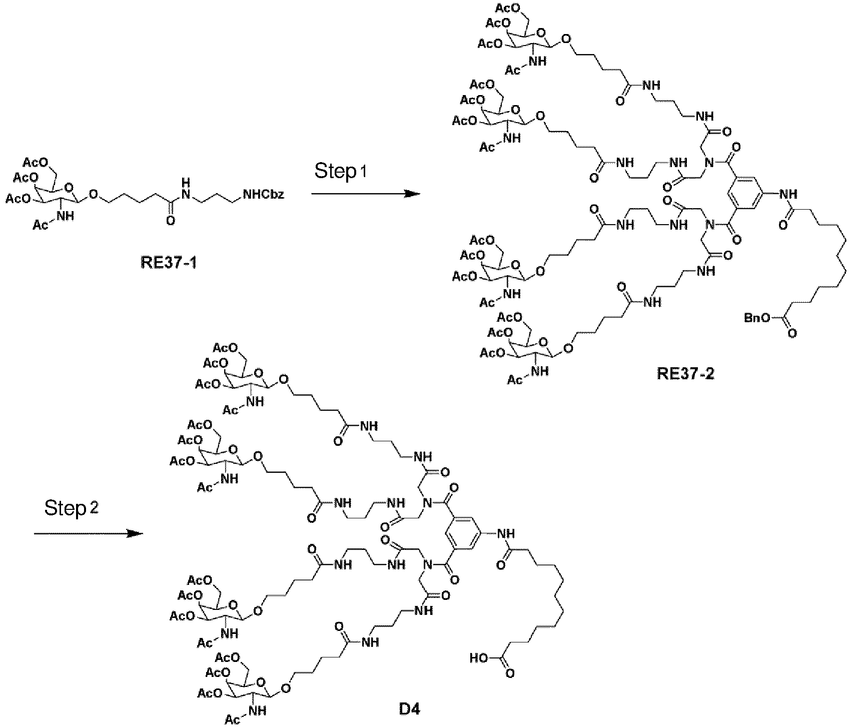

### Step 1 of Reference Example 37

Compound RE37-1 (compound RE13-2 in Reference Example 13, 430 mg, 0.674 mmol) synthesized by the method described in Reference Example 13 was dissolved in N,N'-dimethylformamide (6 mL). To the solution, a 10% palladium-carbon powder (water-containing product, 54.29%; 79 mg) was added at room temperature, and the mixture was stirred for 4 hours in a hydrogen atmosphere. The reaction solution was filtered. Compound RE26-5 (105.0 mg, 0.148 mmol) of Reference Example 26, 1-hydroxybenzotriazole monohydrate (11.31 mg, 0.074 mmol), and 1-(3-dimethylaminopropyl)-3-ethylcarbodiimide hydrochloride (170.0.0 mg, 0.887 mmol) were added to the filtrate, and the mixture was stirred overnight at room temperature. The solvent in the reaction solution was distilled off under reduced pressure, and the residue was purified by reverse-phase column chromatography (water/acetonitrile) to obtain compound RE37-2 (218.1 mg, yield: 56%).
ESI-MS m/z: 1329 (M + 2H)²⁺

### Step 2 of Reference Example 37

Compound RE37-2 (0.210 g, 0.079 mmol) was dissolved in tetrahydrofuran (3.1 mL) and water (1.0 mL). To the solution, a 10% palladium-carbon powder (water-containing product, 54.29%; 39 mg) was added at room temperature, and the mixture was stirred for 4 hours in a hydrogen atmosphere. The reaction solution was filtered, and the solvent was distilled off under reduced pressure to obtain compound D4 (192.7 mg, yield: 95%).
ESI-MS m/z: 1284 (M + 2H) ²⁺
1H-NMR (400 MHz, DMSO-d6, δ): 1.17-1.65 (42H, m), 1.69-2.13 (61H, m), 2.95-3.17 (16H, m), 3.65-3.77 (3H, m), 3.79-3.94 (6H, m), 3.96-4.10 (16H, m), 4.48 (4H, d, J = 8.4 Hz), 4.96 (4H, dd, J = 2.4, 11.2 Hz), 5.21 (4H, d, J = 3.2 Hz), 7.01 (1H, s), 7.64-7.92 (11H, m), 8.26-8.48 (4H, m), 10.14 (1H, s)

### Reference Example 38: Synthesis of compound D5

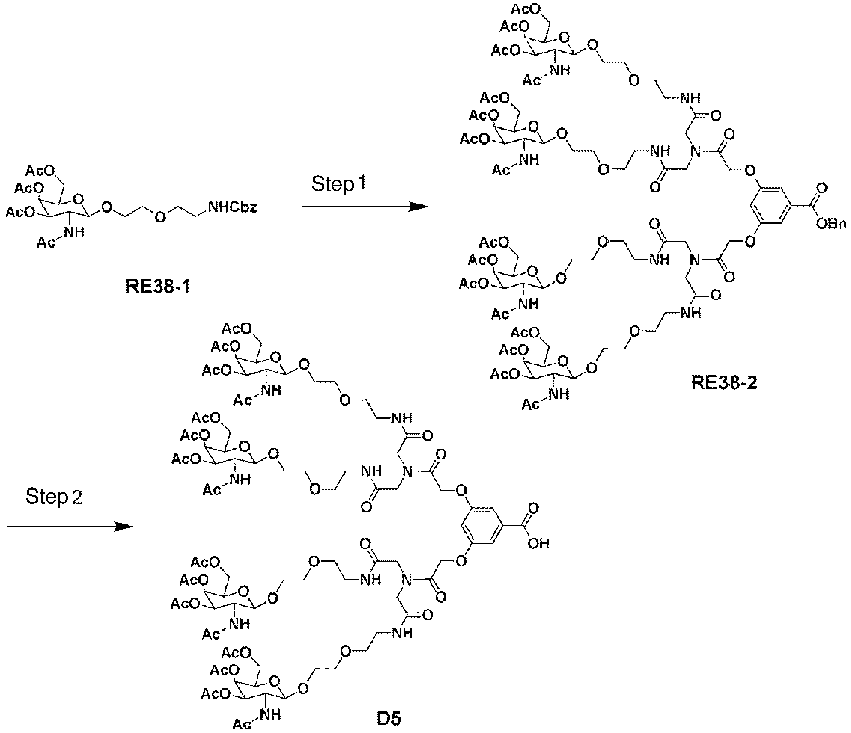

### Step 1 of Reference Example 38

Compound RE38-1 (compound RE12-2 in Reference Example 12, 450 mg, 0.791 mmol) synthesized by the method described in Reference Example 12 was dissolved in N,N'-dimethylformamide (6 mL). To the solution, a 10% palladium-carbon powder (water-containing product, 54.29%; 85 mg) was added at room temperature, and the mixture was stirred for 5 hours in a hydrogen atmosphere. The reaction solution was filtered. Compound RE27-6 (94 mg, 0.158 mmol) of Reference Example 27, 1-hydroxybenzotriazole monohydrate (133.0 mg, 0.871 mmol), and 1-(3-dimethylaminopropyl)-3-ethylcarbodiimide hydrochloride (182.0 mg, 0.950 mmol) were added to the filtrate, and the mixture was stirred overnight at room temperature. The solvent in the reaction solution was distilled off under reduced pressure, and the residue was purified by reverse-phase column chromatography (water/acetonitrile) to obtain compound RE38-2 (99 mg, yield: 28%).
ESI-MS m/z: 1129 (M + 2H)²⁺

### Step 2 of Reference Example 38

Compound RE38-2 (80 mg, 0.035 mmol) was dissolved in tetrahydrofuran (1.7 mL) and water (0.85 mL). To the solution, a 10% palladium-carbon powder (water-containing product, 54.29%; 26 mg) was added at room temperature, and the mixture was stirred for 2 hours in a hydrogen atmosphere. The reaction solution was filtered, and the solvent was distilled off under reduced pressure to obtain compound D5 (57.5 mg, yield: 75%).
ESI-MS m/z: 1084 (M + 2H) ²⁺
¹H-NMR (400 MHz, DMSO-d6, δ): 1.69-2.21 (46H, m), 3.14-3.65 (28H, m), 3.67-4.22 (27H, m), 4.43-4.66 (4H, m), 4.69-4.88 (4H, m), 4.89-5.08 (4H, m), 5.12-5.32 (4H, m), 6.54-6.68 (1H, br), 7.01 (2H, s), 7.78-8.09 (3H, m), 8.13-8.31 (2H, m), 8.58-8.75 (2H, m)

### Reference Example 39: Synthesis of compound D6

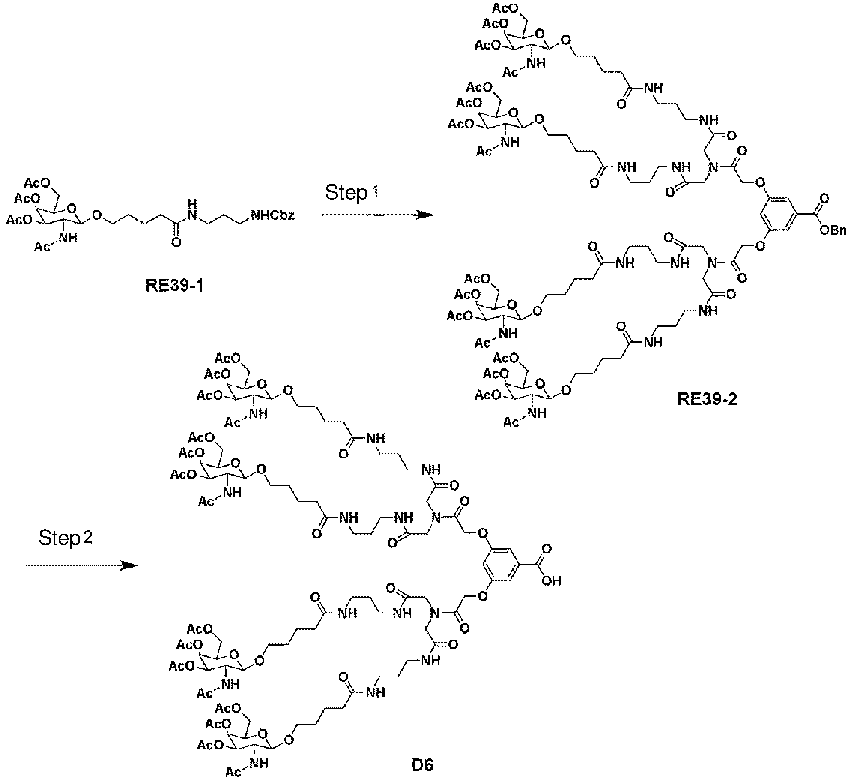

### Step 1 of Reference Example 39

Compound RE39-1 (compound RE13-2 in Reference Example 13, 418 mg, 0.655 mmol) synthesized by the method described in Reference Example 13 was dissolved in N,N'-dimethylformamide (6 mL). To the solution, a 10% palladium-carbon powder (water-containing product, 54.29%; 77 mg) was added at room temperature, and the mixture was stirred for 5 hours in a hydrogen atmosphere. The reaction solution was filtered. Compound RE27-6 (85 mg, 0.144 mmol) synthesized in Reference Example 27, 1-hydroxybenzotriazole monohydrate (121.0 mg, 0.791 mmol), and 1-(3-dimethylaminopropyl)-3-ethylcarbodiimide hydrochloride (165.0 mg, 0.863 mmol) were added to the filtrate, and the mixture was stirred overnight at room temperature. The solvent in the reaction solution was distilled off under reduced pressure, and the residue was purified by reverse-phase column chromatography (water/acetonitrile) to obtain compound RE39-2 (99 mg, yield: 28%).
ESI-MS m/z: 1268 (M + 2H)²⁺

### Step 2 of Reference Example 39

Compound RE39-2 (186 mg, 0.073 mmol) was dissolved in tetrahydrofuran (2.8 mL) and water (0.93 mL). To the solution, a 10% palladium-carbon powder (water-containing product, 54.29%; 40 mg) was added at room temperature, and the mixture was stirred for 2 hours in a hydrogen atmosphere. The reaction solution was filtered, and the solvent was distilled off under reduced pressure to obtain compound D6 (156.7 mg, yield: 87%).
ESI-MS m/z: 1222 (M + 2H) ²⁺
¹H-NMR (400 MHz, DMSO-d6, δ): 1.36-1.62 (27H, m), 1.67-2.17 (64H, m), 2.92-3.21 (15H, m), 3.58-3.77 (2H, m), 3.80-3.95 (7H, m), 3.97-4.13 (15H, m), 4.47 (4H, d, J = 8.8 Hz), 4.88-5.02 (7H, m), 5.10-5.24 (3H, m), 6.95-7.00 (1H, m), 7.26-7.31 (2H, m), 7.72-7.88 (8H, m), 8.10-8.20 (2H, m), 8.51-8.60 (2H, m)

### Reference Example 40

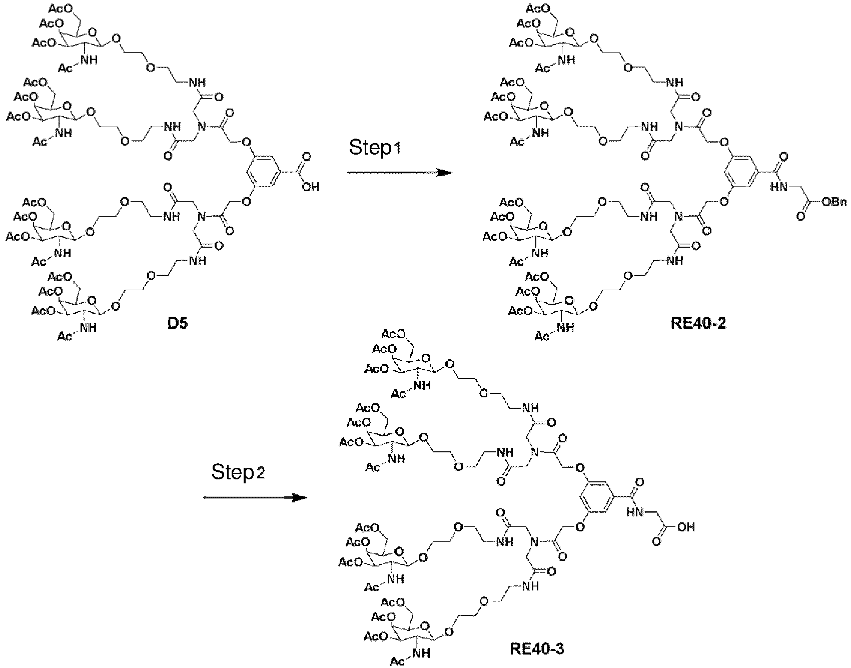

### Step 1 of Reference Example 40

Compound D5 (171 mg, 0.079 mmol) synthesized by the method described in Reference Example 38 was dissolved in N,N'-dimethylformamide (3.4 mL). To the solution, glycine benzyl p-toluenesulfonate (32.0 mg, 0.095 mmol), 1-hydroxybenzotriazole monohydrate (12.09 mg, 0.079 mmol), and 1-(3-dimethylaminopropyl)-3-ethylcarbodiimide hydrochloride (18.16 mg, 0.095 mmol) were added, and the mixture was stirred overnight at room temperature. The solvent in the reaction solution was distilled off under reduced pressure, and the residue was purified by reverse-phase column chromatography (water/acetonitrile) to obtain compound RE40-2 (55.7 mg, yield: 31%).
ESI-MS m/z: 1158 (M + 2H)²⁺

### Step 2 of Reference Example 40

Compound RE40-2 (54 mg, 0.023 mmol) was dissolved in tetrahydrofuran (0.83 mL) and water (0.28 mL). To the solution, a 10% palladium-carbon powder (water-containing product, 54.29%; 18 mg) was added at room temperature, and the mixture was stirred for 2 hours in a hydrogen atmosphere. The reaction solution was filtered, and the solvent was distilled off under reduced pressure to obtain compound RE40-3 (50.1 mg, yield: 97%).
ESI-MS m/z: 1112 (M + 2H) ²⁺
1H-NMR (400 MHz, DMSO-d6, δ): 0.96-1.06 (3H, m), 1.71-2.20 (54H, m), 3.41-3.64 (15H, m), 3.68-4.20 (32H), 4.55 (4H, d, J = 8.4 Hz), 4.81 (4H, s), 4.94-5.02 (4H, m), 5.17-5.25 (4H, m), 6.63-6.76 (2H, m), 6.93-7.02 (2H, m), 7.84-8.00 (3H, m), 8.17-8.30 (2H, m), 8.58-8.70 (2H, m)

### Reference Example 41: Synthesis of compound D7

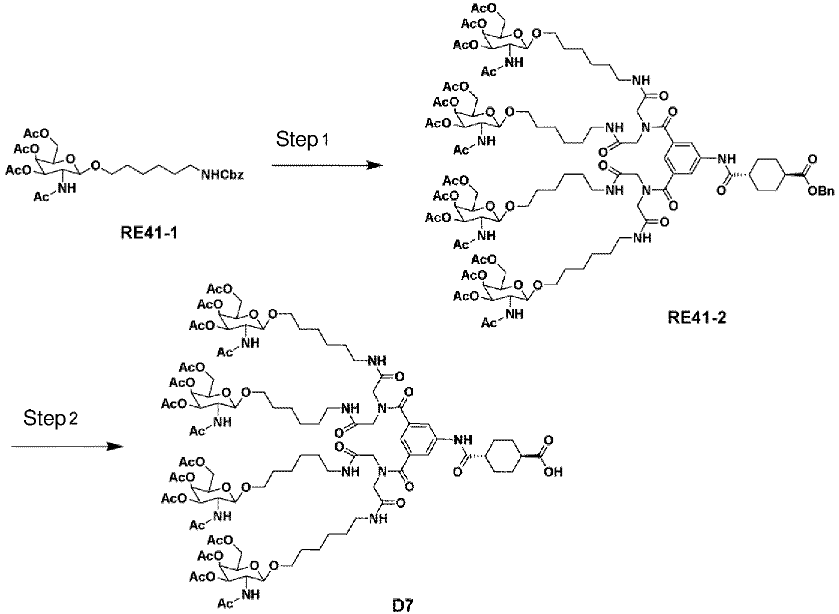

### Step 1 of Reference Example 41

Compound RE41-1 (500 mg, 0.861 mmol) was dissolved in N,N'-dimethylformamide (10 mL). To the solution, a 10% palladium-carbon powder (water-containing product, 54.29%; 92.1 mg) was added at room temperature, and the mixture was stirred for 2 hours in a hydrogen atmosphere. Compound RE27-3 (113 mg, 0.172 mmol) synthesized in Reference Example 27, 1-(3-dimethylaminopropyl)-3-ethylcarbodiimide hydrochloride (198 mg, 1.03 mmol), and 1-hydroxybenzotriazole monohydrate (13.2 mg, 0.086 mmol) were added thereto at room temperature in an argon atmosphere, and the mixture was stirred overnight. The reaction solution was filtered through celite, and the solvent was distilled off under reduced pressure. The residue was purified by silica gel column chromatography (chloroform/methanol = 90/10) and further purified by reverse-phase preparative HPLC (acetonitrile/water) to obtain compound RE41-2 (195 mg, yield: 48%).
ESI-MS m/z: 1186 (M + 2H)²⁺

### Step 2 of Reference Example 41

Compound RE41-2 (194 mg, 0.082 mmol) was dissolved in tetrahydrofuran (2.9 mg) and water (1.0 mL). To the solution, a 10% palladium-carbon powder (water-containing product, 54.29%; 35.7 mg) was added at room temperature, and the mixture was stirred for 8 hours in a hydrogen atmosphere. The reaction solution was filtered, and the solvent was distilled off under reduced pressure to obtain compound D7 (183 mg, yield: 98%).
ESI-MS m/z: 1141 (M + 2H) ²⁺
¹H-NMR (400 MHz, DMSO-d6, δ): 1.21-1.45 (m, 40H), 1.76-2.18 (m, 50H), 3.00-3.09 (m, 8H), 3.40-4.20 (m, 32H), 4.47 (d, J = 8.5 Hz, 4H), 4.96 (dd, J = 3.1, 11.2 Hz, 4H), 5.21 (d, J = 3.1 Hz, 4H), 6.98 (s, 1H), 7.65 (s, 2H), 7.84 (d, J = 9.0 Hz, 4H), 8.31 (brs, 1H), 8.44 (brs, 1H), 10.11 (s, 1H).

### Reference Example 42: Synthesis of compound D8

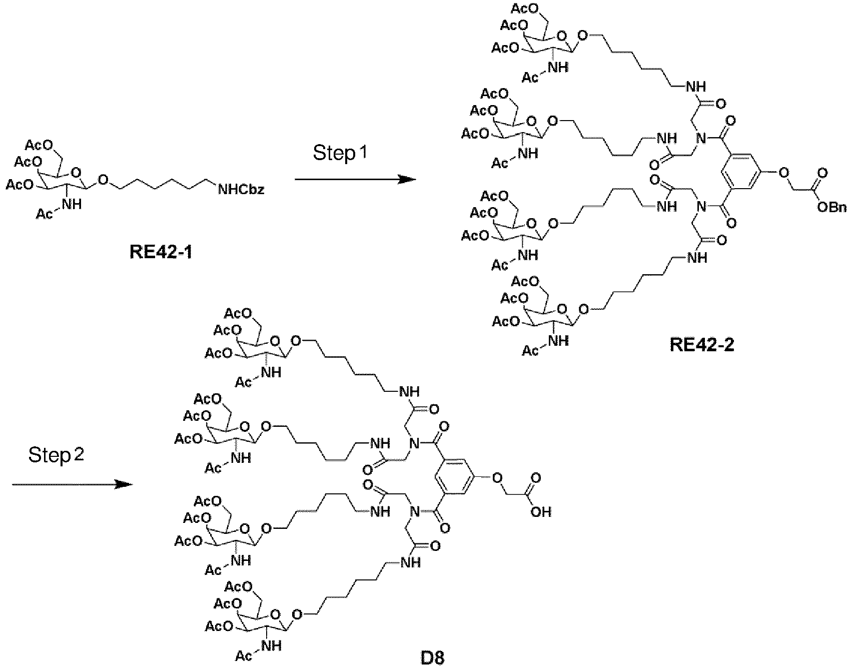

### Step 1 of Reference Example 42

Compound RE42-1 (compound RE11-2 in Reference Example 11, 500 mg, 0.861 mmol) synthesized by the method described in Reference Example 11 was dissolved in N,N'-dimethylformamide (10 mL). To the solution, a 10% palladium-carbon powder (water-containing product, 54.29%; 92.1 mg) was added at room temperature, and the mixture was stirred for 2 hours in a hydrogen atmosphere. Compound RE29-4 (97.0 mg, 0.172 mmol) synthesized in Reference Example 29, 1-(3-dimethylaminopropyl)-3-ethylcarbodiimide hydrochloride (198 mg, 1.03 mmol), and 1-hydroxybenzotriazole monohydrate (13.2 mg, 0.086 mmol) were added thereto at room temperature in an argon atmosphere, and the mixture was stirred overnight. The reaction solution was filtered through celite, and the solvent was distilled off under reduced pressure. The residue was purified by silica gel column chromatography (chloroform/methanol = 85/15) and further purified by reverse-phase preparative HPLC (acetonitrile/water) to obtain compound RE42-2 (179 mg, yield: 46%).
ESI-MS m/z: 1138 (M + 2H)²⁺

### Step 2 of Reference Example 42

Compound RE42-2 (175 mg, 0.077 mmol) was dissolved in tetrahydrofuran (2.6 mg) and water (0.9 mL). To the solution, a 10% palladium-carbon powder (water-containing product, 54.29%; 32.4 mg) was added at room temperature, and the mixture was stirred for 1 hour in a hydrogen atmosphere. The reaction solution was filtered, and the solvent was distilled off under reduced pressure to obtain compound D8 (160 mg, yield: 95%).
ESI-MS m/z: 1093 (M + 2H) ²⁺
¹H-NMR (400 MHz, DMSO-d6, δ): 1.23-1.45 (m, 32H), 1.77 (s, 12H), 1.89 (s, 12H), 1.99 (s, 12H), 2.10 (s, 12H), 3.01-3.11 (m, 8H), 3.69-4.02 (m, 34H), 4.47-4.50 (m, 4H), 4.94-4.98 (m, 4H), 5.21 (d, J = 3.1 Hz, 4H), 6.92 (s, 1H), 6.94 (s, 2H), 7.87 (d, J = 9.4 Hz, 2H), 7.92 (d, J = 8.5 Hz, 2H), 8.33 (brs, 2H), 8.50 (brs, 2H).

### Reference Example 43: Synthesis of compound D9

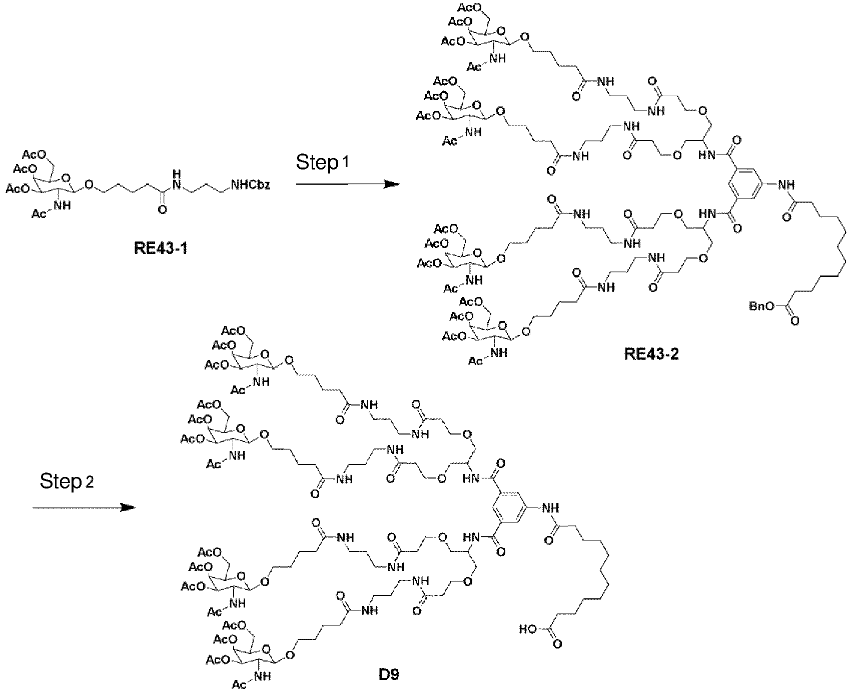

### Step 1 of Reference Example 43

Compound RE43-1 (compound RE13-2 in Reference Example 13, 500 mg, 0.784 mmol) synthesized by the method described in Reference Example 13 was dissolved in N,N'-dimethylformamide (10 mL). To the solution, a 10% palladium-carbon powder (water-containing product, 54.29%; 92.1 mg) was added at room temperature, and the mixture was stirred for 2 hours in a hydrogen atmosphere. Compound RE30-6 (144 mg, 0.157 mmol) synthesized in Reference Example 30, 1-(3-dimethylaminopropyl)-3-ethylcarbodiimide hydrochloride (180 mg, 0.941 mmol), and 1-hydroxybenzotriazole monohydrate (12.0 mg, 0.078 mmol) were added thereto at room temperature in an argon atmosphere, and the mixture was stirred overnight. The reaction solution was filtered through celite, and the solvent was distilled off under reduced pressure. The residue was purified by silica gel column chromatography (chloroform/methanol = 80/20) and further purified by reverse-phase preparative HPLC (acetonitrile/water) to obtain compound RE43-2 (191 mg, yield: 43%).
ESI-MS m/z: 1431 (M + 2H)²⁺

### Step 2 of Reference Example 43

Compound RE43-2 (186 mg, 0.065 mmol) was dissolved in tetrahydrofuran (2.8 mg) and water (0.9 mL). To the solution, a 10% palladium-carbon powder (water-containing product, 54.29%; 34.3 mg) was added at room temperature, and the mixture was stirred for 1 hour in a hydrogen atmosphere. The reaction solution was filtered, and the solvent was distilled off under reduced pressure to obtain compound D9 (174 mg, yield: 97%).
ESI-MS m/z: 1386 (M + 2H) ²⁺
¹H-NMR (400 MHz, DMSO-d6, δ):1.25-4.02 (m, 164H), 4.18-4.26 (m, 2H), 4.47 (d, J = 8.1 Hz, 4H), 4.96 (dd, J = 3.1, 11.2 Hz, 4H), 5.21 (d, J = 3.6 Hz, 4H), 7.74-7.91 (m, 13H), 8.18 (s, 2H), 8.36 (d, J = 7.2 Hz, 2H), 10.27 (brs, 1H) .

### Reference Example 44: Synthesis of compound A2

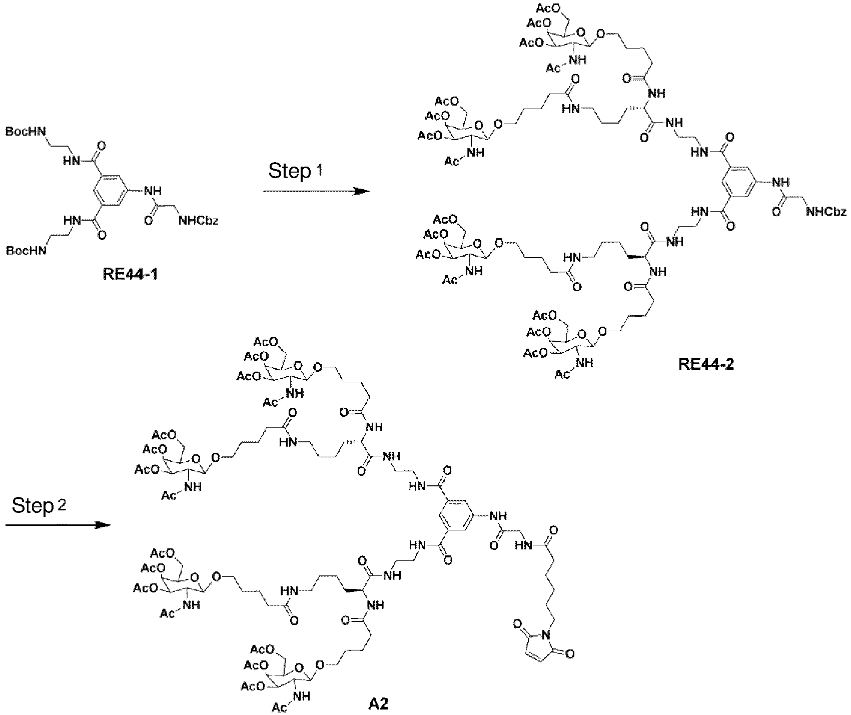

### Step 1 of Reference Example 44

Compound RE44-1 (compound RE31-4 in Reference Example 31, 150 mg, 0.228 mmol) synthesized by the method described in Reference Example 31 was dissolved in dichloromethane (1.5 mL). To the solution, trifluoroacetic acid (1.5 mL) was added at room temperature, and the mixture was stirred for 4 hours.
The reaction solution was concentrated under reduced pressure and subjected to azeotropy with ethyl acetate. The residue was dissolved in N,N'-dimethylformamide (3 mL). To the solution, compound RE14-3 (574 mg, 0.571 mmol) of Reference Example 14, 1-(3-dimethylaminopropyl)-3-ethylcarbodiimide hydrochloride (109 mg, 0.571 mmol), triethylamine (0.159 mL, 1.14 mmol), and 1-hydroxybenzotriazole monohydrate (3.50 mg, 0.023 mmol) were added at room temperature, and the mixture was stirred overnight. The reaction solution was concentrated under reduced pressure, and the residue was purified by silica gel column chromatography (chloroform/methanol = 90/10) and further purified by reverse-phase preparative HPLC (acetonitrile/water) to obtain compound RE44-2 (242 mg, yield: 44%).
ESI-MS m/z: 1216 (M + 2H)²⁺

### Step 2 of Reference Example 44

Compound RE44-2 (242 mg, 0.100 mmol) was dissolved in tetrahydrofuran/water (4/1; 12 mL). To the solution, a 10% palladium-carbon powder (water-containing product, 54.29%; 44.6 mg) was added at room temperature, and the mixture was stirred for 2 hours in a hydrogen atmosphere. 6-Maleimidohexanoic acid (23.2 mg, 0.110 mmol) and 4-(4,6-dimethoxy-1,3,5-triazin-2-yl)-4-methylmorpholinium chloride (55.2 mg, 0.199 mmol) was added thereto at room temperature in an argon atmosphere, and the mixture was stirred overnight. The reaction solution was filtered through celite, and the solvent was distilled off under reduced pressure. The residue was purified using HP20 resin (acetone/water) to obtain compound A2 (97.4 mg, yield: 39%).
ESI-MS m/z: 1245 (M + 2H) ²⁺
¹H-NMR (400 MHz, DMSO-d6, δ):1.14-2.16 (100H, m), 2.96-2.98 (4H, m), 3.24-3.41 (18H, m), 3.69-3.73 (4H, m), 3.83-3.91 (6H, m), 4.14-4.16 (2H, m), 4.47 (4H, d, J = 8.6 Hz), 4.96 (4H, dd, J = 3.6, 11.3 Hz), 5.21 (4H, d, J = 3.2 Hz), 7.01 (2H, s), 7.73-7.75 (2H, m), 7.83-7.94 (7H, m), 8.05-8.08 (2H, m), 8.14-8.20 (3H, m), 8.55-8.56 (2H, m), 10.24 (1H, brs).

### Reference Example 45: Synthesis of compound A3

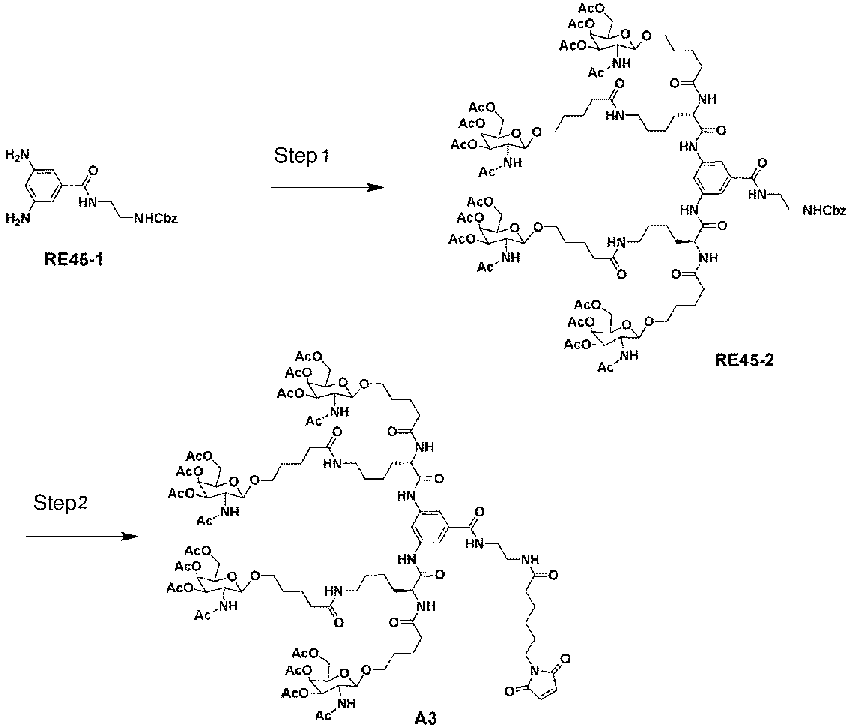

### Step 1 of Reference Example 45

Compound RE45-1 (compound RE32-3 in Reference Example 32, 75.0 mg, 0.228 mmol) synthesized by the method described in Reference Example 32 was dissolved in N,N'-dimethylformamide (3.0 mL). To the solution, compound RE14-3 (574 mg, 0.571 mmol) of Reference Example 14, diisopropylethylamine (0.199 mL, 1.14 mmol), and O-(7-azabenzotriazol-1-yl)-N,N,N',N'-tetramethyluronium hexafluorophosphate (217 mg, 0.571 mmol) were added at room temperature, and the mixture was stirred overnight. The solvent was distilled off under reduced pressure, and the residue was purified by silica gel column chromatography (chloroform/methanol = 90/10) and further purified by reverse-phase preparative HPLC (acetonitrile/water) to obtain compound RE45-2 (202 mg, yield: 38%).
ESI-MS m/z: 1152 (M + 2H)²⁺

### Step 2 of Reference Example 45

Compound RE45-2 (196 mg, 0.085 mmol) was dissolved in tetrahydrofuran/water (4/1; 10 mL). To the solution, a 10% palladium-carbon powder (water-containing product, 54.29%; 36.1 mg) was added at room temperature, and the mixture was stirred for 2 hours in a hydrogen atmosphere. 6-Maleimidohexanoic acid (19.8 mg, 0.094 mmol) and 4-(4,6-dimethoxy-1,3,5-triazin-2-yl)-4-methylmorpholinium chloride (47.0 mg, 0.170 mmol) were added thereto at room temperature in an argon atmosphere, and the mixture was stirred overnight. The reaction solution was filtered through celite, and the solvent was distilled off under reduced pressure. The residue was purified using HP20 resin (acetone/water) to obtain compound A3 (42.4 mg, yield: 21%).
ESI-MS m/z: 1182 (M + 2H)²⁺

### Reference Example 46: Compound D10

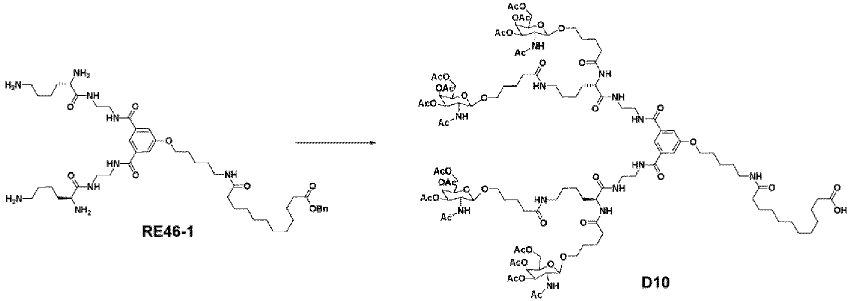

Compound D10 (2.2 g, yield: 58%) was obtained in the same way as in steps 1 and 2 of Reference Example 10 using compound RE46-1 (compound RE34-3 in Reference Example 34) synthesized by the method described in Reference Example 34.
ESI-MS m/z: 2437(M+H)⁺

### Reference Example 47: Synthesis of compound A4

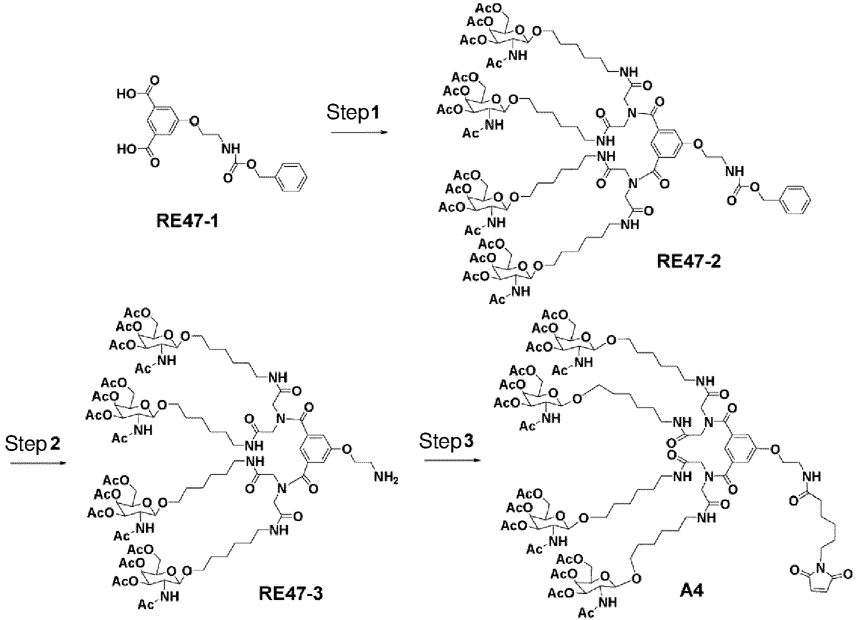

### Step 1 of Reference Example 47

Compound RE47-2 (0.25 g, yield: 39%) was obtained in the same way as in step 3 of Reference Example 3 using compound RE47-1 (compound RE33-4 in Reference Example 33, 0.101 g, 0.282 mmol) synthesized by the method described in Reference Example 33 and compound RE15-2 (0.607 g, 0.613 mmol) of Reference Example 15.
ESI-MS m/z: 2304(M+H)⁺

### Step 2 of Reference Example 47

Compound RE47-3 (0.15 g, yield: 63%) was obtained in the same way as in step 2 of Reference Example 3 using compound RE47-2 (0.255 g, 0.111 mmol).
ESI-MS m/z: 2170(M+H)⁺

### Step 3 of Reference Example 47

Compound A4 (5.5 mg, yield: 24%) was obtained in the same way as in step 2 of Reference Example 5 using compound RE47-3 (20.8 mg, 9.59 µmol).
ESI-MS m/z: 1182(M+2H)²⁺

### Reference Example 48: Synthesis of compound A5

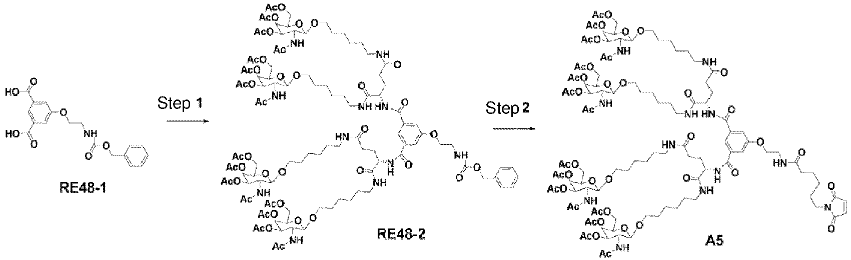

### Step 1 of Reference Example 48

Compound RE48-2 (0.343 g, yield: 53%) was obtained in the same way as in step 3 of Reference Example 3 using compound RE48-1 (compound RE33-4 in Reference Example 33, 0.099 g, 0.277 mmol) synthesized by the method described in Reference Example 33 and compound RE16-3 (0.618 g, 0.615 mmol) synthesized in step 2 of Reference Example 16. ESI-MS m/z: 2333(M+H)⁺

### Step 2 of Reference Example 48

Compound A5 (6.9 mg, yield: 28%) was obtained in the same way as in steps 1 and 2 of Reference Example 10 using compound RE48-2.
ESI-MS m/z: 2392(M+H)⁺

### Reference Example 49: Synthesis of compound A6

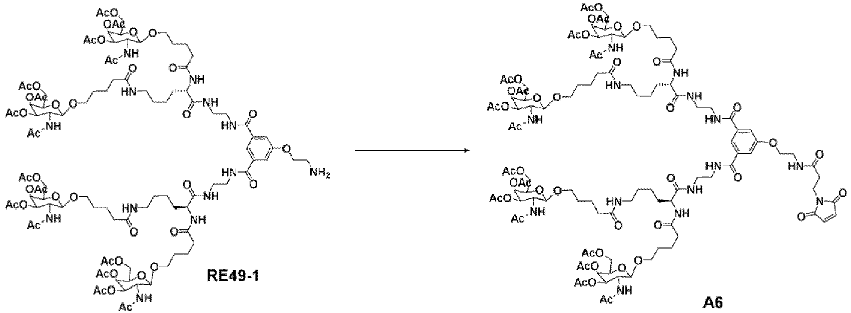

Compound A6 (0.040 g, yield: 78%) was obtained in the same way as in the synthesis of compound A1 of Reference Example 8 using compound RE49-1 (0.048 g, 0.021 mmol) and N-succinimidyl 3-maleimidopropionate (manufactured by Tokyo Chemical Industry Co., Ltd., 0.017 g, 0.064 mmol).
ESI-MS m/z: 2480(M+HCOO)⁻

### Reference Example 50: Synthesis of compound A7

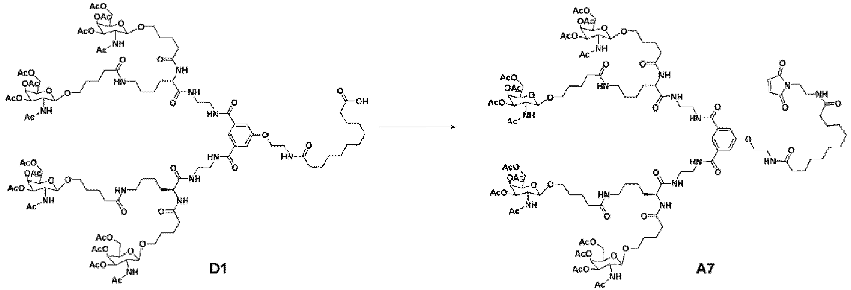

### Step 131

Compound A7 (9.1 mg, yield: 36%) was obtained in the same way as in step 3 of Reference Example 3 using compound D1 (23.6 mg, 9.46 µmol) synthesized by the method described in Reference Example 10 and N-(2-aminoethyl)maleimide trifluoroacetate (manufactured by Sigma-Aldrich Co. LLC, 7.21 mg, 0.028 µmol).
ESI-MS m/z: 1310(M+2H)²⁺

### Reference Example 51

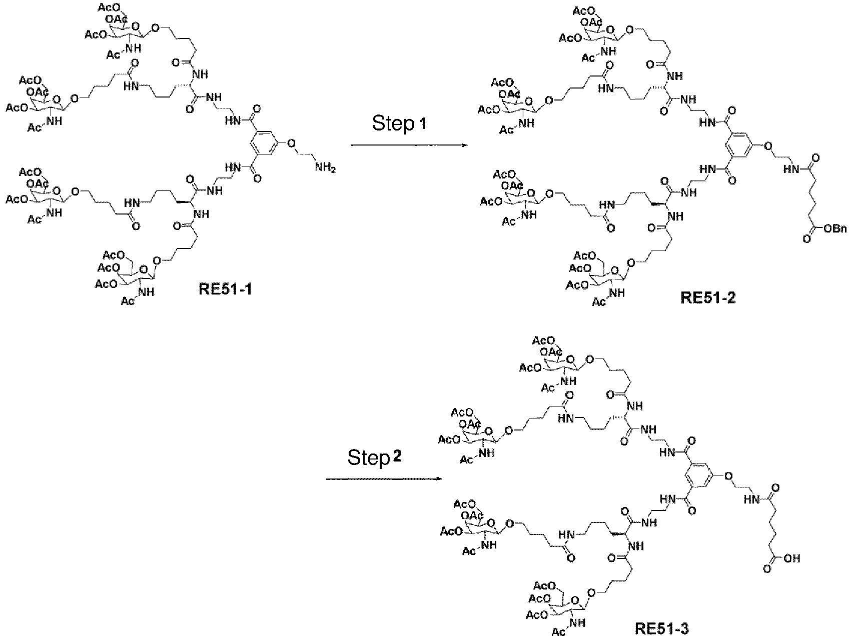

### Step 1 of Reference Example 51

Compound RE51-2 (0.076 g, yield: 56%) was obtained in the same way as in step 1 of Reference Example 4 using compound RE51-1 (0.122 g, 0.054 mmol) synthesized by the method described in Reference Example 7 and hexanoic acid monobenzyl ester synthesized in the same way as the method described in Bioconjugate Chemistry, Vol. 22, p. 690-699, 2011.
ESI-MS m/z: 2503(M+H)⁺

### Step 2 of Reference Example 51

Compound RE51-3 (0.030 g, yield: 40%) was obtained in the same way as in step 1 of Reference Example 3 using compound RE51-2 (0.076 g, 0.03 mmol).
ESI-MS m/z: 2412(M+H)⁺

### Reference Example 52

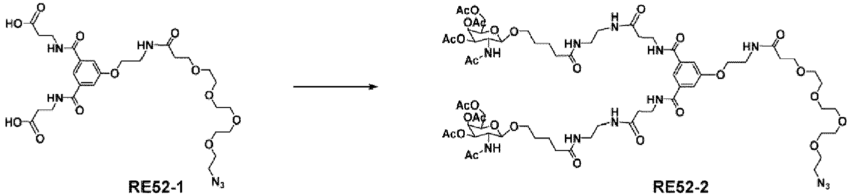

Compound RE52-2 (7 mg, yield: 65%) was obtained in the same way as in step 7 of Reference Example 3 using compound RE52-1 (compound RE6-5 in Reference Example 6, 4.36 mg, 0.006 mmol) synthesized by the method described in Reference Example 6 and compound RE1-4 (10 mg, 0.02 mmol) of Reference Example 1.
ESI-MS m/z: 1581 (M - H)⁻

### Reference Example 53: Synthesis of compound C2

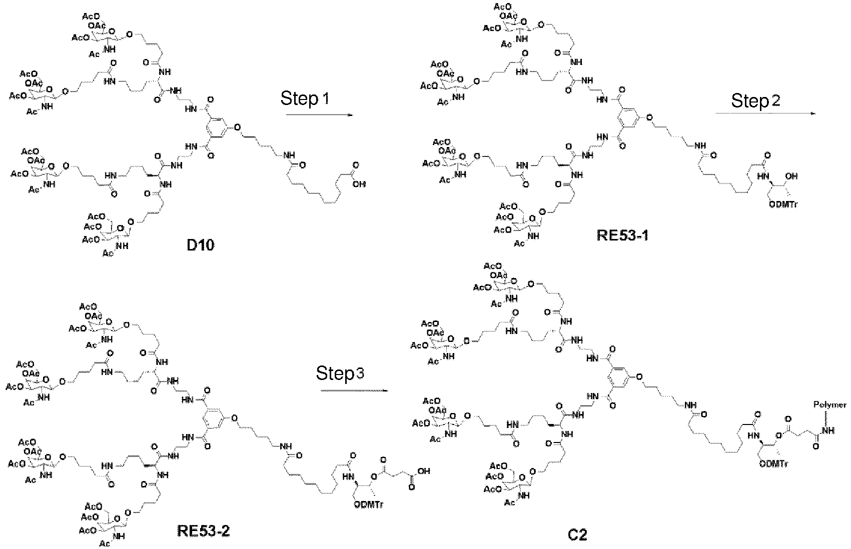

### Step 1 of Reference Example 53

Compound RE53-1 (0.129 g, yield: 55%) was obtained in the same way as in step 3 of Reference Example 10 using compound D10 (0.2011 g, 0.079 mmol) synthesized by the method described in Reference Example 46.
ESI-MS m/z: 2972 (M+HCOO)⁻

### Step 2 of Reference Example 53

A crude product of compound RE53-2 was obtained in the same way as in step 4 of Reference Example 10 using compound RE53-1(0.129 g, 0.044 mmol).
ESI-MS m/z: 1535 (M+HCOOH-2H)²⁻

### Step 3 of Reference Example 53

Compound C2 (19.4 µmol/g, yield: 35%) was obtained in the same way as in step 5 of Reference Example 10 using compound RE53-2 (0.0467 g, 0.013 mmol).

### Reference Example 54: Synthesis of compound C3

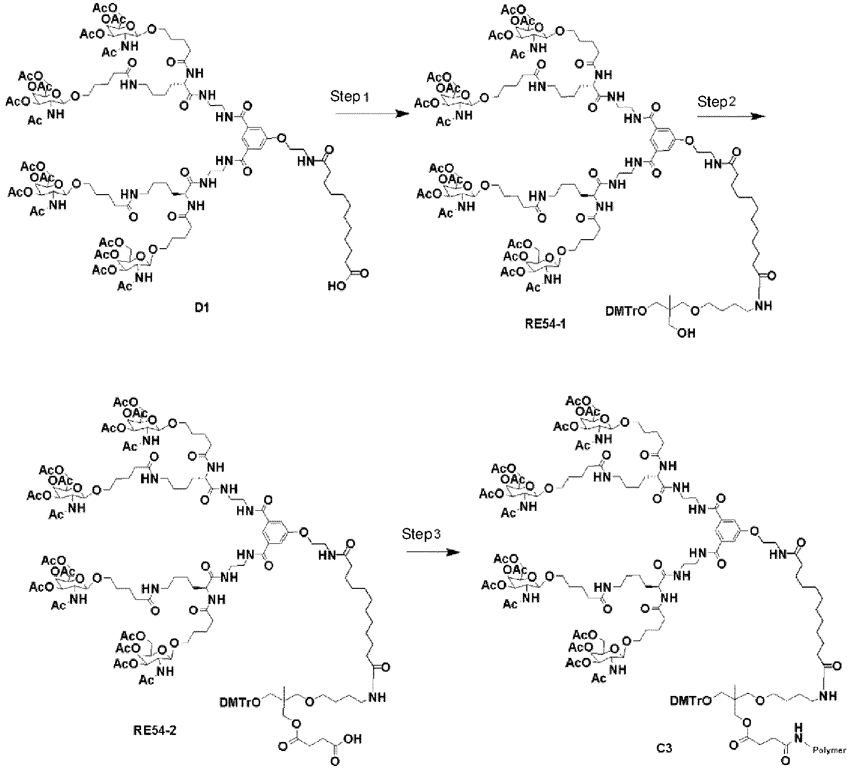

### Step 1 of Reference Example 54

Compound RE54-1 (90 mg, yield: 76%) was obtained in the same way as in step 3 of Reference Example 10 using compound D1 (100 mg, 0.040 mmol) synthesized by the method described in Reference Example 10 and compound RE17-2 of Reference Example 17.
ESI-MS m/z: 1335 (M-DMTr+2H)²⁺

### Step 2 of Reference Example 54

A crude product of compound RE54-2 was obtained in the same way as in step 4 of Reference Example 10 using compound RE54-1 (90 mg, 0.030 mmol).
ESI-MS m/z: 1558 (M+HCOOH-2H)²⁻

### Step 3 of Reference Example 54

Compound C3 (21.5 µmol/g, 2-step yield: 32%) was obtained in the same way as in step 5 of Reference Example 10 using a crude product of compound RE54-2.

### Reference Example 55: Synthesis of compound C4

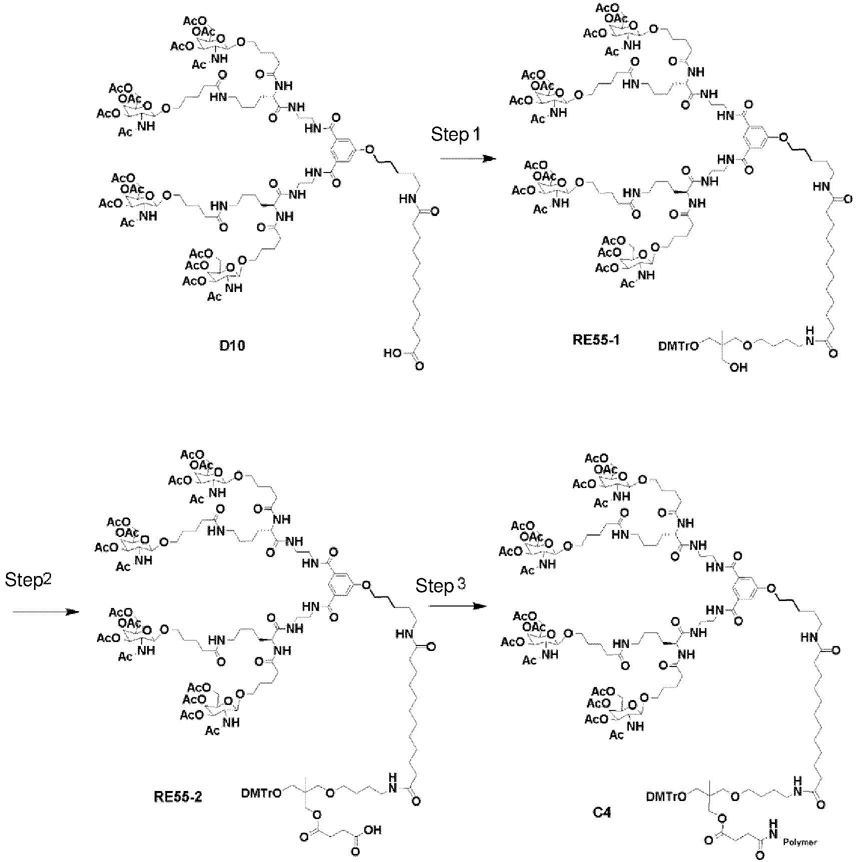

### Step 1 of Reference Example 55

Compound RE55-1 (90 mg, yield: 76%) was obtained in the same way as in step 3 of Reference Example 10 using compound D10 (100 mg, 0.039 mmol) synthesized by the method described in Reference Example 46 and compound RE17-2 synthesized in Reference Example 17.
ESI-MS m/z: 1356 (M+2H)²⁺, detected as a DMTr-deprotected form

### Step 2 of Reference Example 55

A crude product of compound RE55-2 was obtained in the same way as in step 4 of Reference Example 10 using compound RE55-1 (90 mg, 0.030 mmol).
ESI-MS m/z: 1579 (M+HCOOH-2H)²⁻

### Step 3 of Reference Example 55

Compound C4 (17.0 µmol/g, 2-step yield: 26%) was obtained in the same way as in step 5 of Reference Example 10 using a crude product of compound RE55-2.

### Reference Example 56: Synthesis of compound C5

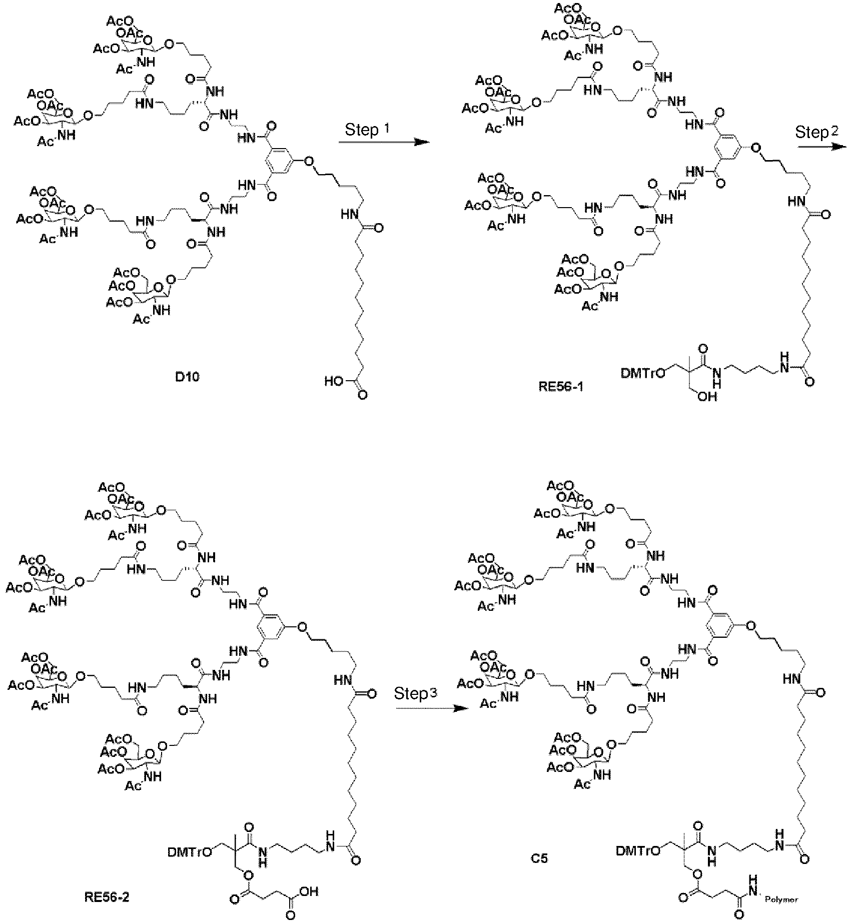

### Step 1 of Reference Example 56

Compound RE56-1 (50 mg, yield: 42%) was obtained in the same way as in step 3 of Reference Example 10 using compound D10 (100 mg, 0.039 mmol) synthesized by the method described in Reference Example 46 and compound RE18-3 synthesized in step 2 of Reference Example 18.
ESI-MS m/z: 1363 (M+2H)²⁺, detected as a DMTr-deprotected form

### Step 2 of Reference Example 56

A crude product of compound RE56-2 was obtained in the same way as in step 4 of Reference Example 10 using compound RE56-1 (50 mg, 0.017 mmol).
ESI-MS m/z: 1587 (M+HCOOH-2H)²⁻

### Step 3 of Reference Example 56

Compound C5 (0.5 µmol/g, 2-step yield: 1%) was obtained in the same way as in step 5 of Reference Example 10 using a crude product of compound RE56-2.

### Reference Example 57: Synthesis of compound C6

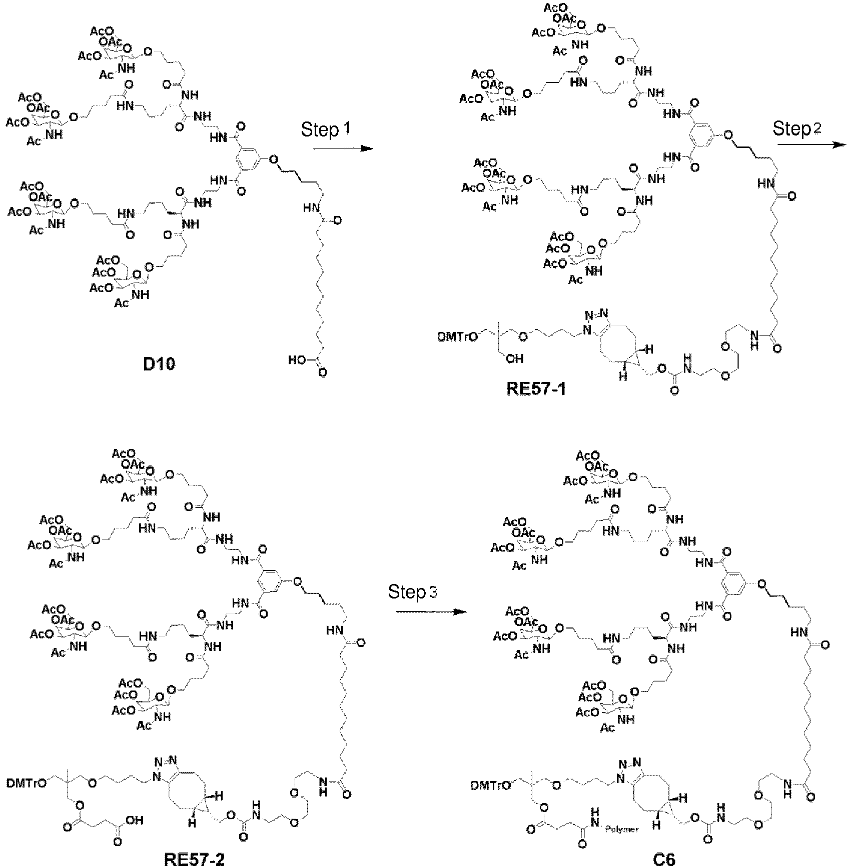

### Step 1 of Reference Example 57

Compound RE57-1 (40 mg, yield: 30%) was obtained in the same way as in step 3 of Reference Example 10 using compound D10 (100 mg, 0.039 mmol) synthesized by the method described in Reference Example 46 and compound RE19-2 of Reference Example 19.
ESI-MS m/z: 1532 (M+2H)²⁺, detected as a DMTr-deprotected form

### Step 2 of Reference Example 57

A crude product of compound RE57-2 was obtained in the same way as in step 4 of Reference Example 10 using compound RE57-1(40 mg, 0.012 mmol).
ESI-MS m/z: 1582 (M+2H)²⁺, detected as a DMTr-deprotected form

### Step 3 of Reference Example 57

Compound C6 (0.2 µmol/g, 2-step yield: 1%) was obtained in the same way as in step 5 of Reference Example 10 using a crude product of compound RE57-2.

### Reference Example 58: Synthesis of compound C7

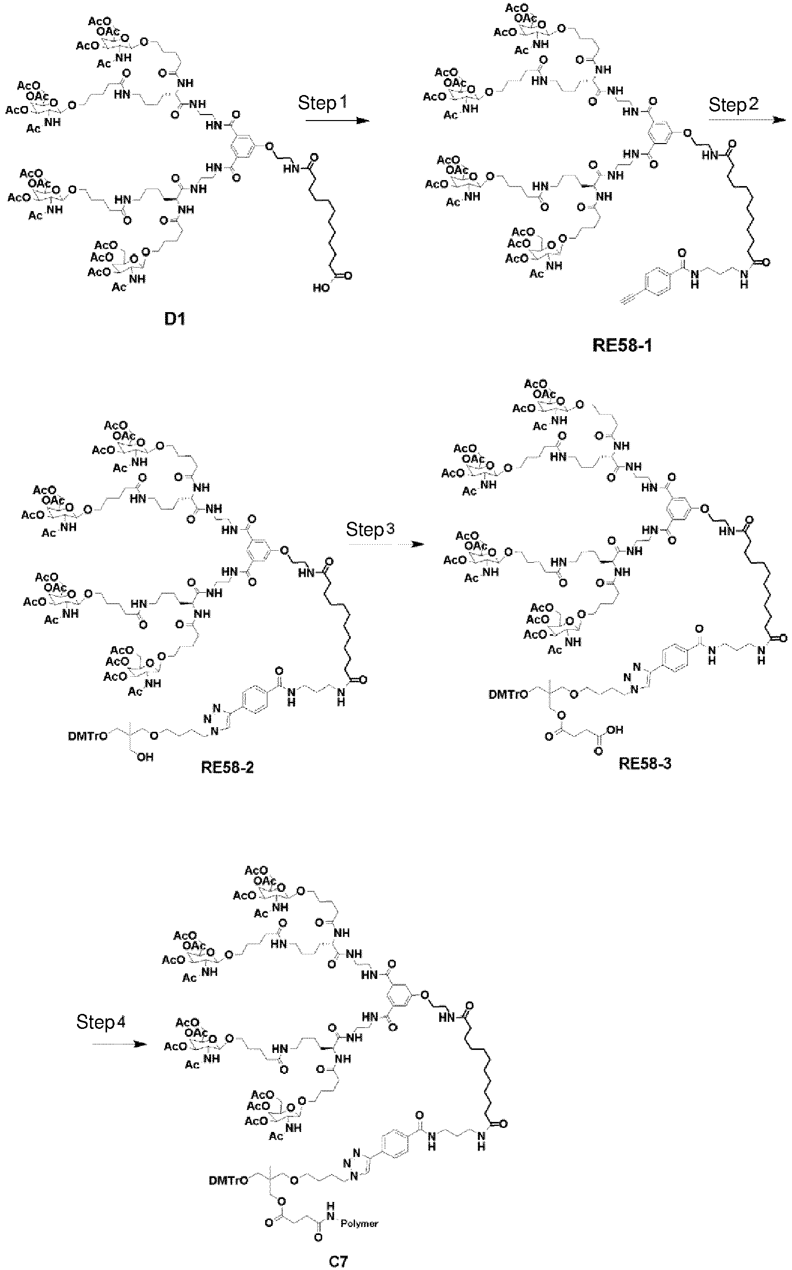

### Step 1 of Reference Example 58

Compound RE58-1 (58 mg, yield: 77%) was obtained in the same way as in step 3 of Reference Example 10 using compound D1 (70 mg, 0.028 mmol) synthesized by the method described in Reference Example 10 and compound RE21-3 of Reference Example 21.
ESI-MS m/z: 1341 (M+2H)²⁺

### Step 2 of Reference Example 58

Compound RE58-1 (58 mg, 0.022 mmol) was dissolved in methanol (0.15 mL). To the solution, compound RE17-1 (16.9 mg, 0.032 mmol) of Reference Example 17 synthesized by the method described in Journal of Organic Chemistry, Vol. 74, p. 6837-6842, 2009, a 1 mol/L aqueous sodium L-ascorbate solution (0.022 mL, 0.022 mmol), a 20 mmol/L aqueous copper(II) sulfate solution (0.011 mL, 0.22 µmol), and a 10 mmol/L solution of tris(2-benzimidazolylmethyl)amine in DMSO (0.022 mL, 0.22 µmol) were added, and the mixture was stirred at room temperature for 3 hours. The reaction solution was purified by silica gel column chromatography (chloroform/methanol = 80/20) to obtain compound RE58-2 (7 mg, yield: 10%).
ESI-MS m/z: 1450 (M+2H)²⁺, detected as a DMTr-deprotected form

### Step 3 of Reference Example 58

A crude product of compound RE58-3 was obtained in the same way as in step 4 of Reference Example 10 using compound RE58-2 (10 mg, 3.13 µmol).
ESI-MS m/z: 1500 (M+2H)²⁺, detected as a DMTr-deprotected form

### Step 4 of Reference Example 58

Compound C7 (8.4 µmol/g, yield: 27%) was obtained in the same way as in step 5 of Reference Example 10 using a crude product of compound RE58-3.

### Reference Example 59

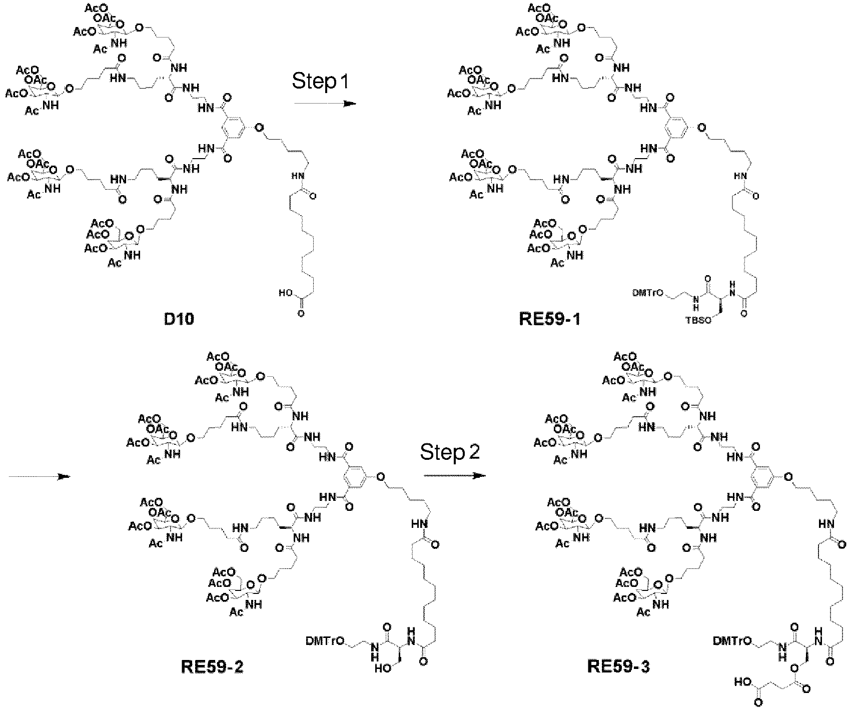

### Step 1 of Reference Example 59

A crude product of compound RE59-1 was obtained in the same way as in step 3 of Reference Example 3 or by the method described in Bioconjugate Chemistry, Vol. 26, p. 1451-1455, 2015 using compound D10 (74.1 mg, 0.029 mmol) synthesized by the method described in Reference Example 46 and compound RE22-2 (15 mg, 0.027 mmol) of Reference Example 22.
ESI-MS m/z: 1392(M +H)⁺, detected as a DMTr-deprotected form

### Step 2 of Reference Example 59

A crude product of compound RE59-2 was obtained by the method described in International Publication No. WO 2015105083 using compound RE59-1 (0.083 g, 0.027 mmol). ESI-MS m/z: 2669(M+H)⁺, detected as a DMTr-deprotected form

### Step 3 of Reference Example 59

A crude product of compound RE59-3 was obtained in the same way as in step 4 of Reference Example 10 using compound RE59-2 (0.08 g, 0.027 mmol).
ESI-MS m/z: 1556(M+HOOH-2H)²⁻
¹H-NMR (400 MHz, MeOD): δ 1.45-1.86 (48H, m), 1.93 (12H, s), 1.94 (12H, s), 2.02 (12H, s), 2.13 (12H, s), 2.16-2.28 (17H, m), 2.48 (4H, s), 3.10-3.16 (6H, m), 3.36-3.56 (19H, m), 3.77 (6H, s), 3.80-3.89 (6H, m), 3.98-4.35 (30H, m), 4.53-4.59 (4H, m), 4.66-4.72 (1H, m), 5.04-5.10 (4H, m), 5.31-5.36 (4H, m), 6.81-6.88 (4H, m), 7.17-7.23 (1H, m), 7.26-7.32 (6H, m), 7.38-7.44 (2H, m), 7.54 (2H, br s), 7.90 (1H, br s).

### Reference Example 60

Compounds described in Tables P-1 and P- 2 were obtained in the same way as in steps 1 and 2 of Reference Example 59 using compound D1, and the structure described in Table Z-1 or compound RE20-4 of Reference Example 20.

The mass spectrometry results of the compounds synthesized in this Example are shown in Table P-3.

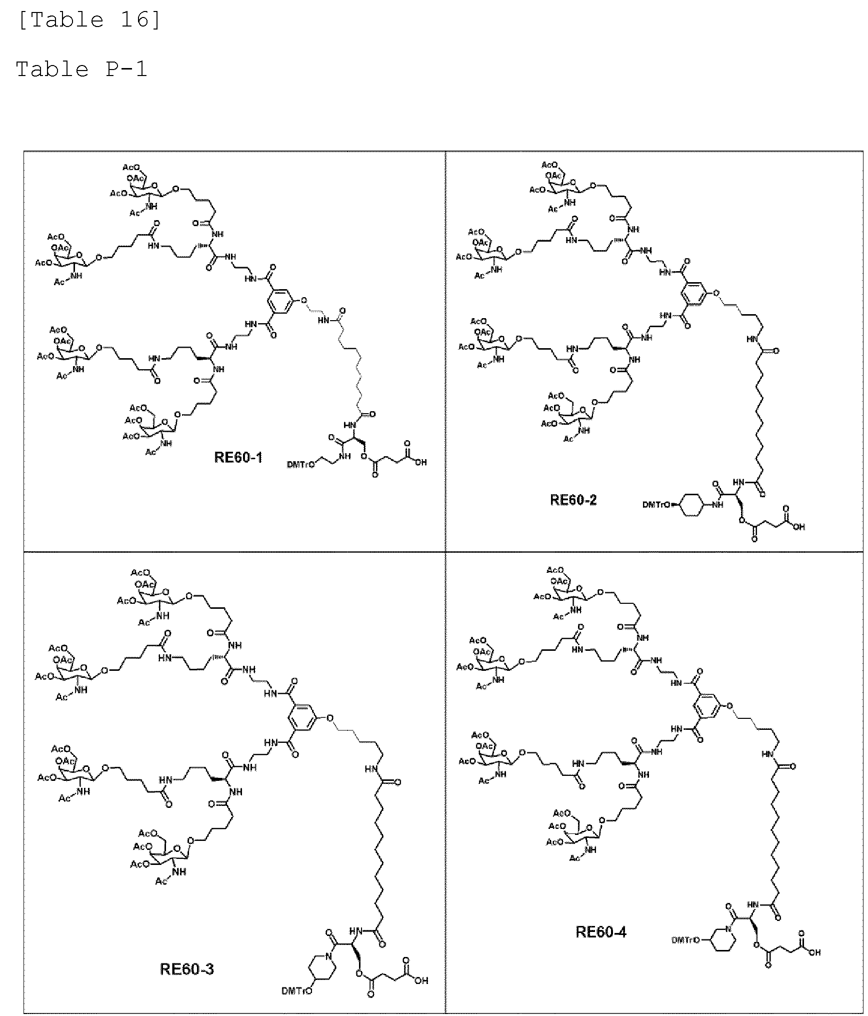

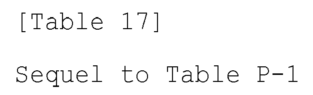

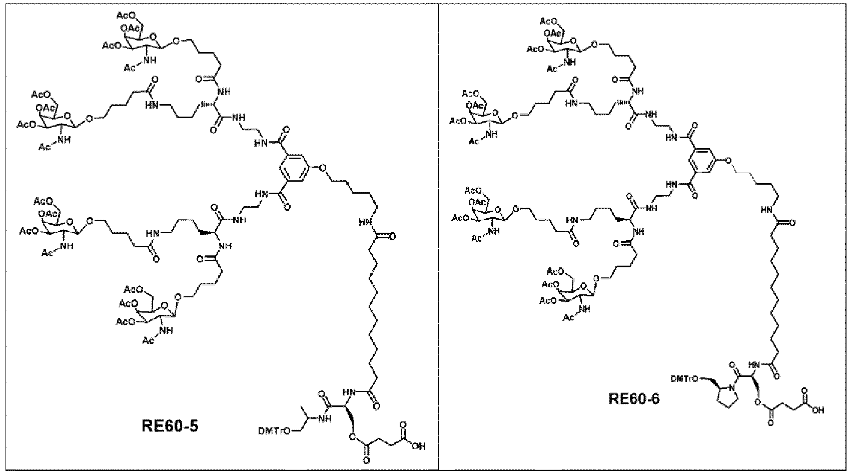

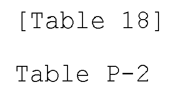

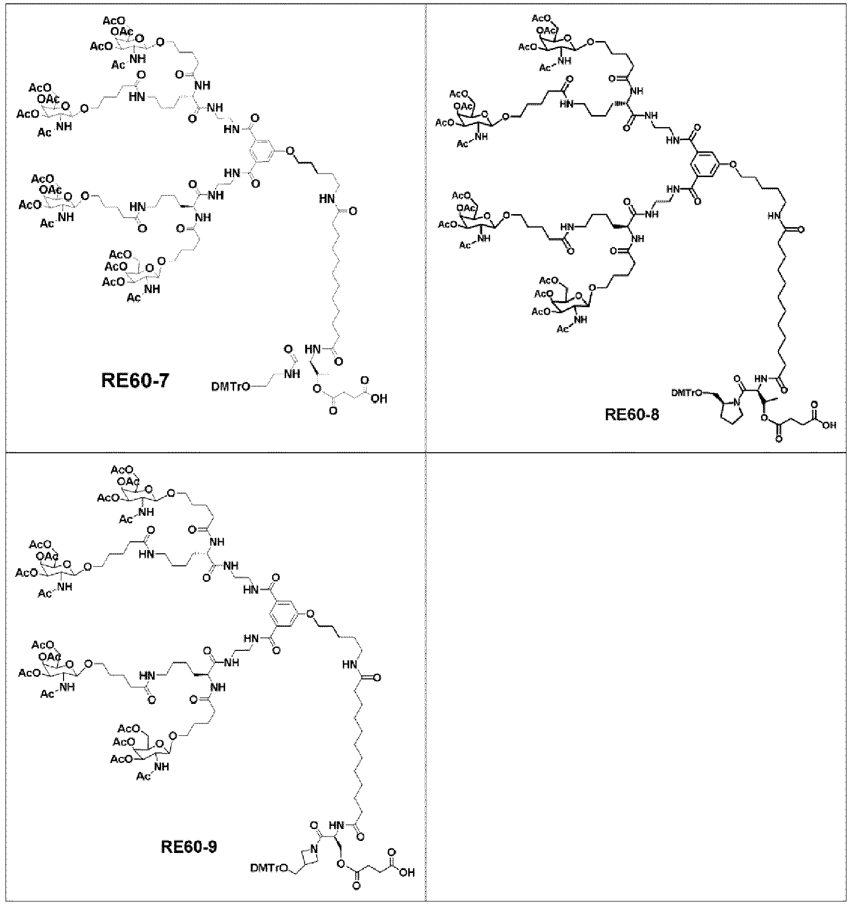

### [Table 19]

**Table P-3**

| Compounds of Reference Example 60 | ESI-MS m/z |
|---|---|
| RE60-1 | 1537 (M+HCOOH-2H)²⁻ |
| RE602 | 1584 (M+HCOOH-2H)²⁻ |
| RE60-3 | 1577 (M+HCOOH-2H)²⁻ |
| RE60-4 | 1577 (M+HCOOH-2H)²⁻ |
| RE60-5 | 1564 (M+HCOOH-2H)²⁻ |
| RE60-6 | 1578 (M+HCOOH-2H)²⁻ |
| RE60-7 | 1564 (M+HCOOH-2H)²⁻ |
| RE60-8 | 1584 (M+HCOOH-2H)²⁻ |
| RE60-9 | 1570 (M+HCOOH-2H)²⁻ |

### Reference Example 61: Synthesis of compound C8

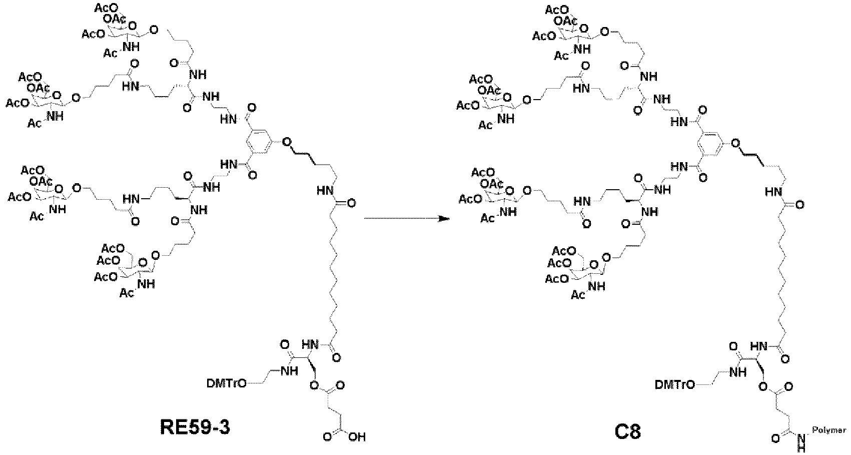

Compound C8 (10.0 µmol/g) was obtained in the same way as in step 5 of Reference Example 10 using compound RE59-3 described in Reference Example 59.

### Reference Example 62: Synthesis of C9 to C17

Compounds described in Tables Q-1 and Q-2 were obtained in the same way as in Reference Example 61 using the compounds described in Tables P-1 and P-2.

The supported amounts of the compounds synthesized in this Example are shown in Table Q-3.

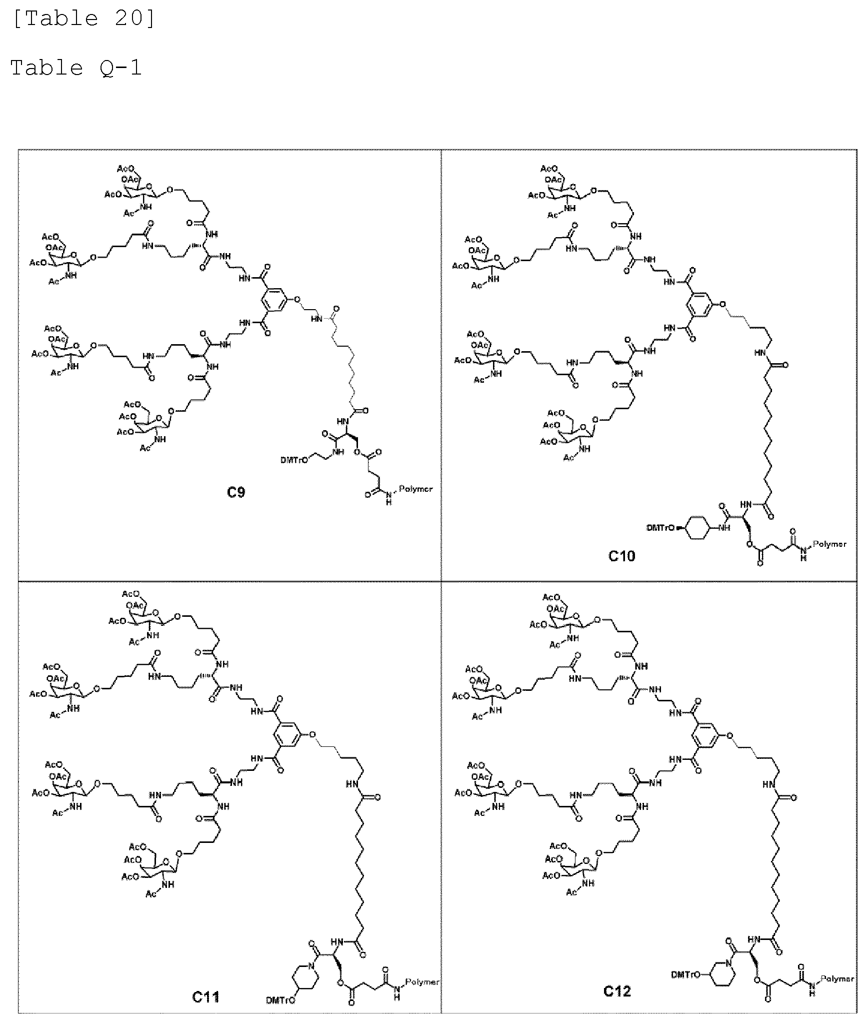

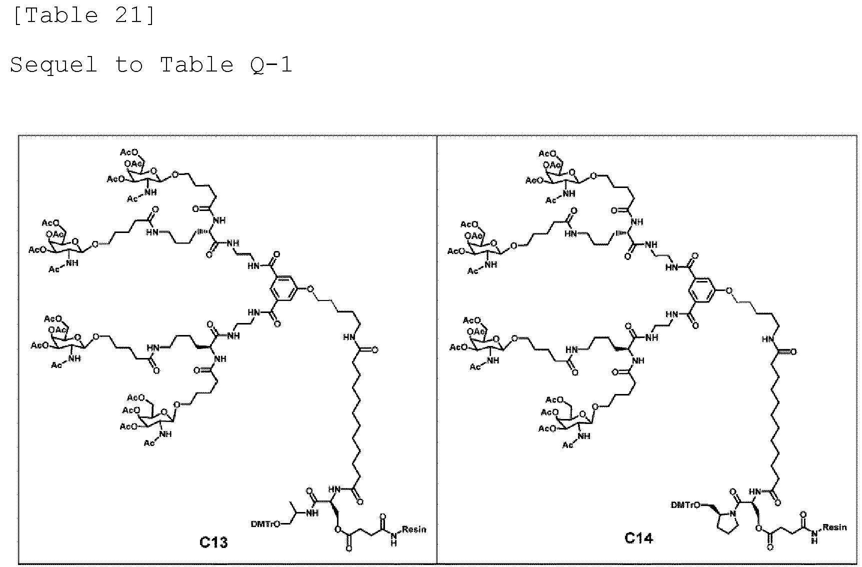

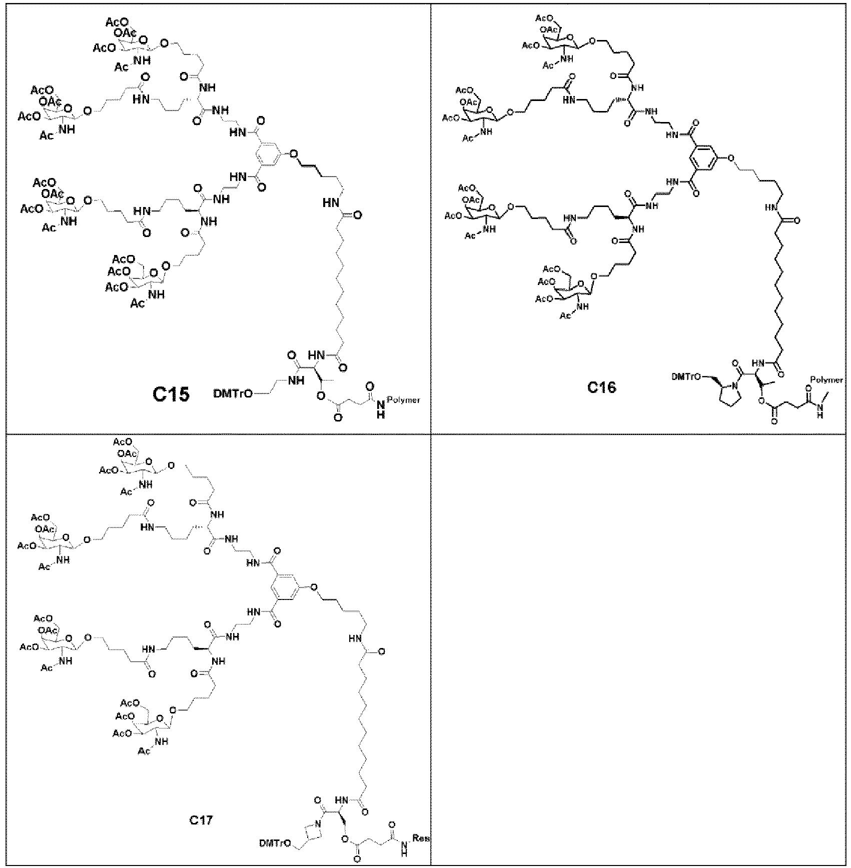

### [Table 23]

**Table Q-3**

| Compound | Supported amount (µmοl/g) |
|---|---|
| C9 | 2.6 |
| C10 | 23.1 |
| C11 | 22.2 |
| C12 | 32.7 |
| C13 | 30.5 |
| C14 | 18.1 |
| C15 | 20.4 |
| C16 | 12.7 |
| C17 | 2.2 |

### Example 1: Synthesis of compounds 1-1 and 1-2

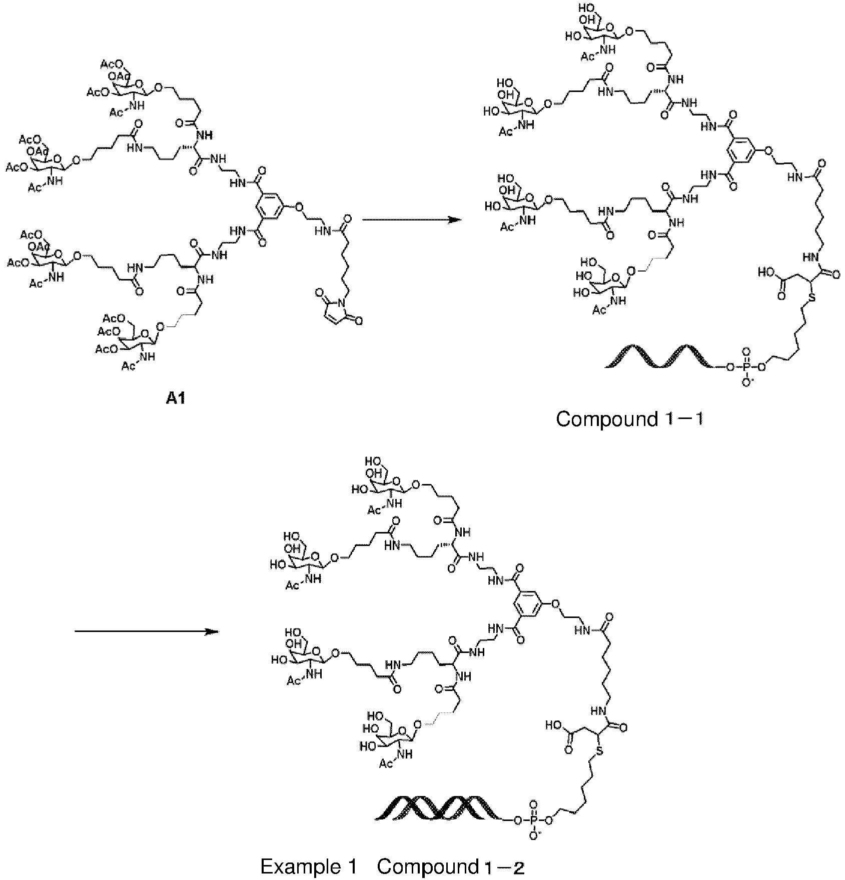

### Step 1

Reference Example compound A1 and a terminally thiolated oligonucleotide synthesized by the method described in Molecules, Vol. 17, p. 13825-13843, 2012 were added and left standing at room temperature for 4 hours. Sodium carbonate was added to the reaction mixture, and the mixture was left standing overnight at 4°C. Compound 1-1 was obtained by purification by any method of anion-exchange chromatography (GE Healthcare Japan Corp., Mono Q 5/50 GL, 10 µm, 5.0 mm x 50 mm, solution A: 10 mmol/L Tris buffer solution/30% acetonitrile, solution B: gradient with 10 mmol/L Tris buffer solution/30% acetonitrile/1 mol/L NaBr) and reverse-phase liquid chromatography (Waters Corp., X Bridge C18, 5 µm, 4.6 mm x 250 mm, 0.1 mol/L triethylammonium acetate buffer solution, solution B: gradient with acetonitrile). The sense strand sequence is as described in Tables R-1 and R-2.

### Step 2

Compound 1-1 (3'-APCS-ssRNA) synthesized in step 1 was concentration-adjusted (50 µmol/L) with a mixed buffer solution (100 mmol/L potassium acetate, 30 mmol/L 2-[4-(2-hydroxyethyl)piperazin-1-yl]ethanesulfonic acid, HEPES)-KOH (pH 7.4), 2 mmol/L magnesium acetate). The sense strand mentioned above and an antisense strand (50 µmol/L) were mixed in equal amounts and left standing at 80°C for 10 minutes. The antisense strand sequence is as described in Tables R-3 and R-4. The temperature was gradually decreased, and the resultant was left standing at 37°C for 1 hour to obtain double-stranded compound 1-2.

### Examples 2 to 10: Synthesis of compounds 2-1 to 10-1 and compounds 2-2 to 10-2

Compounds 2-1 to 10-1 and compounds 2-2 to 10-2 were synthesized in the same way as in Example 1 using oligonucleotides having a nucleotide sequence different from that of Example 1.

### Example 11: Synthesis of compound 11-1 and compound 11-2

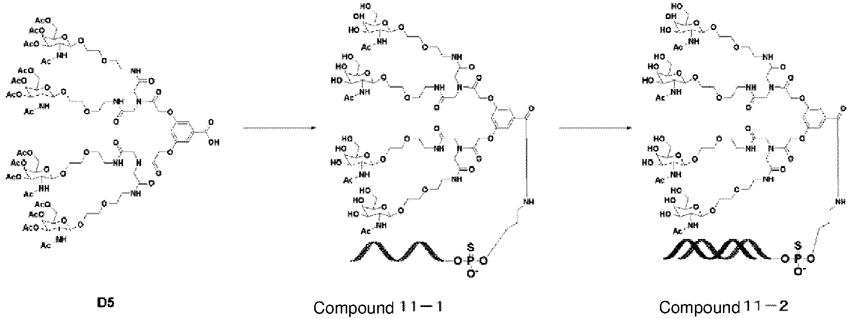

### Step 1

Reference Example compound D5 and a terminally amino group-modified oligonucleotide synthesized by the method described in Molecules, Vol. 17, p. 13825-13843, 2012 were added and reacted by the method described in Bioconjugate Chemistry, Vol. 22, p. 1723-1728, 2011 or Bioconjugate Chemistry, Vol. 26, p. 1451-1455, 2015. Compound 11-1 was obtained by purification by the method described in Example 1.

### Step 2

Compound 11-2 was obtained in the same way as in step 2 of Example 1.

Examples 12 to 43: Synthesis of compounds 12-1 to 43-1 and compounds 12-2 to 43-2

Compounds 12-1 to 43-1 and compounds 12-2 to 43-2 were synthesized in the same way as in Example 11 using oligonucleotides having a nucleotide sequence different from that of Example 11.

The ligand-linker structures of the compounds synthesized in these Examples are shown below.

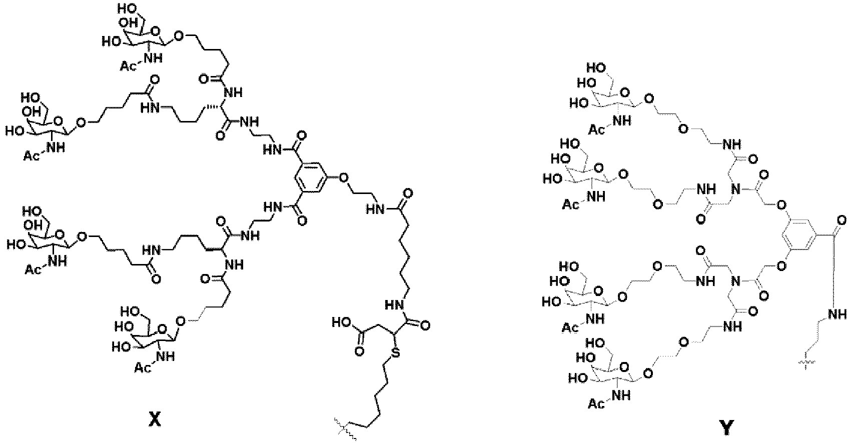

The sequences (sense strands) and mass spectrometry results of the nucleic acid conjugates synthesized in these Examples are shown in Tables R-1 and R-2. In Tables R-1 and R-2, N(M) represents 2'-O-methyl-modified RNA; N(F) represents 2'-fluorine-modified RNA; p represents 5' terminal phosphorylation; ^ represents phosphorothioate; X represents a residue of Reference Example compound A1; and Y represents a residue of Reference Example compound D5. The nucleic acid conjugates are constituted by double-stranded nucleic acids of sense strands consisting of ribonucleotides shown in SEQ ID NOs: 2069 to 2111 and antisense strands consisting of ribonucleotides shown in SEQ ID NOs: 2112 to 2154 (the sense strand represented by SEQ ID NO: n (n = 2069 to 2111) and the antisense strand represented by SEQ ID NO: [n + 43] are paired).

### [Table 24]

**Table R-1**

| Compound | SEQ ID NO: | Sense strand sequence (5'--->3') | Calcd | Found |
|---|---|---|---|---|
| 1-1 | SEQ ID NO: 2069 | U(F)^A(M)^C(F)C(M)U(F)U(M)G(F)U(M)G(F)U(M)U(F)U (F)U(M)C(F)G(M)A(F)G(M)C(F)C(M)U(F)A(M) X | 8970.75 | 8967.77 |
| 2-1 | SEQ ID NO: 2070 | C(F)^C(M)^A(F)G(M)U(F)C(M)C(F)U(M)U(F)U(M)G(F)U (F)G(M)G(F)G(M)A(F)G(M)A(F)G(M)A(F)U(M) X | 9111.87 | 9108.45 |
| 3-1 | SEQ ID NO: 2071 | G(F)^A(M)^G(F)A(M)G(F)A(M)U(F)U(M)G(F)G(M)G(F)G (F)A(M)U(F)U(M)U(F)G(M)U(F)A(M)C(F)A(M)_X | 9199.95 | 9195.61 |
| 4-1 | SEQ ID NO: 2072 | U(F)^G(M)^G(F)G(M)U(F)C(M)U(F)G(M)A(F)G(M)G(F)U (F)C(M)U(F)U(M)G(F)A(M)C(F)U(M)C(F)A(M) X | 9088.83 | 9087.45 |
| 5-1 | SEQ ID NO: 2073 | A(F)^G(M)^A(F)G(M)C(F)A(M)C(F)U(M)U(F)G(M)A(F)A (F)A(M)A(F)U(M)G(F)A(M)A(F)A(M)U(F)G(M) X | 9174.03 | 9172.3 |
| 6-1 | SEQ ID NO: 2074 | G(F)^C(M)^A(F)A(M)C(F)U(M)G(F)G(M)A(F)U(M)A(F)C (F)U(M)A(F)G(M)A(F)U(M)C(F)U(M)U(F)A(M) X | 9063.9 | 9060.12 |
| 7-1 | SEQ ID NO: 2075 | U(F)^C(M)^U(F)G(M)C(F)A(M)G(F)C(M)U(F)C(M)U(F)U (F)U(M)C(F)U(M)U(F)C(M)U(F)U(M)U(F)G(M) X | 8884.65 | 8882.08 |
| 8-1 | SEQ ID NO: 2076 | G(F)^C(M)^A(F)G(M)C(F)U(M)C(F)U(M)U(F)U(M)C(F)U (F)U(M)C(F)U(M)U(F)U(M)G(F)A(M)A(F)U(M) X | 8931.72 | 8929.41 |
| 9-1 | SEQ ID NO: 2077 | C(F)^A(M)^G(F)C(M)U(F)C(M)U(F)U(M)U(F)C(M)U(F)U (F)C(M)U(F)U(M)U(F)G(M)A(F)A(M)U(F)U(M) X | 8892.69 | 8889.39 |
| 10-1 | SEQ ID NO: 2078 | U(F)^U(M)^G(F)A(M)A(F)U(M)U(F)U(M)C(F)C(M)U(F)A (F)U(M)C(F)U(M)G(F)U(M)A(F)U(M)G(F)U(M). X | 8956.74 | 8955.26 |
| 11-1 | SEQ ID NO: 2079 | U(F)^A(M)^C(F)C(M)U(F)U(M)G(F)U(M)G(F)U(M)U(F)U (F)U(M)C(F)G(M)A(F)G(M)C(F)C(M)U(F)A(M)^_Y | 8579.1 | 8577.55 |
| 12-1 | SEQ ID NO: 2080 | C(F)^C(M)^A(F)G(M)U(F)C(M)C(F)U(M)U(F)U(M)G(F)U (F)G(M)G(F)G(M)A(F)G(M)A(F)G(M)A(F)U(M)^_Y | NT | NT |
| 13-1 | SEQ ID NO: 2081 | G(F)^A(M)^G(F)A(M)G(F)A(M)U(F)U(M)G(F)G(M)G(F)G (F)A(M)U(F)U(M)U(F)G(M)U(F)A(M)C(F)A(M)^ Y | NT | NT |
| 14-1 | SEQ ID NO: 2082 | U(F)^G(M)^G(F)G(M)U(F)C(M)U(F)G(M)A(F)G(M)G(F)U (F)C(M)U(F)U(M)G(F)A(M)C(F)U(M)C(F)A(M)^ Y | NT | NT |
| 15-1 | SEQ ID NO: 2083 | A(F)^G(M)^A(F)G(M)C(F)A(M)C(F)U(M)U(F)G(M)A(F)A (F)A(M)A(F)U(M)G(F)A(M)A(F)A(M)U(F)G(M)^ Y | 8782.38 | 8781.1 |
| 16-1 | SEQ ID NO: 2084 | G(F)^C(M)^A(F)A(M)C(F)U(M)G(F)G(M)A(F)U(M)A(F)C (F)U(M)A(F)G(M)A(F)U(M)C(F)U(M)U(F)A(M)^ Y | NT | NT |
| 17-1 | SEQ ID NO: 2085 | U(F)^C(M)^U(F)G(M)C(F)A(M)G(F)C(M)U(F)C(M)U(F)U (F)U(M)C(F)U(M)U(F)C(M)U(F)U(M)U(F)G(M)^ Y | 8493.00 | 8496.61 |
| 18-1 | SEQ ID NO: 2086 | G(F)^C(M)^A(F)G(M)C(F)U(M)C(F)U(M)U(F)U(M)C(F)U (F)U(M)C(F)U(M)U(F)U(M)G(F)A(M)A(F)U(M)^ Y | 8540.07 | 8542.96 |
| 19-1 | SEQ ID NO: 2087 | C(F)^A(M)^G(F)C(M)U(F)C(M)U(F)U(M)U(F)C(M)U(F)U (F)C(M)U(F)U(M)U(F)G(M)A(F)A(M)U(F)U(M)^_Y | 8501.04 | 8501.97 |
| 20-1 | SEQ ID NO: 2088 | U(F)^U(M)^G(F)A(M)A(F)U(M)U(F)U(M)C(F)C(M)U(F)A (F)U(M)C(F)U(M)G(F)U(M)A(F)U(M)G(F)U(M)^ Y | 8565.09 | 8565.86 |
| 21-1 | SEQ ID NO: 2089 | U(F)^A(M)^C(F)C(M)U(F)U(M)G(F)U(M)G(F)U(M)U(F)U (F)U(M)C(F)G(M)A(F)G(M)C(F)C(M)U(M)A(F)^_Y | NT | NT |
| 22-1 | SEQ ID NO: 2090 | U(M)^A(M)^C(M)C(M)U(M)U(M)G(F)U(M)G(F)U(M)U(F)U (F)U(M)C(M)G(M)A(M)G(M)C(M)C(M)U(M)A(M)^ Y | NT | NT |
| 23-1 | SEQ ID NO: 2091 | U(M)^A(M)^C(F)C(M)U(F)U(M)G(F)U(M)G(F)U(M)U(F)U (F)U(M)C(F)G(M)A(F)G(M)C(M)C(M)U(M)A(M)^.Y | NT | NT |
| 24-1 | SEQ ID NO: 2092 | U(M)^G(M)^G(M)G(M)U(M)C(M)U(F)G(M)A(F)G(M)G(F)U (F)C(M)U(M)U(M)G(M)A(M)C(M)U(M)C(M)A(M)^ Y | NT | NT |
| 25-1 | SEQ ID NO: 2093 | U(M)^G(M)^G(F)G(M)U(F)C(M)U(F)G(M)A(F)G(M)G(F)U (F)C(M)U(F)U(M)G(F)A(M)C(M)U(M)C(M)A(M)^ Y | 8733.27 | 8733.46 |

### [Table 25]

**Table R-2**

| Compound | SEQ ID NO: | strand sequence (5'--->3') | Calcd | Found |
|---|---|---|---|---|
| 26-1 | SEQ ID NO: 2094 | A(F)^G(M)^A(F)G(M)C(F)A(M)C(F)U(M)U(F)G(M)A(F)A (F)A(M)A(F)U(M)G(F)A(M)A(F)A(M)U(M)G(F)^_Y | NT | NT |
| 27-1 | SEQ ID NO: 2095 | A (M) ^G (M) ^A (M) G (M) C (M) A (M) C (F) U (M) U (F) G (M) A (F) A (F) A (M) A (M) U (M) G (M) A (M) A (M) A (M) U (M) G (M) ^_Y | NT | NT |
| 28-1 | SEQ ID NO: 2096 | A(M)^G(M)^A(F)G(M)C(F)A(M)C(F)U(M)U(F)G(M)A(F)A (F)A(M)A(F)U(M)G(F)A(M)A(M)A(M)U(M)G(M)^_Y | NT | NT |
| 29-1 | SEQ ID NO: 2097 | U(F)^C(M)^U(F)G(M)C(F)A(M)G(F)C(M)U(F)C(M)U(F)U (F)U(M)C(F)U(M)U(F)C(M)U(F)U(M)U(M)G(F)^_Y | NT | NT |
| 30-1 | SEQ ID NO: 2098 | U (M) ^C (M) ^U (M) G (M) C (M) A (M) G (F) C (M) U (F) C (M) U (F) U (F) U (M) C (M) U (M) U (M) C (M) U (M) U (M) U (M) G (M) ^_Y | NT | NT |
| 31-1 | SEQ ID NO: 2099 | U(M)^C(M)^U(F)G(M)C(F)A(M)G(F)C(M)U(F)C(M)U(F)U (F)U(M)C(F)U(M)U(F)C(M)U(M)U(M)U(M)G(M)^_Y | NT | NT |
| 32-1 | SEQ ID NO: 2100 | G(M)^C(M)^A(M)G(M)C(M)U(M)C(F)U(M)U(F)U(M)C(F)U (F)U(M)C(M)U(M)U(M)U(M)G(M)A(M)A(M)U(M)^_Y | NT | NT |
| 33-1 | SEQ ID NO: 2101 | G(M)^C(M)^A(F)G(M)C(F)U(M)C(F)U(M)U(F)U(M)C(F)U (F)U(M)C(F)U(M)U(F)U(M)G(M)A(M)A(M)U(M)^_Y | 8576.16 | 8574.89 |
| 34-1 | SEQ ID NO: 2102 | C(M)^A(M)^G(M)C(M)U(M)C(M)U(F)U(M)U(F)C(M)U(F)U (F)C(M)U(M)U(M)U(M)G(M)A(M)A(M)U(M)U(M)^_Y | NT | NT |
| 35-1 | SEQ ID NO: 2103 | C(M)^A(M)^G(F)C(M)U(F)C(M)U(F)U(M)U(F)C(M)U(F)U (F)C(M)U(F)U(M)U(F)G(M)A(M)A(M)U(M)U(M)^_Y | 8537.13 | 8537.07 |
| 36-1 | SEQ ID NO: 2104 | U(M)^U(M)^G(M)A(M)A(M)U(M)U(F)U(M)C(F)C(M)U(F)A (F)U(M)C(M)U(M)G(M)U(M)A(M)U(M)G(M)U(M)^_Y | NT | NT |
| 37-1 | SEQ ID NO: 2105 | U(M)^U(M)^G(F)A(M)A(F)U(M)U(F)U(M)C(F)C(M)U(F)A (F)U(M)C(F)U(M)G(F)U(M)A(M)U(M)G(M)U(M)^_Y | NT | NT |
| 38-1 | SEQ ID NO: 2106 | A(F)^G(M)^A(F)G(M)C(F)A(M)C(F)U(M)U(F)G(M)A(F)A (F)A(M)A(F)U(M)G(F)A(M)A(F)A(M)U(F)A(M)^_Y | NT | NT |
| 39-1 | SEQ ID NO: 2107 | A(M)^G(M)^A(M)G(M)C(M)A(M)C(F)U(M)U(F)G(M)A(F)A (F)A(M)A(M)U(M)G(M)A(M)A(M)A(M)U(M)A(M)^_Y | NT | NT |
| 40-1 | SEQ ID NO: 2108 | A(M)^G(M)^A(F)G(M)C(F)A(M)C(F)U(M)U(F)G(M)A(F)A (F)A(M)A(F)U(M)G(F)A(M)A(M)A(M)U(M)A(M)^_Y | NT | NT |
| 41-1 | SEQ ID NO: 2109 | U(F)^C(M)^U(F)G(M)C(F)A(M)G(F)C(M)U(F)C(M)U(F)U (F)U(M)C(F)U(M)U(F)C(M)U(F)U(M)U(F)A(M)^_Y | NT | NT |
| 42-1 | SEQ ID NO: 2110 | U (M) ^C (M) ^U (M) G (M) C (M) A (M) G (F) C (M) U (F) C (M) U (F) U (F) U (M) C (M) U (M) U (M) C (M) U (M) U (M) U (M) A (M) ^_Y | NT | NT |
| 43-1 | SEQ ID NO: 2111 | U(M)^C(M)^U(F)G(M)C(F)A(M)G(F)C(M)U(F)C(M)U(F)U (F)U(M)C(F)U(M)U(F)C(M)U(M)U(M)U(M)A(M)^_Y | NT | NT |

The sequences of the nucleic acid conjugates synthesized in these Examples are shown in Tables R-3 and R-4. In Tables R-3 and R-4, N(M) represents 2'-O-methyl-modified RNA; N(F) represents 2'-fluorine-modified RNA; p represents 5' terminal phosphorylation; and ^ represents phosphorothioate.

### [Table 26]

**Table R-3**

| Compound | SEQ ID NO: | Compound No. of sense strand sequence | Antisense strand sequence (5'--->3') |
|---|---|---|---|
| 1-2 | SEQ ID NO: 2112 | 1-1 | pU(F)^A(M)^G(F)G(M)C(F)U(M)C(F)G(M)A(F)A(M)A(M)A(F )C(M)A(F)C(M)A(F)A(M)G(F)G(M)U(F)A(M)^A(F)^A(M) |
| 2-2 | SEQ ID NO: 2113 | 2-1 | pA(F)^U(M)^C(F)U(M)C(F)U(M)C(F)C(M)C(F)A(M)C(M)A(F )A(M)A(F)G(M)G(F)A(M)C(F)U(M)G(F)G(M)^C(F)^U(M) |
| 3-2 | SEQ ID NO: 2114 | 3-1 | pU(F) ^G(M) ^U(F)A(M)C(F)A(M)A(F)A(M)U(F)C(M)C(M)C(F )C(M)A(F)A(M)U(F)C(M)U(F)C(M)U(F)C(M) ^C(F) ^C(M) |
| 4-2 | SEQ ID NO: 2115 | 4-1 | pU(F)^G(M)^A(F)G(M)U(F)C(M)A(F)A(M)G(F)A(M)C(M)C(F )U(M)C(F)A(M)G(F)A(M)C(F)C(M)C(F)A(M)^C(F)^A(M) |
| 5-2 | SEQ ID NO: 2116 | 5-1 | pC(F)^A(M)^U(F)U(M)U(F)C(M)A(F)U(M)U(F)U(M)U(M)C(F )A(M)A(F)G(M)U(F)G(M)C(F)U(M)C(F)U(M)^C(F)^G(M) |
| 6-2 | SEQ ID NO: 2117 | 6-1 | pU(F)^A(M)^A(F)G(M)A(F)U(M)C(F)U(M)A(F)G(M)U(M)A(F )U(M)C(F)C(M)A(F)G(M)U(F)U(M)G(F)C(M)^U(F)^U(M) |
| 7-2 | SEQ ID NO: 2118 | 7-1 | pC(F)^A(M)^A(F)A(M)G(F)A(M)A(F)G(M)A(F)A(M)A(M)G(F )A(M)G(F)C(M)U(F)G(M)C(F)A(M)G(F)A(M)^U(F)^G(M) |
| 8-2 | SEQ ID NO: 2119 | 8-1 | pA(F)^U(M)^U(F)C(M)A(F)A(M)A(F)G(M)A(F)A(M)G(M)A(F )A(M)A(F)G(M)A(F)G(M)C(F)U(M)G(F)C(M)^A(F)^G(M) |
| 9-2 | SEQ ID NO: 2120 | 9-1 | pA(F)^A(M)^U(F)U(M)C(F)A(M)A(F)A(M)G(F)A(M)A(M)G(F )A(M)A(F)A(M)G(F)A(M)G(F)C(M)U(F)G(M)^C(F)^A(M) |
| 10-2 | SEQ ID NO: 2121 | 10-1 | pA(F)^C(M)^A(F)U(M)A(F)C(M)A(F)G(M)A(F)U(M)A(M)G(F )G(M)A(F)A(M)A(F)U(M)U(F)C(M)A(F)A(M)^A(F)^G(M) |
| 11-2 | SEQ ID NO: 2122 | 11-1 | pU(F) ^A(M) ^G(F)G(M)C(F)U(M)C(F)G(M)A(F)A(M)A(M)A(F )C(M)A(F)C(M)A(F)A(M)G(F)G(M)U(F)A(M) ^A(F) ^A(M) |
| 12-2 | SEQ ID NO: 2123 | 12-1 | pA(F) ^U(M) ^C(F)U(M)C(F)U(M)C(F)C(M)C(F)A(M)C(M)A(F )A(M)A(F)G(M)G(F)A(M)C(F)U(M)G(F)G(M) ^C(F) ^U(M) |
| 13-2 | SEQ ID NO: 2124 | 13-1 | pU(F)^G(M)^U(F)A(M)C(F)A(M)A(F)A(M)U(F)C(M)C(M)C(F )C(M)A(F)A(M)U(F)C(M)U(F)C(M)U(F)C(M)^C(F)^C(M) |
| 14-2 | SEQ ID NO: 2125 | 14-1 | pU(F) ^G(M) ^A(F)G(M)U(F)C(M)A(F)A(M)G(F)A(M)C(M)C(F )U(M)C(F)A(M)G(F)A(M)C(F)C(M)C(F)A(M) ^C(F) ^A(M) |
| 15-2 | SEQ ID NO: 2126 | 15-1 | pC(F)^A(M)^U(F)U(M)U(F)C(M)A(F)U(M)U(F)U(M)U(M)C(F )A(M)A(F)G(M)U(F)G(M)C(F)U(M)C(F)U(M)^C(F)^G(M) |
| 16-2 | SEQ ID NO: 2127 | 16-1 | pU(F) ^A(M) ^A(F) G(M) A(F) U(M) C(F) U(M) A(F) G(M) U(M) A(F ) U(M) C(F) C(M) A(F) G(M) U(F) U(M) G(F) C(M) ^U(F) ^U(M) |
| 17-2 | SEQ ID NO: 2128 | 17-1 | pC(F)^A(M)^A(F)A(M)G(F)A(M)A(F)G(M)A(F)A(M)A(M)G(F )A(M)G(F)C(M)U(F)G(M)C(F)A(M)G(F)A(M)^U(F)^G(M) |
| 18-2 | SEQ ID NO: 2129 | 18-1 | pA(F)^U(M)^U(F)C(M)A(F)A(M)A(F)G(M)A(F)A(M)G(M)A(F )A(M)A(F)G(M)A(F)G(M)C(F)U(M)G(F)C(M)^A(F)^G(M) |
| 19-2 | SEQ ID NO: 2130 | 19-1 | pA(F)^A(M)^U(F)U(M)C(F)A(M)A(F)A(M)G(F)A(M)A(M)G(F )A(M)A(F)A(M)G(F)A(M)G(F)C(M)U(F)G(M)^C(F)^A(M) |
| 20-2 | SEQ ID NO: 2131 | 20-1 | pA(F)^C(M)^A(F)U(M)A(F)C(M)A(F)G(M)A(F)U(M)A(M)G(F )G(M)A(F)A(M)A(F)U(M)U(F)C(M)A(F)A(M)^A(F)^G(M) |
| 21-2 | SEQ ID NO: 2132 | 21-1 | pU(M)^A(F)^G(F)G(M)C(F)U(M)C(F)G(M)A(F)A(M)A(M)A(F )C(M)A(F)C(M)A(F)A(M)G(F)G(M)U(F)A(M)^A(F)^A(M) |
| 22-2 | SEQ ID NO: 2133 | 22-1 | pU(M)^A(F)^G(F)G(F)C(F)U(F)C(F)G(F)A(F)A(M)A(M)A(F )C(M)A(F)C(M)A(F)A(F)G(F)G(F)U(F)A(F)^A(F)^A(M) |
| 23-2 | SEQ ID NO: 2134 | 23-1 | pU(M)^A(F)^G(F)G(M)C(F)U(M)C(F)G(M)A(F)A(M)A(M)A(F )C(M)A(F)C(M)A(F)A(M)G(F)G(M)U(M)A(M)^A(M)^A(M) |
| 24-2 | SEQ ID NO: 2135 | 24-1 | pU(M)^G(F)^A(F)G(F)U(F)C(F)A(F)A(F)G(F)A(M)C(M)C(F )U(M)C(F)A(M)G(F)A(F)C(F)C(F)C(F)A(F)^C(F)^A(M) |
| 25-2 | SEQ ID NO: 2136 | 25-1 | pU(M) ^G(F) ^A(F)G(M)U(F)C(M)A(F)A(M)G(F)A(M)C(M)C(F )U(M)C(F)A(M)G(F)A(M)C(F)C(M)C(M)A(M) ^C(M) ^A(M) |

### [Table 27]

**Table R-4**

| Compound | SEQ ID NO: | Compound No. of sense strand sequence | Antisense strand sequence (5'--->3') |
|---|---|---|---|
| 26-2 | SEQ ID NO: 2137 | 26-1 | pC(M)^A(F)^U(F)U(M)U(F)C(M)A(F)U(M)U(F)U(M)U(M)C(F )A(M)A(F)G(M)U(F)G(M)C(F)U(M)C(F)U(M)^C(F)^G(M) |
| 27-2 | SEQ ID NO: 2138 | 27-1 | pC(M)^A(F)^U(F)U(F)U(F)C(F)A(F)U(F)U(F)U(M)U(M)C(F )A(M)A(F)G(M)U(F)G(F)C(F)U(F)C(F)U(F)^C(F)^G(M) |
| 28-2 | SEQ ID NO: 2139 | 28-1 | pC(M)^A(F)^U(F)U(M)U(F)C(M)A(F)U(M)U(F)U(M)U(M)C(F )A(M)A(F)G(M)U(F)G(M)C(F)U(M)C(M)U(M)^C(M)^G(M) |
| 29-2 | SEQ ID NO: 2140 | 29-1 | pC(M) ^A(F) ^A(F)A(M)G(F)A(M)A(F)G(M)A(F)A(M)A(M)G(F )A(M)G(F)C(M)U(F)G(M)C(F)A(M)G(F)A(M) ^U(F) ^G(M) |
| 30-2 | SEQ ID NO: 2141 | 30-1 | pC(M)^A(F)^A(F)A(F)G(F)A(F)A(F)G(F)A(F)A(M)A(M)G(F )A(M)G(F)C(M)U(F)G(F)C(F)A(F)G(F)A(F)^U(F)^G(M) |
| 31.-2 | SEQ ID NO: 2142 | 31-1 | pC(M) ^A(F) ^A(F)A(M)G(F)A(M)A(F)G(M)A(F)A(M)A(M)G(F )A(M)G(F)C(M)U(F)G(M)C(F)A(M)G(M)A(M) ^U(M) ^G(M) |
| 32-2 | SEQ ID NO: 2143 | 32-1 | pA(M)^U(F)^U(F)C(F)A(F)A(F)A(F)G(F)A(F)A(M)G(M)A(F )A(M)A(F)G(M)A(F)G(F)C(F)U(F)G(F)C(F)^A(F)^G(M) |
| 33-2 | SEQ ID NO: 2144 | 33-1 | pA(M)^U(F)^U(F)C(M)A(F)A(M)A(F)G(M)A(F)A(M)G(M)A(F )A(M)A(F)G(M)A(F)G(M)C(F)U(M)G(M)C(M)^A(M)^G(M) |
| 34-2 | SEQ ID NO: 2145 | 31-1 | pA(M)^A(F)^U(F)U(F)C(F)A(F)A(F)A(F)G(F)A(M)A(M)G(F )A(M)A(F)A(M)G(F)A(F)G(F)C(F)U(F)G(F)^C(F)^A(M) |
| 35-2 | SEQ ID NO: 2146 | 35-1 | pA(M)^A(F)^U(F)U(M)C(F)A(M)A(F)A(M)G(F)A(M)A(M)G(F )A(M)A(F)A(M)G(F)A(M)G(F)C(M)U(M)G(M)^C(M)^A(M) |
| 36-2 | SEQ ID NO: 2147 | 36-1 | pA(M)^C(F)^A(F)U(F)A(F)C(F)A(F)G(F)A(F)U(M)A(M)G(F )G(M)A(F)A(M)A(F)U(F)U(F)C(F)A(F)A(F)^A(F)^G(M) |
| 37-2 | SEQ ID NO: 2148 | 37-1 | pA(M)^C(F)^A(F)U(M)A(F)C(M)A(F)G(M)A(F)U(M)A(M)G(F )G(M)A(F)A(M)A(F)U(M)U(F)C(M)A(M)A(M)^A(M)^G(M) |
| 38-2 | SEQ ID NO: 2149 | 38-1 | pU(F)^A(M)^U(F)U(M)U(F)C(M)A(F)U(M)U(F)U(M)U(M)C(F )A(M)A(F)G(M)U(F)G(M)C(F)U(M)C(F)U(M)^C(F)^G(M) |
| 39-2 | SEQ ID NO: 2150 | 39-1 | pU(M)^A(F)^U(F)U(F)U(F)C(F)A(F)U(F)U(F)U(M)U(M)C(F )A(M)A(F)G(M)U(F)G(F)C(F)U(F)C(F)U(F)^C(F)^G(M) |
| 40-2 | SEQ ID NO: 2151 | 40-1 | pU(M)^A(F)^U(F)U(M)U(F)C(M)A(F)U(M)U(F)U(M)U(M)C(F )A(M)A(F)G(M)U(F)G(M)C(F)U(M)C(M)U(M)^C(M)^G(M) |
| 41-2 | SEQ ID NO: 2152 | 41-1 | pU(F)^A(M)^A(F)A(M)G(F)A(M)A(F)G(M)A(F)A(M)A(M)G(F )A(M)G(F)C(M)U(F)G(M)C(F)A(M)G(F)A(M)^U(F)^G(M) |
| 42-2 | SEQ ID NO: 2153 | 42-1 | pU(M)^A(F)^A(F)A(F)G(F)A(F)A(F)G(F)A(F)A(M)A(M)G(F )A(M)G(F)C(M)U(F)G(F)C(F)A(F)G(F)A(F)^U(F)^G(M) |
| 43-2 | SEQ ID NO: 2154 | 43-1 | pU(M)^A(F)^A(F)A(M)G(F)A(M)A(F)G(M)A(F)A(M)A(M)G(F )A(M)G(F)C(M)U(F)G(M)C(F)A(M)G(M)A(M)^U(M)^G(M) |

### Reference Test Example 1: Measurement of knockdown activity of siRNA against APCS mRNA in human cell

The double-stranded nucleic acids described in Tables 1-1 to 1-13 were synthesized by Sigma-Aldrich Co. LLC and used. Specifically, the double-stranded nucleic acids were prepared by annealing sense strands consisting of ribonucleotides shown in SEQ ID NOs: 2 to 644 and antisense strands consisting of ribonucleotides shown in SEQ ID NOs: 645 to 1287 (the sense strand represented by SEQ ID NO: n (n = 2 to 644) and the antisense strand represented by SEQ ID NO: [n + 643] are paired). A siRNA/RNAiMax mixed solution of each double-stranded nucleic acid and RNAiMax transfection reagent (manufactured by Thermo Fisher Scientific Inc., Catalog No. 13778150) diluted with Opti-MEM I Reduced Serum Medium (manufactured by Thermo Fisher Scientific Inc., Catalog No. 31985070) was added at 20 µL/well to 384-well culture plates. Human ovary cancer-derived cell line RMG-I cells (JCRB Cell Bank, JCRB0172) were inoculated at 10,000 cells/40 µL/well to the 384-well culture plates and cultured at 37°C for 24 hours under 5% CO₂ conditions. The medium used was Ham's F-12 Nutrient Mix medium (manufactured by Thermo Fisher Scientific Inc., Catalog No. 11765-047) containing 10% fetal bovine serum (manufactured by Thermo Fisher Scientific Inc., Catalog No. 10091-148). The final concentration of the double-stranded nucleic acid was set to 1 nM. Then, the cells were washed with DPBS, no calcium, no magnesium (manufactured by Thermo Fisher Scientific Inc., Catalog No. 14190-144), and cDNA was synthesized from each of the plates by the following method using TaqMan(R) Fast Cells-to-CT(TM) kit (manufactured by Thermo Fisher Scientific Inc., Catalog No. 4399003). A solution of Lysis solution (included in the kit) and DNase I (included in the kit) mixed at a ratio of 100:1 was added at 10 µL/well and mixed for 5 minutes. Stop Solution (included in the kit) was added at 1 µL/well and mixed for 2 minutes to prepare RNA extracts. 5 µL of 2 × RT Buffer (included in the kit), 0.5 µL of 20 × RT Enzyme Mix (included in the kit), 2.5 µL of Nuclease-free Water, and 2 µL of the RNA extracts were mixed, and this mixture was reacted at 37°C for 60 minutes and at 95°C for 5 minutes to synthesize cDNA. This cDNA was added at 2 µL/well to MicroAmp(R) EnduraPlate(TM) Optical 384-Well Clear Reaction Plates with Barcode (manufactured by Thermo Fisher Scientific Inc., Catalog No. 4483285), and further, 5 µL of TaqMan(R) Fast Universal PCR Master Mix (manufactured by Thermo Fisher Scientific Inc., Catalog No. 4352042), 2.5 µL of DISTILLED WATER (ULTRAPURE) (manufactured by Thermo Fisher Scientific Inc., Catalog No. 10977015), 0.25 µL of human APCS probe, and 0.25 µL of human ACTB probe were added to each well. The real-time PCR of the human APCS gene and the human ACTB (actin beta) gene was performed using QuantStudio(TM) 7 Flex. ACTB, a constitutively expressed gene, was measured as an internal control and used to correct the APCS gene expression level. The amount of APCS mRNA in RMG-I cells treated with only the transfection reagent without the addition of siRNA was defined as 1.0. The relative expression level of APCS mRNA was calculated when each siRNA was transferred. This experiment was conducted twice. The minimum values of the relative expression level of APCS mRNA are shown in Tables 1-1 to 1-13.

### [Table 28]

**Table 1-1**

| Double-stranded nucleic acid No. | SEQ ID NO: | Sense strand sequence (5--->3') | SEQ ID NO: | Antisense strand sequence (5'--->3') | SEQ ID NO: | Target APCS mRNA sequence | Relative APCS expression level |
|---|---|---|---|---|---|---|---|
| AH0001 | SEQ ID NO: 2 | GCAUGAAUAUCAGACGCUAGG | SEQ ID NO: 645 | UAGCGUCUGAUAUUCAUGCCC | SEQ ID NO: 1288 | GCAUGAAUAUCAGACGCUA | 0.305 |
| AH0002 | SEQ ID NO: 3 | CAUGAAUAUCAGACGCUAGGG | SEQ ID NO: 646 | CUAGCGUCUGAUAUUCAUGCC | SEQ ID NO: 1289 | CAUGAAUAUCAGACGCUAG | 0.391 |
| AH0003 | SEQ ID NO: 4 | AUAUCAGACGUAGGGGGACA | SEQ ID NO: 647 | UCCCCCUAGCGUCUGAUAUUC | SEQ ID NO: 1290 | AUAUCAGACGCUAGGGGGA | 0.427 |
| AH0004 | SEQ ID NO: 5 | CUAGGGGGACAGCCACUGUGU | SEQ ID NO: 648 | ACAGUGGCUGUCCCCCUAGCG | SEQ ID NO: 1291 | CUAGGGGGACAGCCACUGU | 0.405 |
| AH0005 | SEQ ID NO: 6 | AGGGGGACAGCCACUGUGUUG | SEQ ID NO: 649 | ACACAGUGGCUGUCCCCCUAG | SEQ ID NO: 1292 | AGGGGGACAGCCACUGUGU | 0.401 |
| AH0006 | SEQ ID NO: 7 | GGGGACAGCCACUGUGUUGUC | SEQ ID NO: 650 | CAACACACUGGCUGUCCCCCU | SEQ ID NO: 1293 | GGGGAOAGCOAOUGUGUUG | 0.456 |
| AH0007 | SEQ ID NO: 8 | GGGACAGCCACUGUGUUGUCU | SEQ ID NO: 651 | ACAACACAGUGGCUGUCCCCC | SEQ ID NO: 1294 | GGGACAGCCACUGUGUUGU | 0.435 |
| AH0008 | SEQ ID NO: 9 | GGACAGCCACUGUGUUGUCUG | SEQ ID NO: 652 | GACAACACAGUGGCUGUCCCC | SEQ ID NO: 1295 | GGACAGCCACUGUGUUGUC | 0.436 |
| AH0009 | SEQ ID NO: 10 | GACAGCCACUGUGUUGUCUGC | SEQ ID NO: 653 | AGACAACACAGUGGCUGUCCC | SEQ ID NO: 1296 | GACAGCCACUGUGUUGUCU | 0.487 |
| AH0010 | SEQ ID NO: 11 | GCCACUGUGUUGUCUGCUACC | SEQ ID NO: 654 | UAGCAGACAACACAGUGGCUG | SEQ ID NO: 1297 | GCCACUGUGUUGUCUGCUA | 0288 |
| AH0011 | SEQ ID NO: 12 | CCACUGUGUUGUCUGCUACCC | SEQ ID NO: 655 | GUAGCAGACAACACAGUGGCU | SEQ ID NO: 1298 | CCACUGUGUUGUCUGCUAC | 0.382 |
| AH0012 | SEQ ID NO: 13 | CACUGUGUUGUCUGCUACCCU | SEQ ID NO: 656 | GGUAGCAGACAACACAGUGGC | SEQ ID NO: 1299 | CACUGUGUUGUCUGCUACC | 0.490 |
| AH0013 | SEQ ID NO: 14 | CUGUGUUGUCUGCUACCCUCA | SEQ ID NO: 657 | AGGGUAGCAGACAACACAGUG | SEQ ID NO: 1300 | CUGUGUUGUCUGCUACCCU | 0.315 |
| AH0014 | SEQ ID NO : 15 | UGUGUUGUCUGCUACCCUCAU | SEQ ID NO: 658 | GAGGGUAGCAGACAACACAGU | SEQ ID NO: 1301 | UGUGUUGUCUGCUACCCUC | 0.360 |
| AH0015 | SEQ ID NO: 16 | GUGUUGUCUGCUACCCUCAUC | SEQ ID NO: 659 | UGAGGGUAGCAGACAACACAG | SEQ ID NO: 1302 | GUGUUGUCUGCUACCCUCA | 0.275 |
| AH0016 | SEQ ID NO: 17 | UGUUGUCUGCUACCCUCAUCC | SEQ ID NO: 660 | AUGAGGGUAGCAGACAACACA | SEQ ID NO: 1303 | UGUUGUCUGCUACCCUCAU | 0.259 |
| AH0017 | SEQ ID NO: 18 | GUUGUCUGCUACCCUCAUCCU | SEQ ID NO: 661 | GAUGAGGGUAGCAGACAACAC | SEQ ID NO: 1304 | GUUGUCUGCUACCCUCAUC | 0.345 |
| AH0018 | SEQ ID NO: 19 | GUCUGCUACCCUCAUCCUGGU | SEQ ID NO: 662 | CAGGAUGAGGGUAGCAGACAA | SEQ ID NO: 1305 | GUCUGCUACCCUCAUCCUG | 0.406 |
| AH0019 | SEQ ID NO: 20 | CUGCUACCCUCAUCCUGGUCA | SEQ ID NO: 663 | ACCAGGAUGAGGGUAGCAGAC | SEQ ID NO: 1306 | CUGCUACCCUCAUCCUGGU | 0.229 |
| AH0020 | SEQ ID NO: 21 | GCUACCCUCAUCCUGGUCACU | SEQ ID NO: 664 | UGACCAGGAUGAGGGUAGCAG | SEQ ID NO: 1307 | GCUACCCUCAUCCUGGUCA | 0.278 |
| AH0021 | SEQ ID NO: 22 | CUACCCUCAUCCUGGUCACUG | SEQ ID NO: 665 | GUGACCAGGAUGAGGGUAGCA | SEQ ID NO: 1308 | CUACCCUCAUCCUGGUCAC | 0.284 |
| AH0022 | SEQ ID NO: 23 | UACCCUCAUCCUGGUCACUGC | SEQ ID NO: 666 | AGUGACCAGGAUGAGGGUAGC | SEQ ID NO: 1309 | UACCCUCAUCCUGGUCACU | 0.234 |
| AH0023 | SEQ ID NO: 24 | ACCCUCAUCCUGGUCACUGCU | SEQ ID NO: 667 | CAGUGACCAGGAUGAGGGUAG | SEQ ID NO: 1310 | ACCCUCAUCCUGGUCACUG | 0.413 |
| AH0024 | SEQ ID NO: 25 | CCCUCAUCCUGGUCACUGCUU | SEQ ID NO: 668 | GCAGUGACCAGGAUGAGGGUA | SEQ ID NO: 1311 | CCCUCAUCCUGGUCACUGC | 0.268 |
| AH0025 | SEQ ID NO: 26 | CCUCAUCCUGGUCACUGCUUC | SEQ ID NO: 669 | AGCAGUGACCAGGAUGAGGGU | SEQ ID NO: 1312 | CCUCAUCCUGGUCACUGCU | 0.232 |
| AH0026 | SEQ ID NO: 27 | CUCAUCCUGGUCACUGCUUCU | SEQ ID NO: 670 | AAGCAGUGACCAGGAUGAGGG | SEQ ID NO: 1313 | CUCAUCCUGGUCACUGCUU | 0.293 |
| AH0027 | SEQ ID NO: 28 | CAUCCUGGUCACUGCUUCUGC | SEQ ID NO: 671 | AGAAGCAGUGACCAGGAUGAG | SEQ ID NO: 1314 | CAUCCUGGUCACUGCUUCU | 0.158 |
| AH0028 | SEQ ID NO: 29 | AUCCUGGUCACUGCUUCUGCU | SEQ ID NO: 672 | CAGAAGCAGUGACCAGGAUGA | SEQ ID NO: 1315 | AUCCUGGUCACUGCUUCUG | 0.286 |
| AH0029 | SEO ID NO: 30 | UCCUGGUCACUGCUUCUGCUA | SEQ ID NO: 673 | GCAGAAGCAGUGACCAGGAUG | SEQ ID NO: 1316 | UCCUGGUCACUGCUUCUGC | 0.304 |
| AH0030 | SEQ ID NO: 31 | CCUGGUCACUGCUUCUGCUAU | SEQ ID NO: 674 | AGCAGAAGCAGUGACCAGGAU | SEQ ID NO: 1317 | CCUGGUCACUGCUUCUGCU | 0.083 |
| AH0031 | SEQ ID NO: 32 | CUGGUCACUGCUUCUGCUAUA | SEQ ID NO: 675 | UAGCAGAAGCAGUGACCAGGA | SEQ ID NO: 1318 | CUGGUCACUGCUUCUGCUA | 0.132 |
| AH0032 | SEQ ID NO: 33 | UGGUCACUGCUUCUGCUAUAA | SEQ ID NO: 676 | AUAGCAGAAGCAGUGACCAGG | SEQ ID NO: 1319 | UGGUCACUGCUUCUGCUAU | 0.172 |
| AH0033 | SEO ID NO: 34 | GGUCACUGCUUCUGCUAUAAC | SEQ ID NO : 677 | UAUAGCAGAAGCAGUGACCAG | SEQ ID NO: 1320 | GGUCACUGCUUCUGCUAUA | 0117 |
| AH0034 | SEQ ID NO: 35 | GUCACUGCUUCUGCUAUAACA | SEQ ID NO: 678 | UUAUAGCAGAAGCAGUGACCA | SEQ ID NO: 1321 | GUCACUGCUUCUGCUAUAA | 0.174 |
| AH0035 | SEQ ID NO: 36 | CACUGCUUGUGCUAUAACAGC | SEQ ID NO: 679 | UGUUAUAGCAGAAGCAGUGAC | SEQ ID NO: 1322 | CACUGCUUCUGCUAUAACA | 0.082 |
| AH0036 | SEQ ID NO: 37 | ACUGCUUCUGCUAUAACAGCC | SEQ ID NO: 680 | CUGUUAUAGCAGAAGCAGUGA | SEQ ID NO: 1323 | ACUGCUUCUGCUAUAACAG | 0.215 |
| AH0037 | SEO ID NO: 38 | CUGCUUCUGCUAUAACAGCCC | SEQ ID NO: 681 | GCUGUUAUAGCAGAAGCAGUG | SEQ ID NO: 1324 | CUGCUUCUGCUAUAACAGC | 0.082 |
| AH0038 | SEQ ID NO: 39 | GCUUCUGCUAUAACAGCCCUA | SEQ ID NO: 682 | GGGCUGUUAUAGCAGAAGCAG | SEQ ID NO: 1325 | GCUUCUGCUAUAACAGCCC | 0.205 |
| AH0039 | SEO ID NO: 40 | CUUCUGCUAUAACAGCCCUAG | SEQ ID NO: 683 | AGGGCUGUUAUAGCAGAAGCA | SEQ ID NO: 1326 | CUUCUGCUAUAACAGCCCU | 0.165 |
| AH0040 | SEQ ID NO: 41 | UUCUGCUAUAACAGCCCUAGG | SEQ ID NO: 684 | UAGGGCUGUUAUAGCAGAAGC | SEQ ID NO: 1327 | UUCUGCUAUAACAGCCCUA | 0.148 |
| AH0041 | SEQ ID NO:42 | UCUGCUAUAACAGCCCUAGGC | SEQ ID NO: 685 | CUAGGGCUGUUAUAGCAGAAG | SEQ ID NO: 1328 | UCUGCUAUAACAGCCCUAG | 0.371 |
| AH0042 | SEQ ID NO: 43 | CUGCUAUAACAGCCCUAGGCC | SEQ ID NO: 686 | CCUAGGGCUGUUAUAGCAGAA | SEQ ID NO: 1329 | CUGCUAUAACAGCCCUAGG | 0.203 |
| AH0043 | SEO ID NO: 44 | UGCUAUAACAGCCCUAGGCCA | SEQ ID NO: 687 | GCCUAGGGCUGUUAUAGCAGA | SEQ ID NO: 1330 | UGCUAUAACAGCCCUAGGC | 0.419 |
| AH0044 | SEQ ID NO: 45 | GCUAUAACAGCCCUAGGCCAG | SEQ ID NO: 688 | GGCCUAGGGCUGUUAUAGCAG | SEQ ID NO: 1331 | GCUAUAACAGCCCUAGGCC | 0.457 |
| AH0045 | SEQ ID NO: 46 | CUAUAACAGCCCUAGGCCAGG | SEQ ID NO: 689 | UGGCCUAGGGCUGUUAUAGCA | SEQ ID NO: 1332 | CUAUAACAGCCCUAGGCCA | 0.115 |
| AH0046 | SEQ ID NO: 47 | UAUAACAGCCCUAGGCCAGGA | SEQ ID NO: 690 | CUGGCCUAGGGCUGUUAUAGC | SEQ ID NO: 1333 | UAUAACAGCCCUAGGCCAG | 0.300 |
| AH0047 | SEQ ID NO: 48 | AUAACAGCCCUAGGCCAGGAA | SEQ ID NO: 691 | CCUGGCCUAGGGCUGUUAUAG | SEQ ID NO: 1334 | AUAACAGCCCUAGGCCAGG | 0.396 |
| AH0048 | SEQ ID NO: 49 | UAACAGCCCUAGGCCAGGAAU | SEQ ID NO: 692 | UCCUGGCCUAGGGCUGUUAUA | SEQ ID NO: 1335 | UAACAGCCCUAGGCCAGGA | 0.479 |
| AH0049 | SEQ ID NO: 50 | ACAGCCCUAGGCCAGGAAUAU | SEQ ID NO: 693 | AUUCCUGGCCUAGGGCUGUUA | SEQ ID NO: 1336 | ACAGCCCUAGGCCAGGAAU | 0.283 |
| AH0050 | SEQ ID NO: 51 | CAGCCCUAGGCCAGGAAUAUG | SEQ ID NO: 694 | UAUUCCUGGCCUAGGGCUGUU | SEQ ID NO: 1337 | CAGCCCUAGGCCAGGAAUA | 0.293 |

### [Table 29]

**Table 1-2**

| Double-stranded nucleic acid No. | SEQ ID NO: | Sense strand sequence (5'--->3') | SEQ ID NO: | Antisense strand sequence (5'--->3') | SEQ ID NO: | Target APCS mRNA sequence | Relative APCS expression level |
|---|---|---|---|---|---|---|---|
| AH0051 | SEQ ID NO: 52 | AGCCCUAGGCCAGGAAUAGA | SEQ ID NO: 695 | AUAUUCCUGGCCUAGGCUGU | SEQ ID NO: 1338 | AGCCCUAGGCCAGGAAUAU | 0.22 |
| AH0052 | SEQ ID NO: 53 | GCCCUAGGCCAGGAAUAUGAA | SEQ ID NO: 696 | CAUAUUCCUGGGCUAGGGCUG | SEQ ID NO: 1339 | GCCCUAGGCCAGGAAUAUG | 0.189 |
| AH0053 | SEQ ID NO: 54 | CCCUAGGCCAGGAAUAUGAAC | SEQ ID NO: 697 | UCAUAUUCCUGGCCUAGGGCU | SEQ ID NO: 1340 | CCCUAGGCCAGGAAUAUGA | 0.158 |
| AH0054 | SEQ ID NO: 55 | CCUAGGCCAGGAAUAUGAACA | SEQ ID NO: 698 | UUCAUAUUCCUGGCCUAGGGG | SEQ ID NO: 1341 | CCUAGGCCAGGAAUAUGAA | 0.188 |
| AH0055 | SEQ ID NO: 56 | CUAGGCCAGGAAUAUGAACAA | SEQ ID NO: 699 | GUUCAUAUUCCUGGCCUAGGG | SEQ ID NO: 1342 | CUAGGCCAGGAAUAUGAAC | 0.433 |
| AH0056 | SEQ ID NO: 57 | UAGGCCAGGAAUAU GAACAAG | SEQ ID NO: 700 | UGUUCAUAUUCCUGGCCUAGG | SEQ ID NO: 1343 | UAGGCCAGGAAUAUGAACA | 0.242 |
| AH0057 | SEQ ID NO: 58 | AGGCCAGGAAUAUGAACAAGC | SEQ ID NO: 701 | UUGUUCAUAUVCCUGGCCUAG | SEQ ID NO: 1344 | AGGCCAGGAAUAUGAAGAA | 0.107 |
| AH0058 | SEQ ID NO: 59 | GGCCAGGAAUAUGAACAAGCC | SEQ ID NO: 702 | CUUGUUCAUAUUCCUGGCCUA | SEQ ID NO: 1345 | GGCCAGGAAUAUGAACAAG | 0.428 |
| AH0059 | SEQ ID NO: 60 | GCCAGGAAUAUGAACAAGCCG | SEQ ID NO: 703 | GCUUGUUCAUAUUCCUGGCCU | SEQ ID NO: 1346 | GCCAGGAAUAUGAACAAGC | 0.221 |
| AH0060 | SEQ ID NO: 61 | CCAGGAAUAUGAACAAGCCGC | SEQ ID NO: 704 | GGCUUGUUCAUAUUCCUGGCC | SEQ ID NO: 1347 | CCAGGAAUAU GAACAAGCC | 0.242 |
| AH0061 | SEQ ID NO: 62 | CAGGAAUAUGAACAAGCCGCU | SEQ ID NO: 705 | CGGCUUGUUCAUAUUCCUGGC | SEQ ID NO: 1348 | CAGGAAUAUGAACAAGCCG | 0.299 |
| AH0062 | SEO ID NO: 63 | AGGAAUAUGAACAAGCCGCUG | SEQ ID NO: 706 | GCGGCUUGUUCAUAUUCCUGG | SEQ ID NO: 1349 | AGGAAUAUGAACAAGCCGC | 0.309 |
| AH0063 | SEQ ID NO: 64 | GGAAUAUGAACAAGCCGCU GC | SEQ ID NO: 707 | AGCGGCUUGUUCAUAUUCCUG | SEQ ID NO: 1350 | GGAAUAUGAACAAGCCGCU | 0.113 |
| AH0064 | SEQ ID NO: 65 | GAAUAUGAACAAGCCGCUGCU | SEQ ID NO: 708 | CAGCGGCUUGUUCAUAUUCCU | SEQ ID NO: 1351 | GAAUAUGAACAAGCCGCUG | 0.103 |
| AH0065 | SEQ ID NO: 66 | AAUAUGAACAAGCCGCUGCUU | SEQ ID NO: 709 | GCAGCGGCUUGUUCAUAUUCC | SEQ ID NO: 1352 | AAUAUGAACAAGCCGCUGC | 0.444 |
| AH0066 | SEQ ID NO: 67 | AUAUGAACAAGCCGCUGCUUU | SEQ ID NO: 710 | AGCAGCGGCUUGUUCAUAUUC | SEQ ID NO: 1353 | AUAUGAACAAGCCGCUGCU | 0.198 |
| AH0067 | SEQ ID NO: 68 | UAUGAACAAGCCGCUGCUUUG | SEQ ID NO: 711 | AAGCAGCGGCUUGUUCAUAUU | SEQ ID NO: 1354 | UAUGAACAAGCCGCUGCUU | 0.464 |
| AH0068 | SEQ ID NO: 69 | AUGAACAAGCCGCUGCUUUGG | SEO ID NO: 712 | AAAGCAGCGGCUUGUUCAUAU | SEQ ID NO: 1355 | AUGAACAAGCCGCUGCUUU | 0.190 |
| AH0069 | SEQ ID NO: 70 | UGAACAAGCCGCUGCUUUGGA | SEQ ID NO: 713 | CAAAGCAGCGGCUUGUUCAUA | SEQ ID NO: 1356 | UGAACAAGCCGCUGCUUUG | 0.150 |
| AH0070 | SEQ ID NO: 71 | GAACAAGCCGCUGCUUUGGAU | SEQ ID NO: 714 | CCAAAGCAGCGGCUUGUUCAU | SEQ ID NO: 1357 | GAACAAGCCGCUGCUUUGG | 0.149 |
| AH0071 | SEQ ID NO: 72 | AACAAGCCGCUGCUUUGGAUC | SEQ ID NO: 715 | UCCAAAGCAGCGGCUUGUUCA | SEQ ID NO: 1358 | AACAAGCCGCUGCUUUGGA | 0.180 |
| AH0072 | SEO ID NO: 73 | ACAAGCCGCyGCUyUGGAUCU | SEQ ID NO: 716 | AUCCAAAGCAGCGGCUUGUUC | SEQ ID NO: 1359 | ACAAGCCGCUGCUUUGGAU | 0.078 |
| AH0073 | SEO ID NO: 74 | CAAGCCGCUGCUUUGGAUCUC | SEQ ID NO: 717 | GAUCCAAAGCAGCGGCUUGUU | SEQ ID NO: 1360 | CAAGCCGCUGCUUUGGAUC | 0.100 |
| AH0074 | SEQ ID NO: 75 | AAGCCGCUGCUUUGGAUCUCU | SEO ID NO: 718 | AGAUCCAAAGCAGCGGCVUGU | SEQ ID NO: 1361 | AAGCCGCUGCUUUGGAUCU | 0.127 |
| AH0075 | SEQ ID NO: 76 | AGCCGCUGCUUUGGAUCUCUG | SEO ID NO: 719 | GAGAUCCAAAGCAGCGGCU UG | SEQ ID NO: 1362 | AGCCGCUGCUUUGGAUCUC | 0.123 |
| AH0076 | SEQ ID NO: 77 | GCCGCUGCUUUGGAUCUOUGU | SEQ ID NO: 720 | AGAGAUCCAAAGCAGCGGCUU | SEQ ID NO: 1363 | GCCGCUGCUUUGGAUCUCU | 0.047 |
| AH0077 | SEO ID NO: 78 | CCGCUGCUUUGGAUCUCUGUC | SEQ ID NO: 721 | CAGAGAUCCAAAGCAGCGGCU | SEQ ID NO: 1364 | CCGCVGCUUUGGAUCUCUG | 0.054 |
| AH0078 | SEQ ID NO: 79 | CGCUGCUUUGGAUCUCUGUCC | SEO ID NO: 722 | ACAGAGAUCCAAAGCAGCGGC | SEQ ID NO: 1365 | CGCUGCUUUGGAUCUCUGU | 0.064 |
| AH0079 | SEQ ID NO: 80 | GCUGCUUUGGAUCUCUGUCCU | SEQ ID NO: 723 | GACAGAGAUCCAAAGCAGCGG | SEQ ID NO: 1366 | GCUGCUUUGGAUCUCUGUC | 0.090 |
| AH0080 | SEQ ID NO: 81 | CUGCUUUGGAUCUCUGUCCUC | SEQ ID NO: 724 | GGACAGAGAUCCAAAGCAGCG | SEQ ID NO: 1367 | CUGCUUUGGAUCUCUGUCC | 0296 |
| AH0081 | SEO ID NO: 82 | UGCUUUGGAUCUCUGUCCUCA | SEQ ID NO: 725 | AGGACAGAGAUCCAAAGCAGC | SEQ ID NO: 1368 | UGCUUUGGAUCUCUGUCCU | 0.088 |
| AH0082 | SEO ID NO: 83 | GCUUUGGAUCUCUGUCCUCAC | SEQ ID NO: 726 | GAGGACAGAGAUCCAAAGCAG | SEQ ID NO: 1369 | GCUUUGGAUCUCUGUCCUC | 0.131 |
| AH0083 | SEQ ID NO: 84 | CUUUGGAUCVCUGUCCUCACC | SEQ ID NO: 727 | UGAGGACAGAGAUCCAAAGCA | SEQ ID NO: 1370 | CUUUGGAUCUCUGUCCUCA | 0.070 |
| AH0084 | SEQ ID NO: 85 | UUUGGAUCUCUGUCCUCACCA | SEQ ID NO: 728 | GUGAGGACAGAGAUCCAAAGC | SEQ ID NO: 1371 | UUUGGAUCUCUGUCCUCAC | 0.300 |
| AH0085 | SEQ ID NO: 86 | GGAUCUCUGUCCUCACCAGCC | SEQ ID NO: 729 | CUGGUGAGGACAGAGAUCCAA | SEQ D NO: 1372 | GGAUCUCUGUCCUCACCAG | 0.129 |
| AH0086 | SEQ ID NO 87 | GAUCUCUGUCCUCACCAGCCU | SEO ID NO: 730 | GCUGGUGAGGACAGAGAUCCA | SEQ ID NO: 1373 | GAUCUCUGUCCUCACCAGC | 0.483 |
| AH0087 | SEQ ID NO: 88 | CUCUGUCCUCACCAGCCUCCU | SEQ ID NO: 731 | GAGGCUGGUGAGGACAGAGAU | SEO ID NO: 1374 | CUCUGUCCUCACCAGCCUC | 0.263 |
| AH0088 | SEQ ID NO: 89 | CUGUCCUCACCAGCCUCCUGG | SEQ ID NO: 732 | AGGAGGCUGGUGAGGACAGAG | SEQ ID NO: 1375 | CUGUCCUCACCAGCCUCCU | 0.413 |
| AH0089 | SEQ ID NO: 90 | UGUCCUCACCAGCCUCCUGGA | SEQ ID NO: 733 | CAGGAGGCUGGUGAGGACAGA | SEQ ID NO: 1376 | UGUGCUCACCAGCCUCCUG | 0.334 |
| AH0090 | SEQ ID NO: 91 | GUCCUCACCAGCCUCCUGGAA | SEQ ID NO: 734 | CCAGGAGGCUGGUGAGGACAG | SEQ ID NO: 1377 | GUCCUCACCAGCCUCCUGG | 0.295 |
| AH0091 | SEQ ID NO: 92 | UCCUCACCAGCCUCCUGGAAG | SEQ ID NO: 735 | UCCAGGAGGCUGGUGAGGACA | SEQ ID NO: 1378 | UCCUCACCAGCCUCCUGGA | 0.300 |
| AH0092 | SEQ ID NO: 93 | CUCACCAGCCUCCUGGAAGCC | SEQ ID NO: 736 | CUUCCAGGAGGCUGGUGAGGA | SEQ ID NO : 1379 | CUCACCAGCCUCCUGGAAG | 0.243 |
| AH0093 | SEQ ID NO: 94 | UCACCAGCCUCCUGGAAGCCU | SEQ ID NO: 737 | GCUUCCAGGAGGCUGGUGAGG | SEQ ID NO: 1380 | UCACCAGCCUCCUGGAAGC | 0.286 |
| AH0094 | SEQ ID NO: 95 | ACCAGCCUCCUGGAAGCCUUU | SEQ ID NO: 738 | AGGCUUCCAGGAGGCUGGUGA | SEQ ID NO: 1381 | ACCAGCCUCCUGGAAGCCU | 0.365 |
| AH0095 | SEQ ID NO: 96 | CCAGCCUCCUGGAAGCCUUUG | SEQ ID NO: 739 | AAGGCUUCCAGGAGGCUGGUG | SEQ ID NO: 1382 | CCAGCCUCCUGGAAGCCUU | 0060 |
| AH0096 | SEQ ID NO: 97 | CAGCCUCCUGGAAGCCUUUGC | SEQ ID NO: 740 | AAAGGCUUCCAGGAGGCUGGU | SEQ ID NO: 1383 | CAGCCUCCUGGAAGCCUUU | 0.066 |
| AH0097 | SEQ ID NO: 98 | AGCCUCCUGGAAGCCUUUGCU | SEQ ID NO: 741 | CAAAGGCUUCCAGGAGGCUGG | SEO ID NO: 1384 | AGCCUCCUGGAAGCCUUUG | 0.486 |
| AH0098 | SEQ ID NO: 99 | GCCUCCUGGAAGCCUUUGCUC | SEQ ID NO: 742 | GCAAAGGCUUCCAGGAGGCUG | SEQ ID NO: 1385 | GGCUCCUGGAAGGCUUUGC | 0.374 |
| AH0099 | SEQ ID NO: 100 | CCUCCUGGAAGCCUUUGCUCA | SEO ID NO: 743 | AGCAAAGGCUUCCAGGAGGCU | SEQ ID NO: 1386 | CCUCCUGGAAGCCUUUGCU | 0.111 |
| AH0100 | SEQ ID NO: 101 | CUCCUGGAAGCCUUUGCUCAC | SEQ ID NO: 744 | GAGCAAAGGCUUCCAGGAGGC | SEQ ID NO: 1387 | CUCCUGGAAGCCUUUGCUC | 0.310 |

### [Table 30]

**Table 1-3**

| Double-stranded nucleic acid No. | SEQ ID NO: | Sense strand sequence (5'--->3') | SEQ ID NO: | Antisense strand sequence (5'--->3') | SEQ ID NO: | Target APCS mRNA sequence | Relative APCS expression level |
|---|---|---|---|---|---|---|---|
| AH0101 | SEQ ID NO: 102 | UCCUGGAAGCCUUUGCUCACA | SEQ ID NO 745 | UGAGCAAAGGCUUCCAGGAGG | SEQ ID NO : 1388 | UCCUGGAAGCCUUUGCUCA | 0053 |
| AH0102 | SEQ ID NO:103 | CCUGGAAGCCUUUGCUCACAC | SEQ ID NO:746 | GUGAGCAAAGGCUUCCAGGAG | SEQ ID NO 1389 | CCUGGAAGCCUUGCUCAC | 0.086 |
| AH0103 | SEQ ID NO 104 | CUGGAAGGCUUUGCUCACACA | SEQ ID NO 747 | UGUGAGCAAAGGCUUCCAGGA | SEQ ID NO:1380 | CUGGAAGCCUUUGCUCACA | 0.121 |
| AH0104 | SEQ ID NO 105 | GGAAGCCUUUGCUCACAGA | SEQ ID NO 748 | UGUGUGAGCAAAGGCUUCCAG | SEQ ID NO 1391 | GGAAGCCUUUGCUCACACA | 0.161 |
| AH0105 | SEQ ID NO: 106 | GAAGCCUUUGCUCACACAGAC | SEQ ID NO 749 | CUGUGUGAGCAAAGGCUUCCA | SEQ ID NO 1392 | GAAGCCUUUGCUCACACAG | 0.291 |
| AH0106 | SEQ ID NO 107 | AAGCCUUUGCUCACACAGACC | SEQ ID NO 750 | UCUGUGUGAGCAAAGGCUUCC | SEQ ID NO 1393 | AAGCCUUUGCUCACACAGA | 0.065 |
| AH0107 | SEQ ID NO 108 | AGCCUUUGCUCACACAGACCU | SEQ ID NO 751 | GUCUGUGUGAGCAAAGGCU UC | SEQ ID NO: 1394 | AGCCUUUGCUCACACAGAC | 0.136 |
| AH0108 | SEQ ID NO: 109 | GCCUUUGCUCACACAGACCUC | SEQ ID NO: 752 | GGUCUGUGUGAGGAAAGGCUU | SEQ ID NO: 1395 | GCCUUUGCUCACACAGACC | 0.094 |
| AH0109 | SEQ ID NO: 110 | CCUUUGCUCACACAGACCUCA | SEQ ID NO: 753 | AGGUCUGUGUGAGCAAAGGCU | SEQ ID NO: 1396 | CCUUUGCUCACACAGACCU | 0.107 |
| AH0110 | SEQ ID NO: 111 | CUUUGCUCACACAGACCUCAG | SEQ ID NO: 754 | GAGGUCUGUGUGAGCAAAGGC | SEQ ID NO: 1397 | CUUUGCUCACACAGACCUC | 0.254 |
| AH0111 | SEQ ID NO: 112 | UUUGCUCACACAGACCUCAGU | SEQ ID NO: 755 | UGAGGUCUGUGUGAGCAAAGG | SEQ ID NO 1398 | UUUGCUCACACAGACCUCA | 0.334 |
| AH0112 | SEQ ID NO: 113 | UGCUCACACAGACCUCAGUGG | SEQ ID NO 756 | ACUGAGGUCUGUGUGAGCAAA | SEQ ID NO: 1399 | UGCUCACACAGACCUCAGU | 0.302 |
| AH0113 | SEQ ID NO: 114 | CUCACACAGACCUCAGUGGGA | SEQ ID NO: 757 | CCACUGAGGUCUGUGUGAGCA | SEQ ID N0: 1400 | CUCACACAGACCUCAGUGG | 0.490 |
| AH0114 | SEQ ID NO: 115 | CACACAGACCUCAGUGGGAAG | SEQ ID NO: 768 | UCCCACUGAGGUCUGUGUGAG | SEQ ID NO: 1401 | CACACAGACCUCAGUGGGA | 0.109 |
| AH0115 | SEQ ID NO: 116 | ACACAGACCUCAGUGGGAAGG | SEQ ID NO: 759 | UUCCCACUGAGGUCUGUGUGA | SEQ ID NO: 1402 | ACACAGACCUCAGUGGGAA | 0.105 |
| AH0118 | SEQ ID NO: 117 | CACAGACCUCAGUGGGAAGGU | SEQ ID NO: 760 | CUUCCCACUGAGGUCUGUGUG | SEQ ID NO: 1403 | CACAGACCUCAGUGGGAAG | 0.478 |
| AH0117 | SEQ ID NO: 118 | AGACCUCAGUGGGAAGGUGUU | SEQ ID NO: 761 | CACCUUCCCACUGAGGUCUGU | SEQ ID NO: 1404 | AGACCUCAGUGGGAAGGUG | 0.157 |
| AH0118 | SEQ ID NO: 119 | GACCUCAGUGGGAAGGUGUUU | SEQ ID NO: 762 | ACACCUUCCCACUGAGGUCUG | SEQ ID NO: 1405 | GACCUCAGUGGGAAGGUGU | 0.149 |
| AH0119 | SEQ ID NO: 120 | ACCUCAGUGGGAAGGUGUUUG | SEQ ID NO: 763 | AACACCUUCCCACUGAGGUCU | SEQ ID NO: 1406 | ACCUCAGUGGGAAGGUGUU | 0.123 |
| AH0120 | SEQ ID NO: 121 | CCUCAGUGGGAAGGUGUUUGU | SEQ ID NO: 764 | AAACACCUUCCCACUGAGGUC | SEQ ID NO: 1407 | CCUCAGUGGGAAGGUGUUU | 0.261 |
| AH0121 | SEQ ID NO: 122 | CUCAGUGGGAAGGUGUUUGUA | SEQ ID NO: 766 | CAAACACCUUCCCACUGAGGU | SEQ ID NO: 1408 | CUCAGUGGGAAGGUGUUUG | 0.316 |
| AH0122 | SEQ ID NO: 123 | UCAGUGGGAAGGUGUUUGUAU | SEQ ID NO: 766 | ACAAACACCUUCCCACUGAGG | SEQ ID NO: 1409 | UCAGUGGGAAGGUGUUUGU | 0.125 |
| AH0123 | SEQ ID NO: 124 | CAGUGGGAAGGUGUUUGUAUU | SEQ ID NO: 767 | UACAAACACCUUCCCACUGAG | SEQ ID NO: 1410 | CAGUGGGAAGGUGUUUGUA | 0.185 |
| AH0124 | SEQ ID NO: 125 | AGUGGGAAGGUGUUUGUAUUU | SEQ ID NO: 768 | AUACAAACACCUUCCCACUGA | SEQ ID NO: 1411 | AGUGGGAAGGUGUUUGUAU | 0.105 |
| AH0125 | SEQ ID NO: 126 | GUGGGAAGGUGUUUGUAUUUC | SEQ ID NO: 769 | AAUACAAACACCCUCCCACUG | SEQ ID NO: 1412 | GUGGGAAGGUUUGUAUU | 0.101 |
| AH0126 | SEQ ID NO: 127 | UGGGAAGGUGUUUGUAUUUCC | SEQ ID NO: 77O | AAAUACAAACACCUUCCCACU | SEQ ID NO: 1413 | UGGGAAGGUGUUUGUAUUU | 0.150 |
| AH0127 | SEQ ID NO: 128 | GGGAAGGUGUUUGUAUUUCCU | SEQ ID NO: 771 | GAAAUACAAACACCUUCCCAC | SEQ ID NO: 1414 | GGGAAGGUGUUUGUAUUUC | 0.386 |
| AH0128 | SEQ ID NO: 129 | GGAAGGUGUUUGUAUUUCCUA | SEQ ID NO: 772 | GGAAAUACAAACACCUUCCCA | SEQ ID NO: 1415 | GGAAGGUGUUUGUAUUUCC | 0.127 |
| AH0129 | SEQ ID NO: 130 | GAAGGUGUUUGUAUUUCCUAG | SEQ ID NO: 773 | AGGAAAUACAAACACCUUCCC | SEQ ID NO: 1416 | GAAGGUGUUUGUAUUUCCU | 0,077 |
| AH0130 | SEQ ID NO: 131 | AAGGUGUUUGUAUUUCCUAGA | SEQ ID NO: 774 | UAGGAAAUACAAACACCUUCC | SEQ ID NO: 1417 | AAGGUGUUUGUAUUUCCUA | 0.213 |
| AH0131 | SEQ ID NO: 132 | AGGUGUUUGUAUUUCCUAGAG | SEQ ID NO: 775 | CUAGGAAAUACAAACACCUUC | SEQ ID NO: 1418 | AGGUGUUUGUAUUUCCUAG | 0.084 |
| AH0132 | SEQ ID NO: 133 | GGUGUUUGUAUUUCCUAGAGA | SEQ ID NO: 776 | UCUAGGAAAUACAAACACCUU | SEQ ID NO: 1419 | GGUGUUUGUAUUUCCUAGA | 0.112 |
| AH0133 | SEQ ID NO: 134 | GUGUUUGUAUUUCCUAGAGAA | NO: 777 | CUCUAGGAAAUACAAACACCU | SEQ ID NO: 1420 | GUGUUUGUAUUUCCUAGAG | 0.153 |
| AH0134 | SEQ ID NO: 135 | UGUUUGUAUUUCCUAGAGAAU | SEQ ID NO: 778 | UCU CUAGGAAAUACAAACACC | SEQ ID NO: 1421 | UGUUUGUAUUUCCUAGAGA | 0.139 |
| AH0135 | SEQ ID NO: 136 | GUUUGUAUUUCCUAGAGAAUC | SEQ ID NO: 779 | UUCUCUAGGAAAUACAAACAC | SEQ ID NO: 1422 | GUUUGUAUUUCCUAGAGAA | 0.070 |
| AH0136 | SEQ ID NO: 137 | UUUGUAUUUCCUAGAGAAUCU | SEQ ID NO: 780 | AUUCUCUAGGAAAUACAAACA | SEQ ID NO: 1433 | UUUGUAUUUCCUAGAGAAU | 0.125 |
| AH0137 | SEQ ID NO: 138 | UUGUAUUUCCUAGAGAAUCUG | SEQ ID NO: 781 | GAUUCUCUAGGAAAUACAAAC | SEQ ID NO: 1424 | UUGUAUUUCCUAGAGAAUC | 0.451 |
| AH0138 | SEQ ID NO: 139 | UGUAUUUCCUAGAGAAUCUGU | SEQ ID NO: 782 | AGAUUCUCUAGGAAAUACAAA | SEQ ID NO: 1425 | UGUAUUUCCUAGAGAAUCU | 0.204 |
| AH0139 | SEQ ID NO: 140 | GUAUUUCCUAGAGAAUCUGUU | SEQ ID NO: 783 | CAGAUUCUCUAGGAAAUACAA | SEQ ID NO: 1426 | GUAUUUCCUAGAGAAUCUG | 0.071 |
| AH0140 | SEQ ID NO: 141 | UAUUUCCUAGAGAAUCUGUUA | SEQ ID NO: 784 | ACAGAUUCUCUAGGAAAUACA | SEQ ID NO: 1427 | UAUUUCCUAGAGAAUCUGU | 0.242 |
| AH0141 | SEQ ID NO: 142 | AUUUCCUAGAGAAUCUGUUAC | SEQ ID NO: 785 | AACAGAUUCUCUAGGAAAUAC | SEQ ID NO: 1428 | AUUUGCUAGAGAAUCUGUU | 0.123 |
| AH0142 | SEQ ID NO: 143 | UUUCCUAGAGAAUCUGUUACU | SEQ ID NO: 786 | UAACAGAUUCUCUAGGAAAUA | SEQ ID NO: 1429 | UUUCCUAGAGAAUCUGUUA | 0.179 |
| AH0143 | SEQ ID NO: 144 | UCCUAGAGAAUCUGUUACUGA | SEQ ID NO: 787 | AGUAACAGAUUCUCUAGGAAA | SEQ ID NO: 1430 | UCCUAGAGAAUCUGUUACU | 0.311 |
| AH0144 | SEQ ID NO 145 | CCUAGAGAAUCUGUUACUGAU | SEQ ID NO: 788 | CAGUAACAGAUUCUCUAGGAA | SEQ ID NO: 1431 | CCUAGAGAAUCUGUUACUG | 0.137 |
| AH0145 | SEQ ID NO: 146 | CUAGAGAAUCUGUUACUGAUC | SEQ ID NO: 789 | UCAGUAACAGAUUCUCUAGGA | SEQ ID NO: 1432 | CUAGAGAAUCUGUUACUGA | 0.039 |
| AH0146 | SEQ ID NO: 147 | UAGAGAAUCUGUUACUGAUCA | SEQ ID NO: 790 | AUCAGUAACAGAUUCUCUAGG | SEQ ID NO: 1438 | UAGAGAAUCUGUUACUGAU | 0.051 |
| AH0147 | SEQ ID NO: 148 | AGAGAAUCUGUUACUGAUCAU | SEQ ID NO: 791 | GAUCAGUAACAGAUUCUCUAG | SEQ ID NO: 1434 | AGAGAAUCUGUUACUGAUC | 0.361 |
| AH0148 | SEQ ID NO: 149 | GAGAAUCUGUUACUGAUGAUG | SEQ ID NO: 792 | UGAUCAGUAACAGAUUCUCUA | SEQ ID NO: 1435 | GAGAAUCUGUUACUGAUGA | 0.090 |
| AH0149 | SEQ ID NO: 150 | AGAAUCUGUUACUGAUCAUGU | SEQ ID NO: 793 | AUGAUCAGUAACAGAUUCUCU | SEQ ID NO: 1436 | AGAAUCUGUUACUGAUCAU | 0.211 |
| AH0150 | SEQ ID NO: 151 | GAAUCUGUUACUGAUCAUGUA | SEQ ID NO: 794 | CAUGAUCAGUAACAGAUUCUC | SEQ ID NO: 1437 | GAAUCUGUUACUGAUCAUG | 0.183 |

### [Table 31]

**Table 1-4**

| Double-stranded nucleic acid No. | SEQ ID NO: | Sense strand sequence (5'--->3') | SEQ ID NO: | Antisense strand sequence (5'--->3') | SEQ ID NO: | Target APCS mRNA sequence | Relatlve APCS expression level |
|---|---|---|---|---|---|---|---|
| AH0151 | SEQ ID NO: 152 | AAUCUGUUACUGAUCAUGUAA | SEQ ID NO: 795 | ACAUGAUCAGUAACAGAUUCU | SEQ ID NO: 1438 | AAUCUGUUACUGAUCAUGU | 0.477 |
| AH0152 | SEQ ID NO: 153 | AUCUGUUACUGAUCAUGUAAA | SEQ ID NO: 796 | UACAUGAUCAGUAACAGAUUC | SEQ ID NO: 1439 | AUCUGUUACUGAUCAUGUA | 0.103 |
| AH0153 | SEQ ID NO: 154 | UCUGUUACUGAUCAUGUAAAC | SEQ ID NO: 797 | UUACAUGAUCAGUAACAGAUU | SEQ ID NO: 144 | UCUGUUACUGAUCAUGUAA | 0.166 |
| AH0154 | SEQ ID NO: 155 | CUGUUACUGAUCAUGUAAACU | SEQ ID NO: 798 | UUUACAUGAUCAGUAACAGAU | SEQ ID NO: 1441 | CUGUUACUGAUCAUGUAAA | 0.155 |
| AH0155 | SEQ ID NO: 156 | UGUUACUGAUCAUGUAAACUU | SEQ ID NO: 799 | GUUUACAUGAUCAGUAACAGA | SEQ ID NO: 1442 | UGUUACUGAUCAUGUAAAC | 0.260 |
| AH0156 | SEQ ID NO: 157 | GUUACUGAUCAUGUAAACUUG | SEQ ID NO: 800 | AGUUUACAUGAUCAGUAACAG | SEQ ID NO: 1443 | GUUACUGAUCAUGUAAACU | 0.213 |
| AH0167 | SEQ ID NO: 158 | ACUGAUCAUGUAAACUUGAUC | SEQ ID NO: 801 | UCAAGUUUACAUGAUCAGUAA | SEQ ID NO: 1444 | ACUGAUCAUGUAAACUUGA | 0.365 |
| AH0158 | SEQ ID NO: 159 | CUGAUCAUGUAAACUUGAUCA | SEQ ID NO: 802 | AUCAAGUUUACAUGAUCAGUA | SEQ ID NO: 1445 | CUGAUCAUGUAAACUUGAU | 0.167 |
| AH0159 | SEQ ID NO: 160 | UGAUCAUGUAAACUUGAUCAC | SEQ ID NO: 803 | GAUCAAGUUUACAUGAUCAGU | SEQ ID NO: 1446 | UGAUCAUGUAAACUUGAUC | 0.457 |
| AH0160 | SEQ ID NO: 161 | GAUCAUGUAAACUUGAUCACA | SEQ ID NO: 804 | UGAUCAAGUUUACAUGAUCAG | SEQ ID NO: 1447 | GAUCAUGUAAACUUGAUCA | 0.338 |
| AH0161 | SEQ ID NO: 162 | UCAUGUAAACUUGAUCACACC | SEQ ID NO: 805 | UGUGAUCAAGUUUACAUGAUC | SEQ ID NO: 1448 | UCAUGUAAACUUGAUCACA | 0.286 |
| AH0162 | SEQ ID NO: 163 | CAUGUAAACUUGAUCACACCG | SEQ ID NO: 806 | GUGUGAUCAAGUUUACAUGAU | SEQ ID NO: 1449 | CAUGUAAACUUGAUCACAC | 0.485 |
| AH0163 | SEQ ID NO: 164 | UAAACUUGAUCACACCGCUGG | SEQ ID NO: 807 | AGCGGUGUGAUCAAGUUUACA | SEQ ID NO: 1450 | UAAACUUGAUCACACCGCU | 0.355 |
| AH0164 | SEQ ID NO: 165 | AAACUUGAUCACACCGCUGGA | SEQ ID NO: 808 | CAGCGGUGUGAUCAAGUUUAC | SEQ ID NO: 1451 | AAACUUGAUCACACCGCUG | 0360 |
| AH0165 | SEQ ID NO: 166 | ACUUGAUCACACCGCUGGAGA | SEQ ID NO: 809 | UCCAGCGGUGUGAUCAAGUUU | SEQ ID NO: 1452 | ACUUGAUCACACCGCUGGA | 0.212 |
| AH0166 | SEQ ID NO: 167 | UUGAUCACACCGCUGGAGAAG | SEQ ID NO: 810 | UCUCCAGCGGUGUGAUCAAGU | SEQ ID NO: 1453 | UUGAUCACACCGCUGGAGA | 0.493 |
| AH0167 | SEQ ID NO: 168 | UGAUCACACCGCUGGAGAAGC | SEQ ID NO: 811 | UUCUCCAGCGGUGUGUGAUCAAG | SEQ ID NO: 1454 | UGAUCACACCGCUGGAGAA | 0.078 |
| AH0168 | SEQ ID NO: 169 | GAUCACACCGCUGGAGAAGCC | SEQ ID NO: 812 | CUUCUCCAGCGGUGGUGAUCAA | SEQ ID NO: 1455 | GAUCACACCGCUGGAAG | 0.465 |
| AH0169 | SEQ ID NO : 170 | CACACCGCUGGAGAAGCCUCU | SEQ ID NO: 813 | AGGCUUCUCCAGCGGUGUGAU | SEQ ID NO: 1456 | CACACCGCUGGAGAAGCCU | 0.312 |
| AH0170 | SEQ ID NO: 171 | ACACCGCUGGAGAAGCCUCUA | SEQ ID NO: 814 | GAGGCUUCUCCAGCGGUGUGA | SEQ ID NO: 1457 | ACACCGCUGGAGAAGCCUC | 0.208 |
| AH0171 | SEQ ID NO: 172 | CACCGCUGGAGAAGCCUCUAC | SEQ ID NO: 815 | AGAGGCUUCUCCAGCGGUGUG | SEQ ID NO: 1458 | CACCGCUGGAGAAGCCUCU | 0.190 |
| AH0172 | SEQ ID NO: 173 | ACCGCUGGAGAAGCCUCUACA | SEQ ID NO: 816 | UAGAGGCUUCUCCAGCGGUGU | SEQ ID NO: 1459 | ACCGCUGGAGAAGCUA | 0.276 |
| AH0173 | SEQ ID NO: 174 | CCGCUGGAGAAGCCUCUACAG | SEQ ID NO: 817 | GUAGAGGCUUCUCCAGCGGUG | SEQ ID NO: 1460 | CCGCUGGAGAAGCCUCUAC | 0.397 |
| AH0174 | SEQ ID NO: 175 | CGCUGGAGAAGCCUCUACAGA | SEQ ID NO: 818 | UGUAGAGGCUUCUCCAGCGGU | SEQ ID NO: 1461 | CGCUGGAGAAGCCUCUACA | 0.056 |
| AH0175 | SEQ ID NO: 176 | GCUGGAGAAGCCUCUACAGAA | SEQ ID NO: 819 | CUGUAGAGGCUUCUCCAGCGG | SEQ ID NO: 1462 | GCUGGAGAAGCCUCUACAG | 0.335 |
| 4H0176 | SEQ ID NO: 177 | CUGGAGAAGCCUCUACAGAAC | SEQ ID NO: 820 | UCUGUAGAGGCUUCUCCAGCG | SEQ ID NO: 1463 | CUGGAGAAGCCUCUACAGA | 0.080 |
| AH0177 | SEQ ID NO: 178 | UGGAGAAGCCUCUACAGAACU | SEQ ID NO: 821 | UUCUGUAGAGGCUUCUCCAGC | SEQ ID NO: 1464 | UGGAGAAGCCUCUACAGAA | 0.088 |
| AH0178 | SEQ ID NO: 179 | GGAGAAGCCUCUACAGAACUU | SEQ ID NO: 822 | GUUCUGUAGAGGCUUCUCCAG | SEQ ID NO: 1465 | GGAGAAGCCUCUACAGAAC | 0.142 |
| AH0179 | SEQ ID NO: 180 | GAGAAGCCUCUACAGAACUUU | SEQ ID NO: 823 | AGUUCUGUAGAGGCUUCUCCA | SEQ ID NO: 1466 | GAGAAGCCUCUACAGAACU | 0.276 |
| AH0180 | SEQ ID NO: 181 | AGAAGCCUCUACAGAACUUUA | SEQ ID NO: 824 | AAGUUCUGUAGAGGCUUCUCC | SEQ ID NO: 1467 | AGAAGCCUCUACAGAACUU | 0.160 |
| AH0181 | SEQ ID NO: 182 | GAAGCCUCUACAGAACUUUAC | SEQ ID NO: 825 | AAAGUUCUGUAGAGGCUUCUC | SEQ ID NO: 1468 | GAAGCCUCUACAGAACUUU | 0.156 |
| AH0182 | SEQ ID NO: 183 | AAGCCUCUACAGAACUUUACC | SEQ ID NO: 826 | UAAAGUUCUGUAGAGGCUUCU | SEQ ID NO: 1469 | AACCUCUACAGAACUUUA | 0.210 |
| AH0183 | SEQ ID NO: 184 | AGCCUCUACAGAACUUUACCU | SEQ ID NO: 827 | GUAAAGUUCUGUAGAGGCUUC | SEQ ID NO: 1470 | AGCCUCUAGAGACUUUAC | 0.305 |
| AH0184 | SEQ ID NO: 185 | GCCUCUACAGAACUUUACCUU | SEQ ID NO: 828 | GGUAAAGUUCUGUAGAGGCUU | SEQ ID NO: 1471 | GCCUCUACAGAACUUUACC | 0.343 |
| AH0185 | SEQ ID NO: 186 | CCUCUACAGAACUUUACCUUG | SEQ ID NO: 829 | AGGUAAAGUUCUGUAGAGGCU | SEQ ID NO: 1472 | CCUCUACAGAACUUUACCU | 0.178 |
| AH0186 | SEQ ID NO: 187 | CUCUACAGAACUUUACCUUGU | SEQ ID NO: 830 | AAGGUAAAGUUCUGUAGAGGC | SEQ ID NO: 1473 | CUCUACAGAACUUUACCUU | 0.183 |
| AH0187 | SEQ ID NO: 188 | UCUACAGAACUUUACCUUGUG | SEQ ID NO: 831 | CAAGGUAAAGUUCUGUAGAGG | SEQ ID NO: 1474 | UCUACAGAACUUUACCUUG | 0.244 |
| AH0188 | SEQ ID NO: 189 | CUACAGAACUUUACCUUGUGU | SEQ ID NO: 832 | ACAAGGUAAAGUUCUGUAGAG | SEQ ID NO: 1475 | CUACAGAACUUUACCUUGU | 0.079 |
| AH0189 | SEQ ID NO: 190 | UACAGAACUUUACCUUGUGUU | SEQ ID NO: 833 | CACAAGGUAAAGUUCUGUAGA | SEQ ID NO: 1476 | UACAGAACUUUACCUUGUG | 0.219 |
| AH0190 | SEQ ID NO 191 | ACAGAACUUUACCUUGUGUUU | SEQ ID NO: 834 | ACACAAGGUAAAGUUCUGUAG | SEQ ID NO: 1477 | ACAGAACUUUACCUUGUGU | 0.387 |
| AH0191 | SEQ ID NO : 192 | CACAACUUUACCUUGUGUUUU | SEQ ID NO: 835 | AACACAAGGUAAAGUUCUGUA | SEQ ID NO: 1478 | CAGAACUUUACCUUGUGUU | 0.171 |
| AH0192 | SEQ ID NO: 193 | AGAACUUUACCUUGUGUUUUC | SEQ ID NO: 836 | AAACACAAGGUAAAGUUCUGU | SEQ ID NO: 1479 | AGAACUUUACCUUGUGUUU | 0.133 |
| AH0193 | SEQ ID NO: 194 | GAACUUUACCUUGUGUUUUCG | SEQ ID NO: 837 | AAAACACAAGGUAAAGUUCUG | SEQ ID NO: 1480 | GAACUUUACCUUGUGUUUU | 0.081 |
| AH0194 | SEQ ID NO: 195 | AACUUUACCUUGUGUUUUCGA | SEQ ID NO: 838 | GAAAACACAAGGUAAAGUUCU | SEQ ID NO: 1481 | AACUUUACCUUGUGUUUUC | 0.347 |
| AH0195 | SEQ ID NO: 196 | CUUUACCUUGUGUUUUCGAGC | SEQ ID NO: 839 | UCGAAAAQACAAGGUAAAGUU | SEQ ID NO: 1482 | CUUUACCUUGUGUUUCGA | 0.453 |
| AH0196 | SEQ ID NO 197 | ACCUUGUGUUUUCGAGCCUAU | SEQ ID NO: 840 | AGGCUCGAAAACACAAGGUAA | SEQ ID NO: 1483 | ACCUUGUGUUUUCGAGCCU | 0.164 |
| AH0197 | SEQ ID NO: 198 | CCUUGUGUUUUCGAGCCUAUA | SEQ ID NO: 841 | UAGGCUCGAAAACACAAGGUA | SEQ ID NO: 1484 | CCUUGUGUUUUCGAGCCUA | 0.081 |
| AH0198 | SEQ ID NO: 199 | CUUGUGUUUUCGAGCCUAUAG | SEQ ID NO: 842 | AUAGGCUCGAAAACACAAGGU | SEQ ID NO: 1486 | CUUGUGUUUUCGAGCCUAU | 0.110 |
| AH0199 | SEQ ID NO: 200 | UUGUGUUUUCGAGCCUAUAGU | SEQ ID NO: 843 | UAUAGGCUCGAAAACACAAGG | SEQ ID NO: 1486 | UUGUGUUUUCGAGCCUAUA | 0.137 |
| AH0200 | SEQ ID NO: 201 | GUGUUUUCGAGCCUAUAGUGA | SEQ ID NO: 844 | ACUAUAGGCUCGAAAACACAA | SEQ ID NO: 1487 | GUGUUUUCGAGCCUAUAGU | 0.125 |

### [Table 32]

**Table 1-5**

| Double-stranded nucleic acid No. | SEQ ID NO: | Sense strand sequence (5'--->3') | SEQ ID NO: | Antisense strand sequence (5'--->3') | SEQ ID NO: | Target APCS mRNA sequence | Relative APCS expression level |
|---|---|---|---|---|---|---|---|
| AH0201 | SEQ ID NO: 202 | GUUUUCGAGCCUAUAGUGAUC | SEQ ID NO: 845 | UCACUAUAGGCUCGAAAACAC | SEQ ID NO: 1488 | GUUUUCGAGCCUAUAGUGA | 0.074 |
| AH0202 | SEQ ID NO: 203 | UUUUCGAGCCUAUAGUGAUCU | SEQ ID NO: 846 | AUCACUAUAGGCUCGAAAACA | SEQ ID NO: 1489 | UUUUCGAGCCUAUAGUGAU | 0.136 |
| AH0203 | SEQ ID NO: 204 | CGAGCCUAUAGUGAUCUCUCU | SEQ ID NO: 847 | AGAGAUCACUAUAGGCUCGAA | SEQ ID NO: 1490 | CGAGCCUAUAGUGAUCUCU | 0.119 |
| AH0204 | SEQ ID NO: 205 | GAGCCUAUAGUGAUCUCUCUC | SEQ ID NO: 848 | GAGAGAUCACUAUAGGCUCGA | SEQ ID NO: 1491 | GAGCCUAUAGUGAUCUCUC | 0.127 |
| AH0205 | SEQ ID NO: 206 | AGCCUAUAGUGAUCUCUCUCG | SEQ ID NO: 849 | AGAGAGAUCACUAUAGGCUCG | SEQ ID NO: 1492 | AGCCUAUAGUGAUCUCUCU | 0.091 |
| AH0206 | SEQ ID NO: 207 | GCCUAUAGUGAUCUCUCUCGU | SEQ ID NO: 850 | GAGAGAGAUCACUAUAGGCUC | SEQ ID NO: 1493 | GCCUAUAGUGAUCUCUCUC | 0.090 |
| AH0207 | SEQ ID NO: 208 | CCUAUAGUGAUCUCUCUCGUG | SEQ ID NO: 851 | CGAGAGAGAUCACUAUAGGCU | SEQ ID NO: 1494 | CCUAUAGUGAUCUCUCUCG | 0.287 |
| AH0208 | SEQ ID NO: 209 | CUAUAGUGAUCUCUCUCGUGC | SEQ ID NO: 852 | ACGAGAGAGAUCACUAUAGGC | SEQ ID NO: 1495 | CUAUAGUGAUCUCUCUCGU | 0.088 |
| AH0209 | SEQ ID NO: 210 | UAUAGUGAUCUCUCUCGUGCC | SEQ ID NO: 853 | CACGAGAGAGAUCACUAUAGG | SEQ ID NO: 1496 | UAUAGUGAUCUCUCUCGUG | 0.355 |
| AH0210 | SEQ ID NO: 211 | AGUGAUCUCUCUCGUGCCUAC | SEQ ID NO: 854 | AGGCACGAGAGAGAUCACUAU | SEQ ID NO: 1497 | AGUGAUCUCUCUCGUGCCU | 0.089 |
| AH0211 | SEQ ID NO: 212 | GUGAUCUCUCUCGUGCCUACA | SEQ ID NO: 855 | UAGGCACGAGAGAGAUCACUA | SEQ ID NO: 1498 | GUGAUCUCUCUCGUGCCUA | 0.072 |
| AH0212 | SEQ ID NO: 213 | UGAUCUCUCUCGUGCCUACAG | SEQ ID NO: 856 | GUAGGCACGAGAGAGAUCACU | SEQ ID NO: 1499 | UGAUCUCUCUCGUGCCUAC | 0.158 |
| AH0213 | SEQ ID NO: 214 | GAUCUCUCUCGUGCCUACAGC | SEQ ID NO: 857 | UGUAGGCACGAGAGAGAUCAC | SEQ ID NO: 1500 | GAUCUCUCUCGUGCCUACA | 0.126 |
| AH0214 | SEQ ID NO: 215 | CUCUCGUGCCUACAGCCUCUU | SEQ ID NO: 858 | GAGGCUGUAGGCACGAGAGAG | SEQ ID NO: 1601 | CUCUCGUGCCUACAGCCUC | 0.196 |
| AH0215 | SEQ ID NO: 216 | UCUCGUGCCUACAGCCUCUUC | SEQ ID NO: 859 | AGAGGCUGUAGGCACGAGAGA | SEQ ID NO: 1502 | UCUCGUGCCUACAGCCUCU | 0.140 |
| AH0216 | SEQ ID NO: 217 | CUCGUGCCUACAGCCUCUUCU | SEQ ID NO: 860 | AAGAGGCUGUAGGCACGAGAG | SEQ ID NO: 1503 | CUCGUGCCUACAGCCUCUU | 0.198 |
| AH0217 | SEQ ID NO: 218 | CGUGCCUACAGCCUCUUCUCC | SEQ ID NO: 861 | AGAAGAGGCUGUAGGCACGAG | SEQ ID NO: 1504 | CGUGCCUACAGCCUCUUCU | 0.181 |
| AH0218 | SEQ ID NO: 219 | GUGCCUACAGCCUCUUCUCCU | SEQ ID NO: 862 | GAGAAGAGGCUGUAGGCACGA | SEQ ID NO: 1505 | GUGCCUACAGCCUCUUCUC | 0.101 |
| AH0219 | SEQ ID NO: 220 | GCCUACAGCCUCUUCUCCUAC | SEQ ID NO: 863 | AGGAGAAGAGGCUGUAGGCAC | SEQ ID NO: 1506 | GCCUACAGCCUCUUCUCCU | 0.100 |
| AH0220 | SEQ ID NO: 221 | CCUACAGCCUCUUCUCCUACA | SEQ ID NO: 864 | UAGGAGAAGAGGCUGUAGGCA | SEQ ID NO: 1507 | CCUACAGCCUCUUCUCCUA | 0.086 |
| AH0221 | SEQ ID NO: 222 | CUACAGCCUCUUCUCCUACAA | SEQ ID NO: 866 | GUAGGAGAAGAGGCUGUAGGC | SEQ ID NO: 1508 | CUACAGCCUCUUCUCCUAC | 0.153 |
| AH0222 | SEQ ID NO: 223 | UACAGCCUCUUCUCCUACAAU | SEQ ID NO: 866 | UGUAGGAGAAGAGGCUGUAGG | SEQ ID NO: 1509 | UACAGCCUCUUCUCCUACA | 0.221 |
| AH0223 | SEQ ID NO: 224 | ACAGCCUCUUCUCCUACAAUA | SEQ ID NO: 867 | UUGUAGGAGAAGAGGCUGUAG | SEQ ID NO: 1510 | ACAGCCUCUUCUCCUACAA | 0.137 |
| AH0224 | SEQ ID NO: 225 | CAGCCUCUUCUCCUACAAUAC | SEQ ID NO: 868 | AUUGUAGGAGAAGAGGCUGUA | SEQ ID NO: 1511 | CAGCCUCUUCUCCUACAAU | 0.184 |
| AH0225 | SEQ ID NO: 226 | AGCCUCUUCUCCUACAAUACC | SEQ ID NO: 869 | UAUUGUAGGAGAAGAGGCUGU | SEQ ID NO: 1512 | AGCCUCUUCUCCUACAAUA | 0.036 |
| AH0226 | SEQ ID NO: 227 | GCCUCUUCUCOUACAAUACCC | SEQ ID NO: 870 | GUAUUGUAGGAGAAGAGGCUG | SEQ ID NO: 1513 | GCCUCUUCUCCUACAAUAC | 0.172 |
| AH0227 | SEQ ID NO: 228 | CCUCUUCUCCUACAAUACCCA | SEQ ID NO: 871 | GGUAUUGUAGGAGAAGAGGCU | SEQ ID NO: 1514 | CCUCUUCUCCUACAAUACC | 0.116 |
| AH0228 | SEQ ID NO: 229 | UCUUCUCCUACAAUACCCAAG | SEQ ID NO: 872 | UGGGUAUUGUAGGAGAAGAGG | SEQ ID NO: 1515 | UCUUCUCCUACAAUACCCA | 0.450 |
| AH0229 | SEQ ID NO: 230 | UCUCCUACAAUACCCAAGGCA | SEQ ID NO: 873 | CCUUGGGUAUUGUAGGAGAAG | SEQ ID NO: 1516 | UCUCCUACAAUACCCAAGG | 0.486 |
| AH0230 | SEQ ID NO: 231 | CUCCUACAAUACCCAAGGCAG | SEQ ID NO: 874 | GCCUUGGGUAUUGUAGGAGAA | SEQ ID NO: 1617 | CUCCUACAAUACCCAAGGC | 0.303 |
| AH0231 | SEQ ID NO: 232 | UCCUACAAUACCCAAGGCAGG | SEQ ID NO: 875 | UGCCUUGGGUAUUGUAGGAGA | SEQ ID NO: 1518 | UCCUACAAUACCCAAGGCA | 0.206 |
| AH0232 | SEQ ID NO: 233 | CCUACAAUACCCAAGGCAGGG | SEQ ID NO: 876 | CUGCCUUGGGUAUUGUAGGAG | SEQ ID NO: 1519 | CCUACAAUACCCAAGGCAG | 0.324 |
| AH0233 | SEQ ID NO: 234 | CUACAAUACCCAAGGCAGGGA | SEQ ID NO: 877 | CCUGCCUUGGGUAUUGUAGGA | SEQ ID NO: 1520 | CUACAAUACCCAAGGCAGG | 0.223 |
| AH0234 | SEQ ID NO: 235 | CAAUACCCAACGCAGGGAUAA | SEQ ID NO: 878 | AUCCCUGCCUUGGGUAUUGUA | SEQ ID NO: 1521 | CAAUACCCAAGGCAGGGAU | 0.323 |
| AH0235 | SEQ ID NO: 236 | AUACCCAAGGCAGGGAUAAUG | SEQ ID NO: 879 | UUAUCCCUGCCUUGGGUAUUG | SEQ ID NO: 1522 | AUACCCAAGGCAGGGAUAA | 0.207 |
| AH0236 | SEQ ID NO: 237 | UACCCAAGGCAGGGAUAAUGA | SEQ ID NO: 880 | AUUAUCCCUGCCUUGGGUAUU | SEQ ID NO: 1523 | UACCCAAGGCAGGGAUAAU | 0.450 |
| AH0237 | SEQ ID NO: 238 | CCAAGGCAGGGAUAAUGAGCU | SEQ ID NO: 881 | CUCAUUAUCCCUGCCUUGGGU | SEQ ID NO: 1624 | CCAAGGCAGGGAUAAUGAG | 0.424 |
| AH0238 | SEQ ID NO: 239 | AAGGCAGGGAUAAUGAGCUAC | SEQ ID NO: 882 | AGCUCAUUAUCCCUGCCUUGG | SEQ ID NO: 1525 | AAGGCAGGGAUAAUGAGCU | 0.172 |
| AH0239 | SEQ ID NO: 240 | AGGCAGGGAUAAUGAGCUACU | SEQ ID NO: 883 | UAGCUCAUUAUCCCUGCCUUG | SEQ ID NO: 1526 | AGGCAGGGAUAAUGAGCUA | 0.396 |
| AH0240 | SEQ ID NO: 241 | GCAGGGAUAAUGAGCUACUAG | SEQ ID NO: 884 | AGUAGCUCAUUAUCCCUGCCU | SEQ ID NO: 1527 | GCAGGGAUAAUGAGCUACU | 0.280 |
| AH0241 | SEQ ID NO: 242 | CAGGGAUAAUGAGCUACUAGU | SEQ ID NO: 885 | UAGUAGCUCAUUAUCCCUGCC | SEQ ID NO: 1628 | CAGGGAUAAUGAGCUACUA | 0.143 |
| AH0242 | SEQ ID NO: 243 | AGGGAUAAUGAGCUACUAGUU | SEQ ID NO: 886 | CUAGUAGCUCAUUAUCCCUGC | SEQ ID NO: 1529 | AGGGAUAAU GAGCUACUAG | 0.325 |
| AH0243 | SEQ ID NO: 244 | GGGAUAAUGAGCUACUAGUUU | SEQ ID NO: 887 | ACUAGUAGCUCAUUAUCCCUG | SEQ ID NO: 1530 | GGGAUAAUGAGCUACUAGU | 0.085 |
| AH0244 | SEQ ID NO: 245 | GGAUAAUGAGCUACUAGUUUA | SEQ ID NO: 888 | AACUAGUAGCUCAUUAUCCCU | SEQ ID NO: 1531 | GGAUAAUGAGCUACUAGUU | 0.073 |
| AH0245 | SEQ ID NO: 246 | GAUAAUGAGCUACUAGUUUAU | SEQ ID NO: 889 | AAACUAGUAGCUCAUUAUCCC | SEQ ID NO: 1532 | GAUAAUGAGCUACUAGUUU | 0.085 |
| AH0246 | SEQ ID NO: 247 | AUAAUGAGCUACUAGUUUAUA | SEQ ID NO: 890 | UAAACUAGUAGCUCAUUAUCC | SEQ ID NO: 1533 | AUAAUGAGCUACUAGUUUA | 0.115 |
| AH0247 | SEQ ID NO: 248 | UAAUGAGCUACUAGUUUAUAA | SEQ ID NO: 891 | AUAAACUAGUAGCUCAUUAUC | SEQ ID NO: 1534 | UAAUGAGCUACUAGUUUAU | 0.115 |
| AH0248 | SEQ ID NO: 249 | AUGAGCUACUAGUUUAUAAAG | SEQ ID NO: 892 | UUAUAAACUAGUAGCUCAUUA | SEQ ID NO: 1535 | AUGAGCUACUAGUUUAUAA | 0.195 |
| AH0249 | SEQ ID NO: 250 | UGAGCUACUAGUUUAUAAAGA | SEQ ID NO: 893 | UUUAUAAACUAGUAGCUCAUU | SEQ ID NO: 1536 | UGAGCUACUAGUUUAUAAA | 0.124 |
| AH0250 | SEQ ID NO: 251 | GAGCUACUAGUUUAUAAAGAA | SEQ ID NO: 894 | CUUUAUAAACUAGUAGCU CAU | SEQ ID NO: 1537 | GAGCUACUAGUUUAUAAAG | 0.449 |

### [Table 33]

**Table 1-6**

| Double-stranded | SEQ ID NO: | Sense strand sequence (5'--->3') | SEQ ID NO: | Antisense strand sequence (5'--->3') | SEQ ID NO: | Target APCS mRNA sequence | Relative APCS expression level |
|---|---|---|---|---|---|---|---|
| AH0251 | SEQ ID NO: 252 | AGCUACUAGUUUAUAAAGAAA | SEQ ID NO: 895 | UCUUUAUAAACUAGUAGCUCA | SEQ ID NO: 1538 | AGCUACUAGUUUAUAAAGA | 0.131 |
| AH0252 | SEQ ID NO: 253 | GCUACUAGUUUAUAAAGAAAG | SEQ ID NO: 896 | UUCUUUAUAAACUAGUAGCUC | SEQ ID NO: 1539 | GCUACUAGUUUAUUUAGAA | 0.092 |
| AH0253 | SEQ ID NO: 254 | CUACUAGUUUAUAAAGAAAGA | SEQ ID NO: 897 | UUUCUUUAUAAACUAGUAGCU | SEQ ID NO: 1540 | CUACUAGUUUAUAAAGAAA | 0.091 |
| AH0254 | SEQ ID NO: 255 | AGUUUAUAAAGAAAGAGUUGG | SEQ ID NO: 898 | AACUCUUUCUUUAUAAACUAG | SEQ ID NO: 1541 | AGUUUAUAAAGAAAGAGUU | 0.327 |
| AH0255 | SEQ ID NO: 256 | AAGAAAGAGUUGGAGAGUAUA | SEQ ID NO: 899 | UACUCUCCAACUCUUUCUUUA | SEQ ID NO: 1542 | AAGAAAGAGUUGGAGAGUA | 0.114 |
| AH0256 | SEQ ID NO: 257 | AGAAAGAGUUGGAGAGUAUAG | SEQ ID NO: 900 | AUACUCUCCAACUCUUUCUUU | SEQ ID NO: 1543 | AGAAAGAGUUGGAGAGUAU | 0.142 |
| AH0257 | SEQ ID NO: 258 | GAAAGAGUUGGAGAGUAUAGU | SEQ ID NO: 901 | UAUACUCUCCAACUCUUUCUU | SEQ ID NO: 1544 | GAAAGAGUUGGAGAGUAUA | 0.089 |
| AH0258 | SEQ ID NO: 259 | AAGAGUUGGAGAGUAUAGUCU | SEQ ID NO: 902 | ACUAUACUCUCCAACUCUUUC | SEQ ID NO: 1545 | AAGAGUUGGAGAGUAUAGU | 0.292 |
| AH0259 | SEQ ID NO: 260 | AGAGUUGGAGAGUAUAGUCUA | SEQ ID NO: 903 | GACUAUACUCUCCAACUCUUU | SEQ ID NO: 1546 | AGAGUUGGAGAGUAUAGUC | 0.178 |
| AH0260 | SEQ ID NO: 261 | GAGUUGGAGAGUAUAGUCUAU | SEQ ID NO: 904 | AGACUAUACUCUCCAACUCUU | SEQ ID NO: 1547 | GAGUUGGAGAGUAUAGUCU | 0.088 |
| AH0261 | SEQ ID NO: 262 | AGUUGGAGAGUAUAGUCUAUA | SEQ ID NO: 906 | UAGACUAUACUCUCCAACUCU | SEQ ID NO: 1548 | AGUUGGAGAGUAUAGUCUA | 0.034 |
| AH0262 | SEQ ID NO: 263 | GUUGGAGAGUAUAGUCUAUAC | SEQ ID NO: 906 | AUAGACUAUACUCUCCAACUC | SEQ ID NO: 1549 | GUUGGAGAGUAUAGUCUAU | 0.046 |
| AH0263 | SEQ ID NO: 264 | UUGGAGAGUAUAGUCUAUACA | SEQ ID NO: 907 | UAUAGACUAUACUCUCCAACU | SEQ ID NO: 1550 | UUGGAGAGUAUAGUCUAUA | 0.187 |
| AH0264 | SEQ ID NO: 266 | UGGAGAGUAUAGUCUAUACAU | SEQ ID NO: 908 | GUAUAGACUAUACUCUCCAAC | SEQ ID NO: 1551 | UGGAGAGUAUAGUCUAUAC | 0.357 |
| AH0265 | SEQ ID NO: 266 | GGAGAGUAUAGUCUAUACAUU | SEQ ID NO: 909 | UGUAUAGACUAUACUCUCCAA | SEQ ID NO: 1552 | GGAGAGUAUAGUCUAUACA | 0.133 |
| AH0266 | SEQ ID NO: 267 | GAGAGUAUAGUCUAUACAUUG | SEQ ID NO: 910 | AUGUAUAGACUAUACUCUCCA | SEQ ID NO: 1553 | GAGAGUAUAGUCUAUACAU | 0.430 |
| AH0267 | SEQ ID NO: 268 | AGAGUAUAGUCUAUACAUUGG | SEQ ID NO: 911 | AAUGUAUAGACUAUACUCUCC | SEQ ID NO: 1554 | AGAGUAUAGUCUAUACAUU | 0.079 |
| AH0268 | SEQ ID NO: 269 | GAGUAUAGUCUAUACAUUGGA | SEQ ID NO: 912 | CAAUGUAUAGACUAUACUCUC | SEQ ID NO: 1555 | GAGUAUAGUCUAUACAUUG | 0.139 |
| AH0269 | SEQ ID NO: 270 | AGUAUAGUCUAUACAUUGGAA | SEQ ID NO: 913 | CCAAUGUAUAGACUAUACUCU | SEQ ID NO: 1556 | AGUAUAGUCUAUACAUUGG | 0.291 |
| AH0270 | SEQ ID NO: 271 | GUAUAGUCUAUACAUUGGAAG | SEQ ID NO: 914 | UCCAAUGUAUAGACUAUACUC | SEQ ID NO: 1557 | GUAUAGUCUAUACAUUGGA | 0.145 |
| AH0271 | SEQ ID NO: 272 | GUCUAUACAUUGGAAGACACA | SEQ ID NO: 915 | UGUCUUCCAAUGUAUAGACUA | SEQ ID NO: 1558 | GUCUAUACAUUGGAAGACA | 0.249 |
| AH0272 | SEQ ID NO: 273 | CUAUACAUUGGAAGACACAAA | SEQ ID NO: 916 | UGUGUCUUCCAAUGUAUAGAC | SEQ ID NO: 1559 | CUAUACAUUGGAAGACACA | 0.165 |
| AH0273 | SEQ ID NO: 274 | UAUACAUUGGAAGACACAAAG | SEQ ID NO: 917 | UUGUGUCUUCCAAUGUAUAGA | SEQ ID NO: 1560 | UAUACAUUGGAAGACACAA | 0.312 |
| AH0274 | SEQ ID NO: 276 | AUACAUUGGAAGACACAAAGU | SEQ ID NO: 918 | UUUGUGUCUUCCAAUGUAUAG | SEQ ID NO: 1561 | AUACAUUGGAAGACACAAA | 0.296 |
| AH0275 | SEQ ID NO: 276 | ACAUUGGAAGACACAAAGUUA | SEQ ID NO: 919 | ACUUUGUGUCUUCCAAUGUAU | SEQ ID NO: 1562 | ACAUUGGAAGACACAAAGU | 0.362 |
| AH0276 | SEQ ID NO: 277 | CAUUGGAAGACACAAAGUUAC | SEQ ID NO: 920 | AACUUUGUGUCUUCCAAUGUA | SEQ ID NO: 1563 | CAUUGGAAGACACAAAGUU | 0.098 |
| AH0277 | SEQ ID NO: 278 | AUUGGAAGACACAAAGUUACA | SEQ ID NO: 921 | UAACUUUGUGUCUUCCAAUGU | SEQ ID NO: 1564 | AUUGGAAGACACAAAGUUA | 0.165 |
| AH0278 | SEQ ID NO: 279 | UGGAAGACACAAAGUUACAUC | SEQ ID NO: 922 | UGUAACUUUGUGUCUUCCAAU | SEQ ID NO: 1566 | UGGAAGACACAAAGUUACA | 0.257 |
| AH0279 | SEQ ID NO: 280 | GGAAGACACAAAGUUACAUCC | SEQ ID NO: 923 | AUGUAACUUUGUGUCUUCCAA | SEQ ID NO: 1566 | GGAAGACACAAAGUUACAU | 0.191 |
| AH0280 | SEQ ID NO: 281 | GAAGACACAAAGUUACAUCCA | SEQ ID NO: 924 | GAUGUAACUUUGUGUCUUCCA | SEQ ID NO: 1567 | GAAGACACAAAGUUACAUC | 0.353 |
| AH0281 | SEQ ID NO: 282 | AAGACACAAAGUUACAUGCAA | SEQ ID NO: 925 | GGAUGUAACUUUGUGUCUUCC | SEQ ID NO: 1568 | AAGACACAAAGUUACAUCC | 0.390 |
| AH0282 | SEQ ID NO: 283 | AGACACAAAGUUACAUCCAAA | SEQ ID NO: 926 | UGGAUGUAACUUUGUGUCUUC | SEQ ID NO: 1569 | AGACACAAAGUUACAUCCA | 0.281 |
| AH0283 | SEQ ID NO: 284 | GACACAAAGUUACAUCCAAAG | SEQ ID NO: 927 | UUGGAUGUAACUUUGUGUCUU | SEQ ID NO: 1570 | GACACAAAGUUACAUCCAA | 0.059 |
| AH0284 | SEQ ID NO: 285 | ACACAAAGUUACAUCCAAAGU | SEQ ID NO: 928 | UUUGGAUGUAACUUUGUGUCU | SEQ ID NO: 1571 | ACACAAAGUUACAUCCAAA | 0.047 |
| AH0285 | SEQ ID NO: 286 | CACAAAGUUACAUCCAAAGUU | SEQ ID NO: 929 | CUUUGGAUGUAACUUUGUGUC | SEQ ID NO: 1572 | CACAAAGUUACAUCCAAAG | 0.246 |
| AH0286 | SEQ ID NO: 287 | CAAAGUUACAUCCAAAGUUAU | SEQ ID NO: 930 | AACUUUGGAUGUAACUUUGUG | SEQ ID NO: 1573 | CAAAGUUACAUCCAAAGUU | 0.225 |
| AH0287 | SEQ ID NO: 288 | AAAGUUACAUCCAAAGUUAUC | SEQ ID NO: 931 | UAACUUUGGAUGUAACUUUGU | SEQ ID NO: 1574 | AAAGUUACAUCCAAAGUUA | 0.071 |
| AH0288 | SEQ ID NO: 289 | AAGUUACAUCCAAAGUUAUCG | SEQ ID NO: 932 | AUAACUUUGGAUGUAACUUUG | SEQ ID NO:1575 | AAGUUACAUCCAAAGUUAU | 0.088 |
| AH0289 | SEQ ID NO: 290 | AGUUACAUCCAAAGUUAUCGA | SEQ ID NO: 933 | GAUAACUUUGGAUGUAACUUU | SEQ ID NO: 1576 | AGUUACAUCCAAAGUUAUC | 0.276 |
| AH0290 | SEQ ID NO: 291 | GUUACAUCCAAAGUUAUCGAA | SEQ ID NO: 934 | CGAUAACUUUGGAUGUAACUU | SEQ ID NO: 1577 | GUUACAUCCAAAGUUAUCG | 0.240 |
| AH0291 | SEQ ID NO: 292 | UUACAUCCAAAGUUAUCGAAA | SEQ ID NO: 936 | UCGAUAACUUUGGAUGUAACU | SEQ ID NO: 1578 | UUACAUCCAAAGUUAUCGA | 0.352 |
| AH0292 | SEQ ID NO: 293 | UACAUCCAAAGUUAUCGAAAA | SEQ ID NO: 936 | UUCGAUAACUUUGGAUGUAAC | SEQ ID NO: 1579 | UACAUCCAAAGUUAUCGAA | 0.250 |
| AH0293 | SEQ ID NO: 294 | ACAUCCAAAGUUAUCGAAAAG | SEQ ID NO: 937 | UUUCGAUAACUUUGGAUGUAA | SEQ ID NO: 1580 | ACAUCCAAAGUUAUCGAAA | 0.266 |
| AH0294 | SEQ ID NO: 295 | CAUCCAAAGUUAUCGAAAAGU | SEQ ID NO: 938 | UUUUCGAUAACUUUGGAUGUA | SEQ ID NO: 1581 | CAUCCAAAGUUAUCGAAAA | 0.052 |
| AH0295 | SEQ ID NO: 296 | AUCCAAAGUUAUCGAAAAGUU | SEQ ID NO: 939 | CUUUUCGAUAACUUUGGAUGU | SEQ ID NO: 1582 | AUCCAAAGUUAUCGAAAAG | 0.245 |
| AH0296 | SEQ ID NO: 297 | UCCAAAGUUAUCGAAAAGUUC | SEQ ID NO: 940 | ACUUUUCGAUAACUUUGGAUG | SEQ ID NO: 1583 | UCCAAAGUUAUCGAAAAGU | 0.378 |
| AH0297 | SEQ ID NO: 298 | CCAAAGUUAUCGAAAAGUUCC | SEQ ID NO941 | AACUUUUCGAUAACUUUGGAU | SEQ ID NO: 1584 | CCAAAGUUAUCGAAAAGUU | 0.131 |
| AH0298 | SEQ ID NO: 299 | CAAAGUUAUCGAAAAGUUCCC | SEQ ID NO: 942 | GAACUUUUCGAUAACUUUGGA | SEQ ID NO: 1585 | CAAAGUUAUCGAAAAGUUC | 0.062 |
| AH0299 | SEQ ID NO: 300 | CGAAAAGUUCCCGGCUCGAGU | SEQ ID NO943 | UGGAGCCGGGAACUUUUCGAU | SEQ ID NO: 1586 | CGAAAAGUUCCCGGCUCCA | 0.329 |
| AH0300 | SEQ ID NO: 301 | AAAAGUUCCCGGCUCCAGUGC | SEQ ID NO: 944 | ACUGGAGCCGGGAACUUUUCG | SEQ ID NO: 1587 | AAAAGUUCCCGGCUCCAGU | 0.346 |

### [Table 34]

**[Table 1-7]**

| Double-stranded nucleic acid No. | SEQ ID NO: | Sense strand sequence (5'--->3') | SEQ ID NO: | Antisense strand sequence (5'--->3') | SEQ ID NO: | Target APCS mRNA sequence | Relative APCS expression level |
|---|---|---|---|---|---|---|---|
| AH0301 | SEQ ID NO: 302 | CCCGGCUCCAGUGCACAUCUG | SEQ ID NO: 945 | GAUGUGCACUGGAGCCGGGAA | SEQ ID NO: 1588 | CCCGGCUCCAGUGCACAUC | 0.368 |
| AH0302 | SEQ ID NO: 303 | CGGCUCCAGUGCACAUCUGUG | SEQ ID NO: 946 | CAGAUGUGCACUGGAGCCGGG | SEQ ID NO: 1589 | CGGCUCCAGUGCACAUCUG | 0.296 |
| AH0303 | SEQ ID NO: 304 | GGCUCCAGUGCACAUCUGUGU | SEQ ID NO: 947 | ACAGAUGUGCACUGGAGCCGG | SEQ ID NO: 1590 | GGCUCCAGUGCACAUCUGU | 0.273 |
| AH0304 | SEQ ID NO: 305 | GCUCCAGUGCACAUCUGUGUG | SEQ ID NO: 948 | CACAGAUGUGCACUGGAGCCG | SEQ ID NO: 1591 | GCUCCAGUGCACAUCUGUG | 0.075 |
| AH0305 | SEQ ID NO: 306 | CUCCAGUGCACAUCUGUGUGA | SEQ ID NO: 949 | ACACAGAUGUGCACUGGAGCC | SEQ ID NO: 1592 | CUCCAGUGCACAUCUGUGU | 0.280 |
| AH0306 | SEQ ID NO: 307 | CCAGUGCACAUCUGUGUGAGC | SEQ ID NO: 950 | UCACACAGAUGUGCACUGGAG | SEQ ID NO: 1593 | CCAGUGCACAUCUGUGUGA | 0.479 |
| AH0307 | SEQ ID NO: 308 | GUGCACAUCUGUGUGAGCUGG | SEQ ID NO: 951 | AGCUCACACAGAUGUGCACUG | SEQ ID NO: 1594 | GUGCACAUCUGUGUGAGCU | 0.452 |
| AH0308 | SEQ ID NO: 309 | GCACAUCUGUGUGAGCUGGGA | SEQ ID NO952 | CCAGCUCACACAGAUGUGCAC | SEQ ID NO1595 | GCAUCUGUGUGAGCUGG | 0.377 |
| AH0309 | SEQ ID NO: 310 | ACAUCUGUGUGAGCUGGGAGU | SEQ ID NO: 953 | UCCCAGCUCACACAGAUGUGC | SEQ ID NO: 1596 | ACAUCUGUGUGAGCUGGGA | 0.255 |
| AH0310 | SEQ ID NO: 311 | AUCUGUGUGAGCUGGGAGUCC | SEQ ID NO: 954 | ACUCCCAGCUCACACAGAUGU | SEQ ID NO: 1597 | AUCUGUGUGAGCUGGGAGU | 0.317 |
| AH0311 | SEQ ID NO: 312 | UGUGUGAGCUGGGAGUCCUCA | SEQ ID NO: 955 | AGGACUCCCAGCUCACACAGA | SEQ ID NO: 1598 | UGUGUGAGCUGGGAGUCCU | 0.234 |
| AH0312 | SEQ ID NO: 313 | GUGUGAGCUGGGAGUCCUCAU | SEQ ID NO: 956 | GAGGACUCCCAGCUCACACAG | SEQ ID NO: 1599 | GUGUGAGCUGGGAGUCCUC | 0.139 |
| AH0313 | SEQ ID NO: 314 | AGCUGGGAGUCCUCAUCAGGU | SEQ ID NO: 957 | CUGAUGAGGACUCCCAGCUCA | SEQ ID NO: 1600 | AGCUGGGAGUCCUCAUCAG | 0.237 |
| AH0314 | SEQ ID NO: 315 | GCUGGGAGUCCUCAUCAGGUA | SEQ ID NO: 958 | CCUGAUGAGGACUCCCAGCUC | SEQ ID NO: 1601 | GCUGGGAGUCCUCAUCAGG | 0.223 |
| AH0315 | SEQ ID NO: 316 | CUGGGAGUCCUCAUCAGGUAU | SEQ ID NO: 959 | ACCUGAUGAGGACUCCCAGCU | SEQ ID NO: 1602 | CUGGGAGUCCUCAUCAGGU | 0.093 |
| AH0316 | SEQ ID NO: 317 | UGGGAGUCCUCAUCAGGUAUU | SEQ ID NO: 960 | UACCUGAUGAGGACUCCCAGC | SEQ ID NO: 1603 | UGGGAGUCCUCAUCAGGUA | 0.062 |
| AH0317 | SEQ ID NO: 318 | GGGAGUCCUCAUCAGGUAUUG | SEQ ID NO: 961 | AUACCUGAUGAGGACUCCCAG | SEQ ID NO: 1604 | GGGAGUCCUCAUCAGGUAU | 0.218 |
| AH0318 | SEQ ID NO: 319 | GGAGUCCUCAUCAGGUAUUGC | SEQ ID NO: 962 | AAUACCUGAUGAGGACUCCCA | SEQ ID NO: 1605 | GGAGUCCUCAUCAGGUAUU | 0.117 |
| AH0319 | SEQ ID NO: 320 | GAGUCCUCAUCAGGUAUUGCU | SEQ ID NO: 963 | CAAUACCUGAUGAGGACUCCC | SEQ ID NO: 1606 | GAGUCCUCAUCAGGUAUUG | 0.271 |
| AH0320 | SEQ ID NO: 321 | GUCCUCAUCAGGUAUUGCUGA | SEQ ID NO: 964 | AGCAAUACCUGAUGAGGACUC | SEQ ID NO: 1607 | GUCCUCAUCAGGUAUUGCU | 0.076 |
| AH0321 | SEQ ID NO: 322 | UCCUCAUCAGGUAUUGCUGAA | SEQ ID NO: 965 | CAGCAAUACCUGAUGAGGACU | SEQ ID NO: 1608 | UCCUCAUCAGGUAUUGCUG | 0343 |
| AH0322 | SEQ ID NO: 323 | CCUCAUCAGGUAUUGCUGAAU | SEQ ID NO: 966 | UCAGCAAUACCUGAUGAGGAC | SEQ ID NO: 1609 | CCUCAUCAGGUAUUGCUGA | 0.104 |
| AH0323 | SEQ ID NO:324 | CUCAUCAGGUAUUGCUGAAUU | SEQ ID NO: 967 | UUCAGCAAUACCUGAUGAGGA | SEQ ID NO: 1610 | CUCAUCAGGUAUUGCUGAA | 0.105 |
| AH0324 | SEQ ID NO: 325 | UCAUCAGGUAUUGCUGAAUUU | SEQ ID NO: 968 | AUUCAGCAAUACCUGAUGAGG | SEQ ID NO: 1611 | UCAUCAGGUAUUGCUGAAU | 0.246 |
| AH0325 | SEQ ID NO: 326 | CAUCAGGUAUUGCUGAAUUUU | SEQ ID NO: 969 | AAUUCAGCAAUACCUGAUGAG | SEQ ID NO: 1612 | CAUCAGGUAUUGCUGAAUU | 0.137 |
| AH0326 | SEQ ID NO327 | AUCAGGUAUUGCUGAAUUUUG | SEQ ID NO: 970 | AAAUUCAGCAAUACCUGAUGA | SEQ ID NO: 1613 | AUCAGGUAUUGCUGAAUUU | 0.067 |
| AH0327 | SEQ ID NO: 328 | UCAGGUAUUGCUGAAUUUUGG | SEQ ID NO: 971 | AAAAUUCAGCAAUACCUGAUG | SEQ ID NO: 1614 | UCAGGUAUUGCUGAAUUUU | 0.243 |
| AH0328 | SEQ ID NO: 329 | CAGGUAUUGCUGAAUUUUGGA | SEQ ID NO: 972 | CAAAAUUCAGCAAUACCUGAU | SEQ ID NO: 1615 | CAGGUAUUGCUGAAUUUUG | 0.240 |
| AH0329 | SEQ ID NO: 330 | AGGUAUUGCUGAAUUUUGGAU | SEQ ID NO: 973 | CCAAAAUUCAGCAAUACCUGA | SEQ ID NO: 1616 | AGGUAUUGCUGAAUUUUGG | 0.069 |
| AH0330 | SEQ ID NO: 331 | GGUAUUGCUGAAUUUUGGAUC | SEQ ID NO: 974 | UCCAAAAUUCAGCAAUACCUG | SEQ ID NO: 1617 | GGUAUUGCUGAAUUUUGGA | 0.078 |
| AH0331 | SEQ ID NO: 332 | GUAUUGCUGAAUUUUGGAUCA | SEQ ID NO: 975 | AUCCAAAAUUCAGCAAUACCU | SEQ ID NO: 1618 | GUAUUGCUGAAUUUUGGAU | 0.165 |
| AH0332 | SEQ ID NO: 333 | AUUGCUGAAUUUUGGAUCAAU | SEQ ID NO: 976 | UGAUCCAAAAUUCAGCAAUAC | SEQ ID NO: 1619 | AUUGCUGAAUUUUGGAUCA | 0.178 |
| AH0333 | SEQ ID NO: 334 | UGCUGAAUUUUGGAUCAAUGG | SEQ ID NO: 977 | AUUGAUCCAAAAUUCAGCAAU | SEQ ID NO: 1620 | UGCUGAAUUUUGGAUCAAU | 0.220 |
| AH0334 | SEQ ID NO: 335 | GCUGAAUUUUGGAUCAAUGGG | SEQ ID NO: 978 | CAUUGAUCCAAAAUUCAGCAA | SEQ ID NO: 1621 | GCUGAAUUUUGGAUCAAUG | 0160 |
| AH0335 | SEQ ID NO: 336 | CUGAAUUUUGGAUCAAUGGGA | SEQ ID NO: 979 | CCAUUGAUCCAAAAUUCAGCA | SEQ ID NO: 1622 | CUGAAUUUUGGAUCAAUGG | 0.311 |
| AH0336 | SEQ ID NO: 337 | GAAUUUUGGAUCAAUGGGACA | SEQ ID NO: 980 | UCCCAUUGAUGCAAAAUUCAG | SEQ ID NO:1623 | GAAUUUUGGAUCAAUGGGA | 0.130 |
| AH0337 | SEQ ID NO: 338 | GGAUCAAUGGGACACCUUUGG | SEQ ID NO:981 | AAAGGUGUCCCAUUGAUGGAA | SEQ ID NO: BB1624 | GGAUCAAUGGGACACCUUU | 0.129 |
| AH0338 | SEQ ID NO: 339 | CAAUGGGACACCUUUGGUGAA | SEQ ID NO: 982 | CACCAAAGGUGUCCCAUUGAU | SEQ ID NO: 1625 | CAAUGGGACACCUUUGGUG | 0.365 |
| AH0339 | SEQ ID NO: 340 | AAUGGGACACCUUUGGUGAAA | SEQ ID NO: 983 | UCACCAAAGGUGUCCCAUUGA | SEQ ID NO: 1626 | AAUGGGACACCUUUGGUGA | 0.257 |
| AH0340 | SEQ ID NO: 341 | AUGGGACACCUUUGGUGAAAA | SEQ ID NO: 984 | UUCACCAAAGGUGUCCCAUUG | SEQ ID NO: 1627 | AUGGGACACCUUUGGUGAA | 0.333 |
| AH0341 | SEQ ID NO: 342 | UGGGACACCUUUGGUGAAAAA | SEQ ID NO: 985 | UUUCACCAAAGGUGUCCCAUU | SEQ ID NO: 1628 | UGGGACACCUUUGGUGAAA | 0.326 |
| AH0342 | SEQ ID NO: 343 | GGGACACCUUUGGUGAAAAAG | SEQ ID NO: 986 | UUUUCACCAAAGGUGUCCCAU | SEQ ID NO: 1629 | GGGACACCUUUGGUGAAAA | 0.195 |
| AH0343 | SEQ ID NO: 344 | GGACACCUUUGGUGAAAAAGG | SEQ ID NO: 987 | UUUUUCACCAAAGGUGUCCCA | SEQ ID NO: 1630 | GGACACCUUUGGUGAAAAA | 0.122 |
| AH0344 | SEQ ID NO: 345 | ACCUUUGGUGAAAAAGGGUCU | SEQ ID NO: 988 | ACCCUUUUUCACCAAAGGUGU | SEQ ID NO:1631 | ACCUUUGGUGAAAAAGGGU | 0.253 |
| AH0345 | SEQ ID NO: 346 | CCUUUGGUGAAAAAGGGUCUG | SEQ ID NO: 989 | GACCCUUUUUCACCAAAGGUG | SEQ ID NO: 1632 | CCUUUGGUGAAAAAGGGUC | 0.440 |
| AH0346 | SEQ ID NO: 347 | CUUUGGUGAAAAAGGGUCUGC | SEQ ID NO: 990 | AGACCCUUUUUCACCAAAGGU | SEQ ID NO: 1633 | CUUUGGUGAAAAAGGGUCU | 0.125 |
| AH0347 | SEQ ID NO: 348 | GGUGAAAAAGGGUCUGCGACA | SEQ ID NO: 991 | UCGCAGACCCUUUUUCACCAA | SEQ ID NO: 1634 | GGUGAAAAAGGGUCUGCGA | 0.082 |
| AH0348 | SEQ ID NO: 349 | GUGAAAAAGGGUCUGCGACAG | SEQ ID NO: 992 | GUCGCAGACCCUUUUUCACCA | SEQ ID NO: 1635 | GUGAAAAAGGGUCUGCGAC | 0.270 |
| AH0349 | SEQ ID NO: 350 | UGAAAAAGGGUCUGCGACAGG | SEQ ID NO: 993 | UGUCGCAGACCCUUUUUCACC | SEQ ID NO: 1636 | UGAAAAAGGGUCUGCGACA | 0.375 |
| AH0350 | SEQ ID NO: 351 | GAAAAAGGGUCUGCGACAGGG | SEQ ID NO: 994 | CUGUCGCAGACCCUUUUUCAC | SEQ ID NO: 1637 | GAAAAAGGGUCUGCGACAG | 0.376 |

### [Table 35]

**Table 1-8**

| Double-stranded nucleic acid No. | SEQ ID NO: | Sense strand sequence (5'--->3') | SEQ ID NO: | Antisense strand sequence (5'--->3') | SEQ ID NO: | Target APCS mRNA sequence | Relative APCS expression level |
|---|---|---|---|---|---|---|---|
| AH0351 | SEQ ID NO: 352 | AAAGGGUCUGCGACAGGGUUA | SEQ ID NO: 995 | ACCCUGUCGCAGACCCUUUUU | SEQ ID NO: 1638 | AAAGGGUCUGCGACAGGGU | 0.326 |
| AH0352 | SEQ ID NO: 353 | AAGGGUCUGCGACAGGGUUAC | SEQ ID NO: 996 | AACCCUGUCGCAGACCCUUUU | SEQ ID NO: 1639 | AAGGGUCUGCGACAGGGUU | 0.192 |
| AH0353 | SEQ ID NO: 354 | AGGGUCUGCGACAGGGUUACU | SEQ ID NO: 997 | UAACCCUGUCGCAGACCCUUU | SEQ ID NO: 1640 | AGGGUCUGCGACAGGGUUA | 0.106 |
| AH0354 | SEQ ID NO: 355 | GGGUCUGCGACAGGGUUACUU | SEQ ID NO: 998 | GUAACCCUGUCGCAGACCCUU | SEQ ID NO: 1641 | GGGUCUGCGACAGGGUUAC | 0.098 |
| AH0355 | SEQ ID NO: 356 | GGUCUGCGACAGGGUUACUUU | SEQ ID NO: 999 | AGUAACCCUGUCGCAGACCCU | SEQ ID NO: 1642 | GGUCUGCGACAGGGUUACU | 0.195 |
| AH0356 | SEQ ID NO: 357 | GUCUGCGACAGGGUUACUUUG | SEQ ID NO: 1000 | AAGUAACCCUGU CGCAGACCC | SEQ ID NO: 1643 | GUCUGCGACAGGGUUACUU | 0.154 |
| AH0357 | SEQ ID NO: 358 | UCUGCGACAGGGUUACUUUGU | SEQ ID NO: 1001 | AAAGUAACCCUGUCGCAGACC | SEQ ID NO: 1644 | UCUGCGACAGGGUUACUUU | 0.126 |
| AH0358 | SEQ ID NO: 359 | CUGCGACAGGGUUACUUUGUA | SEQ ID NO: 1002 | CAAAGUAACCCUGUCGCAGAC | SEQ ID NO: 1645 | CUGCGACAGGGUUACUUUG | 0.120 |
| AH0359 | SEQ ID NO: 360 | UGCGACAGGGUUACUUUGUAG | SEQ ID NO: 1003 | ACAAAGUAACCCUGUCGCAGA | SEQ ID NO: 1646 | UGCGACAGGGUUACUUUGU | 0.438 |
| AH0360 | SEQ ID NO: 361 | GCGACAGGGUUACUUUGUAGA | SEQ ID NO: 1004 | UACAAAGUAACCCUGUCGCAG | SEQ ID NO: 1647 | GCGACAGGGUUACUUUGUA | 0.104 |
| AH0361 | SEQ ID NO: 362 | CGACAGGGUUACUUUGUAGAA | SEQ ID NO: 1005 | CUACAAAGUAACCCUGUCGCA | SEQ ID NO: 1648 | CGACAGGGUUACUUUGUAG | 0.290 |
| AH0362 | SEQ ID NO: 363 | GACAGGGUUACUUUGUAGAAG | SEQ ID NO: 1006 | UGUACAAAGUAAGGCUGUCGC | SEQ ID NO: 1649 | GACAGGGUUACUUUGUAGA | 0.453 |
| AH0363 | SEQ ID NO: 364 | ACAGGGUUACUUUGUAGAAGC | SEQ ID NO: 1007 | UUCUACAAAGUAACCCUGUCG | SEQ ID NO: 1650 | ACAGGGUUACUUUGUAGAA | 0.467 |
| AH0364 | SEQ ID NO: 365 | CAGGGUUACUUUGUAGAAGCU | SEQ ID NO: 1008 | CUUCUACAAAGUAACCCUGUC | SEQ ID NO: 1651 | CAGGGUUACUUUGUAGAAG | 0.156 |
| AH0365 | SEQ ID NO: 366 | AGGGUUACUUUGUAGAAGCUC | SEQ ID NO: 1009 | GCUUCUACAAAGUAACCCUGU | SEQ ID NO: 1652 | AGGGUUACUUUGUAGAAGC | 0.280 |
| AH0366 | SEQ ID NO: 367 | GGGUUACUUUGUAGAAGCUCA | SEQ ID NO: 1010 | AGCUUCUACAAAGUAACCCUG | SEQ ID NO: 1653 | GGGUUACUUUGUAGAAGCU | 0.179 |
| AH0367 | SEQ ID NO: 368 | GGUUACUUUGUAGAAGCUCAG | SEQ ID NO: 1011 | GAGCUUCUACAAAGUAACCCU | SEQ ID NO: 1654 | GGUUACUUUGUAGAAGCUG | 0.222 |
| AH0368 | SEQ ID NO: 369 | GUUACUUUGUAGAAGCUCAGC | SEQ ID NO: 1012 | UGAGCUUCUACAAAGUAACCC | SEQ ID NO: 1655 | GUUACUUUGUAGAAGCUCA | 0.083 |
| AH0369 | SEQ ID NO: 370 | AGAAGCUCAGCCCAAGAUUGU | SEQ ID NO: 1013 | AAUCUUGGGCUGAGCUUCUAC | SEQ ID NO: 1656 | AGAAGCUCAGCCCAAGAUU | 0.313 |
| AH0370 | SEQ ID NO: 371 | GAAGCUCAGCCCAAGAUUGUC | SEQ ID NO 1014 | CAAUCUUGGGCUGAGCUUCUA | SEQ ID NO: 1657 | GAAGCUCAGCCCAAGAUUG | 0.088 |
| AH0371 | SEQ ID NO: 372 | AAGCUCAGCCCAAGAUUGUCC | SEQ ID NO: 1015 | ACAAUCUUGGGCUGAGCUUCU | SEQ ID NO: 1658 | AAGCUCAGCCCAAGAUUGU | 0.093 |
| AH0372 | SEQ ID NO: 373 | AGCUCAGCCCAAGAUUGUCCU | SEQ ID NO: 1016 | GACAAUCUUGGGCUGAGCUUC | SEQ ID NO: 1659 | AGCUCAGCCCAAGAUUGUC | 0.142 |
| AH0373 | SEQ ID NO: 374 | CUCAGCCCAAGAUUGUCCUGG | SEQ ID NO: 1017 | AGGACAAUCUUGGGCUGAGCU | SEQ ID NO: 1660 | CUCAGCCCAAGAUUGUCCU | 0.255 |
| AH0374 | SEQ ID NO: 375 | AGCCCAAGAUUGUCCUGGGGC | SEQ ID NO: 1018 | CCCAGGACAAUCUUGGGCUGA | SEQ ID NO: 1661 | AGCCCAAGAUUGUCCUGGG | 0.308 |
| AH0375 | SEQ ID NO: 376 | CCAAGAUUGUCCUGGGGCAGG | SEQ ID NO: 1019 | UGCCCCAGGACAAUCUUGGGG | SEQ ID NO: 1662 | CCAAGAUUGUCCUGGGGCA | 0.097 |
| AH0376 | SEQ ID NO: 377 | CAAGAUUGUCCUGGGGCAGGA | SEQ ID NO: 1020 | CUGCCCCAGGACAAUCUUGGG | SEQ ID NO: 1663 | CAAGAUUGUCCUGGGGCAG | 0.316 |
| AH0377 | SEQ ID NO: 378 | AGAUUGUCCUGGGGCAGGAAC | SEQ ID NO: 1021 | UCCUGCCCCAGGACAAUCUUG | SEQ ID NO: 1664 | AGAUUGUCCUGGGGCAGGA | 0.116 |
| AH0378 | SEQ ID NO: 379 | GAUUGUCCUGGGGCAGGAACA | SEQ ID NO: 1022 | UUCCUGCCCCAGGACAAUCUU | SEQ ID NO: 1665 | GAUUGUCCUGGGGCAGGAA | 0.193 |
| AH0379 | SEQ ID NO: 380 | UUGUCCUGGGGCAGGAACAGG | SEQ ID NO: 1023 | UGUUCCUGCCCCAGGACAAUC | SEQ ID NO: 1666 | UUGUCCUGGGGCAGGAACA | 0.432 |
| AH0380 | SEQ ID NO: 381 | UCCUGGGGCAGGAACAGGAUU | SEQ ID NO: 1024 | UCCUGUUCCUGCCCCAGGACA | SEQ ID NO: 1667 | UCCUGGGGCAGGAACAGGA | 0.170 |
| AH0381 | SEQ ID NO: 382 | CUGGGGCAGGAACAGGAUUCC | SEQ ID NO1025 | AAUCCUGUUCCUGCCCCAGGA | SEQ ID NO: 1668 | CUGGGGCAGGAACAGGAUU | 0.448 |
| AH0382 | SEQ ID NO: 383 | UGGGGCAGGAACAGGAUUCCU | SEQ ID NO: 1026 | GAAUCCUGUUCCUGCCCCAGG | SEQ ID NO: 1669 | UGGGGCAGGAACAGGAUUC | 0.494 |
| AH0383 | SEQ ID NO: 384 | GGGGCAGGAACAGGAUUCCUA | SEQ ID NO: 1027 | GGAAUCCUGUUCCUGCCCCAG | SEQ ID NO: 1670 | GGGGCAGGAACAGGAUUCC | 0.244 |
| AH0384 | SEQ ID NO: 385 | GGCAGGAACAGGAUUCCUAUG | SEQ ID NO: 1028 | UAGGAAUCCUGUUCCUGCCCC | SEQ ID NO: 1671 | GGCAGGAACAGGAUUCCUA | 0.074 |
| AH0385 | SEQ ID NO: 386 | GCAGGAACAGGAUUCCUAUGG | SEQ ID NO: 1029 | AUAGGAAUCCUGUUCCUGCCC | SEQ ID NO: 1672 | GCAGGAACAGGAUUCCUAU | 0.146 |
| AH0388 | SEQ ID NO: 387 | CAGGAACAGGAUUCCUAUGGG | SEQ ID NO: 1030 | CAUAGGAAUCCUGUUCCUGCC | SEQ ID NO: 1673 | CAGGAACAGGAUUCCUAUG | 0.280 |
| AH0387 | SEQ ID NO: 388 | GGAACAGGAUUCCUAUGGGGG | SEQ ID NO: 1031 | CCCAUAGGAAUCCUGUUCCUG | SEQ ID NO: 1674 | GGAACAGGAUUCCUAUGGG | 0.260 |
| AH0388 | SEQ ID NO: 389 | GAACAGGAUUCCUAUGGGGGC | SEQ ID NO: 1032 | CCCCAUAGGAAUCCUGUUCCU | SEQ ID NO: 1675 | GAACAGGAUUCCUAUGGGG | 0.437 |
| AH0389 | SEQ ID NO: 390 | ACAGGAUUCCUAUGGGGGCAA | SEQ ID NO: 1033 | GCCCCCAUAGGAAUCCUGUU C | SEQ ID NO: 1676 | ACAGGAUUCCUAUGGGGGC | 0.349 |
| AH0390 | SEQ ID NO: 391 | CAGGAUUCCUAUGGGGGCAAG | SEQ ID NO: 1034 | UGCCCCCAUAGGAAUCCUGUU | SEQ ID NO: 1677 | CAGGAUUCCUAUGGGGGCA | 0.279 |
| AH0391 | SEQ ID NO: 392 | GGAUUCCUAUGGGGGCAAGUU | SEQ ID NO: 1035 | CUUGCCCCCAUAGGAAUCCUG | SEQ ID NO: 1678 | GGAUUCCUAUGGGGGCAAG | 0.153 |
| AH0392 | SEQ ID NO: 393 | GAUUCCUAUGGGGGCAAGUUU | SEQ ID NO: 1036 | ACUUGCCCCCAUAGGAAUCCU | SEQ ID NO: 1679 | GAUUCCUAUGGGGGCAAGU | 0.257 |
| AH0393 | SEQ ID NO: 394 | AUUCCUAUGGGGGCAAGUUUG | SEQ ID NO: 1037 | AACUUGCCCCCAUAGGAAUCC | SEQ ID NO: 1680 | AUUCCUAUGGGGGCAAGUU | 0.373 |
| AH0394 | SEQ ID NO: 395 | UUCCUAUGGGGGCAAGUUUGA | SEQ ID NO: 1038 | AAACUUGCCCCCAUAGGAAUC | SEQ ID NO: 1681 | UUCCUAUGGGGGCAAGUUU | 0.481 |
| AH0395 | SEQ ID NO: 396 | UCCUAUGGGGGCAAGUUUGAU | SEQ ID NO: 1039 | CAAACUUGCCCCCAUAGGAAU | SEQ ID NO: 1682 | UCCUAUGGGGGCAAGUUUG | 0.395 |
| AH0396 | SEQ ID NO: 397 | CCUAUGGGGGCAAGUUUGAUA | SEQ ID NO: 1040 | UCAAACUUGCCCCCAUAGGAA | SEQ ID NO: 1683 | CCUAUGGGGGCAAGUUUGA | 0.272 |
| AH0397 | SEQ ID NO: 398 | CUAUGGGGGCAAGUUUGAUAG | SEQ ID NO: 1041 | AUCAAACUUGCCCCCAUAGGA | SEQ ID NO: 1684 | CUAUGGGGGGAAGUUUGAU | 0.198 |
| AH0398 | SEQ ID NO: 399 | UAUGGGGGCAAGUUUGAUAGG | SEQ ID NO: 1042 | UAUCAAACUUGCCCCCAUAGG | SEQ ID NO: 1685 | UAUGGGGGCAAGUUUGAUA | 0481 |
| AH0399 | SEQ ID NO: 400 | GGGGGCAAGUUUGAUAGGAGC | SEQ ID NO: 1043 | UCCUAUCAAACUUGCCCCCAU | SEQ ID NO: 1686 | GGGGGCAAGUUUGAUAGGA | 0.268 |
| AH0400 | SEQ ID NO: 401 | GGGGCAAGUUUGAUAGGAGCC | SEQ ID NO: 1044 | CUCCUAUCAAACUUGCCCCCA | SEQ ID NO: 1687 | GGGGCAAGUUUGAUAGGAG | 0.100 |

### [Table 36]

**Table 1-9**

| Double-stranded nucleic acid No. | SEQ ID NO: | Sense strand sequence (5'--->3') | SEQ ID NO: | Antisense strand sequence (5'--->3') | SEQ ID NO: | Target APCS mRNA sequence | Relative APCS expression level |
|---|---|---|---|---|---|---|---|
| AH0401 | SEQ ID NO: 402 | GGGCAAGUUUGAUAGGAGCCA | SEQ ID NO: 1045 | GCUCCUAUCAAACUUGCCCCC | SEQ ID NO: 1688 | GGGCAAGUUUGAUAGGAGC | 0.114 |
| AH0402 | SEQ ID NO: 403 | GGCAAGUUUGAUAGGAGCCAG | SEQ ID NO: 1046 | GGCUCCUAUCAAACUUGCCCC | SEQ ID NO: 1689 | GGCAAGUUUGAUAGGAGCC | 0.314 |
| AH0403 | SEQ ID NO: 404 | GCAAGUUUGAUAGGAGCCAGU | SEQ ID NO: 1047 | UGGCUCCUAUCAAACUUGCCC | SEQ ID NO: | GCAAGUUUGAUAGGAGCCA | 0.235 |
| AH0404 | SEQ ID NO: 405 | CAAGUUUGAUAGGAGCCAGUC | SEQ ID NO: 1048 | CUGGCUCGUAUCAAACUUGCC | SEQ ID NO: 1691 | CAAGUUUGAUAGGAGCCAG | 0.274 |
| AH0405 | SEQ ID NO: 406 | AAGUUUGAUAGGAGCCAGUCC | SEQ ID NO: 1049 | ACUGGCUCCUAUCAAACUUGC | SEQ ID NO: 1692 | AAGUUUGAUAGGAGCCAGU | 0.085 |
| AH0406 | SEQ ID NO: 407 | UUGAUAGGAGCCAGUCCUUUG | SEQ ID NO: 1050 | AAGGACUGGCUCCUAUCAAAC | SEQ ID NO: 1693 | UUGAUAGGAGCCAGUCCUU | 0.242 |
| AH0407 | SEQ ID NO: 408 | UGAUAGGAGCCAGUCCUUUGU | SEQ ID NO: 1051 | AAAGGACUGGCUCCUAUCAAA | SEQ ID NO: 1694 | UGAUAGGAGCCAGUCCUUU | 0.239 |
| AH0408 | SEQ ID NO: 409 | GAUAGGAGCCAGUCCUUUGUG | SEQ ID NO: 1052 | CAAAGGACUGGCUCCUAUCAA | SEQ ID NO: 1695 | GAUAGGAGCCAGUCCUUUG | 0.160 |
| AH0409 | SEQ ID NO: 410 | AUAGGAGCCAGUCCUUUGUGG | SEQ ID NO: 1053 | ACAAAGGACUGGCUCCUAUCA | SEQ ID NO: 1696 | AUAGGAGCCAGUCCUUUGU | 0.159 |
| 4H0410 | SEQ ID NO: 411 | AGGAGCCAGUCCUUUGUGGGA | SEQ ID NO: 1054 | CCACAAAGGACUGGCUCCUAU | SEQ ID NO: 1697 | AGGAGCCAGUGCUUUGUGG | 0313 |
| AH0411 | SEQ ID NO: 412 | GGAGCCAGUCCUUUGUGGGAG | SEQ ID NO: 1055 | CCCACAAAGGACUGGCUCCUA | SEQ ID NO: 1698 | GGAGCCAGUCCUUUGUGGG | 0.232 |
| AH0412 | SEQ ID NO: 413 | GAGCCAGUCCUUUGUGGGAGA | SEQ ID NO: 1056 | UCCCACAAAGGACUGGCUCCU | SEQ ID NO: 1699 | GAGCCAGUCCUUUGUGGGA | 0.098 |
| AH0413 | SEQ ID NO: 414 | AGCCAGUCCUUUGUGGGAGAG | SEQ ID NO: 1057 | CUCCCACAAAGGACUGGCUCC | SEQ ID NO: 1700 | AGCCAGUCCUUUGUGGGAG | 0.325 |
| AH0414 | SEQ ID NO: 415 | GCCAGUCCUUUGUGGGAGAGA | SEQ ID NO: 1058 | UCUCCCACAAAGGACUGGCUC | SEQ ID NO: 1701 | GCCAGUCCUUUGUGGGAGA | 0.109 |
| AH0415 | SEQ ID NO: 416 | CCAGUCCUUUGUGGGAGAGAU | SEQ ID NO: 1059 | CUCUCCCACAAAGGACUGGCU | SEQ ID NO: 1702 | CCAGUCCUUUGUGGGAGAG | 0.367 |
| AH0416 | SEQ ID NO: 417 | CAGUCCUUUGUGGGAGAGAUU | SEQ ID NO: 1060 | UCUCUCCCACAAAGGACUGGC | SEQ ID NO: 1703 | CAGUCCUUUGUGGGAGAGA | 0.119 |
| AH0417 | SEQ ID NO: 418 | AGUCCUUGUGGGAGAGAUUG | SEQ ID NO: 1061 | AUCUCUCCCACAAAGGACUGG | SEQ ID NO: 1704 | AGUCCUUUGUGGGAGAGAU | 0.074 |
| AH0418 | SEQ ID NO: 419 | GUCCUUUGUGGGAGAGAUUGG | SEQ ID NO: 1062 | AAUCUCUCCCACAAAGGACUG | SEQ ID NO: 1705 | GUCCUUUGUGGGAGAGAUU | 0.095 |
| AH0419 | SEQ ID NO: 420 | UCCUUUGUGGGAGAGAUUGGG | SEQ ID NO: 1063 | CAAUCUCUCCCACAAAGGACU | SEQ ID NO: 1706 | UCCUUUGUGGGAGAGAUUG | 0.358 |
| AH0420 | SEQ ID NO: 421 | CCUUUGUGGGAGAGAUUGGGG | SEQ ID NO: 1064 | CCAAUCUCUCCCACAAAGGAC | SEQ ID NO: 1707 | CCUUUGUGGGAGAGAUUGG | 0.315 |
| AH0421 | SEQ ID NO: 422 | CUUUGUGGGAGAGAUUGGGGA | SEQ ID NO: 1065 | CCCAAUCUCUCCCACAAAGGA | SEQ ID NO: 1708 | CUUUGUGGGAGAGAUUGGG | 0.301 |
| AH0422 | SEQ ID NO: 423 | UUGUGGGAGAGAUUGGGGAUU | SEQ ID NO: 1066 | UCCCCAAUCUCUCCCACAAAG | SEQ ID NO: 1709 | UUGUGGGAGAGAUUGGGGA | 0.214 |
| AH0423 | SEQ ID NO: 424 | UGUGGGAGAGAUUGGGGAUUU | SEQ ID NO: 1067 | AUCCCCAAUCUCUCCCACAAA | SEQ ID NO: 1710 | UGUGGGAGAGAUUGGGAU | 0.087 |
| AH0424 | SEQ ID NO: 425 | GUGGGAGAGAUUGGGGAUUUG | SEQ ID NO: 1068 | AAUCCCCAAUCUCUCCCACAA | SEQ ID NO: 1711 | GUGGGAGAGAUUGGGGAUU | 0.099 |
| AH0425 | SEQ ID NO426 | UGGGAGAGAUUGGGGAUUUGU | SEQ ID NO: 1069 | AAAUCCCCAAUCUCUCCCACA | SEQ ID NO1712 | UGGGAGAGAUUGGGGAUUU | 0.109 |
| AH0426 | SEQ ID NO: 427 | GGGAGAGAUUGGGGAUUUGUA | SEQ ID NO: 1070 | CAAAUCCCCAAUCUCUCCCAC | SEQ ID NO: 1713 | GGGAGAGAUUGGGGAUUUG | 0.068 |
| AH0427 | SEQ ID NO: 428 | GGAGAGAUUGGGGAUUUGUAC | SEQ ID NO: 1071 | ACAAAUCCCCAAUCUCUCCCA | SEQ ID NO: 1714 | GGAGAGAUUGGGGAUUUGU | 0.071 |
| AH0428 | SEQ ID NO: 429 | GAGAGAUUGGGGAUUUGUACA | SEQ ID NO: 1072 | UACAAAUCCCCAAUCUCUCCC | SEQ ID NO1715 | GAGAGAUUGGGGAUUUGUA | 0.047 |
| AH0429 | SEQ ID NO: 430 | AGAGAUUGGGGAUUUGUACAU | SEQ ID NO: 1073 | GUACAAAUCCCCAAUCUCUCC | SEQ ID NO: 1716 | AGAGAUUGGGGAUUUGUAC | 0.175 |
| AH0430 | SEQ ID NO: 431 | GAGAUUGGGGAUUUGUACAUG | SEQ ID NO: 1074 | UGUACAAAUCCCCAAUCUCUC | SEQ ID NO: 1717 | GAGAUUGGGGAUUUGUACA | 0.079 |
| AH0431 | SEQ ID NO: 432 | AGAUUGGGGAUUUGUACAUGU | SEQ ID NO: 1075 | AUGUACAAAUCCCCAAUCUCU | SEQ ID NO: 1718 | AGAUUGGGGAUUUGUACAU | 0.391 |
| AH0432 | SEQ ID NO: 433 | GGGGAUUUGUACAUGUGGGAC | SEQ ID NO: 1076 | CCCACAUGUACAAAUCCCCAA | SEQ ID NO: 1719 | GGGGAUUUGUACAUGUGGG | 0154 |
| AH0433 | SEQ ID NO: 434 | GGGAUUUGUACAUGUGGGACU | SEQ ID NO: 1077 | UCCCACAUGUACAAAUCCCCA | SEQ ID NO: 1720 | GGGAUUUGUACAUGUGGGA | 0.087 |
| AH0434 | SEQ ID NO: 435 | GGAUUUGUACAUGUGGGACUC | SEQ ID NO: 1078 | GUCCCACAUGUACAAAUCCCC | SEQ ID NO: 1721 | GGAUUUGUACAUGUGGGAC | 0.324 |
| AH0435 | SEQ ID NO: 436 | GAUUUGUACAUGUGGGACUCU | SEQ ID NO: 1079 | ACUCCCACAUGUACAAAUCCC | SEQ ID NO: 1722 | GAUUUGUACAUGUGGGACU | 0.308 |
| AH0436 | SEQ ID NO437 | UGUACAUGUGGGACUCUGUGC | SEQ ID NO1080 | ACAGAGUCCCACAUGUACAAA | SEQ ID NO: 1723 | UGUACAUGUGGGACUCUGU | 0.210 |
| AH0437 | SEQ ID NO: 438 | GUACAUUGGGACUCUGUGCU | SEQ ID NO1081 | CACAGAGUCCCACAUGUACAA | SEQ ID NO: 1724 | GUACAUGUGGGACUCUGUG | 0.384 |
| AH0438 | SEQ ID NO: 439 | ACAUGUGGGACUCUGUGCUGC | SEQ ID NO: 1082 | AGCACAGAGUCCCACAUGUAC | SEQ ID NO: 1725 | ACAUGUGGGACUCUGUGCU | 0.269 |
| AH0439 | SEQ ID NO: 440 | GGGACUCUGUGCUGCCCCCAG | SEQ ID NO: 1083 | GGGGGCAGCACAGAGUCCCAC | SEQ ID NO: 1726 | GGGACUCUGUGCUGCCCCC | 0.463 |
| AH0440 | SEQ ID NO: 441 | GGACUCUGUGCUGCCCCCAGA | SEQ ID NO: 1084 | UGGGGGCAGCACAGAGUCCCA | SEQ ID NO: 1727 | GGACUCUGUGCUGCCCCCA | 0.500 |
| AH0441 | SEQ ID NO: 442 | ACUCUGUGCUGCCCCCAGAAA | SEQ ID NO: 1085 | UCUGGGGGCAGCACAGAGUCC | SEQ ID NO: 1728 | ACUCUGUGCUGCCCCCAGA | 0.308 |
| AH0442 | SEQ ID NO: 443 | CUCUGUGCUGCCCCCAGAAAA | SEQ ID NO: 1086 | UUCUGGGGGCAGCACAGAGUC | SEQ ID NO: 1729 | CUCUGUGCUGCCCCCAGAA | 0.459 |
| AH0443 | SEQ ID NO: 444 | CUGUGCUGCCCCCAGAAAAUA | SEQ ID NO: 1087 | UUUUCUGGGGGCAGCACAGAG | SEQ ID NO: 1730 | CUGUGCUGCCCCCAGAAAA | 0.134 |
| AH0444 | SEQ ID NO: 445 | UGUGCUGCCCCCAGAAAAUAU | SEQ ID NO: 1088 | AUUUUCUGGGGGCAGCACAGA | SEQ ID NO: 1731 | UGUGGUGCCCGCAGAAAU | 0.063 |
| AH0445 | SEQ ID NO: 446 | GUGCUGCCCCCAGAAAAUAUC | SEQ ID NO: 1089 | UAUUUUCUGGGGGCAGCACAG | SEQ ID NO: 1732 | GUGCUGCCCCCAGAAAAUA | 0.433 |
| AH0446 | SEQ ID NO: 447 | GCUGCCCCCAGAAAAUAUCCU | SEQ ID NO: 1090 | GAUAUUUUCUGGGGGCAGCAC | SEQ ID NO: 1733 | GCUGCCCCCAGAAAAUAUC | 0.414 |
| AH0447 | SEQ ID NO: 448 | CCCCCAGAAAAUAUCCUGUCU | SEQ ID NO1091 | ACAGGAUAUUUUCUGGGGGCA | SEQ ID NO: 1734 | CCCCCAGAAAAUAUCCUGU | 0.189 |
| AH0448 | SEQ ID NO: 449 | CCCAGAAAAUAUCCUGUCUGC | SEQ ID NO: 1092 | AGACAGGAUAUUUUCUGGGGG | SEQ ID NO: 1735 | CCCAGAAAAUAUCCUGUCU | 0.489 |
| AH0449 | SEQ ID NO: 450 | CCAUGAAAAUAUCCUGUCUGCC | SEQ ID NO: 1093 | CAGACAGGAUAUUUUCUGGGG | SEQ ID NO: 1736 | CCAGAAAAUAUCCUGUCUG | 0.353 |
| AH 0450 | SEQ ID NO: 451 | CAGAAAAUAUCCUGUCUGCCU | SEQ ID NO: 1094 | GCAGACAGGAUAUUUUCUGGG | SEQ ID NO: 1737 | CAGAAAAUAUCCUGUCUGC | 0.293 |

### [Table 37]

**Table 1-10**

| Double-stranded nucleic acid No. | SEQ ID NO: | Sense strand sequence (5'--->3') | SEQ ID NO: | Antisense strand sequence (5'--->3') | SEQ ID NO: | Target APCS mRNA sequence | Relative APCS expression level |
|---|---|---|---|---|---|---|---|
| AH0451 | SEQ ID NO: 452 | GAAAAUAUCCUGUCCUGCCUAU | SEQ ID NO: 1095 | AGGCAGACAGGAUAUUUUCUG | SEQ ID NO: 1738 | GAAAAUAUCCUGUGGU | 0.121 |
| AH0452 | SEQ ID NO: 453 | AAAAUAUCCUGUCUGCCUAUC | SEQ ID NO: 1096 | UAGGCAGACAGGAUAUUUUCU | SEQ ID NO : 1739 | AAAAUAUCCUGUCUGCCUA | 0.053 |
| AH0453 | SEQ ID NO: 454 | AAAUAUCCUGUCUGCCUAUCA | SEQ ID NO: 1097 | AUAGGCAGACAGGAUAUUUUC | SEQ ID NO: 1740 | AAAUAUCCUGUCUGCCUAU | 0.109 |
| AH0454 | SEQ ID NO: 456 | CCUGUCUGCCUAUCAGGGUAC | SEQ ID NO: 1098 | ACCCUGAUAGGCAGACAGGAU | SEQ ID NO: 1741 | CCUGUCUGCCUAUCAGGGU | 0.298 |
| AH0455 | SEQ ID NO: 456 | CUGUCUGCCUAUGAGGGUACC | SEQ ID NO: 1099 | UACCCUGAUAGGCAGACAGGA | SEQ ID NO: 1742 | CUGUCUGCCUAUCAGGGUA | 0.113 |
| AH0456 | SEQ ID NO:457 | GUCUGCCUAUCAGGGUACCCC | SEQ ID NO: 1100 | GGUACCCUGAUAGGCAGACAG | SEQ ID NO: 1743 | GUCUGCCUAUCAGGGCC | 0.336 |
| AH0457 | SEQ ID NO: 458 | UGCCUAUCAGGGUACCCCUCU | SEQ ID NO : 1101 | AGGGGUACCCUGAUAGGCAGA | SEQ ID NO: 1744 | UGCCUAUCAGGGUACCCCU | 0.251 |
| AH0458 | SEQ ID NO: 469 | GCCUAUCAGGGUACCCCUCUC | SEQ ID NO: 1102 | GAGGGUACCCUGAUAGGCAG | SEQ ID NO: 1745 | GCCUAUCAGGGUACCCCUC | 0.218 |
| AH0459 | SEQ ID NO: 460 | CCUAUCAGGGUACCCCUCUCC | SEQ ID NO: 1103 | AGAGGGGUACCCUGAUAGGCA | SEQ ID NO: 1746 | CCUAUCAGGGUACCCCUCU | 0.403 |
| AH0460 | SEQ ID NO: 461 | CUAUCAGGGUACCCCUCUCCC | SEQ ID NO: 1104 | GAGAGGGGUACCCUGAUAGGC | SEQ ID NO: 1747 | CUAUCAGGGUACCCCUCUC | 0.104 |
| AH0461 | SEQ ID NO: 462 | CAGGGUACCCCUCUCCCUGCC | SEQ ID NO: 1105 | CAGGGAGAGGGGUACCCUGAU | SEQ ID NO: 1748 | CAGGGUACCCCUCUCCCUG | 0.300 |
| AH0462 | SEQ ID NO: 463 | GGGUACCCCUCUCCCUGCCAA | SEQ ID NO: 1106 | GGCAGGGAGAGGGGUACCCUG | SEQ ID NO: 1749 | GGGUACCCCUCUCCCUGCC | 0.463 |
| AH0463 | SEQ ID NO: 464 | GGUACCCCUCUCCCUGCCAAU | SEQ ID NO: 1107 | UGGCAGGGAGAGGGGUACCCU | SEQ ID NO: 1750 | GGUACCCCUCU CCCUGCCA | 0.476 |
| AH0464 | SEQ ID NO: 465 | GUACCCCUCUCCCUGCCAAUA | SEQ ID NO: 1108 | UUGGCAGGGAGAGGGGUACCC | SEQ ID NO: 1751 | GUACCCCUCUCCCUGCCAA | 0.159 |
| AH0465 | SEQ ID NO: 466 | UACCCCUCUCCCUGCCAAUAU | SEQ ID NO: 1109 | AUUGGCAGGGAGAGGGGUACC | SEQ ID NO: 1752 | GUACCCCUCUCCGGG | 0391 |
| AH0466 | SEQ ID NO: 467 | CCCCUCUCCCUGCCAAUAUCC | SEQ ID NO: 1110 | AUAUUGGCAGGGAGAGGGGUA | SEQ ID NO: 1753 | CCCCUCUCCCUGCCAAUAU | 0.389 |
| AH0467 | SEQ ID NO: 468 | CCCUCUCCCUGCCAAUAUCCU | SEQ ID NO: 1111 | GAUAUUGGCAGGGAGAGGGGU | SEQ ID NO: 1754 | CCCUCUCCCUGCCAAUAUC | 0.208 |
| AH0468 | SEQ ID NO: 469 | GUGCGPAAUAUCCUGGACUGGC | SEQ ID NO: 1112 | CAGUCCAGGAUAUUGGCAGGG | SEQ ID NO: 1755 | CUGCCAAUAUCCUGGACUG | 0.333 |
| AH0469 | SEQ ID NO: 470 | CCAAUAUAUCCUGGACUGGCAGG | SEQ ID NO: 1113 | UGCCAGUCCAGAUAUUGGCA | SEQ ID NO: 1756 | CCAAUAUCCUGGACUGGCA | 0.120 |
| AH0470 | SEQ ID NO: 471 | CAAUAUCCUGGACUGGCAGGC | SEQ ID NO: 1114 | CUGCCAGUCCAGGAUAUUGGC | SEQ ID NO: 1757 | CAAUAUCCUGGACUGGCAG | 0.299 |
| AH0471 | SEQ ID NO: 472 | UCCUGGACUGGCAGGCUCUGA | SEQ ID NO: 1115 | AGAGCCUGCCAGUCCAGGAUA | SEQ ID NO: 1758 | UCCUGGACUGGCAGGCUCU | 0.386 |
| AH0472 | SEQ ID NO: 473 | CCUGGACUGGCAGGCUCUGAA | SEQ ID NO: 1116 | CAGAGCCUGCCAGUCCAGGAU | SEQ ID NO: 1759 | CCUGGACUGGCAGGCUGUG | 0.463 |
| AH0473 | SEQ ID NO: 474 | CUGGACUGGCAGGCUCUGAAC | SEQ ID NO: 1117 | UCAGAGCCUGCCAGUCCAGGA | SEQ ID NO: 1760 | CUGGACUGGCAGGCUCUGA | 0.454 |
| AH0474 | SEQ ID NO: 475 | UGGACUGGCAGGCUCUGAACU | SEQ ID NO: 1118 | UUCAGAGCCUGCCAGUCCAGG | SEQ ID NO: 1 761 | UGGACUGGCAGGCUCUGAA | 0.085 |
| AH0475 | SEQ ID NO: 476 | GGACUGGCAGGCUCUGAACUA | SEQ ID NO: 1119 | GUUCAGAGCCUGCCAGUCCAG | SEQ ID NO: 1762 | GGACUGGCAGGCUCUGAAC | 0.383 |
| AH0476 | SEQ ID NO: 477 | GACUGGCAGGCUCUGAACUAU | SEQ ID NO: 1120 | AGUUCAGAGCCUGCCAGUCCA | SEQ ID NO: 1763 | GACUGGCAGGCUCUGAACU | 0.308 |
| AH0477 | SEQ ID NO: 478 | ACUGGCAGGCUCUGAACUAUG | SEQ ID NO: 1121 | UAGUUGAGAGCCUGCCAGUCC | SEQ ID NO: 1764 | ACUGGCAGGCUCUGAACUA | 0.177 |
| AH0478 | SEQ ID NO: 479 | CUGGCAGGCUCUGAACUAUGA | SEQ ID NO: 1122 | AUAGVUUAGAGCCUGCCAGUC | SEQ ID NO: 1765 | CUGGCAGGCUCUGAACUAU | 0.096 |
| AH0479 | SEQ ID NO: 480 | UGGCAGGCUCUGAACUAUGAA | SEQ ID NO: 1123 | CAUAGUUCAGAGCCUGCCAGU | SEQ ID NO: 1766 | UGGCAGGCUCUGAACUAUG | 0.306 |
| AH0480 | SEQ ID NO: 481 | GGCAGGCUCUGAACUAUGAAA | SEQ ID NO: 1124 | UCAUAGUUCAGAGCCUGCCAG | SEQ ID NO: 1767 | GGCAGGCUCUGAACUAUGA | 0.077 |
| AH0481 | SEQ ID NO: 482 | GCAGGCUCUGAACUAUGAAAU | SEQ ID NO: 1125 | UUCAUAGUUCAGAGCCUGCCA | SEQ ID NO: 1768 | GCAGGCUCUGAACUAUGAA | 0.406 |
| AH0482 | SEQ ID NO: 483 | CAGGCUCUGAACUAUGAAAUC | SEQ ID NO: 1126 | UUUCAUAGUUCAGAGCCUGCC | SEQ ID NO: 1769 | CAGGCUCUGAACUAUGAAA | 0.133 |
| AH0483 | SEQ ID NO: 484 | AGGCUCUGAACUAUGAAAUCA | SEQ ID NO: 1127 | AUUUCAUAGUUCAGAGCCUGC | SEQ ID NO: 1770 | AGGCUCUGAACUAUGAAAU | 0.137 |
| AH0484 | SEQ ID NO: 485 | GGCUCUGAACUAUGAAAUCAG | SEQ ID NO: 1128 | GAUUUCAUAGUUCAGAGCCUG | SEQ ID NO: 1771 | GGCUCUGAACUAUGAAAUC | 0.085 |
| AH0485 | SEQ ID NO: 486 | GCUCUGAACUAUGAAAUCAGA | SEQ ID NO: 1129 | UGAUUUCAUAGUUCAGAGCCU | SEQ ID NO: 1772 | GCUCUGAACUAUGAAAUCA | 0.217 |
| AH0486 | SEQ ID NO: 487 | CUCUGAACUAUGAAAUCAGAG | SEQ ID NO: 1130 | CUGAUUUCAUAGUUCAGAGCC | SEQ ID NO: 1773 | CUCUGAACUAUGAAAUCAG | 0.197 |
| AH0487 | SEQ ID NO: 488 | UCUGAACUAUGAAAUCAGAGG | SEQ ID NO: 1131 | UCUGAUUUCAUAGUUCAGAGC | SEQ ID NO: 1774 | UCUGAAGUAUGAAAUCAGA | 0.296 |
| AH0488 | SEQ ID NO: 489 | GAACUAUGAAAUCAGAGGAUA | SEQ ID NO: 1132 | UCCUCUGAUUUCAUAGUUCAG | SEQ ID NO: 1775 | GAACUAUGAAAUCAGAGGA | 0.128 |
| AH0489 | SEQ ID NO: 490 | AACUAUGAAAUCAGAGGAUAU | SEQ ID NO: 1133 | AUCCUCUGAUUUCAUAGUUCA | SEQ ID NO: 1776 | AACUAUGAAAUCAGAGGAU | 0.230 |
| AH0490 | SEQ ID NO: 491 | ACUAUGAAAUCAGAGGAUAUG | SEQ ID NO: 1134 | UAUCCUCUGAUUUCAUAGUUC | SEQ ID NO: 1777 | ACUAUGAAAUCAGAGGAUA | 0.125 |
| AH0491 | SEQ ID NO: 492 | CUAUGAAAUCAGAGGAUAUGU | SEQ ID NO: 1135 | AUAUCCUCUGAUUUCAUAGUU | SEQ ID NO: 1778 | CUAUGAAAUCAGAGGAUAU | 0.104 |
| AH0492 | SEQ ID NO:493 | UAUGAAAUCAGAGGAUAUGUC | SEQ ID NO: 1136 | CAUAUCCUCUGAUUUCAUAGu | SEQ ID NO : 1779 | UAUGAAAUCAGAGGAUAUG | 0.330 |
| AH0493 | SEQ ID NO: 494 | UGAAAUCAGAGGAUAUGUCAU | SEQ ID NO: 1137 | GACAUAUCCUCUGAUUUCAUA | SEQ ID NO: 1780 | UGAAAUCAGAGGAUAUGUC | 0.338 |
| AH0494 | SEQ ID NO: 495 | GAAAUCAGAGGAUAUGUCAUC | SEQ ID NO: 1138 | UGACAUAUCCUCUGAUUUCAU | SEQ ID NO: 1781 | GAAAUCAGAGGAUAUGUCA | 0.043 |
| AH0495 | SEQ ID NO: 496 | AAAUCAGAGGAUAUGUCAUCA | SEQ ID NO: 1139 | AUGACAUAUCCUCUGAUUUCA | SEQ ID NO: 1782 | AAAUCAGAGGAUAUGUCAU | 0.084 |
| AH0496 | SEQ ID NO: 497 | AAUCAGAGGAUAUGUCAUCAU | SEQ ID NO: 1140 | GAUGACAUAUCCUCUGAUUUC | SEQ ID NO: 1783 | AAUCAGAGGAUAUGUCAUC | 0.489 |
| AH0497 | SEQ ID NO:498 | CAGAGGAUAUGUCAUCAUCAA | SEQ ID NO: 1141 | GAUGAUGACAUAUCCUCUGAU | SEQ ID NO: 1784 | CAGAGGAUAUGUCAUCAUC | 0.142 |
| AH0498 | SEQ ID NO: 499 | AGAGGAUAUGUCAUCAUCAAA | SEQ ID NO: 1142 | UGAUGAUGACAUAUCCUCUGA | SEQ ID NO: 1785 | AGAGGAUAUGUCAUCAUCA | 0.079 |
| AH0499 | SEQ ID NO: 500 | GAGGAUAUGUCAUCAUCAAAC | SEQ ID NO: 1143 | UUGAUGAUGACAUAUCCUCUG | SEQ ID NO: 1786 | GAGGAUAUGUCAUCAUCAA | 0.079 |
| AH0500 | SEQ ID NO: 501 | AGGAUAUGUCAUCAUCAAACC | SEQ ID NO: 1144 | UUUGAUGAUGACAUAUGCUCU | SEQ ID NO: 1787 | AGGAUAUGUCAUCAUCAAA | 0.091 |

### [Table 38]

**Table 1-11**

| Double-stranded nucleic acid No. | SEQ ID NO: | Sense strand sequence (5'--->3') | SEQ ID NO: | Antisense strand sequence (5'--->3') | SEQ ID NO: | Target APCS mRNA sequence | Relative APCS expression level |
|---|---|---|---|---|---|---|---|
| AH0501 | SEQ ID NO: 502 | GGAUAUGUCAUCAUCAAACCC | SEQ ID NO: 1145 | GUUUGAUGAUGACAUAUCCUC | SEQ ID NO: 1788 | GGAUAUGUCAUCAUCAAAC | 0.062 |
| AH0502 | SEQ ID NO: 503 | GAUAUGUCAUCAUCAAACCCU | SEQ ID NO:1146 | GGUUUGAUGAUGACAUAUCCU | SEQ ID NO: 1789 | GAUAUGUCAUCAUCAAACC | 0.343 |
| AH0503 | SEQ ID NO: 504 | AUGUCAUCAUCAAACCCUUGG | SEQ ID NO: 1147 | AAGGGUUUGAUGAUGACAUAU | SEQ ID NO: 1790 | AUGUCAUCAUCAAACCCUU | 0.173 |
| AH0504 | SEQ ID NO: 505 | CAUCAUCAAACCCUUGGUGUG | SEQ ID NO: 1148 | CACCAAGGGUUUGAUGAUGAC | SEQ ID NO:1791 | CAUCAUCAAACCCUUGGUG | 0442 |
| AH0505 | SEQ ID NO: 506 | UCAUCAAACCCUUGGUGUGGG | SEQ ID NO: 1149 | CACACCAAGGGUUUGAUGAUG | SEQ ID NO: 1792 | UCAUCAAACCCUUGGUGUG | 0.355 |
| AH0506 | SEQ ID NO: 507 | ACCCUUGGUGUGGGUCUGAGG | SEQ ID NO: 1150 | UCAGACCCACACCAAGGGUUU | SEQ ID NO: 1793 | ACCCUUGGUGUGGGUCUGA | 0.393 |
| AH0507 | SEQ ID NO: 508 | CCCUUGGUGUGGGUCUGAGGU | SEQ ID NO: 1151 | CUCAGACCCACACCAAGGGUU | SEQ ID NO: 1794 | CCCUUGGUGUGGGUCUGAG | 0.273 |
| AH0508 | SEQ ID NO: 509 | CCUUGGUGUGGGUCUGAGGUC | SEQ ID NO: 1152 | CCUCAGACCCACACCAAGGGU | SEQ ID NO: 1795 | CCUUGGUGUGGGUCUGAGG | 0.137 |
| AH0509 | SEQ ID NO: 510 | CUUGGUGUGGGUCUGAGGUCU | SEQ ID NO: 1153 | ACCUCAGACCCACACCAAGGG | SEQ ID NO: 1796 | CUUGGUGUGGGUCUGAGGU | 0.220 |
| AH0510 | SEQ ID NO. 511 | UGGUGUGGGUCUGAGGUCUUG | SEQ ID NO: 1154 | AGACCUCAGACCCACACCAAG | SEQ ID NO: 1797 | UGGUGUGGGUCUGAGGUCU | 0.086 |
| AH0511 | SEQ ID NO: 512 | GGUGUGGGUCUGAGGUCUUGA | SEQ ID NO: 1155 | AAGACCUCAGACCCACACCAA | SEQ ID NO: 1 | GGUGUGGGUCUGAGGUCUU | 0.125 |
| AH0512 | SEQ ID NO:513 | GUGUGGGUCUGAGGUCUUGAC | SEQ ID NO 1156 | CAAGACCUCAGACCCACACCA | SEQ ID NO 1799 | GUGUGGGUCUGAGGUCUUG | 0.066 |
| AH0513 | SEQ ID NO: 514 | UGUGGGUCUGAGGUCUUGACU | SEQ ID NO: 1157 | UCAAGACCUCAGACCCACACC | SEQ ID No: 1800 | UGUGGGUCUGAGGUCUUGA | 0.104 |
| AH0514 | SEQ ID NO: 515 | GUGGGUCUGAGGU CUUGACUC | SEQ ID NO : 1158 | GUCAAGACCUCAGACCCACAC | SEQ ID NO: 1 | GUGGGUGUGAGGUCUUGAC | 0.098 |
| AH0515 | SEQ ID NO: 516 | GGGUCUGAGGCUUGACUCAA | SEQ ID NO: 1159 | GAGUCAAGACCUCAGACCCAC | SEQ ID NO: 1802 | GGGUCUGAGGUCUUGACUC | 0.136 |
| AH0516 | SEQ ID NO: 517 | GGUCUGAGGUCUUGACUCAAC | SEQ ID NO: 1160 | UGAGUCAAGACCUCAGACCCA | SEQ ID NO: 1803 | GGUCUGAGGUCUUGACUCA | 0.072 |
| AH0517 | SEQ ID NO: 518 | GUCUGAGGUCUUGACUCAACG | SEQ ID NO 1161 | UUGAGUCAAGACCUCAGACCC | SEQ ID NO: 1804 | GUCUGAGGUCUUGACUCAA | 0.146 |
| AH0518 | SEQ ID NO: 519 | CUGAGGUCUUGACUCAACGAG | SEQ ID NO: 1162 | CGUUGAGUCAAGACCUCAGAC | SEQ ID NO: 1805 | CUGAGGUCUUGACUCAACG | 0.241 |
| AH0519 | SEQ ID NO: 520 | UGAGGUCUUGACUCAACGAGA | SEQ ID NO: 1163 | UCGUUGAGUCAAGACCUCAGA | SEQ ID NO: 1806 | UGAGGUCUUGACUCAACGA | 0.087 |
| AH0520 | SEQ ID NO 521 | GAGGUCUUGACUCAACGAGAG | SEQ ID NO: 1164 | CUCGUUGAGUCAAGACCUCAG | SEQ ID NO: 1807 | GAGGUCUUGACUCAACGAG | 0.096 |
| AH0521 | SEQ ID NO: 522 | AGGUCUUGACUCAACGAGAGC | SEQ ID NO : 1165 | UCUCGUUGAGUCAAGACCUCA | SEQ ID NO: 1808 | AGGUCUUGACUCAACGAGA | 0.069 |
| AH0522 | SEQ ID NO: 523 | GGUCUUGACUCAACGAGAGCA | SEQ ID NO: 1166 | CUCUCGUUGAGUCAAGACCUC | SEQ ID NO: 1809 | GGUCUUGACUCAACGAGAG | 0.077 |
| AH0523 | SEQ ID NO: 524 | GUCUUGACUCAACGAGAGCAC | SEQ ID NO: 1167 | GCUCUCGUUGAGUCAAGACCU | SEQ ID NO: 1810 | GUCUUGACUCAACGAGAGC | 0.218 |
| AH0524 | SEQ ID NO 525 | UCUUGACUCAACG AGAGCACU | SEQ ID NO: 1168 | UGCUCUCGUUGAGUCAAGACC | SEQ ID NO: 1811 | UCUUGACUCAACGAGAGCA | 0.125 |
| AH0525 | SEQ ID NO: 526 | CUUGACUCAACGAGAGCACUU | SEQ ID NO: 1169 | GUGCUCUCGUUGAGUCAAGAC | SEQ ID NO: 1812 | GUUGACUCAACGAGAGCAC | 0.195 |
| AH0526 | SEQ ID NO: 527 | UUGACUCAACGAGAGCACUUG | SEQ ID NO: 1170 | AGUGCUCUCGUUGAGUCAAGA | SEQ ID NO : 1813 | UUGACUCAACGAGAGGACU | 0.101 |
| AH0527 | SEQ ID NO: 528 | UGACUCAACGAGAGCACUUGA | SEQ ID NO: 1171 | AAGUGCUGUCGUUGAGUCAAG | SEQ ID NO: 1814 | UGACUCAACGAGAGCACUU | 0.189 |
| AH0528 | SEQ ID NO: 529 | GACUCAACGAGAGCACUUGAA | SEQ ID NO: 1172 | CAAGUGCUCUCGUUGAGUCAA | SEQ ID NO: 1815 | GACUCAACGAGAGCACUUG | 0.090 |
| AH0529 | SEQ ID NO: 530 | ACUCAACGAGAGCACUUGAAA | SEQ ID NO: 1173 | UCAAGUGCUCUCGUUGAGUCA | SEQ ID NO: 1816 | ACUCAACGAGAGCACUUGA | 0.104 |
| AM0530 | SEQ ID NO: 531 | CUCAACGAGAGCACUUGAAAA | SEQ ID NO: 1174 | UUCAAGUGCUCUCGUUGAGUC | SEQ ID NO: 1817 | CUCAACGAGAGCACUUGAA | 0.107 |
| AH0531 | SEQ ID NO: 532 | UCAACGAGAGCACUUGAAAAU | SEQ ID NO : 1175 | UUUCAAGUGCUCUCGUUGAGU | SEQ ID NO: 1818 | UCAACGAGAGCACUUGAAA | 0.063 |
| AH0532 | SEQ ID NO: 533 | CAACGAGAGCACUUGAAAAUG | SEQ ID NO: 1176 | UUUUCAAGUGCUCUCGUUGAG | SEQ ID NO: 1819 | CAACGAGAGCACUUGAAAA | 0.066 |
| AH0533 | SEQ ID NO: 534 | AACGAGAGCACUUGAAAAUGA | SEQ ID NO: 1177 | AUUUUCAAGUGCUCUCGUUGA | SEQ ID NO: 1820 | AACGAGAGCACUUGAAAAU | 0.054 |
| AH0534 | SEQ ID NO: 535 | ACGAGAGCACUUGAAAAUGAA | SEQ ID NO: 1178 | CAUUUUCAAGUGCUCUCGUUG | SEQ ID NO: 1821 | ACGAGAGCACUUGAAAAUG | 0.073 |
| AH0535 | SEQ ID NO 536 | CGAGAGCACUUGAAAAUGAAA | SEQ ID NO: 1179 | UCAUUUUCAAGUGCUCUCGUU | SEQ ID NO 1822 | CGAGAGCACUUGAAAAUGA | 0.026 |
| AH0536 | SEQ ID NO: 537 | GAGAGCACUUGAAAAUGAAAU | SEQ ID NO: 1180 | UUCAUUUUCAAGUGCUCUCGU | SEQ ID NO: 1823 | GAGAGCACUUGAAAAUGAA | 0.031 |
| AH0537 | SEQ ID NO : 538 | AGAGCACUUGAAAAUGAAAUG | SEQ ID NO : 1181 | UUUCAUUUUCAAGUGCUCG | SEQ ID NO: 1824 | AGAGCACUUGAAAAGAAA | 0.059 |
| AH0538 | SEQ ID NO: 539 | GAGCACUUGAAAAUGAAAUGA | SEQ ID NO: 1182 | AUUUCAUUUUCAAGUGCUCUC | SEQ ID NO: 1825 | GAGCACUUGAAAAUGAAAU | 0.054 |
| AH0539 | SEQ ID NO : 540 | AGCACUUGAAAAUGAAAUGAC | SEQ ID NO: 1183 | CAUUUCAUUUUCAAGUGCUCU | SEQ ID NO: 1826 | AGCACUUGAAAAUGAAAUG | 0.032 |
| AH0540 | SEQ ID NO: 541 | GCACUUGAAAAUGAAAUGACU | SEQ ID NO: 1184 | UCAUUUCAUUUUCAAGUGCUC | SEQ ID NO: 1827 | GCACUUGAAAAUGAAAUGA | 0.074 |
| AH0541 | SEQ ID NO: 542 | CACUUGAAAAUGAAUGACUG | SEQ ID NO: 1185 | GUCAUUUCAUUUUCAAGUGCU | SEQ ID NO: 1828 | CACUUGAAAAUGAAAUGAC | 0.204 |
| AH0542 | SEQ ID NO : 543 | ACUUGAAAAUGAAAUGACUGU | SEQ ID NO: 1186 | AGUCAUUUCAUUUUCAAGUGC | SEQ ID NO 1829 | ACUUGAAAAUGAAAUGACU | 0.103 |
| AH0543 | SEQ ID NO: 544 | CUUGAAAAUGAAAUGACUGUC | SEQ ID NO: 1187 | CAGUCAUUUCAUUUCAAGUG | SEQ ID NO 1830 | CUUGAAAAUGAAAUGACUG | 0.121 |
| AH0544 | SEQ ID NO: 545 | UUGAAAAUGAAAUGACUGUCU | SEQ ID NO: 1188 | ACAGUCAUUUCAUUUUCAAGU | SEQ ID NO: 1831 | UUGAAAAUGAAAU GACUGU | 0.201 |
| AH0545 | SEQ ID NO: 546 | UGAAAAUGAAAUGACUGUCUA | SEQ ID NO: 1189 | GACAGUCAUUUCAUUUUCAAG | SEQ ID NO 1832 | UGAAAAUGAAAUGACUGUC | 0.445 |
| AH0546 | SEQ ID NO: 547 | GAAAAUGAAAUGACUGUCUAA | SEQ ID NO: 1190 | AGACAGUCAUUUCAUUUUCAA | SEQ ID NO: 1833 | GAAAAUGAAAUGACUGUCU | 0.068 |
| AH0547 | SEQ ID NO: 548 | AAAAUGAAAUGACUGUCUAAG | SEQ ID NO: 1191 | UAGACAGUCAUUUCAUUUUCA | SEQ ID NO: 1834 | AAAAUGAAAUGACUGUCUA | 0.053 |
| AH0548 | SEQ ID NO: 549 | AAAUGAAAUGACUGUCUAAGA | SEQ ID NO: 1192 | UUAGACAGUCAUUUCAUUUC | SEQ ID NO: 1835 | AAAUGAAAUGACUGUCUAA | 0.073 |
| AH0549 | SEQ ID NO: 550 | AAUGAAAUGACUGUCUAAGAG | SEQ ID NO: 1193 | CUUAGACAGUCAUUUCAUUUU | SEQ ID NO: 1836 | AAUGAAAUGACUGUCUAAG | 0.365 |
| AH0550 | SEQ ID NO: 551 | AUGAAAUGACUGUCUAAGAGA | SEQ ID NO: 1194 | UCUUAGACAGUCAUUUCAUUU | SEQ ID NO: 1837 | AUGAAAUGACUGUCUAAGA | 0.395 |

### [Table 39]

**Table 1-12**

| Double-stranded nucleic acid No. | SEQ ID NO: | Sense strand sequence (5'->3') | SEQ ID NO: | Antisense strand sequence (5'--->3') | SEQ ID NO: | Target APCS mRNA sequence | Relative APCS expression level |
|---|---|---|---|---|---|---|---|
| AH0551 | SEQ ID NO: 552 | UGAAAUGACUGUCUAAGAGAU | SEQ ID NO: 1195 | CUCUUAGACAGUCAUUUCAUU | SEQ ID NO:1838 | UGAAAUGACUGUCUAAGAG | 0.471 |
| AH0552 | SEQ ID NO: 553 | GAAAUGACUGUCUAAGAGAUC | SEQ ID NO: 1196 | UCUCUUAGACAGUCAUUUCAU | SEQ ID NO: 1839 | GAAAUGACUGUCUAAGAGA | 0.077 |
| AH0553 | SEQ ID NO: 554 | AAAUGACUGVCUAAGAGAUCU | SEQ ID NO: 1197 | AUCUCUUAGACAGUCAUUUCA | SEQ ID NO: 1840 | AAAUGACUGUCUAAGAGAU | 0.161 |
| AH0554 | SEQ ID NO: 555 | AUGACUGUCUAAGAGAUCUGG | SEQ ID NO: 1198 | AGAUCUCUUAGACAGUCAUUU | SEQ ID NO: 1841 | AUGACUGUCUAAGAGAUCU | 0.244 |
| AH0555 | SEQ ID NO: 556 | UGACUGUCUAAGAGAUCUGGU | SEQ ID NO: 1199 | CAGAUCUCUUAGACAGUCAUU | SEQ ID NO: 1842 | UGACUGUCUAAGAGAUCUG | 0.390 |
| AH0556 | SEQ ID NO: 557 | GACUGUCUAAGAGAUCUGGUC | SEQ ID NO: 1200 | CCAGAUCUCUUAGACAGUCAU | SEQ ID NO: 1843 | GACUGUCUAAGAGAUCUGG | 0.143 |
| AH0557 | SEQ ID NO: 558 | ACUGUCUAAGAGAUCUGGUCA | SEQ ID NO: 1201 | ACCAGAUCUCUUAGACAGUCA | SEQ ID NO: 1844 | ACUGUCUAAGAGAUCUGGU | 0.202 |
| AH0558 | SEQ ID NO: 559 | CUGUCUAAGAGAUCUGGUCAA | SEQ ID NO: 1202 | GACCAGAUCUCUUAGACAGUC | SEQ ID NO: 1845 | CUGUCUAAGAGAUCUGGUC | 0.119 |
| AH0559 | SEQ ID NO: 560 | UGUCUAAGAGAUCUGGUCAAA | SEQ ID NO: 1203 | UGACCAGAUCUCUUAGACAGU | SEQ ID NO: 1846 | UGUCUAAGAGAUCUGGUGA | 0.121 |
| AH0560 | SEQ ID NO: 561 | GUCUAAGAGAUCUCGIICAAAG | SEQ ID NO: 1204 | UUGACCAGAUCUCUUAGACAG | SEQ ID NO: 1847 | GUCUAAGAGAUCUGGUCAA | 0.053 |
| AH0561 | SEQ ID NO: 562 | UCUAAGAGAUCUGGUCAAAGC | SEQ ID NO: 1205 | UUUGACCAGAUCUCUUAGACA | SEQ ID NO: 1848 | UCUAAGAGAUCUGGUCAAAA | 0.081 |
| AH0562 | SEQ ID NO: 563 | CUAAGAGAUCUGGUCAAAGCA | SEQ ID NO: 1206 | CUUUGACCAGAUCUCUUAGAC | SEQ ID NO: 1849 | CUAAGAGAUCUGGUCAAAG | 0.051 |
| AH0563 | SEQ ID NO: 564 | UAAGAUCUGGUCAAAGCAA | SEQ ID NO: 1207 | GCUUUGACCAGAUCUCUUAGA | SEQ ID NO: 1850 | UAAGAGAUCUGGUCAAAGC | 0.363 |
| AH0564 | SEQ ID NO: 565 | AAGAGAUCUGGUCAAAGCAAC | SEQ ID NO: 1208 | UGCUUUGACCAGAUCUCUUAG | SEQ ID NO: 1851 | AAGAGAUCUGGUCAAAGCA | 0.053 |
| AH0565 | SEQ ID NO: 566 | AGAGAUCUGGUCAAAGCAACU | SEQ ID NO: 1209 | UUGCUUUGACCAGAUCUCUUA | SEQ ID NO: 1852 | AGAGAUCUGGUCAAAGCAA | 0.091 |
| AH0566 | SEQ ID NO: 567 | GAGAUCUGGUCAAAGCAACUG | SEQ ID NO: 1210 | GUUGCUUUGACCAGAUCUCUU | SEQ ID NO: 1853 | GAGAUCUGGUCAAAGCAAC | 0.074 |
| AH0567 | SEQ ID NO: 568 | AGAUCUGGUCAAAGCAACUGG | SEQ ID NO: 1211 | AGUUGCUUUGACCAGAUCUCU | SEQ ID NO: 1854 | AGAUCUGGUCAAAGCAACU | 0.096 |
| AH0568 | SEQ ID NO: 569 | GAUCUGGUCAAAGCAACUGGA | SEQ ID NO: 1212 | CAGUUGCUUUGACCAGAUCUC | SEQ ID NO: 1855 | GAUCUGGUCAAAGCAACUG | 0.118 |
| AH0569 | SEQ ID NO: 570 | AUCUGGUCAAAGCAACUGGAU | SEQ ID NO: 1213 | CCAGUUGCUUUGACCAGAUCU | SEQ ID NO: 1856 | AUCUGGUCAAAGCAACUGG | 0.225 |
| AH0570 | SEQ ID NO: 571 | UCUGGUCAAAGCAACUGGAUA | SEQ ID NO: 1214 | UCCAGUUGCUUUGACCAGAUC | SEQ ID NO: 1857 | UCUGGUCAAAGCAACUGGA | 0.105 |
| AH0571 | SEQ ID NO: 572 | CUGGUCAAAGCAACUGGAUAC | SEQ ID NO: 1215 | AUCCAGUUGCUUUGACCAGAU | SEQ ID NO: 1858 | CUGGUCAAAGCAACUGGAU | 0.160 |
| AH0572 | SEQ ID NO: 573 | UGGUCAAAGCAACUGGAUACU | SEQ ID NO: 1216 | UAUCCAGUUGCUUUGACCAGA | SEQ ID NO: 1859 | UGGUCAAAGCAACUGGAUA | 0.093 |
| AH0573 | SEQ ID NO: 574 | GGUCAAAGCAACUGGAUACUA | SEQ ID NO: 1217 | GUAUCCAGUUGCUUUGCCAG | SEQ ID NO: 1860 | GGUCAAAGCAACUGGAUAC | 0.118 |
| AH0574 | SEQ ID NO: 575 | GUCAAAGCAACUGGAUACUAG | SEQ ID NO: 1218 | AGUAUCCAGUUGCUUUGACCA | SEQ ID NO: 1861 | GUCAAAGCAACUGGAUACU | 0.131 |
| AH0575 | SEQ ID NO: 576 | UGAAAGCAACUGGAUACUAGA | SEQ ID NO: 1219 | UAGUAUCCAGUUGCUUUGACC | SEQ ID NO: 1862 | UCAAAGCAACUGGAUACUA | 0.094 |
| AH0576 | SEQ ID NO: 577 | CAAAGCAACUGGAUACUAGAU | SEQ ID NO: 1220 | CUAGUAUCCAGUUGCUUUGAC | SEQ ID NO: 1863 | CAAAGCAACUGGAUACUAG | 0.122 |
| AH0577 | SEQ ID NO: 578 | AAAGCAACUGGAUACUAGAUC | SEQ ID NO: 1221 | UCUAGUAUCCAGUUGCUUUGA | SEQ ID NO: 1864 | AAAGCAACUGGAUACUAGA | 0.089 |
| AH0578 | SEQ ID NO: 579 | AAGQAACUGGAUACUAGAUCU | SEQ ID NO: 1222 | AUCUAGUAUCCAGUUGCUUUG | SEQ ID NO: 1865 | AAGCAACUGGAUACUAGAU | 0.156 |
| AH0579 | SEQ ID NO: 580 | AGCAACUGGAUACUAGAUCUU | SEQ ID NO: 1223 | GAUCUAGUAUCCAGUUGCUUU | SEQ ID NO: 1866 | AGCAACUGGAUACUAGAUC | 0.248 |
| AH0580 | SEQ ID NO: 581 | GCAACUGGAUACUAGAUCUUA | SEQ ID NO: 1224 | AGAUCUAGUAUCCAGUUGCUU | SEQ ID NO: 1867 | GCAACUGGAUACUAGAUCU | 0.089 |
| AH0581 | SEQ ID NO: 582 | CAACUGGAUACUAGAUCUUAC | SEQ ID NO: 1225 | AAGAUCUAGUAUCCAGUUGCU | SEQ ID NO: 1868 | CAACUGGAUACUAGAUCUU | 0.067 |
| AH0582 | SEQ ID NO: 583 | AACUGGAUACUAGAUGUUAGA | SEQ ID NO: 1226 | UAAGAUCUAGUAUCCAGUUGC | SEQ ID NO: 1869 | AACUGGAUACUAGAUCUUA | 0.101 |
| AH0583 | SEQ ID NO: 584 | ACUGGAUACUAGAUCUUACAU | SEQ ID NO: 1227 | GUAAGAUCUAGUAUCCAGUUG | SEQ ID NO: 1870 | ACUGGAUACUAGAUCUUAC | 0.096 |
| AH0584 | SEQ ID NO: 585 | CUGGAUACUAGAUCUUUC | SEQ ID NO: 1228 | UGUAAGAUCUAGUAUCCAGUU | SEQ ID NO: 1871 | CUGGAUACUAGAUCUUACA | 0.075 |
| AH0585 | SEQ ID NO: 586 | UGGAUACUAGAUCUUACAUCU | SEQ ID NO: 1229 | AUGUAAGAUCUAGUAUCCAGU | SEQ ID NO: 1872 | UGGAUACUAGAUCUUACAU | 0.115 |
| AH0586 | SEQ ID NO: 587 | GGAUACUAGAUCUUACAUCUG | SEQ ID NO: 1230 | GAUGUAAGAUCUAGUAUCCAG | SEQ ID NO: 1873 | GGAUACUAGAUCUUACAUC | 0.087 |
| AH0587 | SEQ ID NO: 588 | GAUACUAGAUCUUACAUCUGC | SEQ ID NO: 1231 | AGAUGUAAGAUCUAGUAUCCA | SEQ ID NO: 1874 | GAUACUAGAUCUUACAU CU | 0.069 |
| AH0588 | SEQ ID NO: 589 | AUACUAGAUCUUACAUCUGCA | SEQ ID NO: 1232 | CAGAUGUAAGAUCUAGUAUCC | SEQ ID NO: 1875 | AUACUAGAUCUUACAUGUG | 0.105 |
| AH0589 | SEQ ID NO: 590 | ACUAGAUCUUACAUCUGCAGC | SEQ ID NO: 1233 | UGCAGAUGUAAGAUCUAGUAU | SEQ ID NO: 1876 | ACUAGAUCUUACAUCUGCA | 0.069 |
| AH0590 | SEQ ID NO: 591 | CUAGAUCUUACAUCUGCAGCU | SEQ ID NO: 1234 | CUGCAGAUGUAAGAUCUAGUA | SEQ ID NO: 1877 | CUAGAUCUUACAUCUGCAG | 0.091 |
| AH0591 | SEQ ID NO: 592 | AGAUCUUACAUCUGCAGCUCU | SEQ ID NO: 1235 | AGCUGCAGAUGUAAGAUCUAG | SEQ ID NO: 1878 | AGAUCUUACAUCUGCAGCU | 0.112 |
| AH0592 | SEQ ID NO: 593 | GAUCUUACAUCUGCAGCUCUU | SEQ ID NO 1236 | GAGCUGCAGAUGUAAGAUCUA | SEQ ID NO: 1879 | GAUCUUACAUCUGCAGCUC | 0.096 |
| 4H0593 | SEQ ID NO: 594 | AUCUUACAUCUGCAGCUCUUU | SEQ ID NO: 1237 | AGAGCUGCAGAUGUAAGAUCU | SEQ ID NO: 1880 | AUCUUACAUCUGCAGCUCU | 0.089 |
| AH0594 | SEQ ID NO: 595 | UCUUACAUCUGCAGCUCUUUC | SEQ ID NO 1238 | AAGAGCUGCAGAUGUAAGAUC | SEQ ID NO: 1881 | UCUUACAUCUGCAGCUCUU | 0.083 |
| AH0595 | SEQ ID NO: 596 | CUUACAUCUGCAGCAUCUUUCU | SEQ ID NO: 1239 | AAAGAGCUGCAGAUGUAAGAU | SEQ ID NO: 1882 | CUUACAUCUGCAGCUCUUU | 0.058 |
| AH0596 | SEQ ID NO: 597 | UUACAUCUGCAGCUCUUUCUU | SEQ ID NO: 1240 | GAAAGAGCUGCAGAUGUAAGA | SEQ ID NO: 1883 | UUACAUCUGCAGCUCUUUC | 0.110 |
| AH0597 | SEQ ID NO: 598 | UACAUCUGCAGCUCUUUCUUC | SEQ ID NO: 1241 | AGAAAGAGCUGCAGAUGUAAG | SEQ ID NO: 1884 | UACAUCUGCAGCUCUUUCU | 0.168 |
| AH0598 | SEQ ID NO: 599 | AUCUGCAGCUCUUUCUUCU | SEQ ID NO: 1242 | AAGAAAGAGCUGCAGAUGUAA | SEQ ID NO: 1885 | ACAUCUGCAGCUCUUUCUU | 0.083 |
| AH0599 | SEQ ID NO: 600 | CAUCUGCAGCUCUUUCUUCUU | SEQ ID NO: 1243 | GAAGAAAGAGCUGCAGAUGUA | SEQ ID NO: 1886 | CAUCUGCAGCUCUUUCUUC | 0.072 |
| AH0600 | SEQ ID NO: 601 | AUCUGCAGCUCUUUCUUCUUU | SEQ ID NO: 1244 | AGAAGAAAGAGCUGCAGAUGU | SEQ ID NO: 1887 | AUCUGCAGCUCUUUCUUCU | 0.072 |

### [Table 40]

**Table 1-13**

| Double-stranded nucleic acid No. | SEQ ID NO: | Sense strand sequence (5' --->3') | SEQ ID NO: | Antisense strand sequence (5'--->3') | SEQ ID NO: | Target APCS mRNA sequence | Relative APCS expression level |
|---|---|---|---|---|---|---|---|
| AH0601 | SEQ ID NO: 602 | UCUGCAGCUCUUUCUUCUUUG | SEQ ID NO: 1245 | AAGAAGAAAGAGCUGCAGAUG | SEQ ID NO: 1888 | UCUGCAGCUCUUUCUUCUU | 0.086 |
| AH0602 | SEQ ID NO: 603 | CUGCAGCUCUUUCUUCUUUGA | SEQ ID NO: 1246 | AAAGAAGAAAGAGCUGCAGAU | SEQ ID NO: 1889 | CUGCAGCUCUUUCUUCUUU | 0.097 |
| AH0603 | SEQ ID NO: 604 | UGCAGCUCUUUCUUCUUUGAA | SEQ ID NO: 1247 | CAAAGAAGAAAGAGCUGCAGA | SEQ ID NO: 1890 | UGCAGCUCUUUCUUCUUUG | 0.096 |
| AH0604 | SEQ ID NO: 605 | GCAGCUCUUUCUUCUUUGAAU | SEQ ID NO: 1248 | UCAAAGAAGAAAGAGCUGCAG | SEQ ID NO: 1891 | GCAGCUCUUUCUUCUUUGA | 0.059 |
| AH0605 | SEQ ID NO: 606 | CAGCUCUUUCUUCUUUGAAUU | SEQ ID NO: 1249 | UUCAAAGAAGAAAGAGCUGCA | SEQ ID NO: 1892 | GCAGCUCUUUCUUCUUUGA | 0.054 |
| AH0606 | SEQ ID NO: 607 | AGCUCUUUCUUCUUUGAAUUU | SEQ ID NO: 1250 | AUUCAAAGAAGAAAGAGCUGC | SEQ ID NO: 1893 | AGCUCUUUCUUCUUUGAAU | 0.053 |
| AH0607 | SEQ ID NO: 608 | GCUCUUUCUUCUUUGAAUUUC | SEQ ID NO: 1251 | AAUUCAAAGAAGAAAGAGCUG | SEQ ID NO: 1894 | GCUCUUUCUUCUUUGAAUU | 0.057 |
| AH0608 | SEQ ID NO: 609 | CUCUUUCUUCUUUGAAUUUCC | SEQ ID NO: 1252 | AAAUUCAAAGAAGAAAGAGCU | SEQ ID NO: 1895 | CUCUUUCUUCUUUGAAUUU | 0.068 |
| AH0609 | SEQ ID NO: 610 | UCUUUCUUCUUUGAAUUUCCU | SEQ ID NO: 1253 | GAAAUUCAAAGAAGAAAGAGC | SEQ ID NO: 1896 | UCUUUCUUCUUUGAAUUUC | 0.184 |
| AH0610 | SEQ ID NO: 611 | CUUUCUUCUUUGAAUUUCCUA | SEQ ID NO: 1254 | GGAAAUUCAAAGAAGAAAGAG | SEQ ID NO: 1897 | CUUUCUUCUUUGAAUUUCC | 0.079 |
| AH0611 | SEQ ID NO: 612 | UUUCUUCUUUGAAUUUCCUAU | SEQ ID NO: 1255 | AGGAAAUUCAAAGAAGAAAGA | SEQ ID NO: 1898 | UUUCUUCUUUGAAUUUCCU | 0.124 |
| AH0612 | SEQ ID NO: 613 | UUCUUCUUUGAAUUUCCUAUC | SEQ ID NO: 1256 | UAGGAAAUUCAAAGAAGAAAG | SEQ ID NO: 1899 | UUCUUCUUUGAAUUUCCUA | 0.212 |
| AH0613 | SEQ ID NO: 614 | UCUUCUUUGAAUUUCCUAUCU | SEQ ID NO: 1257 | AUAGGAAAUUCAAAGAAGAAA | SEQ ID NO: 1900 | UCUUCUUUGAAUUUCCUAU | 0.259 |
| AH0614 | SEQ ID NO: 615 | UUCUUUGAAUUUCCUAUCUGU | SEQ ID NO: 1258 | AGAUAGGAAAUUCAAAGAAGA | SEQ ID NO: 1901 | UUCUUUGAAUUUCCUAUCU | 0.171 |
| AH0615 | SEQ ID NO: 616 | UCUUUGAAUUUCCUAUCUGUA | SEQ ID NO: 1259 | CAGAUAGGAAAUU CAAAGAAG | SEQ ID NO: 1902 | UCUUUGAAUUUCCUAUCUG | 0.194 |
| AH0616 | SEQ ID NO: 617 | CUUUGAAUUUCCUAUCUGUAU | SEQ ID NO: 1260 | ACAGAUAGGAAAUUCAAAGAA | SEQ ID NO: 1903 | CUUUGAAUUUCCUAUCUGU | 0.073 |
| AH0617 | SEQ ID NO: 618 | UUUGAAUUUCCUAUCUGUAUG | SEQ ID NO: 1261 | UACAGAUAGGAAAUUCAAAGA | SEQ ID NO: 1904 | UUUGAAUUUCCUAUCUGUA | 0.072 |
| AH0618 | SEQ ID NO: 619 | UUGAAUUUCCUAUCUGUAUGU | SEQ ID NO : 1262 | AUACAGAUAGGAAAUUCAAAG | SEQ ID NO: 1905 | UUGAAUUUCCUAUCUGUAU | 0.104 |
| AH0619 | SEQ ID NO: 620 | UGAAUUUCCUAUCUGUAUGUC | SEQ ID NO: 1263 | CAUACAGAUAGGAAAUUCAAA | SEQ ID NO: 1906 | UGAAUUUCCUAUCUGUAUG | 0.151 |
| AH0620 | SEQ ID NO: 621 | GAAUUUCCUAUCUGUAUGUCU | SEQ ID NO: 1264 | ACAUACAGAUAGGAAAUUCAA | SEQ ID NO: 1907 | GAAUUUCCUAUCUGUAUGU | 0.066 |
| AH0621 | SEQ ID NO: 622 | AUUUCCUAUCUGUAUGUCUGC | SEQ ID NO: 1265 | AGACAUACAGAUAGGAAAUUC | SEQ ID NO: 1908 | AUUUCCUAUCUGUAUGUCU | 0.280 |
| AH0622 | SEQ ID NO: 623 | UUUCCUAUCUGUAUGUCUGCC | SEQ ID NO: 1266 | CAGACAUACAGAUAGGAAAUU | SEQ ID NO: 1909 | UUUCCUAUCUGUAUGUCUG | 0.222 |
| AH0623 | SEQ ID NO: 624 | UUCCUAUCUGUAUGUCUGCCU | SEQ ID NO: 1267 | GCAGACAUACAGAUAGGAAAU | SEQ ID NO: 1910 | UUCCUAUCUGUAUGUCUGC | 0474 |
| AH0624 | SEQ ID NO: 625 | UCCUAUCUGUAUGUCUGCCUA | SEQ ID NO: 1268 | GGCAGACAUACAGAUAGGAAA | SEQ ID NO: 1911 | UCCUAUCUGUAUGUCUGCC | 0.494 |
| AH0625 | SEQ ID NO: 626 | CCUAUCUGUAUGUCUGCCUAA | SEQ ID NO: 1269 | AGGCAGACAUACAGAUAGGAA | SEQ ID NO: 1912 | CCUAUCUGUAUGUCUGCCU | 0.088 |
| AH0626 | SEQ ID NO: 627 | CUAUCUGUAUGUCUGCCUAAU | SEQ ID NO: 1270 | UAGGCAGACAUACAGAUAGGA | SEQ ID NO: 1913 | CUAUCUGUAUGUCUGCCUA | 0073 |
| AH0627 | SEQ ID NO: 628 | UAUCUGUAUGUCUGCCUAAUU | SEQ ID NO: 127 1 | UUAGGCAGACAUACAGAUAGG | SEQ ID NO: 1914 | UAUCUGUAUGUCUGCCUAA | 0.114 |
| AH0628 | SEQ ID NO: 629 | AUCUGUAUGUCUGCCUAAUUA | SEQ ID NO: 1272 | AUUAGGCAGACAUACAGAUAG | SEQ ID NO: 1915 | AUCUGUAUGUCUGCCUAAU | 0.073 |
| AH0629 | SEQ ID NO: 630 | UCUGUAUGUCUGCCUAAUUAA | SEQ ID NO: 1273 | AAUUAGGCAGACAUACAGAUA | SEQ ID NO: 1916 | UCUGUAUGUCUGCCUAAUU | 0.132 |
| AH0630 | SEQ ID NO: 631 | CUGUAUGUCUGCCUAAUUAAA | SEQ ID NO: 1274 | UAAUUAGGCAGACAUACAGAU | SEQ ID NO: 1917 | CUGUAUGUCUGCCUAAUUA | 0.051 |
| AH0631 | SEQ ID NO: 632 | UGUAUGUCUGCCUAAUUAAAA | SEQ ID NO: 1275 | UUAAUUAGGCAGACAUACAGA | SEQ ID NO: 1918 | UGUAUGUCUGCCUAAUUAA | 0.109 |
| AH0632 | SEQ ID NO: 633 | GUAUGUCUGCCUAAUUAAAAA | SEQ ID NO: 1276 | UUUAAUUAGGCAGACAUACAG | SEQ ID NO: 1919 | GUAUGUCUGCCUAAUUAAA | 0.091 |
| AH0633 | SEQ ID NO: 634 | UAUGUCUGCCUAAUUAAAAAA | SEQ ID NO: 1277 | UUUUAAUUAGGCAGACAUACA | SEQ ID NO: 1920 | UAUGUCUGCCUAAUUAAAA | 0.083 |
| AH0634 | SEQ ID NO: 635 | AUGUCUGCCUAAUUAAAAAAA | SEQ ID NO: 1278 | UUUUUAAUUAGGCAGACAUAC | SEQ ID NO: 1921 | AUGUCUGCCUAAUUAAAAA | 0.081 |
| AH0635 | SEQ ID NO: 636 | UGUCUGCCUAAUUAAAAAAAU | SEQ ID NO: 1279 | UUUUUUAAUUAGGCAGACAUA | SEQ ID NO: 1922 | UGUCUGCCUAAUUAAAAAA | 0.113 |
| AH0636 | SEQ ID NO: 637 | GUCUGCCUAAUUAAAAAAAUA | SEQ ID NO: 1280 | UUUUUUUAAUUAGGCAGACAU | SEQ ID NO: 1923 | GUCUGCCUAAUUAAAAAAA | 0.179 |
| AH0637 | SEQ ID NO: 638 | UCUGCCUAAUUAAAAAAAUAU | SEQ ID NO: 1281 | AUUUUUUUAAUUAGGCAGACA | SEQ ID NO: 1 924 | UCUGCCUAAUUAAAAAAAU | 0113 |
| AH0638 | SEQ ID NO: 639 | CUGCCUAAUUAAAAAAAUAUA | SEQ ID NO: 1282 | UAUUUUUUUAAUUAGGCAGAC | SEQ ID NO: 1925 | CUGCCUAAUUAAAAAAAUA | 0.043 |
| AH0639 | SEQ ID NO: 640 | UGCCUAAUUAAAAAAAUAUAU | SEQ ID NO: 1283 | AUAUUUUUUUAAUUAGGCAGA | SEQ ID NO: 1926 | UGCCUAAUUAAAAAAAUAU | 0.151 |
| AH0640 | SEQ ID NO: 641 | GCCUAAUUAAAAAAAUAUAUA | SEQ ID NO: 1284 | UAUAUUUUUUUAAUUAGGCAG | SEQ ID NO: 1927 | GCCUAAUUAAAAAAAUAUA | 0.191 |
| AH0641 | SEQ ID NO: 642 | CCUAAUUAAAAAAAUAUAUAU | SEQ ID NO: 1285 | AUAUAUUUUUUUAAUUAGGCA | SEQ ID NO: 1928 | CCUAAUUAAAAAAAUAUAU | 0.361 |
| AH0642 | SEQ ID NO: 643 | CUAAUUAAAAAAAUAUAUAUU | SEQ ID NO: 1286 | UAUAUAUUUUUUUAAUUAGGC | SEQ ID NO: 1929 | CUAAUUAAAAAAAUAUAUA | 0.287 |
| AH0643 | SEQ ID NO: 644 | UAAAAAAAUAUAUAUUGUAUU | SEQ ID NO: 1287 | UACAAUAUAUAUUUUUUUAAU | SEQ ID NO: 1930 | UAAAAAAAUAUAUAUUGUA | 0.376 |

The nucleotide sequence of full-length APCS 2nd strand cDNA is shown in Table 2.

### [Table 41]

**Table 2**

| | |
|---|---|
| SEQ ID NO:1 | gggcatgaatatcagacgctagggggacagccactgtgttgtctgctaccctcatcctggtcactgcttctgctataa cagccctaggccaggaatatgaacaagccgctgctttggatctctgtcctcaccagcctcctggaagcctttgctca cacagacctcagtgggaaggtgtttgtatttcctagagaatctgttactgatcatgtaaacttgatcacaccgctggag aagcctctacagaactttaccttgtgttttcgagcctatagtgatctctctcgtgcctacagcctcttctcctacaata cccaaggcagggataatgagctactagtttataaagaaagagttggagagtatagtctatacattggaagacacaaagt tacatccaaagttatcgaaaagttcccggctccagtgcacatctgtgtgagctgggagtcctcatcaggtattgctga attttggatcaatgggacacctttggtgaaaaagggtctgcgacagggttactttgtagaagctcagcccaagattgtc ctggggcaggaacaggattcctatgggggcaagtttgataggagccagtcctttgtgggagagattggggatttgtacat gtgggactctgtgctgcccccagaaaatatcctgtctgcctatcagggtacccctctccctgccaatatcctggact ggcaggctctgaactatgaaatcagaggatatgtcatcatcaaaccccttggtgtgggtctgaggtcttgactcaacgag agcacttgaaaatgaaatgactgtctaagagatctggtcaaagcaactggatactagatcttacatctgcagctctttc ttctttgaatttcctatctgtatgtctgcctaattaaaaaaatatatattgtattatgctacctgcaaaaaaaaaaaaaaaa aaaaaaaaaaaaaa |

Reference Test Example 2: Measurement of knockdown activity of modified siRNA against APCS mRNA in human cell - 1

Sense strand nucleic acids consisting of ribonucleotides shown in SEQ ID NOs: 1931 to 1974, antisense strand nucleic acids consisting of ribonucleotides shown in SEQ ID NOs: 1975 to 2018, and double-stranded nucleic acids prepared by annealing these strands (the sense strand represented by SEQ ID NO: n (n = 1931 to 1974) and the antisense strand represented by SEQ ID NO: [n + 44] are paired) were synthesized by commission to GeneDesign, Inc.

The relative expression level of APCS mRNA was calculated by the same operation as in Reference Test Example 1 except that: the final concentration of each synthesized double-stranded nucleic acid was set to 1 nM or 0.1 nM; and the double-stranded nucleic acid was transferred to RMG-1 cells, which were then cultured for 24 hours. This experiment was conducted four times. Median values of the relative expression level of APCS mRNA are shown in Tables M1-1 and Ml-2. In Tables M1-1 and Ml-2, N(M) represents 2'-O-methyl-RNA, and N(F) represents 2'-F-RNA.

### [Table 42]

**Table M1-1**

| Double-stranded nucleic acid No | SEQ ID NO: | Sense strand sequence (5'--->3') | SEQ ID NO: | Antisense strand sequence (5'--->3') | Relative APCS expression level 1 nM | Relative APCS expression level D.1 nM |
|---|---|---|---|---|---|---|
| AH0644 | SEQ ID NO: 1931 | U(F)A(M)C(F)C(M)U(F)U(M)G(F)U(M)G(F)U(M)U(F)U(F)U (M)C(F)G(M)A(F)G(M)C(F)C(M)U(F)A(M) | SEQ ID NO: 1975 | J(F)A(M)G(F)G(M)C(F)U(M)C(F)G(M)A(F)A(M)A(M)A(F)C M)A(F)C(M)A(F)A(M)G(F)G(M)U(F)A(M)A(F)A(M) | 0.057 | 0.098 |
| AH0645 | SEQ ID NO: 1932 | U(F)G(M)C(F)C(M)U(F)A(M)C(F)A(M)G(F)C(M)C(F)U(F)C (M)U(F)U(M)C(F)U(M)C(F)C(M)U(F)A(M) | SEQ ID NO: 1976 | U(F)A(M)G(F)G(M)A(F)G(M)A(F)A(M)G(F)A(M)G(M)G(F)C M)U(F)G(M)U(F)A(M)G(F)G(M)C(F)A(M)C(F)G(M) | 0.083 | 0.130 |
| AH0646 | SEQ ID NO: 1933 | A(F)C(M)A(F)G(M)C(F)C(M)U(F)C(M)U(F)U(M)C(F)U(F)C (M)C(F)U(M)A(F)C(M)A(F)A(M)U(F)A(M) | SEQ ID NO: 1977 | U(F)A(M)U(F)U(M)G(F)U(M)A(F)G(M)G(F)A(M)G(M)A(F)A (M)G(F)A(M)G(F)G(M)C(F)U(M)G(F)U(M)A(F)G(M) | 0.101 | 0.257 |
| AH0647 | SEQ ID NO: 1934 | G(F)U(M)C(F)C(M)U(F)C(M)A(F)U(M)C(F)A(M)G(F)G(F)U (M)A(F)U(M)U(F)G(M)C(F)U(M)G(F)A(M) | SEQ ID NO: 1978 | U(F)C(M)A(F)G(M)C(F)A(M)A(F)U(M)A(F)C(M)C(M)U(F)G (M)A(F)U(M)G(F)A(M)G(F)G(M)A(F)C(M)U(F)C(M) | 0.135 | 0.293 |
| AH0648 | SEQ ID NO: 1935 | A(F)G(M)G(F)A(M)G(F)C(M)C(F)A(M)G(F)U(M)C(F)C(F)U (M)U(F)U(M)G(F)U(M)G(F)G(M)G(F)A(M) | SEQ ID NO: 1979 | U(F)C(M)C(F)C(M)A(F)C(M)A(F)A(M)A(F)G(M)G(M)A(F)C (M)U(F)G(M)G(F)C(M)U(F)C(M)C(F)U(M)A(F)U(M) | 0.177 | 0.355 |
| AH0649 | SEQ ID NO: 1936 | G(F)A(M)G(F)C(M)C(F)A(M)G(F)U(M)C(F)C(M)U(F)U(F)U (M)G(F)U(M)G(F)G(M)G(F)A(M)G(F)A(M) | SEQ ID NO: 1980 | U(F)C(M)U(F)C(M)C(F)C(M)A(F)C(M)A(F)A(M)A(M)G(F)G (M)A(F)C(M)U(F)G(M)G(F)C(M)U(F)C(M)C(F)U(M) | 0.084 | 0.196 |
| AH0650 | SEQ ID NO: 1937 | C(F)C(M)A(F)G(M)U(F)C(M)C(F)U(M)U(F)U(M)G(F)U(F)G (M)G(F)G(M)A(F)G(M)A(F)G(M)A(F)U(M) | SEQ ID NO: 1981 | A(F)U(M)C(F)U(M)C(F)U(M)C(F)C(M)C(F)A(M)C(M)A(F)A (M)A(F)G(M)G(F)A(M)C(F)U(M)G(F)G(M)C(F)U(M) | 0.065 | 0.126 |
| AH0651 | SEQ ID NO: 1938 | C(F)A(M)G(F)U(M)C(F)C(M)U(F)U(M)U(F)G(M)U(F)G(F)G (M)G(F)A(M)G(F)A(M)G(F)A(M)U(F)U(M) | SEQ ID NO: 1982 | A(F)A(M)U(F)C(M)U(F)C(M)U(F)C(M)C(F)C(M)A(M)C(F)A (M)A(F)A(M)G(F)G(M)A(F)C(M)U(F)G(M)G(F)C(M) | 0.093 | 0.193 |
| AH0652 | SEQ ID NO: 1939 | U(F)G(M)G(F)G(M)A(F)G(M)A(F)G(M)A(F)U(M)U(F)G(F)G (M)G(F)G(M)A(F)U(M)U(F)U(M)G(F)U(M) | SEQ ID NO: 1983 | A(F)C(M)A(F)A(M)A(F)U(M)C(F)C(M)C(F)C(M)A(M)A(F)U (M)C(F)U(M)C(F)U(M)C(F)C(M)C(F)A(M)C(F)A(M) | 0.170 | 0.380 |
| AH0653 | SEQ ID NO: 1940 | G(F)G(M)G(F)A(M)G(F)A(M)G(F)A(M)U(F)U(M)G(F)G(F)G (M)G(F)A(M)U(F)U(M)U(F)G(M)U(F)A(M) | SEQ ID NO: 1984 | U(F)A(M)C(F)A(M)A(F)A(M)U(F)C(M)C(F)C(M)C(M)A(F)A (M)U(F)C(M)U(F)C(M)U(F)C(M)C(F)C(M)A(F)C(M) | 0.152 | 0.300 |
| AH0654 | SEQ ID NO: 1941 | G(F)A(M)G(F)A(M)G(F)A(M)U(F)U(M)G(F)G(M)G(F)G(F)A (M)U(F)U(M)U(F)G(M)U(F)A(M)C(F)A(M) | SEQ ID NO: 1985 | U(F)G(M)U(F)A(M)C(F)A(M)A(F)A(M)U(F)C(M)C(M)C(F)C (M)A(F)A(M)U(F)C(M)U(F)C(M)U(F)C(M)C(F)C(M) | 0.094 | 0.176 |
| AH0655 | SEQ ID NO: 1942 | U(F)C(M)C(F)U(M)G(F)U(M)C(F)U(M)G(F)C(M)C(F)U(F)A (M)U(F)C(M)A(F)G(M)G(F)G(M)U(F)A(M) | SEQ ID NO: 1986 | U(F)A(M)C(F)C(M)C(F)U(M)G(F)A(M)U(F)A(M)G(M)G(F) C(M)A(F)G(M)A(F)C(M)A(F)G(M)G(F)A(M)U(F)A(M) | 0.123 | 0.332 |
| AH0656 | SEQ ID NO: 1943 | C(F)A(M)G(F)G(M)C(F)U(M)C(F)U(M)G(F)A(M)A(F)C(F)U (M)A(F)U(M)G(F)A(M)A(F)A(M)U(F)C(M) | SEQ ID NO: 1987 | G(F)A(M)U(F)U(M)U(F)C(M)A(F)U(M)A(F)G(M)U(M)U(F) C(M)A(F)G(M)A(F)G(M)C(F)C(M)U(F)G(M)C(F)C(M) | 0.098 | 0.176 |
| AH0657 | SEQ ID NO: 1944 | G(F)A(M)G(F)G(M)A(F)U(M)A(F)U(M)G(F)U(M)C(F)A(F)U (M)C(F)A(M)U(F)C(M)A(F)A(M)A(F)C(M) | SEQ ID NO: 1988 | G(F)U(M)U(F)U(M)G(F)A(M)U(F)G(M)A(F)U(M)G(M)A(F) C(M)A(F)U(M)A(F)U(M)C(F)C(M)U(F)C(M)U(F)G(M) | 0.147 | 0.338 |
| AH0658 | SEQ ID NO: 1945 | U(F)G(M)G(F)G(M)U(F)C(M)U(F)G(M)A(F)G(M)G(F)U(F)C (M)U(F)U(M)G(F)A(M)C(F)U(M)C(F)A(M) | SEQ ID NO: 1989 | U(F)G(M)A(F)G(M)U(F)C(M)A(F)A(M)G(F)A(M)C(M)C(F)U (M)C(F)A(M)G(F)A(M)C(F)C(M)C(F)A(M)C(F)A(M) | 0.103 | 0.203 |
| AH0659 | SEQ ID NO: 1946 | A(F)C(M)U(F)C(M)A(F)A(M)C(F)G(M)A(F)G(M)A(F)G(F)C (M)A(F)C(M)U(F)U(M)G(F)A(M)A(F)A(M) | SEQ ID NO: 1990 | U(F)U(M)U(F)C(M)A(F)A(M)G(F)U(M)G(F)C(M)U(M)C(F) U(M)C(F)G(M)U(F)U(M)G(F)A(M)G(F)U(M)C(F)A(M) | 0.066 | 0.084 |
| AH0660 | SEQ ID NO: 1947 | C(F)U(M)C(F)A(M)A(F)C(M)G(F)A(M)G(F)A(M)G(F)C(F)A (M)C(F)U(M)U(F)G(M)A(F)A(M)A(F)A(M) | SEQ ID NO: 1991 | U(F)U(M)U(F)U(M)C(F)A(M)A(F)G(M)U(F)G(M)C(M)U(F) C(M)U(F)C(M)G(F)U(M)U(F)G(M)A(F)G(M)U(F)C(M) | 0.067 | 0.066 |
| AH0661 | SEQ ID NO: 1948 | U(F)C(M)A(F)A(M)C(F)G(M)A(F)G(M)A(F)G(M)C(F)A(F)C (M)U(F)U(M)G(F)A(M)A(F)A(M)A(F)U(M) | SEQ ID NO: 1992 | A(F)U(M)U(F)U(M)U(F)C(M)A(F)A(M)G(F)U(M)G(M)C(F) U(M)C(F)U(M)C(F)G(M)U(F)U(M)G(F)A(M)G(F)U(M) | 0.058 | 0.074 |
| AH0662 | SEQ ID NO: 1949 | A(F)A(M)C(F)G(M)A(F)G(M)A(F)G(M)C(F)A(M)C(F)U(F)U (M)G(F)A(M)A(F)A(M)A(F)U(M)G(F)A(M) | SEQ ID NO: 1993 | U(F)C(M)A(F)U(M)U(F)U(M)U(F)C(M)A(F)A(M)G(M)U(F) G(M)C(F)U(M)C(F)U(M)C(F)G(M)U(F)U(M)G(F)A(M) | 0.046 | 0.091 |
| AH0663 | SEQ ID NO: 1950 | A(F)C(M)G(F)A(M)G(F)A(M)G(F)C(M)A(F)C(M)U(F)U(F)G (M)A(F)A(M)A(F)A(M)U(F)G(M)A(F)A(M) | SEQ ID NO: 1994 | U(F)U(M)C(F)A(M)U(F)U(M)U(F)U(M)C(F)A(M)A(M)G(F) U(M)G(F)C(M)U(F)C(M)U(F)C(M)G(F)U(M)U(F)G(M) | 0.053 | 0.068 |
| AH0664 | SEQ ID NO: 1951 | C(F)G(M)A(F)G(M)A(F)G(M)A(F)G(M)C(F)A(M)C(F)U(M)U(F)G(F)A (M)A(F)A(M)A(F)U(M)G(F)A(M)A(F)A(M) | SEQ ID NO: 1995 | U(F)U(M)U(F)C(M)A(F)U(M)U(F)U(M)U(F)C(M)A(M)A(F) G(M)U(F)G(M)C(F)U(M)C(F)U(M)C(F)G(M)U(F)U(M) | 0.057 | 0.076 |
| AH0665 | SEQ ID NO: 1952 | G(F)A(M)G(F)A(M)G(F)C(M)A(F)C(M)U(F)U(M)G(F)A(F)A (M)A(F)A(M)U(F)G(M)A(F)A(M)A(F)U(M) | SEQ ID NO: 1996 | A(F)U(M)U(F)U(M)C(F)A(M)U(F)U(M)U(F)U(M)C(M)A(F)A (M)G(F)U(M)G(F)C(M)U(F)C(M)U(F)C(M)G(F)U(M) | 0.062 | 0.082 |

### [Table 43]

**Table M1-2**

| Double-stranded nucleic acid No. | SEQ ID NO: | Sense strand sequence (5'--->3') | SEQ ID NO: | Antisense strand sequence (**5'**--->3') | Relative APCS expression level 1 nM | Relative APCS expression level 0.1 nM |
|---|---|---|---|---|---|---|
| AH0666 | SEQ ID NO: 1953 | A(F)G(M)A(F)G(M)C(F)A(M)C(F)U(M)U(F)G(M)A(F)A(F)A( M)A(F)U(M)G(F)A(M)A(F)A(M)U(F)G(M) | SEQ ID NO: 1997 | C(F)A(M)U(F)U(M)U(F)C(M)A(F)U(M)U(F)U(M)U(M)C(F) A(M)A(F)G(M)U(F)G(M)C(F)U(M)C(F)U(M)C(F)G(M) | 0.062 | 0.083 |
| AH0667 | SEQ ID NO: 1954 | C(F)U(M)A(F)A(M)G(F)A(M)G(F)A(M)U(F)C(M)U(F)G(F)G (M)U(F)C(M)A(F)A(M)A(F)G(M)C(F)A(M) | SEQ ID NO: 1998 | U(F)G(M)C(F)U(M)U(F)U(M)G(F)A(M)C(F)C(M)A(M)G(F) A(M)U(F)C(M)U(F)C(M)U(F)U(M)A(F)G(M)A(F)C(M) | 0.093 | 0.131 |
| AH0668 | SEQ ID NO: 1955 | G(F)U(M)G(F)G(M)U(F)C(M)A(F)A(M)A(F)G(M)C(F)A(F)A (M)C(F)U(M)G(F)G(M)A(F)U(M)A(F)C(M) | SEQ ID NO: 1999 | G(F)U(M)A(F)U(M)C(F)C(M)A(F)G(M)U(F)U(M)G(M)C(F) U(M)U(F)U(M)G(F)A(M)C(F)C(M)A(F)G(M)A(F)U(M) | 0.151 | 0.319 |
| AH0669 | SEQ ID NO: 1956 | A(F)A(M)G(F)C(M)A(F)A(M)C(F)U(M)G(F)G(M)A(F)U(F)A( M)C(F)U(M)A(F)G(M)A(F)U(M)C(F)U(M) | SEQ ID NO: 2000 | A(F)G(M)A(F)U(M)C(F)U(M)A(F)G(M)U(F)A(M)U(M)C(F)C (M)A(F)G(M)U(F)U(M)G(F)C(M)U(F)U(M)U(F)G(M) | 0.083 | 0.126 |
| AH0670 | SEQ ID NO: 1957 | A(F)G(M)C(F)A(M)A(F)C(M)U(F)G(M)G(F)A(M)U(F)A(F)C (M)U(F)A(M)G(F)A(M)U(F)C(M)U(F)U(M) | SEQ ID NO: 2001 | A(F)A(M)G(F)A(M)U(F)C(M)U(F)A(M)G(F)U(M)A(M)U(F)C (M)C(F)A(M)G(F)U(M)U(F)G(M)C(F)U(M)U(F)U(M) | 0.071 | 0.129 |
| AH0671 | SEQ ID NO: 1958 | G(F)C(M)A(F)A(M)C(F)U(M)G(F)G(M)A(F)U(M)A(F)C(F)U (M)A(F)G(M)A(F)U(M)C(F)U(M)U(F)A(M) | SEQ ID NO: 2002 | U(F)A(M)A(F)G(M)A(F)U(M)C(F)U(M)A(F)G(M)U(M)A(F)U (M)C(F)C(M)A(F)G(M)U(F)U(M)G(F)C(M)U(F)U(M) | 0.115 | 0.181 |
| AH0672 | SEQ ID NO: 1959 | A(F)U(M)C(F)U(M)U(F)A(M)C(F)A(M)U(F)C(M)U(F)G(F)C (M)A(F)G(M)C(F)U(M)C(F)U(M)U(F)U(M) | SEQ ID NO: 2003 | A(F)A(M)A(F)G(M)A(F)G(M)C(F)U(M)G(F)C(M)A(M)G(F)A (M)U(F)G(M)U(F)A(M)A(F)G(M)A(F)U(M)C(F)U(M) | 0.072 | 0.093 |
| AH0673 | SEQ ID NO: 1960 | U(F)A(M)C(F)A(M)U(F)C(M)U(F)G(M)C(F)A(M)G(F)C(F)U (M)C(F)U(M)U(F)U(M)C(F)U(M)U(F)C(M) | SEQ ID NO: 2004 | G(F)A(M)A(F)G(M)A(F)A(M)A(F)G(M)A(F)G(M)C(M)U(F)G (M)C(F)A(M)G(F)A(M)U(F)G(M)U(F)A(M)A(F)G(M) | 0.089 | 0.112 |
| AH0674 | SEQ ID NO: 1961 | A(F)C(M)A(F)U(M)C(F)U(M)G(F)C(M)A(F)G(M)C(F)U(F)C (M)U(F)U(M)U(F)C(M)U(F)U(M)C(F)U(M) | SEQ ID NO: 2005 | A(F)G(M)A(F)A(M)G(F)A(M)A(F)A(M)G(F)A(M)G(M)C(F)U (M)G(F)C(M)A(F)G(M)A(F)U(M)G(F)U(M)A(F)A(M) | 0.067 | 0.082 |
| AH0675 | SEQ ID NO: 1962 | C(F)A(M)U(F)C(M)U(F)G(M)C(F)A(M)G(F)C(M)U(F)C(F)U (M)U(F)U(M)C(F)U(M)U(F)C(M)U(F)U(M) | SEQ ID NO: 2006 | A(F)A(M)G(F)A(M)A(F)G(M)A(F)A(M)A(F)G(M)A(M)G(F)C (M)U(F)G(M)C(F)A(M)G(F)A(M)U(F)G(M)U(F)A(M) | 0.098 | 0.114 |
| AH0676 | SEQ ID NO: 1963 | A(F)U(M)C(F)U(M)G(F)C(M)A(F)G(M)C(F)U(M)C(F)U(F) (M)U(F)C(M)U(F)U(M)C(F)U(M)U(F)U(M) | SEQ ID NO: 2007 | A(F)A(M)A(F)G(M)A(F)A(M)G(F)A(M)A(F)A(M)G(M)A(F)G( M)C(F)U(M)G(F)C(M)A(F)G(M)A(F)U(M)G(F)U(M) | 0.093 | 0144 |
| AH0677 | SEQ ID NO: 1964 | U(F)C(M)U(F)G(M)C(F)A(M)G(F)C(M)U(F)C(M)U(F)U(F)U (M)C(F)U(M)U(F)C(M)U(F)U(M)U(F)G(M) | SEQ ID NO: 2008 | C(F)A(M)A(F)A(M)G(F)A(M)A(F)G(M)A(F)A(M)A(M)G(F)A( M)G(F)C(M)U(F)G(M)C(F)A(M)G(F)A(M)U(F)G(M) | 0.084 | 0.093 |
| AH0678 | SEQ ID NO: 1965 | C(F)U(M)G(F)C(M)A(F)G(M)C(F)U(M)C(F)U(M)U(F)U(F)C (M)U(F)U(M)C(F)U(M)U(F)U(M)G(F)A(M) | SEQ ID NO: 2009 | U(F)C(M)A(F)A(M)A(F)G(M)A(F)A(M)G(F)A(M)A(M)A(F)G (M)A(F)G(M)C(F)U(M)G(F)C(M)A(F)G(M)A(F)U(M) | 0.089 | 0.100 |
| AH0679 | SEQ ID NO: 1966 | U(F)G(M)C(F)A(M)G(F)C(M)U(F)C(M)U(F)U(M)U(F)C(F)U (M)U(F)C(M)U(F)U(M)U(F)G(M)A(F)A(M) | SEQ ID NO: 2010 | U(F)U(M)C(F)A(M)A(F)A(M)G(F)A(M)A(F)G(M)A(M)A(F)A (M)G(F)A(M)G(F)C(M)U(F)G(M)C(F)A(M)G(F)A(M) | 0.101 | 0.158 |
| AH0680 | SEQ ID NO: 1967 | G(F)C(M)A(F)G(M)C(F)U(M)C(F)U(M)U(F)U(M)C(F)U(F)U (M)C(F)U(M)U(F)U(M)G(F)A(M)A(F)U(M) | SEQ ID NO: 2011 | A(F)U(M)U(F)C(M)A(F)A(M)A(F)G(M)A(F)A(M)G(M)A(F)A (M)A(F)G(M)A(F)G(M)C(F)U(M)G(F)C(M)A(F)G(M) | 0.086 | 0.112 |
| AH0681 | SEQ ID NO: 1968 | C(F)A(M)G(F)C(M)U(F)C(M)U(F)U(M)U(F)C(M)U(F)U(F)C (M)U(F)U(M)U(F)G(M)A(F)A(M)U(F)U(M) | SEQ ID NO: 2012 | A(F)A(M)U(F)U(M)C(F)A(M)A(F)A(M)G(F)A(M)A(M)G(F)A (M)A(F)A(M)G(F)A(M)G(F)C(M)U(F)G(M)C(F)A(M) | 0.088 | 0.119 |
| AH0682 | SEQ ID NO: 1969 | U(F)U(M)G(F)A(M)A(F)U(M)U(F)U(M)C(F)C(M)U(F)A(F)U (M)C(F)U(M)G(F)U(M)A(F)U(M)G(F)U(M) | SEQ ID NO: 2013 | A(F)C(M)A(F)U(M)A(F)C(M)A(F)G(M)A(F)U(M)A(M)G(F)G (M)A(F)A(M)A(F)U(M)U(F)C(M)A(F)A(M)A(F)G(M) | 0.052 | 0.064 |
| AH0683 | SEQ ID NO: 1970 | U(F)C(M)C(F)U(M)A(F)U(M)C(F)U(M)G(F)U(M)A(F)U(F)G (M)U(F)C(M)U(F)G(M)C(F)C(M)U(F)A(M) | SEQ ID NO: 2014 | U(F)A(M)G(F)G(M)C(F)A(M)G(F)A(M)C(F)A(M)U(M)A(F)C (M)A(F)G(M)A(F)U(M)A(F)G(M)G(F)A(M)A(F)A(M) | 0.062 | 0.089 |
| AH0684 | SEQ ID NO: 1971 | A(F)U(M)C(F)U(M)G(F)U(M)A(F)U(M)G(F)U(M)C(F)U(F)G (M)C(F)C(M)U(F)A(M)A(F)U(M)U(F)A(M) | SEQ ID NO: 2015 | U(F)A(M)A(F)U(M)U(F)A(M)G(F)G(M)C(F)A(M)G(M)A(F)C (M)A(F)U(M)A(F)C(M)A(F)G(M)A(F)U(M)A(F)G(M) | 0.055 | 0.083 |
| AH0685 | SEQ ID NO: 1972 | U(F)C(M)U(F)G(M)U(F)A(M)U(F)G(M)U(F)C(M)U(F)G(F)C (M)C(F)U(M)A(F)A(M)U(F)U(M)A(F)A(M) | SEQ ID NO: 2016 | U(F)U(M)A(F)A(M)U(F)U(M)A(F)G(M)G(F)C(M)A(M)G(F)A (M)C(F)A(M)U(F)A(M)C(F)A(M)G(F)A(M)U(F)A(M) | 0.073 | 0.099 |
| AH0686 | SEQ ID NO: 1973 | C(F)U(M)G(F)U(M)A(F)U(M)G(F)U(M)C(F)U(M)G(F)C(F)C (M)U(F)A(M)A(F)U(M)U(F)A(M)A(F)A(M) | SEQ ID NO: 2017 | U(F)U(M)U(F)A(M)A(F)U(M)U(F)A(M)G(F)G(M)C(M)A(F)G (M)A(F)C(M)A(F)U(M)A(F)C(M)A(F)G(M)A(F)U(M) | 0.148 | 0.305 |
| AH0687 | SEQ ID NO: 1974 | G(F)U(M)C(F)U(M)G(F)C(M)C(F)U(M)A(F)A(M)U(F)U(F)A (M)A(F)A(M)A(F)A(M)A(F)A(M)U(F)A(M) | SEQ ID NO: 2018 | U(F)A(M)U(F)U(M)U(F)U(M)U(F)U(M)U(F)A(M)A(M)U(F) U(M)A(F)G(M)G(F)C(M)A(F)G(M)A(F)C(M)A(F)U(M) | 0.078 | 0.158 |

### Reference Test Example 3: Measurement of knockdown activity of modified siRNA against APCS mRNA in human cell - 2

The double-stranded nucleic acids described in Table Ml-3 were synthesized by GeneDesign, Inc. and used. Specifically, the double-stranded nucleic acids were prepared by annealing sense strands consisting of ribonucleotides shown in SEQ ID NOs: 2019 to 2043 and antisense strands consisting of ribonucleotides shown in SEQ ID NOs: 2044 to 2068 (the sense strand represented by SEQ ID NO: n (n = 2019 to 2043) and the antisense strand represented by SEQ ID NO: [n + 25] are paired). A siRNA/RNAiMax mixed solution of each double-stranded nucleic acid and RNAiMax transfection reagent (manufactured by Thermo Fisher Scientific Inc., Catalog No. 13778150) diluted with Opti-MEM I Reduced Serum Medium (manufactured by Thermo Fisher Scientific Inc., Catalog No. 31985070) was added at 20 µL/well to 96-well culture plates. Human ovary cancer-derived cell line RMG-I cells (JCRB Cell Bank, JCRB0172) were inoculated at 10,000 cells/60 µL/well to the 96-well culture plates and cultured at 37°C for 24 hours under 5% CO₂ conditions. The medium used was Ham's F-12 Nutrient Mix medium (manufactured by Thermo Fisher Scientific Inc., Catalog No. 11765-047) containing 10% fetal bovine serum (manufactured by Thermo Fisher Scientific Inc., Catalog No. 10091-148). The final concentration of the double-stranded nucleic acid was set to 1 or 0.1 nM. Then, the cells were washed with DPBS, no calcium, no magnesium (manufactured by Thermo Fisher Scientific Inc., Catalog No. 14190-144), and the recovery of total RNA from each of the plates and the preparation of cDNA through reverse-transcription reaction using the obtained total RNA as a template were performed using SuperPrep Cell Lysis & RT Kit for qPCR (manufactured by Toyobo Co., Ltd., Catalog No. SCQ-101) according to the method described in the instruction attached to the product. This cDNA was added at 3 µL/well to MicroAmp Optical 384-well plate (manufactured by Applied Biosystems, Inc., Catalog No. 4309849), and further, 10 µL of TaqMan Gene Expression Master Mix (manufactured by Applied Biosystems, Inc., Catalog No. 4369016), 6 µL of UltraPure Distilled Water (manufactured by Thermo Fisher Scientific Inc., Catalog No. 10977-015), and 1 µL of human APCS probe or 1 µL of human GAPDH (D-glyceraldehyde-3-phosphate dehydrogenase) probe were added to each well. The real-time PCR of the human APCS gene and the human GAPDH gene was performed using QuantStudio 12K Flex real-time PCR system (manufactured by Thermo Fisher Scientific Inc.). GAPDH, a constitutively expressed gene, was measured as an internal control and used to correct the APCS gene expression level. The amount of APCS mRNA in RMG-I cells treated with only the transfection reagent without the addition of siRNA was defined as 1.0. The relative expression level of APCS mRNA was calculated when each siRNA was transferred. This experiment was conducted twice. Average values of the relative expression level of APCS mRNA are shown in Table Ml-3. In Table Ml-3, N(M) represents 2'-O-methyl-modified RNA, and N(F) represents 2'-fluorine-modified RNA.

### [Table 44]

**Table Ml-3**

| SEQ ID NO: Sense strand sequence (5'--->3') | | SEQ ID NO: | Antisense strand sequence (5'--->3') | Relative APCS expression level 1 nM | Relative APCS expression level 0.1 nM |
|---|---|---|---|---|---|
| SEQ ID NO: 2019 | U(F)A(M)C(F)C(M)U(F)U(M)G(F)U(M)G(F)U(M)U(F)U(F)U(M)C( F)G(M)A(F)G(M)C(F)C(M)U(M)A(F) | SEQ ID NO: 2044 | U(M)A(F)G(F)G(M)C(F)U(M)C(F)G(M)A(F)A(M)A(M)A(F)C(M)A( F)C(M)A(F)A(M)G(F)G(M)U(F)A(M)A(F)A(M) | 0.288 | 0.986 |
| SEQ ID NO: 2020 | U(M)A(M)C(M)C(M)U(M)U(M)G(F)U(M)G(F)U(M)U(F)U(F)U(M)C (M)G(M)A(M)G(M)C(M)C(M)U(M)A(M) | SEQ ID NO: 2045 | U(M)A(F)G(F)G(F)C(F)U(F)C(F)G(F)A(F)A(M)A(M)A(F)C(M)A(F) C(M)A(F)A(F)G(F)G(F)U(F)A(F)A(F)A(M) | 0.053 | 0.132 |
| SEQ ID NO: 2021 | U(M)A(M)C(F)C(M)U(F)U(M)G(F)U(M)G(F)U(M)U(F)U(F)U(M)C( F)G(M)A(F)G(M)C(M)C(M)U(M)A(M) | SEQ ID NO: 2046 | U(M)A(F)G(F)G(M)C(F)U(M)C(F)G(M)A(F)A(M)A(M)A(F)C(M)A( F)C(M)A(F)A(M)G(F)G(M)U(M)A(M)A(M)A(M) | 0.089 | 0.208 |
| SEQ ID NO: 2022 | U(M)G(M)G(M)G(M)U(M)C(M)U(F)G(M)A(F)G(M)G(F)U(F)C(M)U (M)U(M)G(M)A(M)C(M)U(M)C(M)A(M) | SEQ ID NO: 2047 | U(M)G(F)A(F)G(F)U(F)C(F)A(F)A(F)G(F)A(M)C(M)C(F)U(M)C(F) A(M)G(F)A(F)C(F)C(F)C(F)A(F)C(F)A(M) | 0.106 | 0.264 |
| SEQ ID NO: 2023 | U(M)G(M)G(F)G(M)U(F)C(M)U(F)G(M)A(F)G(M)G(F)U(F)C(M)U( F)U(M)G(F)A(M)C(M)U(M)C(M)A(M) | SEQ ID NO: 2048 | U(M)G(F)A(F)G(M)U(F)C(M)A(F)A(M)G(F)A(M)C(M)C(F)U(M)C( F)A(M)G(F)A(M)C(F)C(M)C(M)A(M)C(M)A(M) | 0.098 | 0.134 |
| SEQ ID NO: 2024 | A(F)G(M)A(F)G(M)C(F)A(M)C(F)U(M)U(F)G(M)A(F)A(F)A(M)A(F )U(M)G(F)A(M)A(F)A(M)U(M)G(F) | SEQ ID NO: 2049 | C(M)A(F)U(F)U(M)U(F)C(M)A(F)U(M)U(F)U(M)U(M)C(F)A(M)A( F)G(M)U(F)G(M)C(F)U(M)C(F)U(M)C(F)G(M) | 0.029 | 0.040 |
| SEQ ID NO: 2025 | A(M)G(M)A(M)G(M)C(M)A(M)C(F)U(M)U(F)G(M)A(F)A(F)A(M)A( M)U(M)G(M)A(M)A(M)A(M)U(M)G(M) | SEQ ID NO: 2050 | C(M)A(F)U(F)U(F)U(F)C(F)A(F)U(F)U(F)U(M)U(M)C(F)A(M)A(F) G(M)U(F)G(F)C(F)U(F)C(F)U(F)C(F)G(M) | 0.044 | 0.201 |
| SEQ ID NO: 2026 | A(M)G(M)A(F)G(M)C(F)A(M)C(F)U(M)U(F)G(M)A(F)A(F)A(M)A( F)U(M)G(F)A(M)A(M)A(M)U(M)G(M) | SEQ ID NO: 2051 | C(M)A(F)U(F)U(M)U(F)C(M)A(F)U(M)U(F)U(M)U(M)C(F)A(M)A( F)G(M)U(F)G(M)C(F)U(M)C(M)U(M)C(M)G(M) | 0.014 | 0.187 |
| SEQ ID NO: 2027 | U(F)C(M)U(F)G(M)C(F)A(M)G(F)C(M)U(F)C(M)U(F)U(F)U(M)C( F)U(M)U(F)C(M)U(F)U(M)U(M)G(F) | SEQ ID NO: 2052 | C(M)A(F)A(F)A(M)G(F)A(M)A(F)G(M)A(F)A(M)A(M)G(F)A(M)G(F )C(M)U(F)G(M)C(F)A(M)G(F)A(M)U(F)G(M) | 0.053 | 0.054 |
| SEQ ID NO: 2028 | U(M)C(M)U(M)G(M)C(M)A(M)G(F)C(M)U(F)C(M)U(F)U(F)U(M)C (M)U(M)U(M)C(M)U(M)U(M)U(M)G(M) | SEQ ID NO: 2053 | C(M)A(F)A(F)A(F)G(F)A(F)A(F)G(F)A(F)A(M)A(M)G(F)A(M)G(F) C(M)U(F)G(F)C(F)A(F)G(F)A(F)U(F)G(M) | 0.045 | 0.098 |
| SEQ ID NO: 2029 | U(M)C(M)U(F)G(M)C(F)A(M)G(F)C(M)U(F)C(M)U(F)U(F)U(M)C( F)U(M)U(F)C(M)U(M)U(M)U(M)G(M) | SEQ ID NO: 2054 | C(M)A(F)A(F)A(M)G(F)A(M)A(F)G(M)A(F)A(M)A(M)G(F)A(M)G(F )C(M)U(F)G(M)C(F)A(M)G(M)A(M)U(M)G(M) | 0.077 | 0.264 |
| SEQ ID NO: 2030 | G(M)C(M)A(M)G(M)C(M)U(M)C(F)U(M)U(F)U(M)C(F)U(F)U(M)C (M)U(M)U(M)U(M)G(M)A(M)A(M)U(M) | SEQ ID NO: 2055 | A(M)U(F)U(F)C(F)A(F)A(F)A(F)G(F)A(F)A(M)G(M)A(F)A(M)A(F) G(M)A(F)G(F)C(F)U(F)G(F)C(F)A(F)G(M) | 0.070 | 0.298 |
| SEQ ID NO: 2031 | G(M)C(M)A(F)G(M)C(F)U(M)C(F)U(M)C(F)U(M)U(F)U(M)C(F)U(F)U(M)C( F)U(M)U(F)U(M)G(M)A(M)A(M)U(M) | SEQ ID NO: 2056 | A(M)U(F)U(F)C(M)A(F)A(M)A(F)G(M)A(F)A(M)G(M)A(F)A(M)A(F )G(M)A(F)G(M)C(F)U(M)G(M)C(M)A(M)G(M) | 0.053 | 0.049 |
| SEQ ID NO: 2032 | C(M)A(M)G(M)C(M)U(M)C(M)U(F)U(M)U(F)C(M)U(F)(F)C(M)U (M)U(M)U(M)G(M)A(M)A(M)U(M)U(M) | SEQ ID NO: 2057 | A(M)A(F)U(F)U(F)C(F)A(F)A(F)A(F)G(F)A(M)A(M)G(F)A(M)A(F) A(M)G(F)A(F)G(F)C(F)U(F)G(F)C(F)A(M) | 0.060 | 0.096 |
| SEQ ID NO: 2033 | C(M)A(M)G(F)C(M)U(F)C(M)U(F)U(M)U(F)C(M)U(F)U(F)C(M)U( F)U(M)U(F)G(M)A(M)A(M)U(M)U(M) | SEQ ID NO: 2058 | A(M)A(F)U(F)U(M)C(F)A(M)A(F)A(M)G(F)A(M)A(M)G(F)A(M)A(F )A(M)G(F)A(M)G(F)C(M)U(M)G(M)C(M)A(M) | 0.023 | 0.075 |
| SEQ ID NO: 2034 | U(M)U(M)G(M)A(M)A(M)U(M)U(F)U(M)C(F)C(M)U(F)A(F)U(M)C (M)U(M)G(M)U(M)A(M)U(M)G(M)U(M) | SEQ ID NO: 2059 | A(M)C(F)A(F)U(F)A(F)C(F)A(F)G(F)A(F)U(M)A(M)G(F)G(M)A(F) A(M)A(F)U(F)U(F)C(F)A(F)A(F)A(F)G(M) | 0.055 | 0.100 |
| SEQ ID NO: 2035 | U(M)U(M)G(F)A(M)A(F)U(M)U(F)U(M)C(F)C(M)U(F)A(F)U(M)C( F)U(M)G(F)U(M)A(M)U(M)G(M)U(M) | SEQ ID NO: 2060 | A(M)C(F)A(F)U(M)A(F)C(M)A(F)G(M)A(F)U(M)A(M)G(F)G(M)A( F)A(M)A(F)U(M)U(F)C(M)A(M)A(M)A(M)G(M) | 0.026 | 0.075 |
| SEQ ID NO: 2036 | AGAGCACUUGAAAUGAAAUA | SEQ ID NO: 2061 | UAUUUCAUUUUCAAGUGCUCUCG | 0.037 | 0.085 |
| SEQ ID NO: 2037 | A(F)G(M)A(F)G(M)C(F)A(M)C(F)U(M)U(F)G(M)A(F)A(F)A(M)A(F )U(M)G(F)A(M)A(F)A(M)U(F)A(M) | SEQ ID NO: 2062 | U(F)A(M)U(F)U(M)U(F)C(M)A(F)U(M)U(F)U(M)U(M)C(F)A(M)A( F)G(M)U(F)G(M)C(F)U(M)C(F)U(M)C(F)G(M) | 0.039 | 0.060 |
| SEQ ID NO: 2038 | A(M)G(M)A(M)G(M)C(M)A(M)C(F)U(M)U(F)G(M)A(F)A(F)A(M)A( M)U(M)G(M)A(M)A(M)A(M)U(M)A(M) | SEQ ID NO: 2063 | U(M)A(F)U(F)U(F)U(F)C(F)A(F)U(F)U(F)U(M)U(M)C(F)A(M)A(F) G(M)U(F)G(F)C(F)U(F)C(F)U(F)C(F)G(M) | 0.073 | 6.030 |
| SEQ ID NO: 2039 | A(M)G(M)A(F)G(M)C(F)A(M)C(F)U(M)U(F)G(M)A(F)A(F)A(M)A( F)U(M)G(F)A(M)A(M)A(M)U(M)A(M) | SEQ ID NO: 2064 | U(M)A(F)U(F)U(M)U(F)C(M)A(F)U(M)U(F)U(M)U(M)C(F)A(M)A( F)G(M)U(F)G(M)C(F)U(M)C(M)U(M)C(M)G(M) | 0.019 | 0.062 |
| SEQ ID NO: 2040 | UCUGCAGCUCUUUCUUCUUUA | SEQ ID NO: 2065 | UAAAGAAGAAAGAGCUGCAGAUG | 0.037 | 0.049 |
| SEQ ID NO: 2041 | U(F)C(M)U(F)G(M)C(F)A(M)G(F)C(M)U(F)C(M)U(F)U(F)U(M)C( F)U(M)U(F)C(M)U(F)U(M)U(F)A(M) | SEQ ID NO: 2066 | U(F)A(M)A(F)A(M)G(F)A(M)A(F)G(M)A(F)A(M)A(M)G(F)A(M)G(F )C(M)U(F)G(M)C(F)A(M)G(F)A(M)U(F)G(M) | 0.040 | 0.111 |
| SEQ ID NO: 2042 | U(M)C(M)U(M)G(M)C(M)A(M)G(F)C(M)U(F)C(M)U(F)U(F)U(M)C (M)U(M)U(M)C(M)U(M)U(M)U(M)A(M). | SEQ ID NO: 2067 | U(M)A(F)A(F)A(F)G(F)A(F)A(F)G(F)A(F)A(M)A(M) G(F)A(M)G(F) C(M)U(F)G(F)C(F)A(F)G(F)A(F)U(F)G(M) | 0.167 | 0.072 |
| SEQ ID NO: 2043 | U(M)C(M)U(F)G(M)C(F)A(M)G(F)C(M)U(F)C(M)U(F)U(F)U(M)C( F)U(M)U(F)C(M)U(M)U(M)U(M)A(M) | SEQ ID NO: 2068 | U(M)A(F)A(F)A(M)G(F)A(M)A(F)G(M)A(F)A(M)A(M)G(F)A(M)G(F )C(M)U(F)G(M)C(F)A(M)G(M)A(M)U(M)G(M) | 0.055 | 0.210 |

### Test Example 1 APCS mRNA knockdown test of nucleic acid conjugate against human primary liver cell

The *in vitro* knockdown activity of each nucleic acid conjugate obtained in Examples 1 to 43 was measured in human primary liver cells. Each nucleic acid conjugate diluted with Opti-MEM (manufactured by Thermo Fisher Scientific Inc., 31985) such that the final concentration of this nucleic acid conjugate was 300, 30, 3 or 0.3 nmol/L was dispensed at 20 µL/well to a 96-well culture plate coated with collagen I (manufactured by Corning Inc., Catalog No. 356407). Then, human primary liver cells (manufactured by Biopredic International, Catalog No. HEP187) suspended in a plating medium (manufactured by Biopredic International, Catalog No. LV0304-2) were inoculated at 10,000 cells/80 µL/well thereto and cultured at 37°C for 6 hours under 5% CO₂ conditions. Then, the culture supernatant was carefully removed, and an incubation medium (manufactured by Biopredic International, Catalog No. LV0304-2) was added thereto so that each nucleic acid conjugate was applied to the human primary liver cells. 20 µL of Opti-MEM was applied to human primary liver cells, which were in turn used as a negative control group. The cells supplemented with each nucleic acid conjugate were cultured at 37°C for 18 hours in a 5% CO₂ incubator and washed with ice-cooled phosphate-buffered saline (DPBS) (manufactured by Nacalai Tesque, Inc., Catalog No. 14249-95). The recovery of total RNA from each of the plates and the preparation of cDNA through reverse-transcription reaction using the obtained total RNA as a template were performed using SuperPrep Cell Lysis & RT Kit for qPCR (manufactured by Toyobo Co., Ltd., Catalog No. SCQ-101) according to the method described in the instruction attached to the product. This cDNA was added at 3 µL/well to MicroAmp Optical 384-well plate (manufactured by Applied Biosystems, Inc., Catalog No. 4309849), and further, 10 µL of TaqMan Gene Expression Master Mix (manufactured by Applied Biosystems, Inc., Catalog No. 4369016), 6 µL of UltraPure Distilled Water (manufactured by Thermo Fisher Scientific Inc., Catalog No. 10977-015), and 1 µL of human APCS probe (manufactured by Applied Biosystems, Inc.) or 1 µL of human GAPDH probe (manufactured by Applied Biosystems, Inc.) were added to each well. The real-time PCR of the human APCS gene and the human GAPDH gene was performed using QuantStudio 12K Flex real-time PCR system (manufactured by Thermo Fisher Scientific Inc.) according to the method described in the attached instruction manual. GAPDH, a constitutively expressed gene, was measured as an internal control and used to correct the APCS gene expression level. The semiquantitative value of APCS mRNA in the negative control measured in the same way as above was defined as 1.0. The relative expression level of APCS mRNA was calculated when each nucleic acid conjugate was transferred. This experiment was conducted twice. Average values of the relative expression level of APCS mRNA are shown in Table S1.

### [Table 45]

**Table S1: APCS mRNA expression rate**

| Compound | 300nM | 30nM | 3nM | 0.3nM |
|---|---|---|---|---|
| 1-2 | 0.127 | 0.223 | 0.451 | 0.852 |
| 2-2 | 0.309 | 0.459 | 0.596 | 0.977 |
| 3-2 | 0.366 | 0.516 | 0.766 | 0.919 |
| 4-2 | 0.254 | 0.386 | 0.596 | 0.874 |
| 5-2 | 0.075 | 0.096 | 0.141 | 0.451 |
| 6-2 | 0.377 | 0.499 | 0.669 | 0.841 |
| 7-2 | 0.187 | 0.314 | 0.652 | 0.950 |
| 8-2 | 0.180 | 0.273 | 0.509 | 0.893 |
| 9-2 | 0.145 | 0.272 | 0.456 | 0.885 |
| 10-2 | 0.087 | 0.125 | 0.258 | 0.682 |
| 11-2 | 0.130 | 0.244 | 0.676 | 0.987 |
| 12-2 | NT | NT | NT | NT |
| 13-2 | NT | NT | NT | NT |
| 14-2 | NT | NT | NT | NT |
| 15-2 | 0.051 | 0.056 | 0.204 | 0.731 |
| 16-2 | NT | NT | NT | NT |
| 17-2 | 0.122 | 0.220 | 0.920 | 0.965 |
| 18-2 | 0.184 | 0.374 | 0.993 | 0.988 |
| 19-2 | 0.144 | 0.286 | 0.989 | 1.218 |
| 20-2 | 0.064 | 0.120 | 0.395 | 0.925 |
| 21-2 | NT | NT | NT | NT |
| 22-2 | NT | NT | NT | NT |
| 23-2 | NT | NT | NT | NT |
| 24-2 | NT | NT | NT | NT |
| 25-2 | 0.177 | 0.337 | 0.874 | 1.021 |
| 26-2 | NT | NT | NT | NT |
| 27-2 | NT | NT | NT | NT |
| 28-2 | NT | NT | NT | NT |
| 29-2 | NT | NT | NT | NT |
| 30-2 | NT | NT | NT | NT |
| 31-2 | NT | NT | NT | NT |
| 32-2 | NT | NT | NT | NT |
| 33-2 | 0.046 | 0.082 | 0.381 | 0.880 |
| 34-2 | NT | NT | NT | NT |
| 35-2 | 0.051 | 0.076 | 0.442 | 0.984 |
| 36-2 | NT | NT | NT | NT |
| 37-2 | NT | NT | NT | NT |
| 38-2 | NT | NT | NT | NT |
| 39-2 | NT | NT | NT | NT |
| 40-2 | NT | NT | NT | NT |
| 41-2 | NT | NT | NT | NT |
| 42-2 | NT | NT | NT | NT |
| 43-2 | NT | NT | NT | NT |

As is evident from Table S1, each nucleic acid conjugate inhibited the mRNA expression of the APCS gene after being added to human primary liver cells.

Test Example 2 *In vivo* knockdown test of nucleic acid conjugate in mouse

An *in vivo* knockdown test was conducted on each nucleic acid conjugate obtained in Examples 1 to 43 by the following method. Each nucleic acid conjugate was diluted with phosphate-buffered saline (DPBS) (manufactured by Nacalai Tesque, Inc.) according to the test and used. The mice used were human liver cell-transplanted PXB mice (PXB/human Hepatocyte repopulated cDNA-uPA/SCID Tg, obtained from PhoenixBio Co., Ltd.). The nucleic acid conjugate was evaluated for its effect on human APCS. After acclimatization of the PXB mice, each nucleic acid conjugate was subcutaneously administered to the mice. The dose was 10 mg/kg, and the dose was 5 mL/kg. For a control group, DPBS alone was subcutaneously administered to the mice. Six days before the administration and 1, 2, 3, 6, 10 and 13 days after the administration, blood was collected and centrifuged at 1500 × g to obtain serum. The serum thus obtained was used to evaluate the amount of human APCS in blood using human SAP ELISA (SAP, Human, ELISA kit, manufactured by Hycult Biotech Inc., #HK331-02). ELISA was carried out according to the attached manual. Changes in human APCS concentration in blood of each mouse group are shown in Figure 1. The APCS expression ratio (%) of the nucleic acid conjugate administration group to the DPBS administration group is shown in Table S2.

### [Table 46]

**Table S2: APCS protein expression rate (vs. DPBS administration group) on 13 days after administration at dose of 10 mg/kg**

| Compound | Expression rate |
|---|---|
| 1-2 | NT |
| 2-2 | NT |
| 3-2 | NT |
| 4-2 | NT |
| 5-2 | 18.52 |
| 6-2 | NT |
| 7-2 | NT |
| 8-2 | NT |
| 9-2 | NT |
| 10-2 | NT |
| 11-2 | NT |
| 12-2 | NT |
| 13-2 | NT |
| 14-2 | NT |
| 15-2 | NT |
| 16-2 | NT |
| 17-2 | NT |
| 18-2 | NT |
| 19-2 | NT |
| 20-2 | NT |
| 21-2 | NT |
| 22-2 | NT |
| 23-2 | NT |
| 24-2 | NT |
| 25-2 | NT |
| 26-2 | NT |
| 27-2 | NT |
| 28-2 | NT |
| 29-2 | NT |
| 30-2 | NT |
| 31-2 | NT |
| 32-2 | NT |
| 33-2 | 31.35 |
| 34-2 | NT |
| 35-2 | 31.31 |
| 36-2 | NT |
| 37-2 | NT |
| 38-2 | NT |
| 39-2 | NT |
| 40-2 | NT |
| 41-2 | NT |
| 42-2 | NT |
| 43-2 | NT |

As is evident from Table S2, the nucleic acid conjugate of the present invention was found to decrease APCS protein expression in mouse peripheral blood by administration to mice expressing human APCS.

### Industrial Applicability

The nucleic acid conjugate of the present invention can be used for treating an amyloid-related disease *in vivo* by administration to a mammal.

## Claims

1. A nucleic acid conjugate represented by the following formula 1: 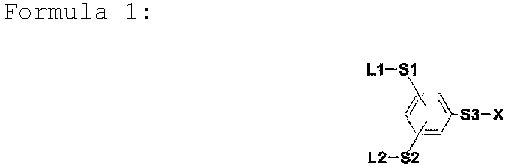 wherein
X is a double-stranded nucleic acid consisting of a sense strand and an antisense strand and comprising a duplex region of at least 11 base pairs, wherein
in the double-stranded nucleic acid, an oligonucleotide strand having a chain length of 17 to 30 nucleotides in the antisense strand is complementary to any of target APCS mRNA sequences described in Tables 1-1 to 1-13, and
a 3' end or a 5' end of the sense strand binds to S3,
L1 and L2 are each independently a sugar ligand, and
S1, S2 and S3 are each independently a linker.

2. The nucleic acid conjugate according to claim 1, wherein the nucleic acid conjugate has a structure represented by the following formula 2: 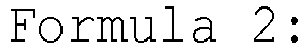 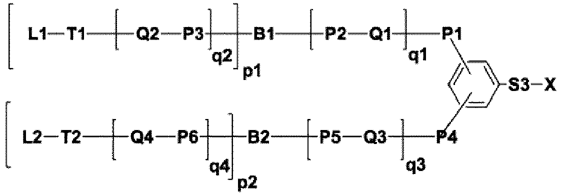 wherein
X, L1, L2 and S3 are each as defined above,
P1, P2, P3, P4, P5 and P6, and T1 and T2 are each independently absent, or -CO-, -NH-, -O-, -S-, -O-CO-, - S-CO-, -NH-CO-, -CO-O-, -CO-S- or -CO-NH-,
Q1, Q2, Q3 and Q4 are each independently absent, or substituted or unsubstituted alkylene having 1 to 12 carbon atoms or -(CH₂CH₂O)ₙ-CH₂CH₂- wherein n is an integer of 0 to 99,
B1 and B2 are each independently a bond, or any structure represented by the following formula 2-1, wherein each of the terminal dots in each structure is a binding site to P2 or P3, or P5 or P6, and ml, m2, m3 and m4 are each independently an integer of 0 to 10: 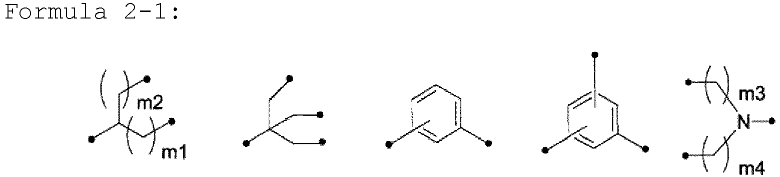
p1 and p2 are each independently an integer of 1, 2 or 3, and
ql, q2, q3 and q4 are each independently an integer of 0 to 10,
provided that when each of p1 and p2 is an integer of 2 or 3, each P3 and P6, Q2 and Q4, T1 and T2 or L1 and L2 are the same or different, and when q1 to q4 are 2 to 10, combinations -[P2-Q1]-, -[Q2-P3]-, -[P5-Q3]- or -[Q4-P6]- are the same or different.

3. The nucleic acid conjugate according to claim 2, wherein P1 and P4 are each independently -CO-NH-, -NH-CO- or -O-.

4. The nucleic acid conjugate according to claim 2 or 3, wherein -[P2-Q1]_{q1}- and -[P5-Q3]_{q3}- are each independently absent, or any structure represented by the following formulas 3-1 to 3-3: 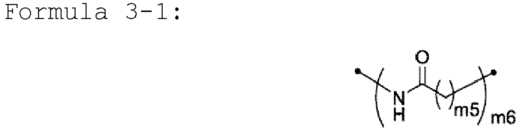 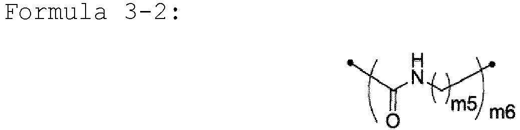 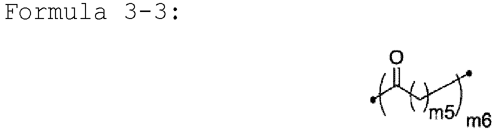 wherein
m5 and m6 are each independently an integer of 0 to 10, and each of the terminal dots in the structures of formulas 3-1 to 3-3 is a binding site to B1 or B2, or P1 or P4.

5. The nucleic acid conjugate according to any one of claims 2 to 4, wherein the nucleic acid conjugate has any structure represented by the following formulas 4-1 to 4-9: 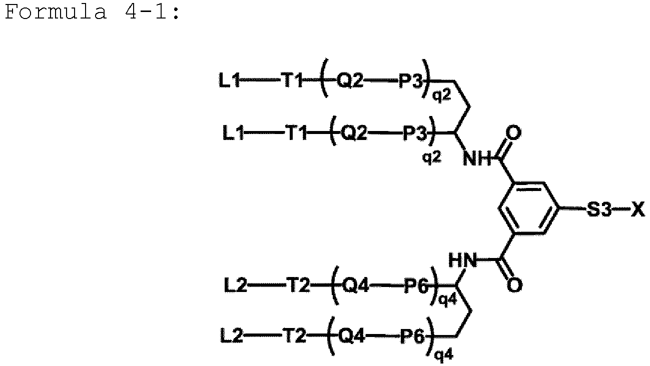 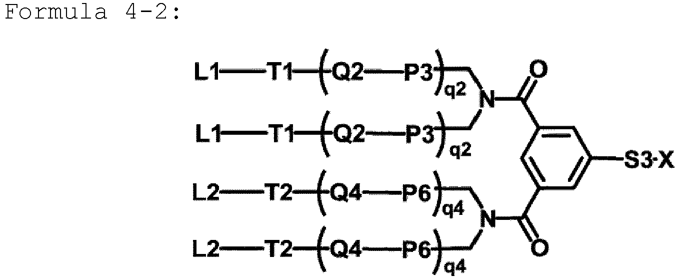 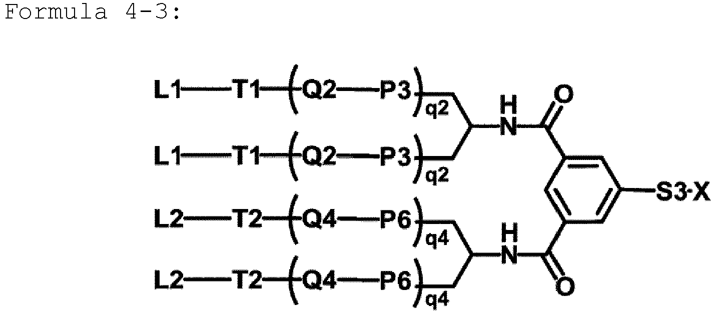 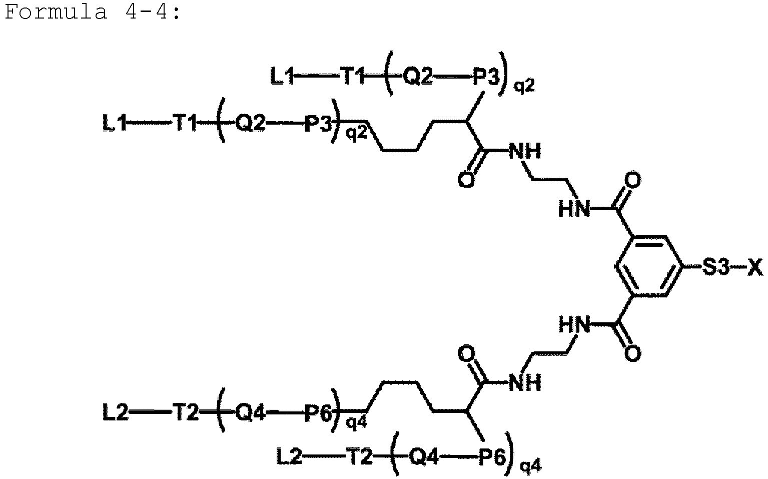 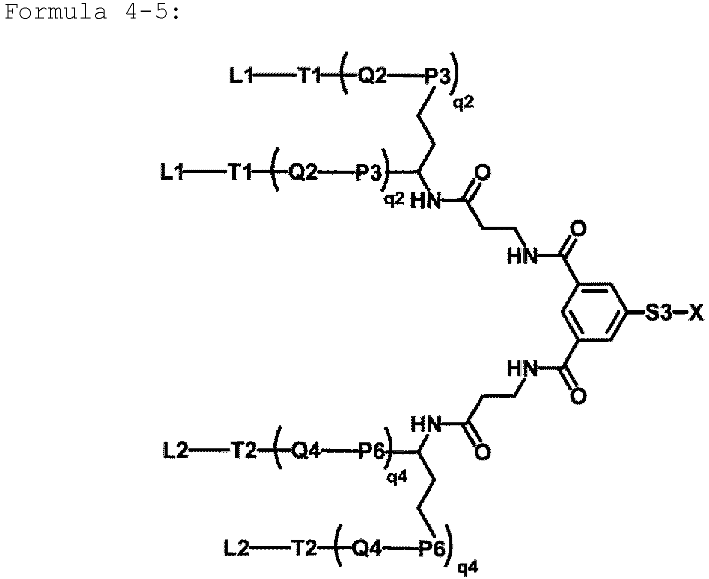 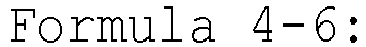 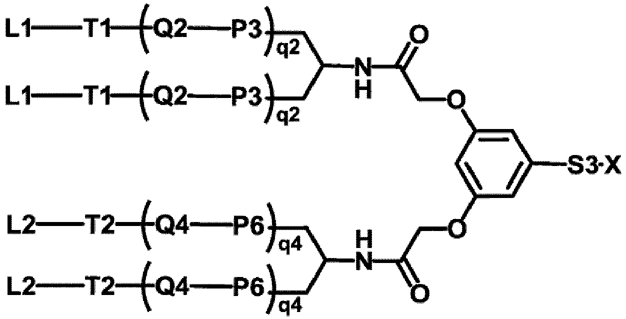 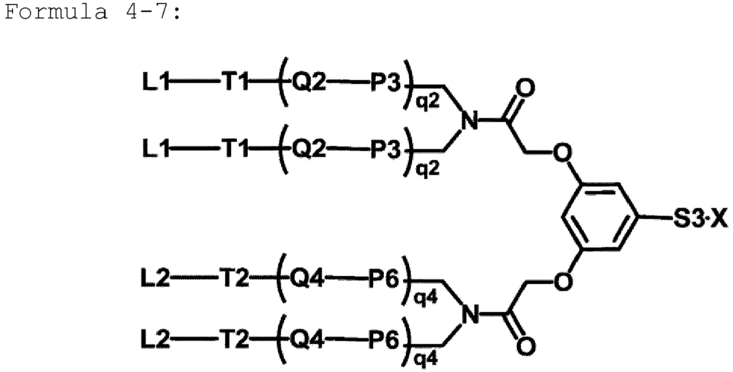 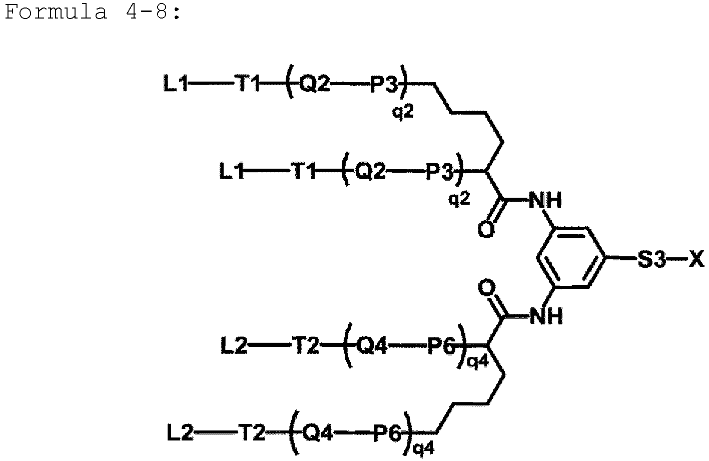 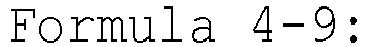 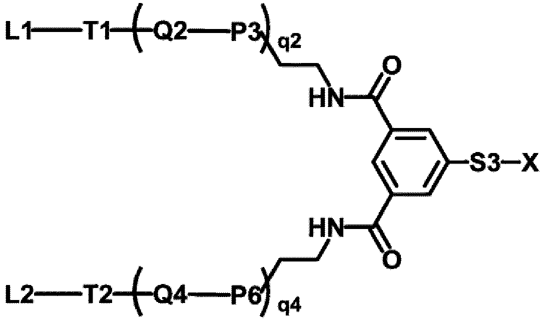 Wherein
X, L1, L2, S3, P3, P6, T1, T2, Q2, Q4, q2 and q4 are each as defined above.

6. The nucleic acid conjugate according to claim 1, wherein the nucleic acid conjugate has a structure represented by the following formula 5: 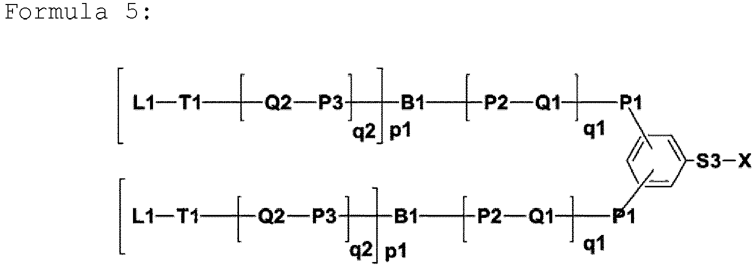 wherein
X, S3, P1, P2, P3, Q1, Q2, B1, T1, L1, p1, q1 and q2 are each as defined above.

7. The nucleic acid conjugate according to claim 6, wherein P1 is -CO-NH-, -NH-CO- or -O-.

8. The nucleic acid conjugate according to claim 6 or 7, wherein the nucleic acid conjugate has any structure represented by the following formulas 6-1 to 6-9: 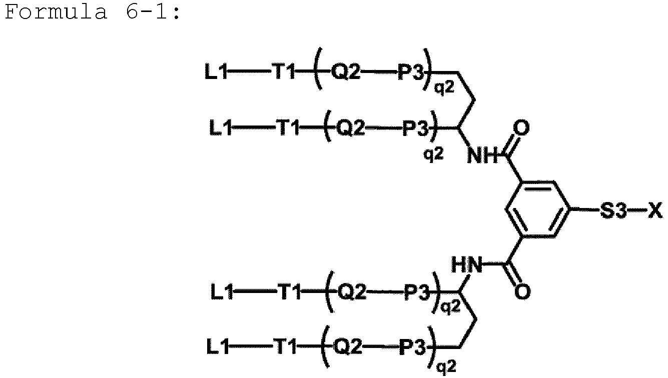 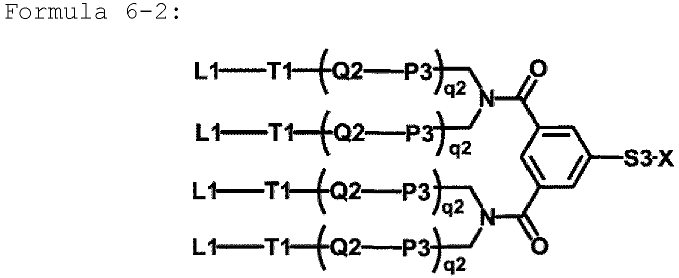 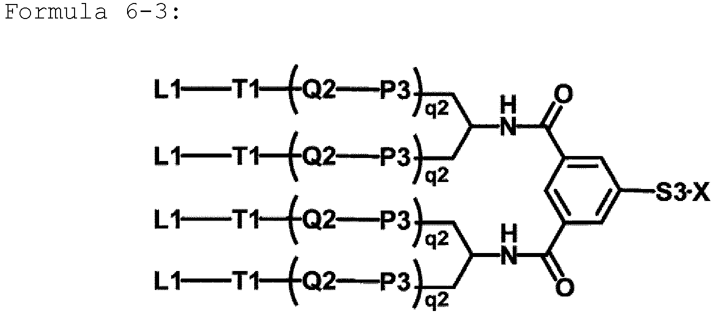 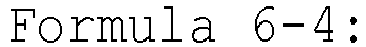 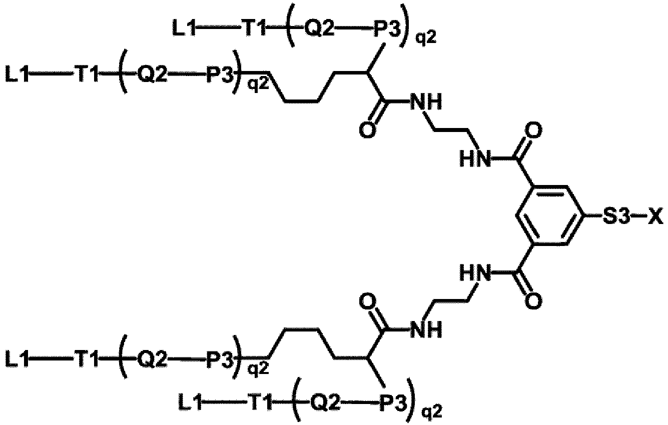 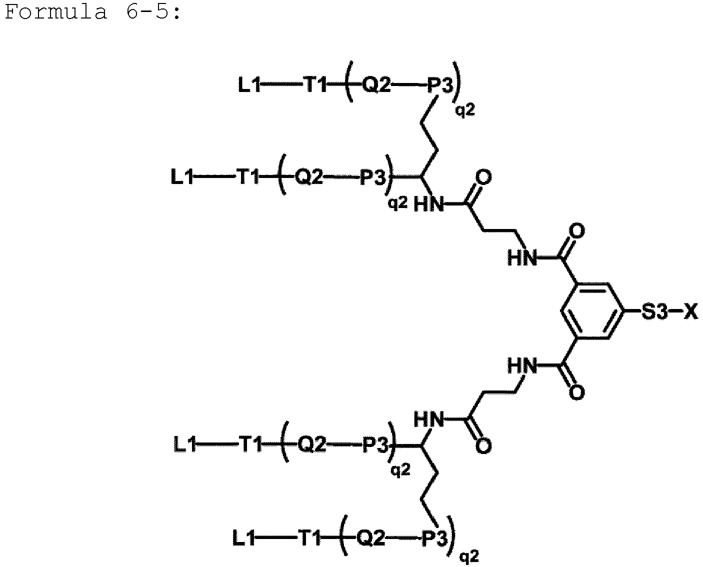 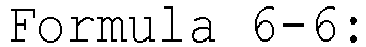 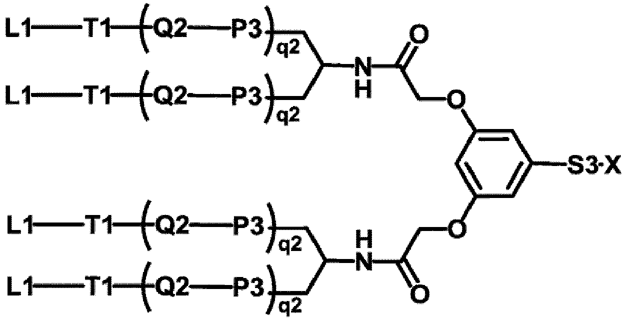 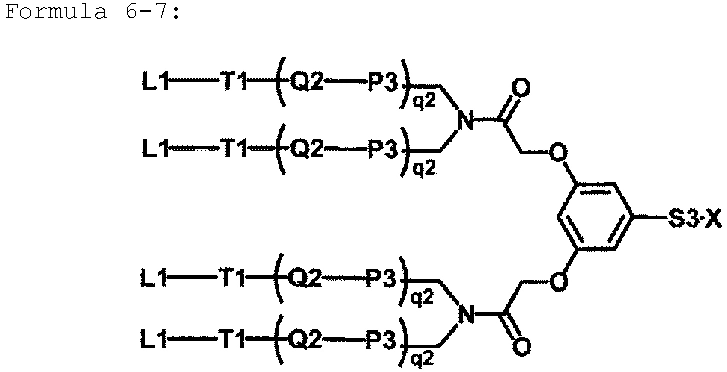 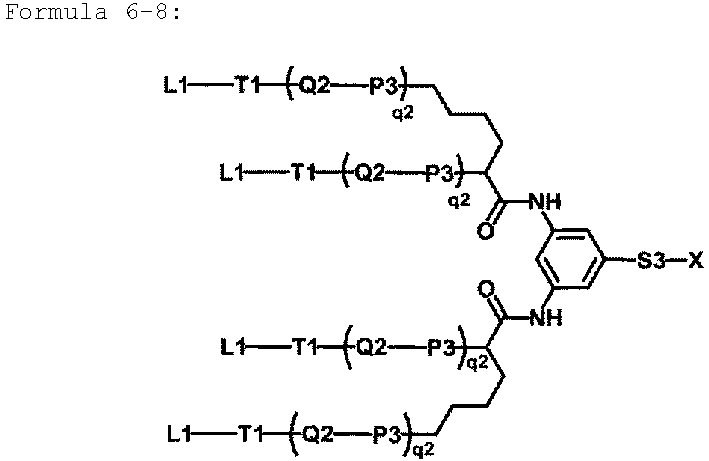 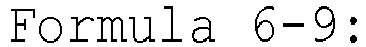 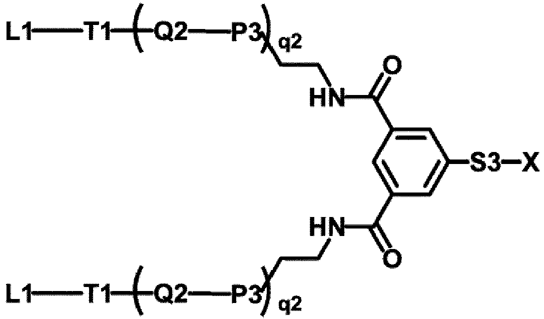 wherein
X, S3, P3, Q2, T1, L1 and q2 are each as defined above.

9. The nucleic acid conjugate according to any one of claims 2 to 5, wherein the nucleic acid conjugate has any structure represented by the following formulas 7-1 to 7-9: 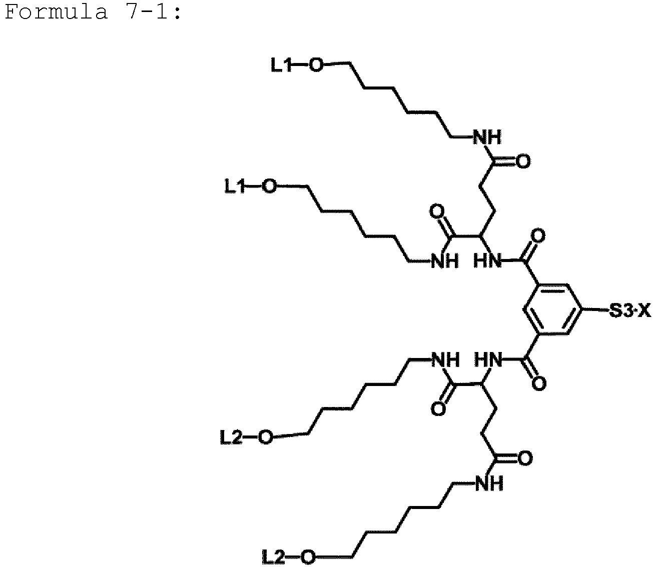 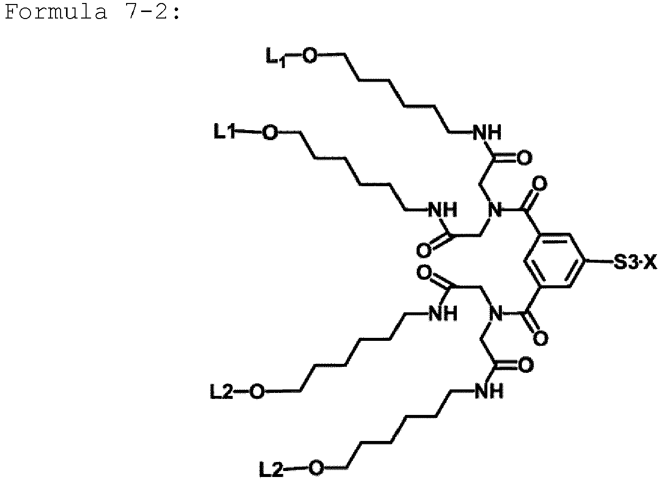 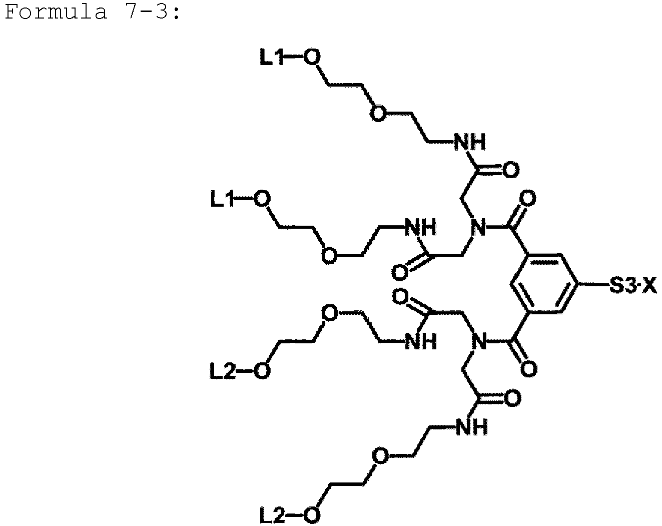 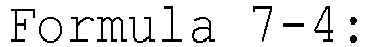 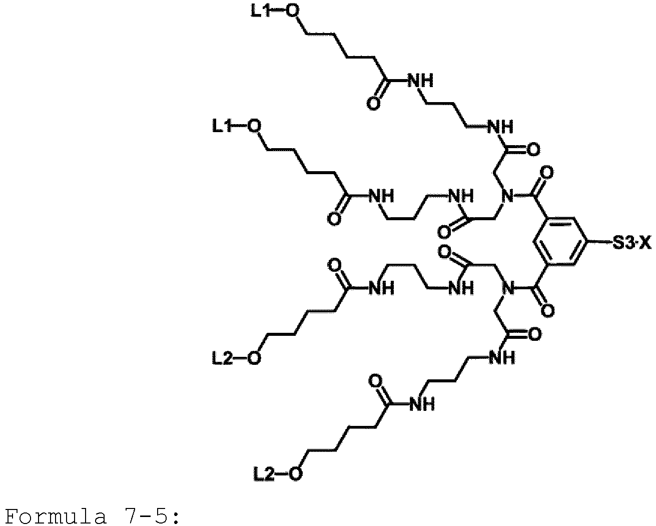 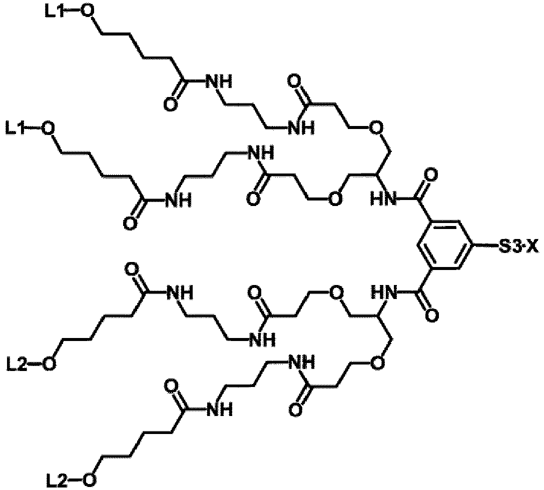 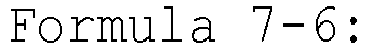 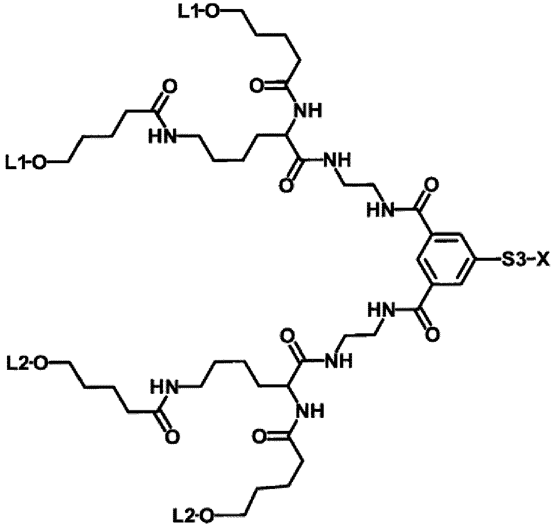 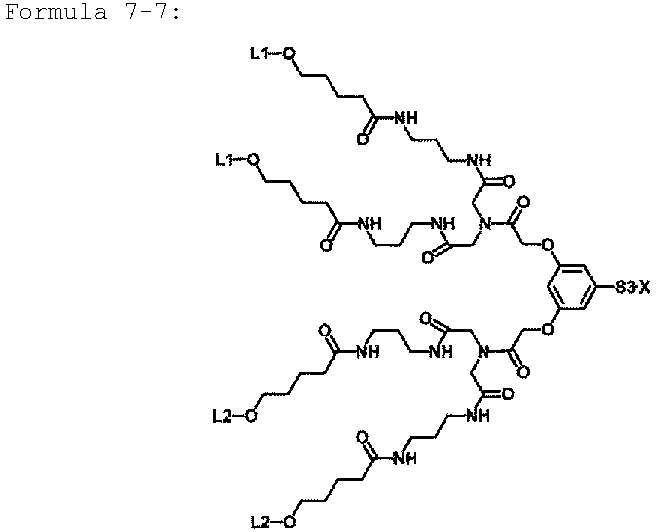 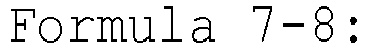 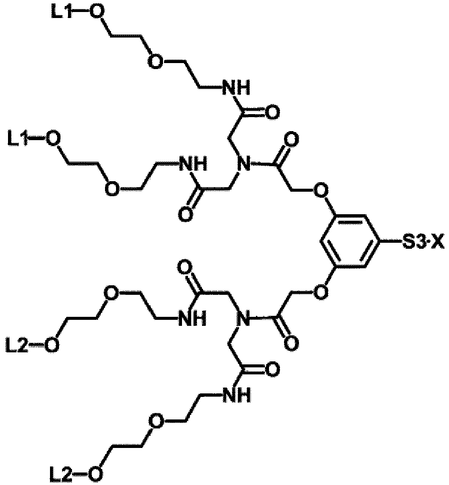 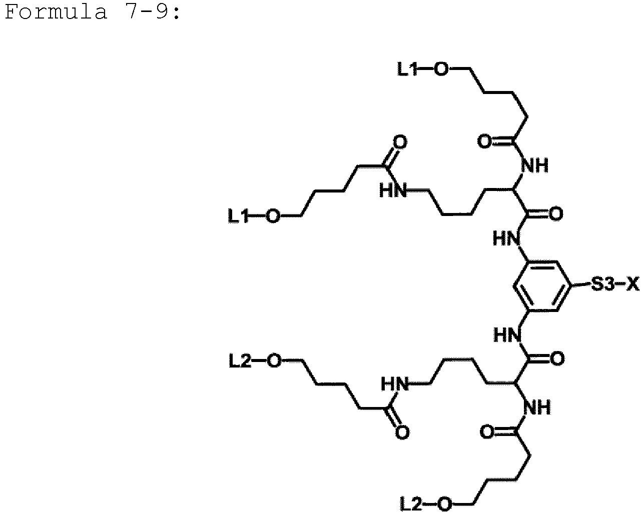 wherein
X, S3, L1 and L2 are each as defined above.

10. The nucleic acid conjugate according to any one of claims 1 to 9, wherein the sugar ligand is N-acetylgalactosamine.

11. The nucleic acid conjugate according to any one of claims 1 to 10, wherein the double-stranded nucleic acid comprises a modified nucleotide.

12. The nucleic acid conjugate according to any one of claims 1 to 11, wherein the 3' end of the sense strand and the 5' end of the antisense strand each form a blunt end.

13. The nucleic acid conjugate according to claim 11, wherein the double-stranded nucleic acid comprises a nucleotide modified at the sugar moiety.

14. The nucleic acid conjugate according to any one of claims 1 to 13, wherein the nucleic acid conjugate has a structure represented by the following formula 7-8-1: 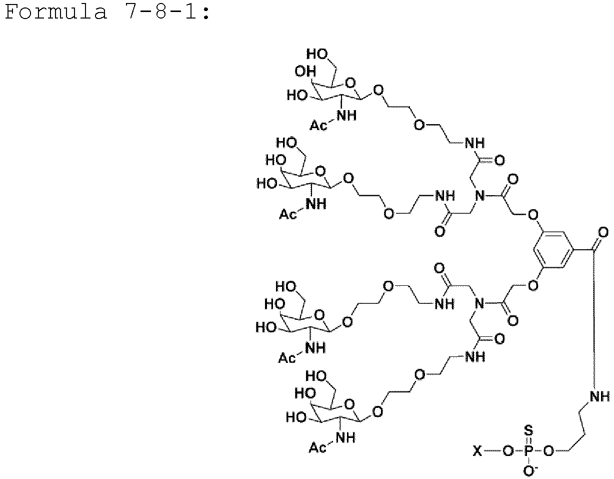 wherein X is as defined above.

15. The nucleic acid conjugate according to any one of claims 1 to 14, wherein X is a pair of sense strand/antisense strand selected from the group consisting of sense strands/antisense strands described in Tables 1-1 to 1-13.

16. The nucleic acid conjugate according to any one of claims 1 to 14, wherein X is a pair of sense strand/antisense strand selected from the group consisting of sense strands/antisense strands described in Tables M1-1 to M1-3, R-1 to R-2 and R-3 to R-4.

17. A pharmaceutical composition comprising a nucleic acid conjugate according to any one of claims 1 to 16.

18. The pharmaceutical composition according to claim 17, wherein the pharmaceutical composition is for transfer into a cell.

19. The pharmaceutical composition according to claim 17 or 18, wherein the pharmaceutical composition is intravenously administered or subcutaneously administered.

20. A method for treating or preventing a disease, comprising administering a nucleic acid conjugate according to any one of claims 1 to 16 or a pharmaceutical composition according to any one of claims 17 to 19 to a patient in need thereof.

21. A method for inhibiting the expression of APCS gene, comprising transferring a double-stranded nucleic acid into a cell using a nucleic acid conjugate according to any one of claims 1 to 16 or a pharmaceutical composition according to any one of claims 17 to 19.

22. A method for treating an amyloid-related disease, comprising administering a nucleic acid conjugate according to any one of claims 1 to 16 or a pharmaceutical composition according to any one of claims 17 to 19 to a mammal.

23. A medicament for use in the treatment of an amyloid-related disease, comprising a nucleic acid conjugate according to any one of claims 1 to 16 or a pharmaceutical composition according to any one of claims 17 to 19.

24. A therapeutic agent for an amyloid-related disease, comprising a nucleic acid conjugate according to any one of claims 1 to 16 or a pharmaceutical composition according to any one of claims 17 to 19.

25. The treatment method according to claim 22, wherein the amyloid-related disease is a disease caused by a disorder mediated by amyloid fibrils containing APCS.

26. The medicament according to claim 23, wherein the amyloid-related disease is a disease caused by a disorder mediated by amyloid fibrils containing APCS.

27. The therapeutic agent according to claim 24, wherein the amyloid-related disease is a disease caused by a disorder mediated by amyloid fibrils containing APCS.
